# EUROPEAN PATENT SPECIFICATION

(11) **EP 2 531 200 B1**
(45) Date of publication and mention of the grant of the patent: **12.07.2017**
(21) Application number: 11740284.2
(22) Date of filing: 02.02.2011
(51) Int. Cl.: A61K 9/22, A61K 31/66

(54) **CRYSTALLIZATION METHOD AND BIOAVAILABILITY**
KRISTALLISATIONSVERFAHREN UND BIOVERFÜGBARKEIT
PROCÉDÉ DE CRISTALLISATION ET BIODISPONIBILITÉ

(30) Priority: 10.05.2010 US 333041 P; 30.07.2010 US 847568; 03.09.2010 US 379814 P; 30.07.2010 WO PCT/US2010/043892; 29.06.2010 US 359544 P; 26.10.2010 US 455778 P; 30.07.2010 WO PCT/US2010/043916; 29.03.2010 US 318503 P; 10.05.2010 US 333028 P; 11.03.2010 US 312879 P; 06.02.2010 US 302110 P
(43) Date of publication of application: 12.12.2012
(73) Proprietor: Grünenthal GmbH, 52078 Aachen (DE)
(72) Inventor: HANNA, Mazen, Lutz, FL 33549 (US); SHAN, Ning, Tampa, FL 33613 (US); CHENEY, Miranda, L., Tampa, FL 33637 (US); WEYNA, David, R., Tampa, FL 33612 (US); HOUCK, Raymond, Oakmont, PA 15139 (US)
(74) Representative: Eder, Michael
(86) International application number: PCT/US2011/023427
(87) International publication number: WO 2011/097269

(56) References cited:
- WO-A1-2011/014781
- WO-A2-2005/005447
- WO-A2-2012/071517
- CN-B- 102 070 668
- US-A1- 2004 220 264
- US-A1- 2006 068 010
- US-A1- 2007 021 618
- MCNAMARA ET AL.: 'Use of a Glutaric Acid Cocrystal to Improve Oral Bioavailability of a Low Solubility API' PHARMACEUTICAL RESEARCH vol. 23, no. 8, August 2006, pages 1888 - 1897

## Description

### FIELD OF THE INVENTION

This disclosure pertains to improvement of the aqueous solubility and permeability of poorly permeable and sparingly water soluble drug compounds through generating novel crystalline forms of such drugs. The novel forms include but are not limited to cocrystals, salts, hydrates, solvates, solvates of salts, and mixtures thereof. Methods for the preparation and pharmaceutical compositions suitable for drug delivery systems that include one or more of these new forms are disclosed.

### BACKGROUND OF THE INVENTION

Many Biopharmaceutics Classification System (BCS) class III or IV drugs suffer from the lack of gastrointestinal (GI) tract membrane permeability leading to poor oral bioavailability. Different strategies have been implemented to improve the permeability and subsequently the oral bioavailability of such drugs. For example, the U.S. patent application 20060068010 describes a formulation method for improving the permeability of drugs and subsequently increasing their bioavailability by granulation of the physical solid mixture of the drug with one or more amino acids, at least one inter-granular hydrophilic polymer, and an additional immediate release excipient. Another application WO 200602009 A1 disclosed an increase in the oral bioavailability of poorly permeable bisphosphonate drugs; risedronate, an exemplary bisphosphonate, was mixed with a chelating agent such as ethylenediaminetetraacetic acid (EDTA) and other excipients to make an oral dosage form with enhanced bioavailability. In another application, WO 2007093226 describes a method for improving the bioavailability of ibandronate by generating a physical mixture of the drug together with a modified amino acid (acylation or sulphonation of the amino group with phenyl or cyclohexyl) and other excipients. Another application, WO 2003007916 A1, reports a gastric retention system to improve the bioavailability of a poorly permeable drug, alendronate, which was orally formulated with vitamin D and released an hour after the immediate release of vitamin D. WO 2006080780 discloses yet another method to improve the permeability and bioavailability of alendronate by mixing it with a biocompatible cationic polymer (i.e. water soluble chitosan) with up to a 10:1 weight ratio of the chitosan to the drug, while the resulting mixture can be formulated into a solid or liquid oral dosage form. An additional method of improving permeability of drug materials was discussed in the U.S. patent application 2007/014319 A1, where an oral dosage form was formulated by a powder mixture of a bisphosphonic acid (e.g. zoledronic acid) together with an inactive ingredient (either an ester of a medium chain fatty acid or a lipophilic polyethylene glycol ester). A similar approach was disclosed in the US application 2007/0238707 A1 where a medium chain length fatty acid or its derivative (6-20 carbon atom fatty acid chain) was physically mixed with a poorly permeable drug (e.g. zoledronic acid) in a capsule that was enterically coated.

Zoledronic acid, known as (1-hydroxy-2-imidazol-1-yl-1-phosphono-ethyl)phosphonic acid, is depicted by the following chemical structure:

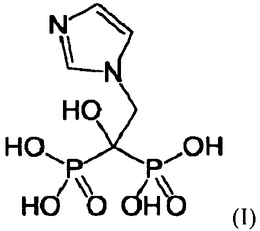

Zoledronic acid is a third generation bisphosphonate which far exceeds the previous generations in terms of efficacy and is used predominately for indications of osteoporosis, Paget's disease, hypercalcemia, and inhibition of bone metastasis. It was originally developed by Novartis and marketed as the monohydrate under the brand names Zometa® and Reclast®. Zoledronic acid was first approved in 2000 for the treatment of hypercalcemia in Canada. It was later approved for use in the US for hypercalcemia in 2001, for multiple myeloma and bone metastases from solid tumors in 2002, and for osteoporosis and Paget's disease in 2007. Clinical trials have also been conducted and are on-going to explore the use of zoledronic acid in neoadjuvant or adjuvant cancer therapy, Coleman, et al., British J Cancer 2010;102(7):1099-1105*,* Gnant, et al., New England J Medicine. 2009, 360 (17):679-691 and Davies, et al. J Clinical Oncology, 2010, 28(7s): Abstract 8021. Zoledronic acid is administered as an intravenous (IV) dose of 4 mg over 15 minutes for hypercalcemia of malignancy, multiple myeloma, and bone metastases from solid tumors, while an IV dose of 5 mg over 15 minutes is used for osteoporosis and Paget's disease.

Zoledronic acid is sparingly soluble in water and 0.1 N HCl solution but is freely soluble in 0.1 N NaOH. Zoledronic acid is practically insoluble in various organic solvents. Much effort has been taken to generate novel oral formulations of zoledronic acid through crystallization and metal salt formation to improve its aqueous solubility, permeability, and subsequent oral bioavailability. A crystalline trihydrate was disclosed in the U.S. Patent application 2006/0178439 A1 and world patent application WO2007/032808. Seven hydrated forms, an amorphous form, three monosodium salts, and eleven disodium salts with varying degrees of hydration of zoledronic acid were also disclosed in the patent application WO2005/005447 A2. Zoledronate metal salts including Na⁺, Mg²⁺, Zn²⁺ were reported in the journal of Drugs of the Future (Sorbera et al, Drugs of the Future, 2000, 25(3): 259-268). Zoledronate, zoledronic, or zoledronic salt represents the ionic form of zoledronic acid. Patent application WO2008/064849 A1 from Novartis disclosed additional metal salts including two Ca²⁺ salts, two Zn²⁺ salts, one Mag²⁺ salt, as well as a monohydrate, a trihydrate, an amorphous form, and an anhydrous form.

According to the US Food and Drug Administration (FDA) Summary Basis of Approval (SBA) for zoledronic acid, the poor oral bioavailability (approximately 1%), is partially due to its poor permeability in the GI tract. It was also noted that insoluble metal complexes were formed in the upper intestines, most commonly with calcium. Zoledronic acid has also been shown to cause severe gastric and intestinal irritations.

All of the above attempts to improve the oral bioavailability of zoledronic acid were either focused on improving the aqueous solubility by generating novel solid forms, or by mixing the drug with an inactive ingredient that has enhanced GI tract permeability. The improvement of aqueous solubility failed to improve the bioavailability of zoledronic acid, since the formation of insoluble zoledronate calcium complexes is unlikely to be prevented. On the other hand, powder mixtures of the poorly permeable drug with inactive permeability enhancers improved the bioavailability of the drug. This approach of mixing different materials with different particle sizes and size distributions could result in poor blend/physical mixture uniformity. Constituents of the mixture could also segregate during transportation or with shaking and vibration. Additionally, the powder blends require rigorous batch-to-batch consistency to ensure the uniformity of the blend batches.

The upward trend in the use of oral drugs continues especially in light of the goal to decrease the overall cost of healthcare. Orally administered drugs are becoming more preferred in various therapeutic areas including oncology. Clearly, there is an opportunity to create oral dosage forms of drugs with poor aqueous solubility and/or poor permeability. One such example is zoledronic acid which is only approved for intravenous administration due to its low oral bioavailability, resulting from poor permeability. By using pharmaceutically acceptable and/or approved coformers to hydrogen or ionically bond with an API, novel molecular complexes (e.g. cocrystals, salts, solvates, and mixtures thereof) with improved solubility and/or permeability can be created. These novel molecular complexes could be used in the development of novel oral dosage forms of BCS Class III and IV drugs.

### SUMMARY OF THE INVENTION

The present invention addresses the issue of low oral bioavailability using two approaches. The first approach represents a deliberate molecular design in the form of a molecular complex comprising drug and certain excipient(s) (coformer(s)) in a single crystalline structure. The benefit of such a design can reduce batch to batch blend uniformity and particle segregation problems that powder blends often suffer from. In addition, this invention simplifies the manufacturing of a solid dosage form (comprised of drug and excipient) such that the final solid dosage form is, in one embodiment, a particulate or powder of the molecular complex. Additionally, the resulting molecular complexes possess very different physicochemical properties compared to the parent drug or coformer or the physical mixture thereof. These properties include but are not limited to melting point, thermal and electrical conductivity, aqueous solubility, rate of dissolution and permeability across the GI tract membrane. The second approach targets the issue of low permeability of BCS class III and IV drugs. The approach involves combining a low permeability drug with an amino acid which can increase permeability and subsequent oral bioavailability.

The present disclosure is directed towards generating new forms of APIs, e.g., zoledronic acid, with improved physicochemical properties, such as improved aqueous solubility, rate of dissolution, and, particularly, improved permeability resulting in enhanced bioavailability. The present invention is more specifically directed to the subject matter set out in the appended claims.

One aspect of the present invention includes novel pharmaceutical compositions comprising crystalline molecular complexes of zoledronic acid in the form of cocrystals, salts, cocrystals of salts and solvates (including hydrates and mixed solvates) thereof and molecular complex coformers selected from lysine and glycine and pharmaceutically acceptable excipients, further comprising an additional coformer selected from lysine and/or glycine, wherein the mass ratio of additional coformer to molecular complex coformer is between about 2:1 to about 5000:1. In addition, the disclosure further includes processes of making and methods for using the same. In this aspect the role of the 'additional' or 'excess' coformer is as a functional excipient. The additional coformer of the invention is particularly lysine or glycine, and more particularly lysine, wherein the coformer, lysine or glycine, more particularly lysine, increases the oral bioavailability of BGS Class III and IV drugs. such as zoledronic acid.

In one aspect the molar ratio of coformer to API is about 1:1. In another aspect the coformer is in molar excess to the API. In one embodiment the molar ratio of coformer to API is between about 2:1 and 10:1. In another embodiment the ratio is between about 2:1 and about 5:1. In another embodiment the ratio is about 2:1. In another embodiment the ratio is about 3:1. In another embodiment the ratio is about 4:1. In another embodiment the ratio is about 5:1

In one aspect the API is in molar excess to the coformer. In one embodiment the molar ration of API to coformer is between about 2:1 and about 10:1. In another embodiment the molar ratio is between about 2:1 and about 5:1. In another embodiment the molar ratio is about 2:1. In another embodiment the molar ratio is about 3:1. In another embodiment the molar ratio is about 4:1. In another embodiment the molar ratio is about 5:1.

In this aspect the composition of the present invention further comprises 'additional coformer' that is not in the form of a molecular complex with the API wherein the mass ratio of the additional coformer to the molecular complex coformer is between about 2:1 to about 5000:1. In one embodiment the additional coformer and the coformer that forms a molecular complex with the API (i.e., the 'molecular complex coformer') are the same. In another embodiment the additional coformer and the molecular complex coformer are different. In another embodiment the additional coformer is crystalline. In another embodiment the additional coformer is amorphous. In one embodiment the amount of additional coformer in the composition is greater than the amount of molecular complex coformer. In some embodiments the ratio is between about 1000:1 to about 5000:1. In another embodiment the ratio is between about 1000:1 to about 4000:1. In another embodiment the ratio is between about 2000:1 to about 4000:1. In another embodiment the ratio is between about 1000:1 to about 2000:1. In another embodiment the ratio is between about 100:1 to about 2000:1. In another embodiment the ratio is between about 100:1 to about 1000:1. In another embodiment the ratio is between about 100:1 to about 750:1. In another embodiment the ratio is between about 100:1 to about 500:1. In another embodiment the ratio is between about 100:1 to about 275:1. In another embodiment the ratio is between about 200:1 to about 275:1. In another embodiment the ratio is between about 175:1 to about 275:1. In another embodiment the ratio is between about 150:1 to about 250:1. In another embodiment the ratio is between about 100:1 to about 250:1. In another embodiment the ratio is between about 100:1 to about 200:1. In another embodiment the ratio is between about 50:1 to about 200:1. In another embodiment the ratio is between about 50:1 to about 150:1. In another embodiment the ratio is between about 50:1 to about 100:1. In another embodiment the ratio is between about 2:1 to about 100:1. In another embodiment the ratio is between about 5:1 to about 100:1. In another embodiment the ratio is between about 10:1 to about 100:1. In another embodiment the ratio is between about 11:1 to about 100:1. In another embodiment the ratio is between about 25:1 to about 100:1. In another embodiment the ratio is between about 50:1 to about 100:1. In another embodiment the ratio is between about 75:1 to about 100:1. In another embodiment the ratio is between about 2:1 to about 50:1. In another embodiment the ratio is between about 5:1 to about 50:1. In another embodiment the ratio is between about 10:1 to about 50:1. In another embodiment the ratio is between about 11:1 to about 50:1. In another embodiment the ratio is between about 12:1 to about 50:1. In another embodiment the ratio is between about 13:1 to about 50:1. In another embodiment the ratio is between about 14:1 to about 50:1. In another embodiment the ratio is between about 15:1 to about 50:1. In another embodiment the ratio is between about 25:1 to about 50:1. In another embodiment the ratio is between about 35:1 to about 50:1.

The present invention discloses various compositions in Table 11.

It was found that the coformer of the present invention increases the oral bioavailability of the API. In one embodiment the coformer increases oral bioavailability of the API by at least 10%. In one embodiment the coformer increases oral bioavailability of the API by at least 25%. In one embodiment the coformer increases oral bioavailability of the API by at least 75%. In one embodiment the coformer increases oral bioavailability of the API by at least two fold. In one embodiment the coformer increases oral bioavailability of the API by at least three fold. In one embodiment the coformer increases oral bioavailability of the API by at least five fold.

In another aspect the coformer increases the Cₘₐₓ of the API. In one embodiment the coformer increases Cₘₐₓ of the API by at least 10%. In one embodiment the coformer increases Cₘₐₓ of the API by at least 25%. In one embodiment the coformer increases Cₘₐₓ of the API by at least 75%. In one embodiment the coformer increases Cₘₐₓ of the API by at least two fold. In one embodiment the coformer increases Cₘₐₓ of the API by at least three fold. In one embodiment the coformer increases Cₘₐₓ of the API by at least five fold.

In another aspect the coformer reduces the time to the Tₘₐₓ of the API. In one embodiment the coformer reduces the time to the Tₘₐₓ of the API by at least 10%. In one embodiment the coformer reduces the time to the Tₘₐₓ of the API by at least 25%. In one embodiment the coformer reduces the time to the Tₘₐₓ of the API by at least 75%. In one embodiment the coformer reduces the time to the Tₘₐₓ of the API by at least two fold. In one embodiment the coformer reduces the time to the Tₘₐₓ of the API by at least three fold. In one embodiment the coformer reduces the time to the Tₘₐₓ of the API by at least five fold.

In another aspect the coformer increases the permeability of the API in the small intestine. In one embodiment the coformer increases the permeability of the API by at least 10%. In one embodiment the coformer increases the permeability of the API by at least 25%. In one embodiment the coformer increases the permeability of the API by at least 75%. In one embodiment the coformer increases the permeability of the API by at least two fold. In one embodiment the coformer increases the permeability of the API by at least three fold. In one embodiment the coformer increases the permeability of the API by at least five fold.

In some embodiments the pharmaceutical composition is an oral dosage form. In some embodiments the pharmaceutical composition is a unit dose.

Also described herein is a method of treating or preventing a disease for which the API is indicated, the method comprising the step of administering to a patient in need of the API a therapeutically effective amount of a pharmaceutical composition of the present invention. In some instances the method is for treating such a disease. In some instances the method is for preventing such as disease.

Another aspect of the present invention provides for a pharmaceutical composition of the present invention for use in treating or preventing a disease for which the API is indicated. In one aspect the medicament is for use in treating such a disease. In another aspect the medicament is for use in preventing such a disease.

The various aspects and embodiments of the present invention expressly provide for combinations in any consistent manner since providing for all such combinations would unduly lengthen the specification. For example, the ranges provided for the amount of API or coformer apply to any one of the individual API-coformer combination and accordingly, each of which should be considered a specific embodiment of the present invention. To list each such API or coformer combination for each range would needlessly lengthen the specification.

The following detailed description, including Examples, which proceeds with reference to the accompanying drawings and tables are meant to be illustrative, not limiting, of the invention.

### BRIEF DESCRIPTION OF THE DRAWINGS

FIG. 1 shows PXRD diffractograms of: (A = zoledronic acid, sodium zoledronic salt and water complex), (B = NaCl), (Z1 = Zoledronic acid monohydrate), (Z3 = Zoledronic acid trihydrate).
FIG. 2 is an FTIR spectrum of a complex comprising zoledronic acid, sodium zoledronic salt, and water.
FIG. 3 shows PXRD diffractograms of: (C = ammonium zoledronic salt and water complex), (Z1 = Zoledronic acid monohydrate), and (Z3 = Zoledronic acid trihydrate).
FIG. 4 is an FTIR spectrum of ammonium zoledronic salt and water complex.
FIG. 5 shows PXRD diffractograms of: (D = zoledronic, L-lysine, and water complex), (E = L-lysine), (Z1 = Zoledronic acid monohydrate), and (Z3 = Zoledronic acid trihydrate).
FIG. 6 is an FTIR spectrum of zoledronic, L-lysine, and water complex.
FIG. 7 shows PXRD diffractograms of: (F = zoledronic, DL-lysine, and water complex), (G = DL-lysine), (Z1 = Zoledronic acid monohydrate), and (Z3 = Zoledronic acid trihydrate).
FIG. 8 is an FTIR spectrum of zoledronic, DL-lysine, and water complex.
FIG. 9 shows PXRD diffractograms of: (H = zoledronic acid, zoledronic, DL-lysine, ethanol, and water complex), (G = DL-lysine), (Z1 = Zoledronic acid monohydrate), (Z3 = Zoledronic acid trihydrate).
FIG. 10 is an FTIR spectrum of zoledronic acid, zoledronic, DL-lysine, ethanol, and water complex.
FIG. 11 shows PXRD diffractograms of: (I = zoledronic, nicotinamide, and water complex), (J = nicotinamide), (Z1 = Zoledronic acid monohydrate), and (Z3 = Zoledronic acid trihydrate).
FIG. 12 is an FTIR spectrum of zoledronic, nicotinamide, and water complex.
FIG. 13 shows PXRD diffractograms of: (K = zoledronic, adenine, and water complex), (L = adenine), (Z1 = Zoledronic acid monohydrate), (Z3 = Zoledronic acid trihydrate).
FIG. 14 is an FTIR spectrum of zoledronic, adenine, and water complex.
FIG. 15 shows PXRD diffractograms of: (M = zoledronic and glycine complex), (N = glycine), (Z1 = Zoledronic acid monohydrate), and (Z3 = Zoledronic acid trihydrate).
FIG. 16 is an FTIR spectrum of zoledronic and glycine complex.
FIG. 17 shows PXRD diffractograms of: (O = zoledronic diammonia water complex), (Z1 = Zoledronic acid monohydrate), and (Z3 = Zoledronic acid trihydrate).
FIG. 18 is an FTIR spectrum of zoledronic diammonia water complex.
FIG. 19 shows PXRD diffractograms of: (P = zoledronic, DL-lysine, and water complex), (G = DL-lysine), (Z1 = Zoledronic acid monohydrate), and (Z3 = Zoledronic acid trihydrate).
FIG. 20 is an FTIR spectrum of zoledronic, DL-lysine, and water complex.
FIG. 21 shows PXRD diffractograms of: (R = zoledronic, DL-lysine, and water complex), (G = DL-lysine), (Z1 = Zoledronic acid monohydrate), and (Z3 = Zoledronic acid trihydrate).
FIG. 22 is an FTIR spectrum of zoledronic, DL-lysine, and water complex.
FIG. 23 shows PXRD diffractograms of: (R = zoledronic, DL-lysine, and water complex), (G = DL-lysine), (Z1 = Zoledronic acid monohydrate), and (Z3 = Zoledronic acid trihydrate).
FIG. 24 is an FTIR spectrum of zoledronic, DL-lysine, and water complex.
FIG. 25 shows PXRD diffractograms of: (Q = zoledronic, L-lysine, and water complex), (E = L-lysine), (Z1 = Zoledronic acid monohydrate), and (Z3 = Zoledronic acid trihydrate).
FIG. 26 is an FTIR spectrum of zoledronic, L-lysine, and water complex.
FIG. 27 shows the 24 hr rat plasma PK profile of parent zoledronic acid and zoledronic acid complexes delivered via IV, oral, and intraduodenal (ID) routes.
FIG. 28 shows the 4 hr rat plasma PK profile of parent zoledronic acid and zoledronic acid complexes delivered orally.
FIG. 29 shows the 4 hr rat plasma PK profile of parent zoledronic acid and zoledronic acid complexes delivered ID.
FIG. 30 shows the 24 hr rat plasma PK profile of parent zoledronic acid and zoledronic acid complexes delivered by oral gavage.
FIG. 31 shows the 4 hr rat plasma PK profile of parent zoledronic acid and zoledronic acid complexes delivered orally.
FIG. 32 shows the 4 hr rat plasma PK profile of parent zoledronic acid and selected zoledronic acid complexes delivered orally.
FIG. 33 shows the dog serum PK profile of parent zoledronic acid and zoledronic acid complexes delivered IV and orally.
FIG. 34 shows the 4 hr dog serum PK profile of parent zoledronic acid and zoledronic acid complexes delivered IV and orally.
FIG. 35 shows the dog serum PK profile of parent zoledronic acid and zoledronic acid complexes delivered IV and orally, using enteric and non-enteric coated capsules.
FIG. 36 shows the 6 hr dog serum PK profile of parent zoledronic acid and zoledronic acid complexes delivered IV and orally, using enteric and non-enteric coated capsules.
Fig. 37 shows the dog serum PK data for the enteric and non-enteric coated hard gelatin capsules.
FIG. 38 shows the 24 hr dog serum PK profile of zoledronic acid complexes delivered IV and orally.
FIG. 39 shows the 4 hr dog serum PK profile of zoledronic acid complexes delivered IV and orally.
FIG. 40 shows the 4 hr dog serum PK profile of zoledronic acid complexes delivered orally.
FIG. 41 shows the 24 hr dog serum PK profile of zoledronic acid complexes delivered orally.
FIG. 42 shows the 4 hr dog serum PK profile of zoledronic acid complex delivered orally.
FIG. 43 shows the 24 hr dog serum PK profile of zoledronic acid complex delivered orally.
FIG. 44 shows the 4 hr dog serum PK profile of zoledronic acid complex with excess coformer delivered orally.
FIG. 45 shows the 24 hr dog serum PK profile of zoledronic acid complex with excess coformer delivered orally.

### DETAILED DESCRIPTION OF THE PREFERRED EMBODIMENTS

Novel API forms and formulations provide an opportunity to improve the performance characteristics of a pharmaceutical product. The present disclosure relates to new forms of active pharmaceutical ingredients (APIs) with improved physicochemical properties, such as improved aqueous solubility, rate of dissolution, and, particularly, increased permeability and bioavailability.

The term 'active pharmaceutical ingredient(s)' or 'API(s)' refers to the substance in a pharmaceutical drug that is biologically active.

As used herein, the terms 'treat', 'treating' or 'treatment' means to alleviate, reduce or abrogate one or more symptoms or characteristics of a disease and may be curative, palliative, prophylactic or slow the progression of the disease. The term 'therapeutically effective amount' is intended to mean that amount of drug that will elicit a desired biological or pharmacological response, i.e., an amount sufficient to treat said disease.

The term 'patient' includes mammals, especially humans. In one embodiment the patient is a human. In another embodiment the patient is a human male. In another embodiment the patient is a human female.

The term 'excipient' refers to a pharmaceutically acceptable, inactive substance used as a carrier for the pharmaceutically active ingredient(s) and includes antiadherents, binders, coatings, disintegrants, fillers, diluents, flavors, bulkants, colours, glidants, dispersing agents, wetting agents, lubricants, preservatives, sorbents and sweeteners. The choice of excipient(s) will depend on factors such as the particular mode of administration and the nature of the dosage form. The term 'functional excipient' refers to an excipient that improves the oral bioavailability of a drug, e.g., by increasing absorption, e.g., increasing paracellular and/or transcellular permeability, or increasing aqueous solubility.

The term 'oral bioavailability' is defined as AUCₒᵣₐₗ · dose_{i.v}/ AUC_{i.v}.· doseₒᵣₐₗ · 100%.

The term 'significant' or 'significantly' is determined by t-test at 0.05 level of significance.

The term 'molecular complex' refers to a material comprised of two or more unique molecules (in the case of a cocrystal) or ions (in the case of a salt) that are bonded together, and wherein one of the molecule/ions is an API and another of the molecule/ions is a coformer. The API and coformer are bonded either through ionic bonds (in the case of a salt) or hydrogen bonds (in the case of a cocrystal), or a combination of both ionic and hydrogen bonds in the case of a cocrystal of a salt. Other modes of molecular recognition may also be present including, pi-stacking, guest-host complexation and van der Waals interactions. The term also includes solvates, including hydrates, thereof.

The term 'cocrystal' refers to a crystalline material comprised of two or more unique molecules that are solids at room temperature, wherein one of the molecules is an API and one of the molecules is a coformer, wherein the API and coformer are both solids at room temperature and are bonded together by hydrogen bonds. Other modes of molecular recognition may also be present including, pi-stacking, guest-host complexation and van der Waals interactions. The term includes solvates of cocrystals, i.e., a solvated cocrystal, including hydrates of the same:

The term 'salt' refers to an ionic compound resulting from the neutralization reaction of an acid and a base, and in the case of a composition of the present invention, whereby one of the ions is an API and one of the ions, of an opposite charge, is a coformer, whereby the product is neutral (without a net charge).

The term 'coformer' refers to either (or both) a 'molecular complex coformer' or an 'additional coformer' ('excess coformer'). The term 'molecular complex coformer' refers to a coformer that is a component of a molecular complex with an API. The terms 'additional coformer' or 'excess coformer' refers to a coformer of the present invention that is not bound to the API as part of a molecular complex, i.e., wherein the coformer is a 'functional excipient'. An 'additional coformer' or 'excess coformer' may be present in addition to a 'molecular complex coformer,' or may be present in the absence of a 'molecular complex coformer,' (e.g., when an API is a free acid or free base).

The term 'unit dose' refers to the amount of API administered to a patient in a single dose.

The present invention describes pharmaceutical compositions with increased permeability. Increased permeability is achieved through the addition of a coformer to a pharmaceutical composition comprising an API, wherein the coformer is an amino acid.

In some instances the API is in the form of a molecular complex with the amino acid or other coformer. In another aspect a portion of the amino acid is in the form of a molecular complex with the API (as a molecular complex coformer) and a portion is not bound to the API (as an additional coformer). In some instances the API-amino acid molecular complex is a cocrystal. In some instances the API and amino acid molecular complex is a salt. In some instances the salt is crystalline. In some instances the amino acid not bound to the API is crystalline (as an additional coformer only).

The present invention also describes a pharmaceutical composition comprising an amino acid and an API, wherein the API is a BCS Class III or IV drug.

In some instances the amino acid is a standard amino acid. In particular instances the amino acid is isoleucine, alanine, leucine, asparagine, lysine, aspartic acid, methionine, cysteine, phenylalanine, glutamic acid, threonine, glutamine, tryptophan, glycine, valine, proline, serine, tyrosine arginine or histidine. In another instance the amino acid is selenocysteine, ornithine or taurine. In further particular instances the amino acid is the L-form (e.g., L-lysine). In other particular instances the amino acid is the D-form (e.g., D-lysine). In other particular instances the amino acid is the DL-form (e.g., DL-lysine).

The pharmaceutical compositions of the present invention have increased permeability of the API (compared to the corresponding composition without the molecular complex coformer selected from lysine and glycine). In one embodiment the compositions have increased paracellular transport of the API. In another embodiment the compositions have increased transcellular transport of the API. The increase in permeability results in an increase in bioavailability of the API. Thus the compositions of the present invention are particularly advantageous for oral dosage forms.

In one aspect the pharmaceutical compositions of the present invention have increased the oral bioavailability of the API (compared to the corresponding composition without molecular complex coformer selected from lysine and glycine). In one embodiment the increase in oral bioavailability is at least 10%. In another embodiment the increase in oral bioavailability is at least 25%. In another embodiment the increase in oral bioavailability is at least 50%. In another embodiment the increase in oral bioavailability is at least 75%. In another embodiment the increase in oral bioavailability is at least two fold. In another embodiment the increase in oral bioavailability is at least three fold.

In one embodiment the increase in oral bioavailability is achieved without the need of additional excipients, e.g., an intra-granular hydrophilic polymer.

Also described herein is a method of enhancing the permeability of an API comprising the step of combining the API with an amino acid to form a pharmaceutical composition of the present invention. In one instance the API is a BCS Class III or IV drug and the amino acid is L-lysine. In another instance the API is a BCS Class III or IV drug and the amino acid is DL-lysine. In another instance the API is a BCS Class III or IV drug and the amino acid is D-lysine. In another instance the API is a BCS Class III or IV drug and the amino acid is glycine.

In one aspect the pharmaceutical composition is an oral dosage form. In one embodiment the oral dosage form is a solid oral dosage form. In one embodiment the oral dosage form is a liquid oral dosage form. In one embodiment the liquid oral dosage form is a solution. In another embodiment the liquid oral dosage form is a suspension. In one embodiment the oral dosage form is a semi-solid oral dosage form.

In another aspect the pharmaceutical composition is a unit dose. In one embodiment the unit dose comprises at least 100mg of the molecular complex coformer (an amino acid selected from lysine and glycine). In another embodiment the unit dose comprises at least 250mg of amino acid. In another embodiment the unit dose comprises at least 500mg of amino acid. In another embodiment the unit dose comprises at least 750mg of amino acid. In another embodiment the unit dose comprises at least 100mg of amino acid. In another embodiment the unit dose comprises at least 1250mg of amino acid. In another embodiment the unit dose comprises at least 1750mg of amino acid. In another embodiment the unit dose comprises at least 2000mg of amino acid. In another embodiment the unit dose comprises at least 2500mg of amino acid. In another embodiment the unit dose comprises at least 3000mg of amino acid. In another embodiment the unit dose comprises at least 3500mg of amino acid. In another embodiment the unit dose comprises at least 4000mg of amino acid. In another embodiment the unit dose comprises at least 4500mg of amino acid. In another embodiment the unit dose comprises at least 5000mg of amino acid. In another embodiment the unit dose comprises at least 6000mg of amino acid. In another embodiment the unit dose comprises at least 7000mg of amino acid. In another embodiment the unit dose comprises at least 8000mg of amino acid. In another embodiment the unit dose comprises at least 9000mg of amino acid. In another embodiment the unit dose comprises at least 10g of amino acid. In another embodiment the unit dose comprises at least 11g of amino acid. In another embodiment the unit dose comprises at least 12g of amino acid. In another embodiment the unit dose comprises at least 13g of amino acid. In another embodiment the unit dose comprises at least 14g of amino acid. In another embodiment the unit dose comprises at least 15g of amino acid. In another embodiment the unit dose comprises at least 16g of amino acid. In another embodiment the unit dose comprises at least 17g of amino acid. In another embodiment the unit dose comprises at least 18g of amino acid. In another embodiment the unit dose comprises at least 19g of amino acid. In another embodiment the unit dose comprises at least 20g of amino acid. In another embodiment the unit dose comprises between about 50 to about 5000mg of amino acid. In another embodiment the unit dose comprises between about 100 to about 1000mg of amino acid. In another embodiment the unit dose comprises between about 1250 to about 5000mg of amino acid. In another embodiment the unit dose comprises between about 2000 to about 5000mg of amino acid. In another embodiment the unit dose comprises between about 3000 to about 5000mg of amino acid. In another embodiment the unit dose comprises between about 1250 to about 3000mg of amino acid. In another embodiment the unit dose comprises between about 1250 to about 2500mg of amino acid. In another embodiment the unit dose comprises between about 1g to about 20g of amino acid. In another embodiment the unit dose comprises between about 1250mg to about 20g of amino acid. In another embodiment the unit dose comprises between about 1500mg to about 20g of amino acid. In another embodiment the unit dose comprises between about 1g to about 10g of amino acid. In another embodiment the unit dose comprises between about 1250mg to about 10g of amino acid. In another embodiment the unit dose comprises between about 1500mg to about 10g of amino acid. In another embodiment the unit dose comprises between about 1g to about 5g of amino acid. In another embodiment the unit dose comprises between about 1250mg to about 5g of amino acid. In another embodiment the unit dose comprises between about 1500mg to about 5g of amino acid. In another embodiment the unit dose comprises between about 5g to about 15g of amino acid. In another embodiment the unit dose comprises between about 5g to about 10g of amino acid. In another embodiment the unit dose comprises between about 7g to about 10g of amino acid. In another embodiment the unit dose comprises between about 10g to about 20g of amino acid. In another embodiment the unit dose comprises between about 10g to about 15g of amino acid. In another embodiment the unit dose comprises between about 10g to about 12.5g of amino acid. In another embodiment the unit dose comprises between about 12.5g to about 20g of amino acid. In another embodiment the unit dose comprises between about 12.5g to about 17.5g of amino acid. In another embodiment the unit dose comprises between about 15g to about 20g of amino acid. In another embodiment the unit dose comprises between about 17.5g to about 20g of amino acid. In another embodiment the unit dose comprises between about 1g to about 2g of amino acid. In another embodiment the unit dose comprises between about 1g to about 2g of amino acid. In another embodiment the unit dose comprises between about 1g to about 2g of amino acid. In one embodiment the amino acid is lysine. In another embodiment the amino acid is glycine. In another embodiment the amino acid is L-lysine. In another embodiment the amino acid is DL-lysine. In another embodiment the amino acid is D-lysine.

Also described herein is a method of treating or preventing a disease for which an API is indicated, the method comprising the step of administering to a patient in need of the API a therapeutically effective amount of a pharmaceutical composition of the present invention comprising the API. In one instance the method is for treating such a disease. In another instance the method is for preventing such as disease. Another aspect of the present invention provides for a medicament comprising a pharmaceutical composition of the present invention for use in treating or preventing a disease for which the API is indicated. In one embodiment the medicament is for use in treating such a disease. In another embodiment the medicament is for use in preventing such a disease.

### Bisphosphonic Acids

A portion of the present disclosure relates to new crystalline forms and compositions of bisphosphonic acids. Bisphosphonic acids described herein include zoledronic acid, clodronic acid, tiludronic acid, pamidronic acid, alendronic acid, risedronic acid or ibandronic acid.

For example, a number of novel zoledronic acid forms and compositions with improved properties have been synthesized, characterized, and disclosed herein. Of particular interest are novel crystalline forms of zoledronic acid and compositions comprising zoledronic acid and a standard amino acid with enhanced permeability.

The results with bisphosphonic acids, e.g., zoledronic acid, are both surprising and unexpected. For example, it is known that bisphosphonic acids form insoluble complexes with metal ions such as Ca²⁺. Two means of depleting Ca²⁺ in the small intestine would be to either chelate the metal ion or cause its absorption before it could bind the bisphosphonic acid. Lysine and glycine however, are unable to form a coordinate covalent bond with Ca²⁺ based on their structure. At the physiological pH of the small intestine, which is about 6-6.5 in the duodenum and about 7.5 in the jejunum and ileum, lysine has a net positive charge. Even at a pH of ≥10.5, it will carry only a net negative charge of -1. Similarly, glycine can at most have a net negative charge of -1, occurring at about pH of ≥9.7, and thus, cannot form a coordinate covalent bond with Ca²⁺. At physiological pH, glycine is neutral. Alternatively, if lysine or glycine were acting to increase absorption of Ca²⁺ in the intestine, one would expect that the amino acid would have to be released into the small intestine long before the bisphosphonic acid in order provide enough time for the small intestine to absorb the Ca²⁺ present in the GI tract. PCT publication WO 03/007916 teaches that a Ca²⁺ absorption activator needs to be released into the small intestine at least one hour before the bisphosphonic acid. The compositions of the present invention, on the other hand, do not have additional formulation requirements. The compositions do not require the bisphosphonic acid to be formulated as a delayed release. Further the compositions do not have particular granulation requirements. For example, the compositions do not have to be granulated with a hydrophilic polymer as do the compositions of PCT publication WO 06/039499.

Further unexpected and surprising is the extent to which the compositions of the present invention improve the oral bioavailability of bisphosphonic acids. For example, an oral bioavailability of greater than 8% has been achieved with zoledronic acid (see Leg 37). The data predicts an oral bioavailability well over this with increasing amounts of amino acid. The ability to achieve such high levels of oral bioavailability has the distinct advantage of being able to lower the dose of the drug, thereby increasing safety to the patient. In the case of bisphosphonic acids, side effects center on severe esophageal and GI irritation and ulceration that are worse when stringent dosing guidelines are not followed. A lower dose of bisphosphonic acid should result in reduced esophageal and GI irritation or ulceration and thus, increased safety to the patient. Accordingly, one aspect of the invention is an oral dosage form of a pharmaceutical composition of the present invention, wherein said pharmaceutical composition has an improved safety profile over the corresponding marketed formulation: in the case of zoledronic acid, marketed as ZOMETA as a 4 mg dose for hypercalcemia of malignancy, metastatic bone disease, osteoporosis, and Paget's disease and marketed as RECLAST as a 5mg annual dose for postmenopausal osteoporosis. Another aspect of the invention is an oral dosage form of a pharmaceutical composition of the present invention wherein said pharmaceutical composition has reduced esophageal and GI irritation or ulceration over the corresponding zoledronic acid or its marketed formulation. Another aspect of the invention is an oral dosage form of a pharmaceutical composition of the present invention, wherein the permeability of said pharmaceutical composition is less effected by food compared to that of the corresponding marketed oral formulation.

Schematic diagrams for zoledronic acid:amino acid complexes (a zoledronic acid:lysine complex and a zoledronic acid:glycine complex, two embodiments of the invention) are shown below. The diagrams show a molecular structure of the complex and possible interactions between the constituents of the complex which is different from the physical mix of the constituents.

### Zoledronic acid : lysine complex

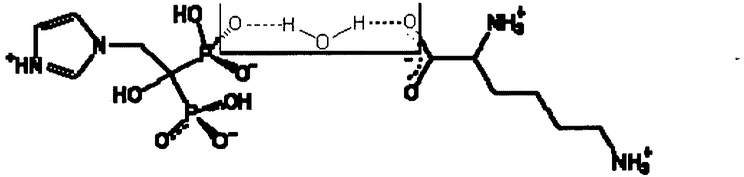

### Zoledronic acid : glycine complex

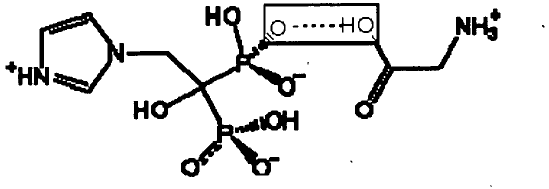

These represent one of the arrangements in which the molecules of the drug and the standard amino acids coformers could interact to form a stable complex that, even when stressed thermally in an elevated relative humidity (RH) environment, have not displayed any signs of deterioration or disintegration to its original constituents. Such stability can be attributed to the hydrogen bonding (dashed line in the box) or ionic interactions in these molecular complexes. When packing in a crystal structure these complexes exhibit a very different spatial arrangement in comparison to that of its constituents or their physical mix as indicated by their powder X-ray diffraction (PXRD) patterns and therefore would possess different, unpredictable physicochemical properties.

The present disclosure includes new forms and formulations of bisphosphonic acids including zoledronic acid, with improved physicochemical properties, such as improved, safety, stability, aqueous solubility, rate of dissolution, permeability, and/or enhanced bioavailability.

The present disclosure includes novel molecular complexes of bisphosphonic acids (e.g., zoledronic acid) in the form of cocrystals, salts, mixed cocrystal-salts and solvates (e.g. hydrates), as well as combinations of such materials. In addition, the disclosure further includes methods for the preparation of such molecular complexes.

In one embodiment the molecular complex is a salt. In another embodiment the molecular complex is a cocrystal. In another embodiment the molecular complex is a crystalline two-component molecular complex between zoledronic acid and a single coformer selected from lysine and glycine. In another embodiment the molecular complex is a crystalline three-component molecular complex comprising the zoledronic acid and at least one coformer. In a further embodiment the crystalline three-component molecular complex consists of the zoledronic acid, a first coformer and a second (different) coformer. In a further embodiment the crystalline three-component molecular complex consists of the zoledronic acid, a coformer and a solvent. In a further embodiment the solvent is water.

In one aspect the molar ratio of coformer to zoledronic acid in the molecular complex is about 1:1. In another aspect the coformer is in molar excess to the zoledronic acid. In one embodiment the molar ratio of coformer to zoledronic acid is between about 2:1 and 10:1. In a further embodiment the molar ratio is between about 2:1 and about 5:1. In a further embodiment the molar ratio is about 2:1. In another embodiment the molar ratio is about 3:1. In another embodiment the molar ratio is about 4:1. In another embodiment the molar ratio is about 5:1. In another aspect the zoledronic acid is in molar ratio to the coformer. In one embodiment the molar ratio is between about 2:1 and about 10:1. In another embodiment the molar ratio is between about 2:1 and about 5:1. In another embodiment the molar ratio is about 2:1. In another embodiment the molar ratio is about 3:1. In another embodiment the molar ratio is about 4:1. In another embodiment the molar ratio is about 5:1.

In one embodiment the additional coformer and the coformer that forms a molecular complex with the zoledronic acid, i.e., the molecular complex coformer, are the same. In another embodiment the additional coformer and the molecular complex coformer, are different. In another embodiment the additional coformer is crystalline. In another embodiment the additional coformer is amorphous. The mass ratio of the additional coformer to the molecular complex coformer is generally between about 2:1 to about 5000:1. In another embodiment the mass ratio of additional coformer to molecular complex coformer is between about 1000:1 to about 5000:1. In another embodiment the mass ratio of additional coformer to molecular complex coformer is between about 1000:1 to about 4000:1. In another embodiment the mass ratio of additional coformer to molecular complex coformer is between about 2000:1 to about 4000:1. In another embodiment the mass ratio of additional coformer to molecular complex coformer is between about 1000:1 to about 2000:1. In another embodiment the mass ratio of additional coformer to molecular complex coformer is between about 100:1 to about 2000:1. In another embodiment the mass ratio of additional coformer to molecular complex coformer is between about 100:1 to about 1000:1. In another embodiment the mass ratio of additional coformer to molecular complex coformer is between about 100:1 to about 750:1. In another embodiment the mass ratio of additional coformer to molecular complex coformer is between about 100:1 to about 500:1. In another embodiment the mass ratio of additional coformer to molecular complex coformer is between about 100:1 to about 275:1. In another embodiment the mass ratio of additional coformer to molecular complex coformer is between about 200:1 to about 275:1. In another embodiment the mass ratio of additional coformer to molecular complex coformer is between about 175:1 to about 275:1. In another embodiment the mass ratio of additional coformer to molecular complex coformer is between about 150:1 to about 250:1. In another embodiment the mass ratio of additional coformer to molecular complex coformer is between about 100:1 to about 250:1. In another embodiment the mass ratio of additional coformer to molecular complex coformer is between about 100:1 to about 200:1. In another embodiment the mass ratio of additional coformer to molecular complex coformer is between about 50:1 to about 200:1. In another embodiment the mass ratio of additional coformer to molecular complex coformer is between about 50:1 to about 150:1. In another embodiment the mass ratio of additional coformer to molecular complex coformer is between about 50:1 to about 100:1. In another embodiment the mass ratio of additional coformer to molecular complex coformer is between about 2:1 to about 100:1. In another embodiment the mass ratio of additional coformer to molecular complex coformer is between about 5:1 to about 100:1. In another embodiment the mass ratio of additional coformer to molecular complex coformer is between about 10:1 to about 100:1. In another embodiment the mass ratio of additional coformer to molecular complex coformer is between about 11:1 to about 100:1. In another embodiment the mass ratio of additional coformer to molecular complex coformer is between about 25:1 to about 100:1. In another embodiment the mass ratio of additional coformer to molecular complex coformer is between about 50:1 to about 100:1. In another embodiment the mass ratio of additional coformer to molecular complex coformer is between about 75:1 to about 100:1. In another embodiment the mass ratio of additional coformer to molecular complex coformer is between about 2:1 to about 50:1. In another embodiment the mass ratio of additional coformer to molecular complex coformer is between about 5:1 to about 50:1. In another embodiment the mass ratio of additional coformer to molecular complex coformer is between about 10:1 to about 50:1. In another embodiment the mass ratio of additional coformer to molecular complex coformer is between about 11:1 to about 50:1. In another embodiment the mass ratio of additional coformer to molecular complex coformer is between about 15:1 to about 50:1. In another embodiment the mass ratio of additional coformer to molecular complex coformer is between about 25:1 to about 50:1. In another embodiment the mass ratio of additional coformer to molecular complex coformer is between about 35:1 to about 50:1.

Also described herein is a composition comprising a bisphosphonic acid and a coformer, wherein the bisphosphonic acid and coformer are not associated in a molecular complex, i.e., a composition comprising additional conformer but not molecular complex coformer. In one embodiment the amount of additional coformer present in the composition is in excess to the amount of bisphosphonic acid present in the composition. In another embodiment the mass ratio of the additional coformer to bisphosphonic acid is between about 2:1 to about 5000:1. In another embodiment the mass ratio of additional coformer to bisphosphonic acid is between about 1000:1 to about 5000:1. In another embodiment the mass ratio of additional coformer to bisphosphonic acid is between about 1000:1 to about 4000:1. In another embodiment the mass ratio of additional coformer to bisphosphonic acid is between about 2000:1 to about 4000:1. In another embodiment the mass ratio of additional coformer to bisphosphonic acid is between about 1000:1 to about 2000:1. In another embodiment the mass ratio of additional coformer to bisphosphonic acid is between about 100:1 to about 2000:1. In another embodiment the mass ratio of additional coformer to bisphosphonic acid is between about 100:1 to about 1000:1. In another embodiment the mass ratio of additional coformer to bisphosphonic acid is between about 100:1 to about 750:1. In another embodiment the mass ratio of additional coformer to bisphosphonic acid is between about 100:1 to about 500:1. In another embodiment the mass ratio of additional coformer to bisphosphonic acid is between about 100:1 to about 275:1. In another embodiment the mass ratio of additional coformer to bisphosphonic acid is between about 200:1 to about 275:1. In another embodiment the mass ratio of additional coformer to bisphosphonic acid is between about 175:1 to about 275:1. In another embodiment the mass ratio of additional coformer to bisphosphonic acid is between about 150:1 to about 250:1. In another embodiment the mass ratio of additional coformer to bisphosphonic acid is between about 100:1 to about 250:1. In another embodiment the mass ratio of additional coformer to bisphosphonic acid is between about 100:1 to about 200:1. In another embodiment the mass ratio of additional coformer to bisphosphonic acid is between about 50:1 to about 200:1. In another embodiment the mass ratio of additional coformer to bisphosphonic acid is between about 50:1 to about 150:1. In another embodiment the mass ratio of additional coformer to bisphosphonic acid is between about 50:1 to about 100:1. In another embodiment the mass ratio of additional coformer to bisphosphonic acid is between about 2:1 to about 100:1. In another embodiment the mass ratio of additional coformer to bisphosphonic acid is between about 5:1 to about 100:1. In another embodiment the mass ratio of additional coformer to bisphosphonic acid is between about 10:1 to about 100:1. In another embodiment the mass ratio of additional coformer to bisphosphonic acid is between about 11:1 to about 100:1. In another embodiment the mass ratio of additional coformer to bisphosphonic acid is between about 25:1 to about 100:1. In another embodiment the mass ratio of additional coformer to bisphosphonic acid is between about 50:1 to about 100:1. In another embodiment the mass ratio of additional coformer to bisphosphonic acid is between about 75:1 to about 100:1. In another embodiment the mass ratio of additional coformer to bisphosphonic acid is between about 2:1 to about 50:1. In another embodiment the mass ratio of additional coformer to bisphosphonic acid is between about 5:1 to about 50:1. In another embodiment the mass ratio of additional coformer to bisphosphonic acid is between about 10:1 to about 50:1. In another embodiment the mass ratio of additional coformer to bisphosphonic acid is between about 11:1 to about 50:1. In another embodiment the mass ratio of additional coformer to bisphosphonic acid is between about 15:1 to about 50:1. In another embodiment the mass ratio of additional coformer to bisphosphonic acid is between about 25:1 to about 50:1. In another embodiment the mass ratio of additional coformer to bisphosphonic acid is between about 35:1 to about 50:1. In another embodiment the mass ratio of additional coformer to bisphosphonic acid is at least 2:1. In another embodiment the mass ratio of additional coformer to bisphosphonic acid is at least 5:1. In another embodiment the mass ratio of additional coformer to bisphosphonic acid is at least 10:1. In another embodiment the mass ratio of additional coformer to bisphosphonic acid is at least 11:1. In another embodiment the mass ratio of additional coformer to bisphosphonic acid is at least 15:1. In another embodiment the mass ratio of additional coformer to bisphosphonic acid is at least 25:1. In another embodiment the mass ratio of additional coformer to bisphosphonic acid is at least 35:1. In another embodiment the mass ratio of additional coformer to bisphosphonic acid is at least 50:1. In another embodiment the mass ratio of additional coformer to bisphosphonic acid is at least 65:1. In another embodiment the mass ratio of additional coformer to bisphosphonic acid is at least 75:1. In another embodiment the mass ratio of additional coformer to bisphosphonic acid is at least 85:1. In another embodiment the mass ratio of additional coformer to bisphosphonic acid is at least 100:1. In another embodiment the mass ratio of additional coformer to bisphosphonic acid is at least 125:1. In another embodiment the mass ratio of additional coformer to bisphosphonic acid is at least 150:1. In another embodiment the mass ratio of additional coformer to bisphosphonic acid is at least 175:1. In another embodiment the mass ratio of additional coformer to bisphosphonic acid is at least 200:1. In another embodiment the mass ratio of additional coformer to bisphosphonic acid is at least 225:1. In another embodiment the mass ratio of additional coformer to bisphosphonic acid is at least 250:1. In another embodiment the mass ratio of additional coformer to bisphosphonic acid is at least 275:1, In another embodiment the mass ratio of additional coformer to bisphosphonic acid is at least 500:1. In another embodiment the mass ratio of additional coformer to bisphosphonic acid is at least 750:1. In another embodiment the mass ratio of additional coformer to bisphosphonic acid is at least 1000:1. In another embodiment the mass ratio of additional coformer to bisphosphonic acid is at least 2000:1. In another embodiment the mass ratio of additional coformer to bisphosphonic acid is at least 3000:1. In another embodiment the mass ratio of additional coformer to bisphosphonic acid is at least 4000:1. In one embodiment the bisphosphonic acid is zoledronic acid. In another embodiment the bisphosphonic acid is clodronic acid. In another embodiment the bisphosphonic acid is tiludronic acid. In another embodiment the bisphosphonic acid is pamidronic acid. In another embodiment the bisphosphonic acid is alendronic acid. In another embodiment the bisphosphonic acid is risedronic acid. In another embodiment the bisphosphonic acid is ibandronic acid. Also described herein are compositions as defined in Tables 12, 13, 14, and 15.

Also described herein is a method of increasing aqueous solubility of a bisphosphonic acid (e.g., zoledronic acid), compared with the free acid, comprising the step of combining a bisphosphonic acid with a coformer and forming a composition of the present invention. In one embodiment the method comprises the step of forming a molecular complex of the present invention. In another embodiment the method comprises the step of combining a bisphosphonic acid (including salts, cocrystals, solvates and prodrugs) with an amino acid. In one embodiment the bisphosphonic acid is zoledronic acid. In another embodiment the bisphosphonic acid is clodronic acid. In another embodiment the bisphosphonic acid is tiludronic acid. In another embodiment the bisphosphonic acid is pamidronic acid. In another embodiment the bisphosphonic acid is alendronic acid. In another embodiment the bisphosphonic acid is risedronic acid. In another embodiment the bisphosphonic acid is ibandronic acid. In another embodiment the bisphosphonic acid is zoledronic acid and the amino acid is lysine or glycine. In another embodiment the bisphosphonic acid is zoledronic acid and the amino acid is L-lysine. In another embodiment the bisphosphonic acid is zoledronic acid and the amino acid is DL-lysine. In another embodiment the bisphosphonic acid is zoledronic acid and the amino acid is D-lysine. In another embodiment the aqueous solubility of the composition comprising zoledronic acid is at least 5mg/ml. In another embodiment the aqueous solubility of the composition comprising zoledronic acid is at least 10mg/ml. In another embodiment the aqueous solubility of the composition comprising zoledronic acid is at least 13mg/ml.

Also described herein is a molecular complex, wherein said complex is a crystalline zoledronic acid, sodium zoledronate and water complex characterized by an X-ray powder diffraction pattern having peaks at about 8.1, 13.3, 21.5, 24.6, and 25.6 ±0.2 degrees two-theta.

Also described herein is a molecular complex, wherein said complex is a crystalline ammonium zoledronic acid salt and water complex characterized by an X-ray powder diffraction pattern having strong peaks at about 11.0, 14.6, 15.4, 19.9, and 29.4 ±0.2 degrees two-theta.

Also described herein is a molecular complex, wherein said complex is a zoledronic acid diammonia water complex characterized by an X-ray powder diffraction pattern having strong peaks at about 12.2, 13.0, 14.1, 17.1, and 19.3 ±0.2 degrees two-theta.

In one embodiment the molecular complex is a crystalline zoledronic acid, L-lysine, and water complex characterized by an X-ray powder diffraction pattern having peaks at about 9.0, 14.4, 18.1, 26.0, and 29.6 ±0.2 degrees two-theta.

In another embodiment the molecular complex is a crystalline zoledronic acid, L-lysine, and water complex characterized by an X-ray powder diffraction pattern comprising peaks at about 9.6, 10.7, 14.3, 21.4, 23.5 ±0.2 degrees two theta. I

n another embodiment the molecular complex is a crystalline zoledronic acid, DL-lysine and water complex characterized by an X-ray powder diffraction pattern comprising peaks at about 8.3, 11.8, 12.3, 15.8, and 20.8 ±0.2 degrees two-theta.

In another embodiment the molecular complex is a crystalline zoledronic acid, DL-lysine, and water complex characterized by an X-ray powder diffraction pattern comprising peaks at about 9.1, 14.7, 18.0, 21.2, and 26.0 ±0.2 degrees two-theta.

In another embodiment the molecular complex is a crystalline zoledronic acid, DL-lysine, and water complex characterized by an X-ray powder diffraction pattern comprising peaks at about 9.7, 10.8, 14.4, 18.9, 21.4 ±0.2 degrees two theta.

In another embodiment the molecular complex is a crystalline zoledronic acid, DL-lysine, ethanol, and water complex characterized by an X-ray powder diffraction pattern comprising peaks at about 8.8, 9.7, 17.6, 23.1, and 26.5 ±0.2 degrees two-theta.

Also described herein is a molecular complex, wherein said complex is a crystalline zoledronic acid, adenine, and water complex characterized by an X-ray powder diffraction pattern comprising peaks at about 13.6 15.9, 19.7, 27.9, and 29.5 ±0.2 degrees two-theta.

Also described herein is a molecular complex, wherein said complex is a crystalline zoledronic acid, nicotinamide, and water complex characterized by an X-ray powder diffraction pattern comprising peaks at about 13.1, 15.2, 21.0, 23.9, and 26.5 ±0.2 degrees two-theta.

In another particular embodiment the zoledronic and glycine crystalline form is characterized by an X-ray powder diffraction pattern comprising peaks at about 10.2, 17.8, 19.9, 22.9, and 28.1 ±0.2 degrees two-theta.

In one embodiment the pharmaceutical composition is a solid dosage form. In another embodiment the pharmaceutical composition is a liquid dosage form. In another embodiment the pharmaceutical composition is an oral dosage form. In another embodiment the pharmaceutical composition is a unit dose. In another embodiment the unit dose is a single tablet or capsule. In another embodiment the unit dose is two tablets or capsules. In another embodiment the unit dose is in the form of a particulate material, e.g., a granulated particulate material or powder. In another embodiment the unit dose is enclosed in a sachet, a disposable one time use package. In another embodiment the unit dose is in the form of a solution. In another embodiment the unit dose is in the form of a suspension. In another embodiment the unit dose is an effervescent formulation. In one aspect of an oral dosage form both the zoledronic acid and the additional coformer are formulated to have the same release profile. In another embodiment both the zoledronic acid and the additional coformer are formulated to have an enteric release profile. In another embodiment the zoledronic acid is formulated to have an enteric release profile. In another embodiment both the zoledronic acid and the additional coformer are formulated to have a sustained release profile. In another embodiment the zoledronic acid is formulated to have a sustained release profile. In another embodiment both the additional coformer is formulated to have a sustained release profile. In another embodiment both the zoledronic acid and the additional coformer are formulated to have a delayed + sustained release profile. In another embodiment the zoledronic acid is formulated to have a delayed + sustained release profile. In another embodiment the additional coformer is formulated to have a delayed + sustained release profile. In one embodiment, the sustained release is a first-order release. In another embodiment the sustained release is a zero-order release. In another embodiment the zoledronic acid and the additional coformer are formulated a biphasic release. In one embodiment the Tₘₐₓ of the zoledronic acid is reached within one hour of the Tₘₐₓ of the coformer. In another embodiment the Tₘₐₓ of the zoledronic acid is reached within 45 minutes of the Tₘₐₓ of the coformer. In another embodiment the Tₘₐₓ of the zoledronic acid is reached within 30 minutes of the Tₘₐₓ of the coformer. In another embodiment the Cₘₐₓ of the zoledronic acid is reached within one hour of the Cₘₐₓ of the coformer. In another embodiment the Cₘₐₓ of the zoledronic acid is reached within 45 minutes of the Cₘₐₓ of the coformer. In another embodiment the Cₘₐₓ of the zoledronic acid is reached within 30 minutes of the Cₘₐₓ of the coformer. In another embodiment the Cₘₐₓ and Tₘₐₓ for the coformer occurs less than one hour before the Cₘₐₓ and Tₘₐₓ of the zoledronic acid. In another embodiment, the Cₘₐₓ and Tₘₐₓ for the coformer occur less than 45 minutes before the Cₘₐₓ and Tₘₐₓ of the zoledronic acid. In another embodiment, the Cₘₐₓ and Tₘₐₓ for the coformer occur less than 30 minutes before the Cₘₐₓ and Tₘₐₓ of the bisphosphonic acid. In another embodiment, the Cₘₐₓ and Tₘₐₓ for the zoledronic acid occurs before the Cₘₐₓ and Tₘₐₓ of the coformer.

The pharmaceutical compositions generally contain about 1% to about 99% by weight of at least one novel molecular complex of zoledronic acid) with the remaining 99% to 1% by weight of a comprising one or more coformers and one or more suitable pharmaceutical excipients.

In one embodiment the unit dose of zoledronic acid comprises between about 50 to about 5000mg of lysine. In another embodiment the unit dose of zoledronic acid comprises between about 100 to about 1000mg of lysine. In another embodiment the unit dose of zoledronic acid comprises between about 1250 to about 5000mg of lysine. In another embodiment the unit dose of zoledronic acid comprises between about 2000 to about 5000mg of lysine. In another embodiment the unit dose of zoledronic acid comprises between about 3000 to about 5000mg of lysine. In another embodiment the unit dose of zoledronic acid comprises between about 1250 to about 3000mg of lysine. In another embodiment the unit dose of zoledronic acid comprises between about 1250 to about 2500mg of lysine. In another embodiment the unit dose of zoledronic acid comprises between about 1g to about 20g of lysine. In another embodiment the unit dose of zoledronic acid comprises between about 1250mg to about 20g of lysine. In another embodiment the unit dose of zoledronic acid comprises between about 1500mg to about 20g of lysine. In another embodiment the unit dose of zoledronic acid comprises between about 1g to about 10g of lysine. In another embodiment the unit dose of zoledronic acid comprises between about 1250mg to about 10g of lysine. In another embodiment the unit dose of zoledronic acid comprises between about 1500mg to about 10g of lysine. In another embodiment the unit dose of zoledronic acid comprises between about 1g to about 5g of lysine. In another embodiment the unit dose of zoledronic acid comprises between about 1250mg to about 5g of lysine. In another embodiment the unit dose of zoledronic acid comprises between about 1500mg to about 5g of lysine. In another embodiment the unit dose of zoledronic acid comprises between about 5g to about 15g of lysine. In another embodiment the unit dose of zoledronic acid comprises between about 5g to about 10g of lysine. In another embodiment the unit dose of zoledronic acid comprises between about 7g to about 10g of lysine. In another embodiment the unit dose of zoledronic acid comprises between about 10g to about 20g of lysine. In another embodiment the unit dose of zoledronic acid comprises between about 10g to about 15g of lysine. In another embodiment the unit dose of zoledronic acid comprises between about 10g to about 12.5g of lysine. In another embodiment the unit dose of zoledronic acid comprises between about 12.5g to about 20g of lysine. In another embodiment the unit dose of zoledronic acid comprises between about 12.5g to about 17.5g of lysine. In another embodiment the unit dose of zoledronic acid comprises between about 15g to about 20g of lysine. In another embodiment the unit dose of zoledronic acid comprises between about 17.5g to about 20g of lysine. In another embodiment the unit dose of zoledronic acid comprises between about 1g to about 2g of lysine. In another embodiment the unit dose of zoledronic acid comprises between about 1g to about 2g of lysine. In another embodiment the unit dose of zoledronic acid comprises between about 1g to about 2g of lysine. In another embodiment a unit dose of a zoledronic acid pharmaceutical composition comprises at least 100mg of lysine. In another embodiment the unit dose of zoledronic acid comprises at least 250mg of lysine. In another embodiment the unit dose of zoledronic acid comprises at least 500mg of lysine. In another embodiment the unit dose of zoledronic acid comprises at least 750mg of lysine. In another embodiment the unit dose of zoledronic acid comprises at least 1000mg of lysine. In another embodiment the unit dose of zoledronic acid comprises at least 1100mg of lysine. In another embodiment the unit dose of zoledronic acid comprises at least 1200mg of lysine. In another embodiment the unit dose of zoledronic acid comprises at least 1250mg of lysine. In another embodiment the unit dose of zoledronic acid comprises at least 1500mg of lysine. In another embodiment the unit dose of zoledronic acid comprises at least 1750mg of lysine. In another embodiment the unit dose of zoledronic acid comprises at least 1900mg of lysine. In another embodiment the unit dose of zoledronic acid comprises at least 2000mg of lysine. In another embodiment the unit dose of zoledronic acid comprises at least 2500mg of lysine. In another embodiment the unit dose of zoledronic acid comprises at least 3000mg of lysine. In another embodiment the unit dose of zoledronic acid comprises at least 3500mg of lysine. In another embodiment the unit dose of zoledronic acid comprises at least 4000mg of lysine. In another embodiment the unit dose of zoledronic acid comprises at least 4500mg of lysine. In another embodiment the unit dose of zoledronic acid comprises at least 5000mg of lysine. In another embodiment the unit dose of zoledronic acid comprises at least 6000mg of lysine. In another embodiment the unit dose of zoledronic acid comprises at least 7000mg of lysine. In another embodiment the unit dose of zoledronic acid comprises at least 8000mg of lysine. In another embodiment the unit dose of zoledronic acid comprises at least 9000mg of lysine. In another embodiment the unit dose of zoledronic acid comprises at least 10g of lysine. In another embodiment the unit dose of zoledronic acid comprises at least 11g of lysine. In another embodiment the unit dose of zoledronic acid comprises at least 12g of lysine. In another embodiment the unit dose of zoledronic acid comprises at least 13g of lysine. In another embodiment the unit dose of zoledronic acid comprises at least 14g of lysine. In another embodiment the unit dose of zoledronic acid comprises at least 15g of lysine. In another embodiment the unit dose of zoledronic acid comprises at least 16g of lysine. In another embodiment the unit dose of zoledronic acid comprises at least 17g of lysine. In another embodiment the unit dose of zoledronic acid comprises at least 18g of lysine. In another embodiment the unit dose of zoledronic acid comprises at least 19g of lysine. In another embodiment the unit dose of zoledronic acid comprises at least 20g of lysine. In one embodiment the lysine in the unit dose of zoledronic acid is L-lysine. In another embodiment the lysine in the unit dose of zoledronic acid is DL-lysine. In another embodiment the lysine in the unit dose of zoledronic acid is D-lysine. In one embodiment a unit dose of a zoledronic acid pharmaceutical composition comprises at least 100mg of glycine. In another embodiment the unit dose of zoledronic acid comprises at least 250mg of glycine. In another embodiment the unit dose of zoledronic acid comprises at least 500mg of glycine. In another embodiment the unit dose of zoledronic acid comprises at least 750mg of glycine. In another embodiment the unit dose of zoledronic acid comprises at least 1000mg of glycine. In another embodiment the unit dose of zoledronic acid comprises at least 1100mg of glycine. In another embodiment the unit dose of zoledronic acid comprises at least 1200mg of glycine. In another embodiment the unit dose of zoledronic acid comprises at least 1250mg of glycine. In another embodiment the unit dose of zoledronic acid comprises at least 1500mg of glycine. In another embodiment the unit dose of zoledronic acid comprises at least 1750mg of glycine. In another embodiment the unit dose of zoledronic acid comprises at least 1900mg of glycine. In another embodiment the unit dose of zoledronic acid comprises at least 2000mg of glycine. In another embodiment the unit dose of zoledronic acid comprises at least 2500mg of glycine. In another embodiment the unit dose of zoledronic acid comprises at least 3000mg of glycine. In another embodiment the unit dose of zoledronic acid comprises at least 3500mg of glycine. In another embodiment the unit dose of zoledronic acid comprises at least 4000mg of glycine. In another embodiment the unit dose of zoledronic acid comprises at least 4500mg of glycine. In another embodiment the unit dose of zoledronic acid comprises at least 5000mg of glycine. In another embodiment the unit dose of zoledronic acid comprises at least 6000mg of glycine. In another embodiment the unit dose of zoledronic acid comprises at least 7000mg of glycine. In another embodiment the unit dose of zoledronic acid comprises at least 8000mg of glycine. In another embodiment the unit dose of zoledronic acid comprises at least 9000mg of glycine. In another embodiment the unit dose of zoledronic acid comprises at least 10g of glycine. In another embodiment the unit dose of zoledronic acid comprises at least 11g of glycine. In another embodiment the unit dose of zoledronic acid comprises at least 12g of glycine. In another embodiment the unit dose of zoledronic acid comprises at least 13g of glycine. In another embodiment the unit dose of zoledronic acid comprises at least 14g of glycine. In another embodiment the unit dose of zoledronic acid comprises at least 15g of glycine. In another embodiment the unit dose of zoledronic acid comprises at least 16g of glycine. In another embodiment the unit dose of zoledronic acid comprises at least 17g of glycine. In another embodiment the unit dose of zoledronic acid comprises at least 18g of glycine. In another embodiment the unit dose of zoledronic acid comprises at least 19g of glycine. In another embodiment the unit dose of zoledronic acid comprises at least 20g of glycine. In another embodiment the unit dose of zoledronic acid comprises between about 50 to about 5000mg of glycine. In another embodiment the unit dose of zoledronic acid comprises between about 100 to about 1000mg of glycine. In another embodiment the unit dose of zoledronic acid comprises between about 1250 to about 5000mg of glycine. In another embodiment the unit dose of zoledronic acid comprises between about 2000 to about 5000mg of glycine. In another embodiment the unit dose of zoledronic acid comprises between about 3000 to about 5000mg of glycine. In another embodiment the unit dose of zoledronic acid comprises between about 1250 to about 3000mg of glycine. In another embodiment the unit dose of zoledronic acid comprises between about 1250 to about 2500mg of glycine. In another embodiment the unit dose of zoledronic acid comprises between about 1g to about 20g of glycine. In another embodiment the unit dose of zoledronic acid comprises between about 1250mg to about 20g of glycine. In another embodiment the unit dose of zoledronic acid comprises between about 1500mg to about 20g of glycine. In another embodiment the unit dose of zoledronic acid comprises between about 1g to about 10g of glycine. In another embodiment the unit dose of zoledronic acid comprises between about 1250mg to about 10g of glycine. In another embodiment the unit dose of zoledronic acid comprises between about 1500mg to about 10g of glycine. In another embodiment the unit dose of zoledronic acid comprises between about 1g to about 5g of glycine. In another embodiment the unit dose of zoledronic acid comprises between about 1250mg to about 5g of glycine. In another embodiment the unit dose of zoledronic acid comprises between about 1500mg to about 5g of glycine. In another embodiment the unit dose of zoledronic acid comprises between about 5g to about 15g of glycine. In another embodiment the unit dose of zoledronic acid comprises between about 5g to about 10g of glycine. In another embodiment the unit dose of zoledronic acid comprises between about 7g to about 10g of glycine. In another embodiment the unit dose of zoledronic acid comprises between about 10g to about 20g of glycine. In another embodiment the unit dose of zoledronic acid comprises between about 10g to about 15g of glycine. In another embodiment the unit dose of zoledronic acid comprises between about 10g to about 12.5g of glycine. In another embodiment the unit dose of zoledronic acid comprises between about 12.5g to about 20g of glycine. In another embodiment the unit dose of zoledronic acid comprises between about 12.5g to about 17.5g of glycine. In another embodiment the unit dose of zoledronic acid comprises between about 15g to about 20g of glycine. In another embodiment the unit dose of zoledronic acid comprises between about 17.5g to about 20g of glycine. In another embodiment the unit dose of zoledronic acid comprises between about 1g to about 2g of glycine. In another embodiment the unit dose of zoledronic acid comprises between about 1g to about 2g of glycine. In another embodiment the unit dose of zoledronic acid comprises between about 1g to about 2g of glycine.

In one aspect a unit dose of a zoledronic acid pharmaceutical composition comprising zoledronic acid and lysine or glycine has an oral bioavailability of at least 3%. In another embodiment the composition has an oral bioavailability of at least 5%. In another embodiment the composition has an oral bioavailability of at least 8%. In one embodiment the amino acid is L-lysine and the oral bioavailability is at least 3%. In one embodiment the amino acid is L-lysine and the oral bioavailability is at least 5%. In one embodiment the amino acid is L-lysine and the oral bioavailability is at least 8%. In one embodiment the amino acid is DL-lysine and the oral bioavailability is at least 3%. In one embodiment the amino acid is DL-lysine and the oral bioavailability is at least 5%. In one embodiment the amino acid is DL-lysine and the oral bioavailability is at least 8%. In one embodiment the amino acid is D-lysine and the oral bioavailability is at least 3%. In one embodiment the amino acid is D-lysine and the oral bioavailability is at least 5%. In one embodiment the amino acid is D-lysine and the oral bioavailability is at least 8%. In one embodiment the amino acid is glycine and the oral bioavailability is at least 3%. In one embodiment the amino acid is glycine and the oral bioavailability is at least 5%. In one embodiment the amino acid is glycine and the oral bioavailability is at least 8%.

In one aspect the majority of the increase in oral bioavailability is due to the presence of the coformer, whether as part of a molecular complex or as additional coformer. In one embodiment the coformer is the only component of a pharmaceutical composition comprising a bisphosphonic acid-coformer molecular complex that significantly increases the oral bioavailability of the molecular complex. In one embodiment the lysine or glycine added as an excipient is the only component of a pharmaceutical composition comprising a bisphosphonic acid that increases the oral bioavailability of the molecular complex. In one embodiment the increase in oral bioavailability is achieved without the need of additional excipients, e.g., an intra-granular hydrophilic polymer.

In one aspect a unit dose of a zoledronic acid pharmaceutical composition comprising zoledronic acid and lysine or glycine is no more than 4.1mg/kg and is at least equivalent in efficacy to a 4mg unit dose of the marketed form ZOMETA (or its equivalent) administered intravenously. In one embodiment a unit dose of a zoledronic acid pharmaceutical composition comprising zoledronic acid and lysine or glycine is no more than 2.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine or glycine is no more than 1.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine or glycine is no more than 1mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine or glycine is no more than 0.75mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine or glycine is no more than 0.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine or glycine is no more than 0.3mg/kg and is at least equivalent in efficacy to a 4mg unit dose of the marketed form ZOMETA (or its equivalent) administered intravenously. In one embodiment a unit dose of a zoledronic acid pharmaceutical composition comprising zoledronic acid and lysine is no more than 4.1mg/kg and is at least equivalent in efficacy to a 4mg unit dose of the marketed form ZOMETA (or its equivalent) administered intravenously. In one embodiment a unit dose of a zoledronic acid pharmaceutical composition comprising zoledronic acid and lysine is no more than 2.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine is no more than 1.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine is no more than 1mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine is no more than 0.75mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine is no more than 0.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and lysine is no more than 0.3mg/kg and is at least equivalent in efficacy to a 4mg unit dose of the marketed form ZOMETA (or its equivalent) administered intravenously. In further particular embodiments the unit dose consists of or consists essentially of zoledronic acid and lysine. In one embodiment a unit dose of a zoledronic acid pharmaceutical composition comprising zoledronic acid and L-lysine is no more than 4.1mg/kg and is at least equivalent in efficacy to a 4mg unit dose of the marketed form ZOMETA (or its equivalent) administered intravenously. In one embodiment a unit dose of a zoledronic acid pharmaceutical composition comprising zoledronic acid and L-lysine is no more than 2.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and L-lysine is no more than 1.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and L-lysine is no more than 1mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and L-lysine is no more than 0.75mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and L-lysine is no more than 0.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and L-lysine is no more than 0.3mg/kg and is at least equivalent in efficacy to a 4mg unit dose of the marketed form ZOMETA (or its equivalent) administered intravenously. In further particular embodiments the unit dose consists of or consists essentially of zoledronic acid and L-lysine. In one embodiment a unit dose of a zoledronic acid pharmaceutical composition comprising zoledronic acid and DL-lysine is no more than 4.1mg/kg and is at least equivalent in efficacy to a 4mg unit dose of the marketed form ZOMETA (or its equivalent) administered intravenously. In one embodiment a unit dose of a zoledronic acid pharmaceutical composition comprising zoledronic acid and DL-lysine is no more than 2.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and DL-lysine is no more than 1.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and DL-lysine is no more than 1mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and DL-lysine is no more than 0.75mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and DL-lysine is no more than 0.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and DL-lysine is no more than 0.3mg/kg and is at least equivalent in efficacy to a 4mg unit dose of the marketed form ZOMETA (or its equivalent) administered intravenously. In further particular embodiments the unit dose consists of or consists essentially of zoledronic acid and DL-lysine. In one embodiment a unit dose of a zoledronic acid pharmaceutical composition comprising zoledronic acid and D-lysine is no more than 4.1mg/kg and is at least equivalent in efficacy to a 4mg unit dose of the marketed form ZOMETA (or its equivalent) administered intravenously. In one embodiment a unit dose of a zoledronic acid pharmaceutical composition comprising zoledronic acid and D-lysine is no more than 2.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and D-lysine is no more than 1.5mg/kg and is at least equivalent in efficacy to a 4mg unit dose of the marketed form ZOMETA (or its equivalent) administered intravenously. In another embodiment a unit .dose of a zoledronic acid pharmaceutical composition comprising zoledronic acid and D-lysine is no more than 1mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and D-lysine is no more than 0.75mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and D-lysine is no more than 0.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and D-lysine is no more than 0.3mg/kg and is at least equivalent in efficacy to a 4mg unit dose of the marketed form ZOMETA (or its equivalent) administered intravenously. In further particular embodiments the unit dose consists of or consists essentially of zoledronic acid and D-lysine. In one embodiment a unit dose of a zoledronic acid pharmaceutical composition comprising zoledronic acid and glycine is no more than 4.1mg/kg and is at least equivalent in efficacy to a 4mg unit dose of the marketed form ZOMETA (or its equivalent) administered intravenously. In one embodiment a unit dose of a zoledronic acid pharmaceutical composition comprising zoledronic acid and glycine is no more than 2.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and glycine is no more than 1.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and glycine is no more than 1mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and glycine is no more than 0.75mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and glycine is no more than 0.5mg/kg and is at least equivalent in efficacy to a 4mg unit dose of the marketed form ZOMETA (or its equivalent) administered intravenously. In another embodiment a unit dose of a zoledronic acid pharmaceutical composition comprising zoledronic acid and glycine is no more than 0.3mg/kg and is at least equivalent in efficacy to a 4mg unit dose of the marketed form ZOMETA (or its equivalent) administered intravenously. In further particular embodiments the unit dose consists of or consists essentially of zoledronic acid and glycine.

Another aspect of the present invention provides for a medicament comprising a pharmaceutical composition of the present invention for use in treating or preventing a disease for which zoledronic acid is indicated. In one embodiment the disease is selected from osteoporosis, hypercalcemia, cancer induced bone metastasis, Paget's disease, adjuvant cancer therapy or neoadjuvant cancer therapy. In one embodiment the medicament is for use in treating such a disease. In another embodiment the medicament is for use in preventing such a disease.

Also described herein are complexes of a bisphosphonic acid (e.g., zoledronic acid) with sodium, ammonium, ammonia, L-lysine, DL-lysine, nicotinamide, adenine and glycine which are capable of complexing in the solid-state, for example, through dry or solvent-drop grinding (liquid assisted grinding), heating or solvent evaporation of their solution in single or mixed solvent systems, slurry suspension, supercritical fluids or other techniques known to a person skilled in the art.

In one instance the present invention describes a zoledronic and nicotinamide complex to be made by dissolving both compounds in a water:ethylacetate (1:1 v/v) mixture and allowing the solvent to evaporate to form crystalline material.

In another instance, the present invention describes a zoledronic and glycine solid complex made by dissolving both compounds in water, and allowing the solvent to evaporate to form crystalline material.

In another instance, the present invention describes a molecular complex of zoledronic acid and a coformer selected from sodium, ammonium, ammonia, L-lysine, DL-lysine, nicotinamide, adenine or glycine, suitable for a pharmaceutical formulation than can be delivered orally to the human body.

### Pharmaceutical Compositions

A pharmaceutical composition of the invention may be in any pharmaceutical form, for example, a tablet, capsule, particulate material, e.g., granulated particulate material or a powder, oral liquid suspension, oral liquid solution, an injectable solution, a lyophilized material for reconstitution, suppository, topical, or transdermal.

In one aspect the invention provides for a composition comprising a micronized molecular complex of the present invention. In one embodiment the micronized molecular complex is zoledronic, DL-lysine and water molecular complex. In other embodiment the composition further comprises excess micronized cocrystal former (e.g., DL-lysine).

Another embodiment of the invention provides micronized novel zoledronic acid complex (zoledronic, DL-lysine and water) where the particle mean size diameter is 5 microns by volume.

Another aspect of the invention provides micronized excess coformer (e.g, DL-lysine) where the mean particle size diameter is 5 microns by volume.

Generally, the oral dosage forms described herein will contain from about 1 mg to about 500 mg of an API (e.g, bisphosphonic acid) on an anhydrous weight basis, depending on the particular API administered. In one aspect the oral dosage form is a unit dose of zoledronic acid. In one embodiment the unit dose is between about 10 mg to about 400 mg. In another embodiment the unit dose is between about 10 mg to about 100 mg. In another embodiment the unit dose is between about 100 mg to about 400 mg. In another embodiment the unit dose is between about 50 mg to about 100 mg. In another embodiment the unit dose is between about 10 mg to about 50 mg. In another embodiment the unit dose is between about 1 mg to about 10 mg. In one embodiment the zoledronic acid is dosed on a daily basis. In another embodiment the zoledronic acid is dosed twice weekly. In one embodiment the zoledronic is dosed on a weekly basis. In one embodiment the time between doses is ten days. In another embodiment the time between doses is two weeks. In another embodiment the time between doses is three weeks. In another embodiment the time between doses is four weeks. In another embodiment the time between doses is one month. In another embodiment the time between doses is six weeks. In another embodiment the time between doses is eight weeks. In another embodiment the time between doses is two months. In one embodiment the zoledronic acid is dosed no more frequent than once in a three month period. In one embodiment the zoledronic acid is dosed no more frequent than once in a six month period. In one embodiment the zoledronic acid is dosed no more frequent than once in a year. In one embodiment a course of treatment is between one month and one year. In another embodiment a course of treatment is between one month and six months. In one embodiment a course of treatment is between one month and three months. In one embodiment a course of treatment is between three months and six months. In one embodiment a course of treatment is one month. In another embodiment a course of treatment is two months. In another embodiment a course of treatment is three months.

The API (whether in the form of a molecular complex or as a free acid or base) and additional coformer combinations of the present invention (e.g., a zoledronic acid, L-lysine, and water complex and excess lysine) may be administered together or sequentially in single or multiple doses.

In one aspect the API and excess coformer are administered as a fixed dose combination product (e.g., a tablet containing both the molecular complex and excess coformer). In one embodiment the fixed dose combination product is a tablet or a capsule. In another embodiment the fixed dose combination product is a liquid solution or suspension. In another embodiment the fixed dose combination product is a particulate material, e.g., powder. In another embodiment the fixed dose combination product is a particulate material and is enclosed in a sachet. In another embodiment the fixed dose combination product is administered in single doses as part of a therapeutic treatment program or regimen. In another embodiment the fixed dose combination product is administered in multiple doses as part of a therapeutic treatment program or regimen.

In another aspect the API and excess coformer are administered as separate unit doses (e.g., two different tablets) but as part of the same therapeutic treatment program or regimen. In one embodiment, the API and excess coformer are administered simultaneously. In another embodiment the API and excess coformer are administered sequentially. In another embodiment the excess coformer is administered before the API. In another embodiment the API and excess coformer are administered in a single dose as part of the same therapeutic treatment program or regimen. In another embodiment the API and/or excess coformer is administered in multiple doses as part of the same therapeutic treatment program or regimen.

The compositions and dosage forms described herein can be administered via any conventional route of administration. In one embodiment the route of administration is oral.

Examples of suitable oral compositions of the present invention include tablets, capsules, troches, lozenges, suspensions, solutions, dispersible powders or granules, emulsions, syrups and elixirs.

Examples of fillers and diluents of the present invention include, for example, sodium carbonate, lactose, sodium phosphate and plant cellulose (pure plant filler). A range of vegetable fats and oils may be used in soft gelatin capsules. Other examples of fillers of the present invention include sucrose, glucose, mannitol, sorbitol, and magnesium stearate.

Examples of granulating and disintegrants of the present invention include corn starch and alginic acid, crosslinked polyvinyl pyrrolidone, sodium starch glycolate or crosslinked sodium carboxymethyl cellulose (crosscarmellose).

Examples of binding agents of the present invention include starch, gelatin, acacia, cellulose, cellulose derivatives, such as methyl cellulose, microcrystalline cellulose and hydroxypropyl cellulose, polyvinylpyrrolidone, sucrose, polyethylene glycol, lactose, or sugar alcohols like xylitol, sorbitol and maltitol.

Examples of lubricants of the present invention include magnesium stearate, stearic acid and talc.

Examples of coatings of the present invention include a hydroxy propylmethylcellulose (HPMC) and gelatin.

Tablets of the present invention may be uncoated or coated by known techniques. Such coatings may delay disintegration and thus, absorption in the gastrointestinal tract and thereby provide a sustained action over a longer period.

The pharmaceutical compositions of the present invention comprising an API and excess coformer may be formulated such that the API and excess coformer have the same release profile or different release profiles. In one aspect the API and excess coformer have the same release profile. In one embodiment a pharmaceutical composition comprising zoledronic acid and an amino acid (e.g., lysine) are formulated such that the zoledronic acid and amino acid have the same release profile. In another embodiment the zoledronic acid is released within one hour of the amino acid. In another embodiment the zoledronic acid is released within 30 minutes of the amino acid. In another embodiment the zoledronic acid is released within 15 minutes of the amino acid. In another embodiment the zoledronic acid is released before the amino acid. In another aspect the API is released before the excess coformer. In another aspect the excess coformer is released before the API. In another aspect the amino acid is released before the API.

The pharmaceutical compositions of the present invention may be formulated as a sustained release formulation such that the API or excess coformer is released over a longer period of time that it would be if formulated as an immediate release formulation. In one embodiment the excess coformer is formulated as a sustained release formulation. In another embodiment the API is formulated as a sustained release formulation. In another embodiment both the API and the excess coformer are formulated as a sustained release formulation.

The pharmaceutical compositions of the present invention may also be formulated as an immediate release formulation. In one embodiment the excess coformer is formulated as an immediate release formulation. In another embodiment the API is formulated as an immediate release formulation. In another embodiment both the API and the excess coformer are formulated as an immediate release formulation.

The pharmaceutical compositions of the present invention may further be formulated as a 'delayed + sustained release' formulation, a formulation intended to delay release of a drug until the dosage form has passed through the stomach, followed by sustained release of the drug in the small intestine. Such a release profile can be achieved, e.g., through coating of multiparticulates or hydrophilic matrix tablets with pH-dependent coating polymers, or coating with combinations of pH dependent coating polymers and extended release barrier membrane systems. In one embodiment the excess coformer is formulated as a delayed + sustained release formulation. In another embodiment the API is formulated as a delayed + sustained release formulation.

The pharmaceutical compositions of the present invention may further be formulated as an "enteric release" formulation, a formulation intended to delay release of a drug until the dosage form has passed through the stomach, followed by rapid release of the drug in the proximal small intestine. Such a release profile can be achieved through coating of particles or granules within a tablet or capsule or coating of the tablets with pH-dependent polymeric coating systems. In one embodiment the excess coformer is formulated as an enteric release formulation. In another embodiment the API is formulated as an enteric release formulation. In another embodiment the pharmaceutical composition is an enterically coated dosage form. In one embodiment enterically coated dosage form is an enterically coated hard gelatin capsule. In another embodiment enterically coated dosage form is an enterically coated soft or hard gelatin capsule. In another embodiment the enterically coated dosage form is an enterically coated tablet.

The term "modified enteric release" refers to a formulation that allows for a small portion of a drug dose to be released into the stomach, with the remainder of release occurring rapidly upon passage of the dosage form into the small intestine. Such a release profile can be achieved through the use of hydrophilic pore formers in pH dependent enteric coatings. In one embodiment the excess coformer is formulated as a modified enteric release formulation. In another embodiment the API is formulated as a modified enteric release formulation. In another embodiment both the excess coformer and the API are formulated as a modified enteric release formulation.

The term "biphasic release" refers to a formulation whereby a drug is released in a biphasic manner rather than a single phase. It also refers to a formulation where two different components, e.g., the excess coformer and API of the present invention, are released in a biphasic manner rather than a single phase. For example, a first dose may be released as an immediate release dose fraction, while a second dose is released as an extended release phase. Examples of such systems can be found as bilayer tablets, drug layered matrices, or multiparticulate combinations with different release profiles. In one embodiment the excess coformer is formulated as a biphasic release formulation. In another embodiment the molecular complex is formulated as a biphasic release formulation.

In another embodiment the excess coformer and molecular complex are formulated as a biphasic formulation, wherein said excess coformer and said API are formulated to be released in different phases thereby forming a biphasic release profile. In another embodiment the excess coformer and API are formulated as a biphasic release formulation, wherein said excess coformer is formulated to be released as a first phase and said API is formulated to be released as a second phase. In another embodiment the pharmaceutical composition of the present invention is formulated as a bilayer tablet comprising a first layer and a second layer, wherein said first layer comprises an excess coformer and an excipient, and wherein said second layer comprises an API and an excipient.

In another embodiment the pharmaceutical composition of the present invention is formulated as a multiparticulate formulation, i.e., a formulation comprising multiple particles. In one embodiment the API and excess coformer are in the same particle.

In another embodiment the pharmaceutical composition of the present invention is formulated as a tablet or capsule comprising a multiparticulate combination, said multiparticulate combination comprising a first multiparticulate formulation and a second multiparticulate formulation, wherein said first multiparticulate formulation comprises an excess coformer and, optionally, one or more excipient, and wherein said second multiparticulate formulation comprises a API and, optionally, one or more excipient.

In one embodiment the particles comprising the API, excess coformer or both API and excess coformer have a mean size diameter by volume of between about 1 and about 1000 microns. In one embodiment the particles have a mean size of between about 1 and about 100 microns. In one embodiment the particles have a mean size of between about 1 and about 10 microns. In one embodiment the particles have a mean size of between about 1 and about 5 microns. In one embodiment the particles have a mean size of between about 100 and about 1000 microns. In one embodiment the particles have a mean size of between about 100 and about 500 microns. In one embodiment the particles have a mean size of between about 200 and about 400 microns. In one embodiment the particles have a mean size of between about 300 and about 500 microns.

The term "Cₘₐₓ" refers to the maximum plasma concentration of a drug after administration.

In one embodiment, the excess coformer and API are formulated as a biphasic release formulation, wherein said excess coformer is formulated to be released as a first phase and said API is formulated to be released as a second phase, and wherein a Cₘₐₓ of said excess coformer occurs less than 60 minutes before a Cₘₐₓ of said API. In another embodiment, the Cₘₐₓ for said excess coformer occurs less than 45 minutes before the Cₘₐₓ of said API. In another embodiment, the Cₘₐₓ for said excess coformer occurs less than 30 minutes before the Cₘₐₓ of said API. In another embodiment, the Cₘₐₓ for said excess coformer occurs before the Cₘₐₓ of said API. In another embodiment, the Cₘₐₓ for said API occurs before the Cₘₐₓ of said excess coformer. In a particular embodiment wherein the pharmaceutical composition comprises a bisphosphonic acid, e.g., zoledronic acid and an amino acid, e.g., lysine, the Cₘₐₓ for said amino acid occurs less than 60 minutes before the Cₘₐₓ of said bisphosphonic acid. In another embodiment, the Cₘₐₓ for the amino acid occurs less than 45 minutes before the Cₘₐₓ of the bisphosphonic acid. In another embodiment, the Cₘₐₓ for the amino acid occurs less than 30 minutes before the Cₘₐₓ of the bisphosphonic acid. In another embodiment, the Cₘₐₓ for the bisphosphonic acid occurs before the Cₘₐₓ of the amino acid. In one embodiment, the excess coformer and API are formulated as a biphasic release formulation, wherein said excess coformer is formulated to be released as a first phase and said API is formulated to be released as a second phase, and wherein a Tₘₐₓ of said excess coformer occurs less than 60 minutes before a Tₘₐₓ of said API. In another embodiment, the Tₘₐₓ for said excess coformer occurs less than 45 minutes before the Tₘₐₓ of said API. In another embodiment, the Tₘₐₓ for said excess coformer occurs less than 30 minutes before the Tₘₐₓ of said API. In another embodiment, the Tₘₐₓ for said excess coformer occurs before the Tₘₐₓ of said API. In another embodiment, the Tₘₐₓ for said API occurs before the Tₘₐₓ of said excess coformer. In a particular embodiment wherein the pharmaceutical composition comprises a bisphosphonic acid, e.g., zoledronic acid and an amino acid, e.g., lysine, the Tₘₐₓ for said amino acid occurs less than 60 minutes before the Tₘₐₓ of said bisphosphonic acid. In another embodiment, the Tₘₐₓ for the amino acid occurs less than 45 minutes before the Tₘₐₓ of the bisphosphonic acid. In another embodiment, the Tₘₐₓ for the amino acid occurs less than 30 minutes before the Tₘₐₓ of the bisphosphonic acid. In another embodiment, the Tₘₐₓ for the bisphosphonic acid occurs before the Tₘₐₓ of the amino acid.

In one embodiment, the excess coformer and API are formulated as a biphasic release formulation, wherein said excess coformer is formulated to be released as a first phase and said API is formulated to be released as a second phase, and wherein a Cₘₐₓ and Tₘₐₓ of said excess coformer occurs less than 60 minutes before a Cₘₐₓ and Tₘₐₓ of said API. In another embodiment, the Cₘₐₓ and Tₘₐₓ for said excess coformer occurs less than 45 minutes before the Cₘₐₓ and Tₘₐₓ of said API. In another embodiment, the Cₘₐₓ and Tₘₐₓ for said excess coformer occurs less than 30 minutes before the Cₘₐₓ and Tₘₐₓ of said API. In another embodiment, the Cₘₐₓ and Tₘₐₓ for said excess coformer occurs before the Cₘₐₓ and Tₘₐₓ of said API. In another embodiment, the Cₘₐₓ and Tₘₐₓ for said API occurs before the Cₘₐₓ and Tₘₐₓ of said excess coformer. In a particular embodiment wherein the pharmaceutical composition comprises a bisphosphonic acid, e.g., zoledronic acid and an amino acid, e.g., lysine, the Cₘₐₓ and Tₘₐₓ for said amino acid occurs less than 60 minutes before the Cₘₐₓ and Tₘₐₓ of said bisphosphonic acid. In another embodiment, the Cₘₐₓ and Tₘₐₓ for the amino acid occur less than 45 minutes before the Cₘₐₓ and Tₘₐₓ of the bisphosphonic acid. In another embodiment, the Cₘₐₓ and Tₘₐₓ for the amino acid occur less than 30 minutes before the Cₘₐₓ and Tₘₐₓ of the bisphosphonic acid. In another embodiment, the Cₘₐₓ and Tₘₐₓ for the bisphosphonic acid occur before the Cₘₐₓ and Tₘₐₓ of the amino acid.

In one embodiment the excess coformer and API are formulated as a biphasic release formulation in a fixed dose combination product (e.g., in a single tablet). In one embodiment the excess coformer and API are each formulated as a multi-particulate formulation and combined to form a fixed dose combination product. In one embodiment the dosage form is a capsule comprising a first multiparticulate formulation of said excess coformer and a second multiparticulate formulation of said API as a fixed dose combination product. In another embodiment the fixed dose combination product is a bilayer tablet comprising a first layer and a second layer, wherein said first layer comprises an excess coformer and said second layer comprises an API.

In another embodiment, the API and excess coformer are formulated into a bilayer, whereby the API and matrix-forming material are combined and compressed to form a sustained release layer, and the excess coformer is blended with one or more agents and forms a second layer. In one embodiment the excess coformer layer is an immediate release formulation. In another embodiment the bilayer dosage form is enteric coated. In another embodiment the excess coformer layer and/or the API layer, is an enteric release formulation

The term "first-order release" refers to where the rate of elimination of drug from plasma is proportional to the plasma concentration of the drug. In one embodiment the excess coformer is released from the pharmaceutical composition as a first-order release. In one embodiment the API is released from the pharmaceutical composition as a first-order release. In one embodiment both the excess coformer and API are released from the pharmaceutical composition as a first-order release.

The term "zero order release" refers to the ability to deliver a drug at a rate which is independent of time and concentration of the drug within a pharmaceutical dosage form. Zero order mechanism.ensures that a steady amount of drug is released over time, minimizing potential peak/trough fluctuations and side effects, while maximizing the amount of time the drug concentrations remain within the therapeutic window (efficacy). Osmotic tablet formulations, coated tablet matrices, and the use of polymer combinations in hydrophilic matrices, for example, can be utilized to provide zero order drug release profiles. In one embodiment the excess coformer is released from the pharmaceutical composition as a zero-order release. In one embodiment the API is released from the pharmaceutical composition as a zero-order release. In one embodiment both the excess coformer and API are released from the pharmaceutical composition as a zero-order release.

Compounds useful for modifying a release profile of a drug are well known in the art. For example, a time delay material such as glyceryl monostearate or glyceryl distearate may be employed. The dosage form may also be coated by the techniques (e.g., those described in the U.S. Pat. Nos. 4,256,108; 4,166,452 and 4,265,874, each incorporated by reference in their entireties) to form osmotic therapeutic tablets for controlled release. Other controlled release technologies are also available and are included herein. Typical ingredients that are useful to slow the release of the drug in sustained release tablets include various cellulosic compounds, such as methylcellulose, ethylcellulose, propylcellulose, hydroxypropylcellulose, hydroxyethylcellulose, hydroxypropylmethylcellulose, microcrystalline cellulose, starch and the like. Various natural and synthetic materials are also of use in sustained release formulations. Examples include alginic acid and various alginates, polyvinyl pyrrolidone, tragacanth, locust bean gum, guar gum, gelatin, various long chain alcohols, such as cetyl alcohol and beeswax.

One embodiment of the invention includes a sustained release tablet that comprises the API in combination with one or more of the cellulosic compounds noted above, compressed into a sustained release tablet to form a polymer matrix. In another embodiment, the API and matrix-forming material are combined and compressed to form a sustained release core, and the excess coformer is blended with one or more coating agents and coated onto the outer surface of the core.

In one embodiment, the excess coformer is provided as a combined first immediate release dose and a second sustained release dose. The sustained release dose can be, for example, zero-order or first order. In certain embodiments the second dose has a lag time wherein the drug is released from the second dose at about 30 minutes, in another embodiment 1 hour, in another embodiment 1.5 hours, in another embodiment 2 hours, in another embodiment 2.5 hours, in another embodiment 3 hours, in another embodiment 3.5 hours and in another embodiment 4 hours after administration. The initial dose may be the same or different amount from the second dose.

In one aspect, the API is provided as a combined first immediate release dose and a second sustained release dose. The sustained release dose can be, for example, zero-order or first order. In certain embodiments the second dose has a lag time where drug is released from the second dose at about 30 minutes, in another embodiment 1 hour, in another embodiment 1.5 hours, in another embodiment 2 hours, in another embodiment 2.5 hours, in another embodiment 3 hours, in another embodiment 3.5 hours and in another embodiment 4 hours after administration. The initial dose may be the same or different from the second dose.

In one aspect, the excess coformer and API is provided in a combined single unit dose whereby the excess coformer is provided as an immediate release dose and API as a sustained release dose. The API sustained release dose can be, for example, zero-order or first order. In one embodiment the API second dose has a lag time where drug is released at about 30 minutes, in another embodiment 1 hour, in another embodiment 1.5 hours, in another embodiment 2 hours, in another embodiment 2.5 hours, in another embodiment 3 hours, in another embodiment 3.5 hours and in another embodiment 4 hours after administration.

Typical release time frames for sustained release tablets in accordance with the present invention range from about 1 to as long as about 48 hours, preferably about 4 to about 24 hours, and more preferably about 8 to about 16 hours.

Hard gelatin capsules constitute another solid dosage form for oral use. Such capsules similarly include the active ingredients mixed with carrier materials as described above. Soft gelatin capsules include the active ingredients mixed with water-miscible solvents such as propylene glycol, PEG and ethanol, or an oil such as peanut oil, liquid paraffin or olive oil. Aqueous suspensions are also contemplated as containing the active material in admixture with excipients suitable for the manufacture of aqueous suspensions. Such excipients include suspending agents, for example sodium carboxymethylcellulose, methylcellulose, hydroxypropylmethylcellulose, sodium alginate, polyvinylpyrrolidone, tragacanth and acacia; dispersing or wetting agents, e.g., lecithin; preservatives, e.g., ethyl, or n-propyl parahydroxybenzoate, colorants, flavors, sweeteners and the like.

Dispersible powders and granules suitable for preparation of an aqueous suspension by the addition of water provide the active ingredients in admixture with a dispersing or wetting agent, suspending agent and one or more preservatives. Suitable dispersing or wetting agents and suspending agents are exemplified by those already mentioned above. Aqueous solutions, suspensions, syrups and elixirs may also be formulated.

The techniques and approaches set forth in the present disclosure can further be used by the person of ordinary skill in the art to prepare variants thereof, said variants are considered to be part of the inventive disclosure.

### EXAMPLES

Molecular complexes of zoledronic acid and sodium, ammonium, ammonia, L-lysine, DL-lysine, nicotinamide, adenine, and glycine have been made and are characterized by their PXRD patterns and FTIR spectra disclosed herein. Further, *in vivo* data in rats concerning the oral bioavailability of zoledronic acid delivered orally, intravenously, and intraduodenally have been generated as well as PK profiles of the parent compound.

Zoledronic acid as a starting material used in all experiments in this disclosure was supplied by Farmkemi Limited (Wuhan Pharma Chemical Co.), China with purity of ca. 98% and was purified further via recrystallization from water. All other pure chemicals (Analytical Grade) were supplied by Sigma-Aldrich and used without further purification.

Enteric coating of gelatin capsules was contracted out to AzoPharma, Hollywood, FL, USA. A 10% w/w coating solution of Eudragit L 100-55, and triethyl citrate, 9.09 and 0.91 w/w% respectively, in purified water and acetone was used in the Vector LDCS pan coater to achieve a uniform coating layer on the capsules. The coating uniformity and functionality for duodenal delivery was tested by 2 hr dissolution in simulated gastric fluid stirred at 75rpm and 37°C. All capsules remained closed for the duration of this test.

Micronization was carried out at the Jet Pulverizer Company (NJ, USA) using a three inch diameter mill.

### Solid phase characterization

Analytical techniques used to observe the crystalline forms include powder X-ray diffraction (PXRD) and Fourier transform infrared spectroscopy (FTIR). The particular methodology used in such analytical techniques should be viewed as illustrative, and not limiting in the context of data collection. For example, the particular instrumentation used to collect data may vary; routine operator error or calibration standards may vary; sample preparation method may vary (for example, the use of the KBr disk or Nujol mull technique for FTIR analysis).

Fourier Transform FTIR Spectroscopy (FTIR): FTIR analysis was performed on a Perkin Elmer Spectrum 100 FTIR spectrometer equipped with a solid-state ATR accessory. Powder X-Ray Diffraction (PXRD): All zoledronic acid molecular complex products were observed by a D-8 Bruker X-ray Powder Diffractometer using Cu Ka (λ = 1.540562 Å), 40kV, 40mA. The data were collected over an angular range of 3° to 40° 2θ in continuous scan mode at room temperature using a step size of 0.05° 2θ and a scan speed of 6.17 °/min.

Laser scattering particle size analysis: All micronized samples were tested using the Horiba LA950 laser scattering particle size analyzer, dry method using air at pressure of 0.3MPA to fluidize the micronized samples before flowing in the path of a laser beam. The micronized samples were further tested using light microscopy to verify the Horiba results.

### Example 1: Preparation of zoledronic acid, sodium zoledronic salt, and water complex.

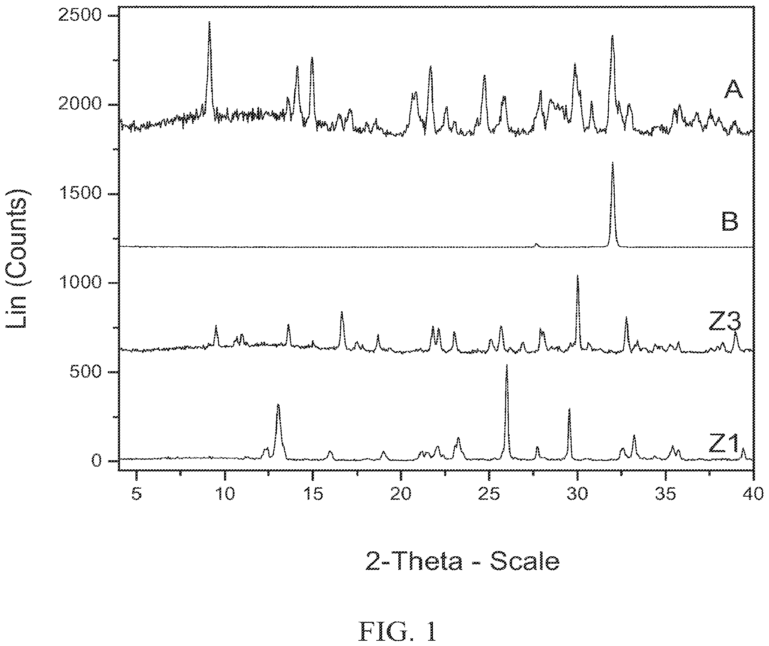

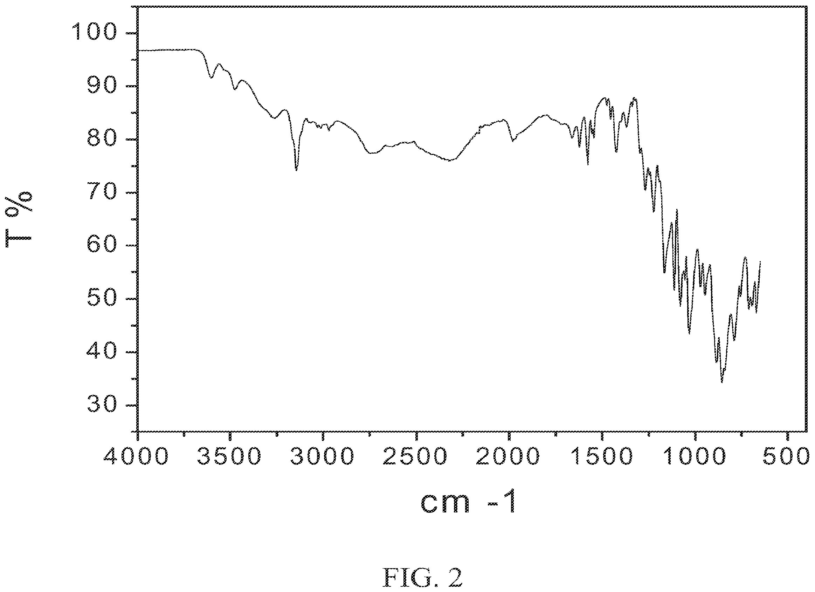

200 mg of zoledronic acid was slurried with 180 mg of sodium chloride in 1mL of 1:1 ethanol:water overnight. The material was filtered and rinsed. The particulate material was gathered and stored in a screw cap vial for subsequent analysis. The material was characterized by PXRD and FTIR corresponding to FIG. 1 and FIG. 2, respectively.

### Example 2: Preparation of ammonium zoledronic salt and water complex.

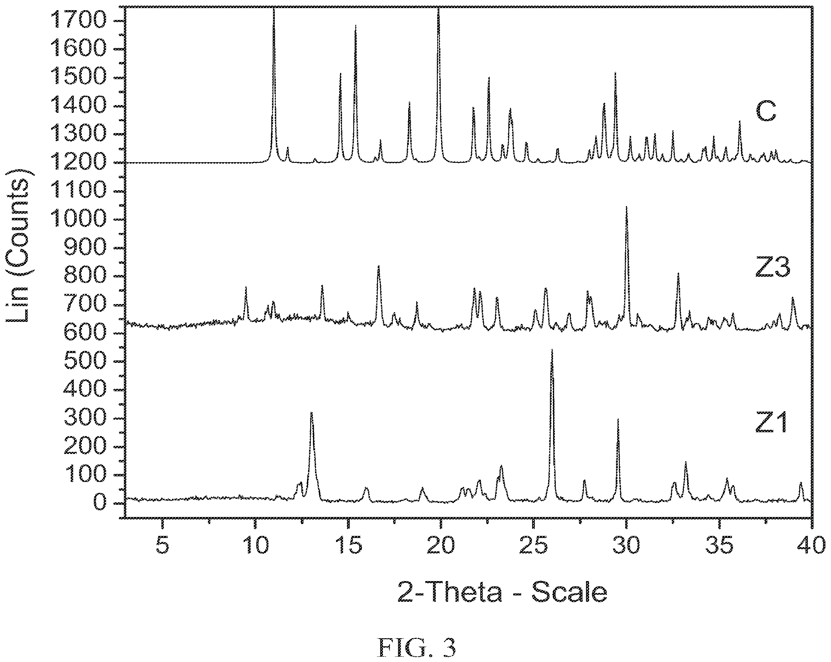

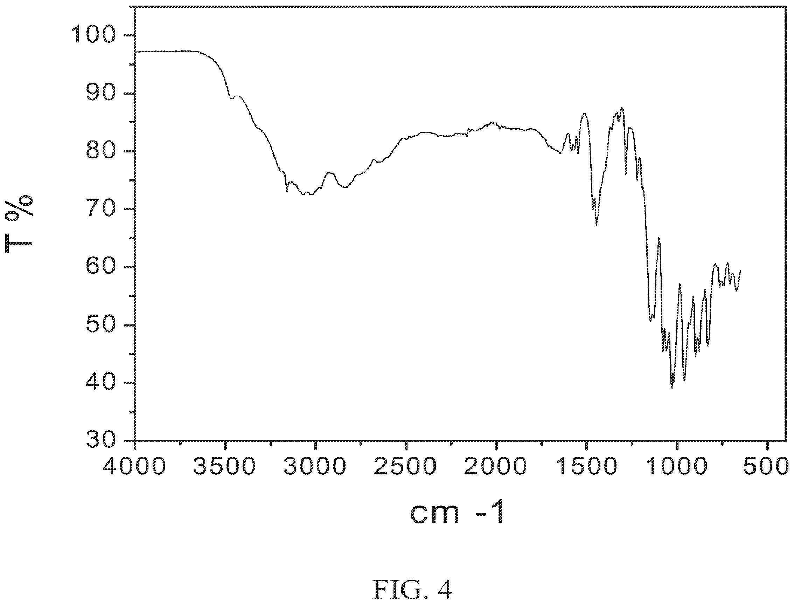

300 mg of zoledronic acid was slurried in 7N ammonia in methanol overnight. The material was filtered and rinsed. The particulate material was dissolved in water and left to evaporate at ambient conditions to obtain colorless plates after 1 week. The material was characterized by PXRD and FTIR corresponding to FIG. 3 and FIG. 4, respectively.

### Example 3: Preparation of zoledronic, L-lysine, and water complex.

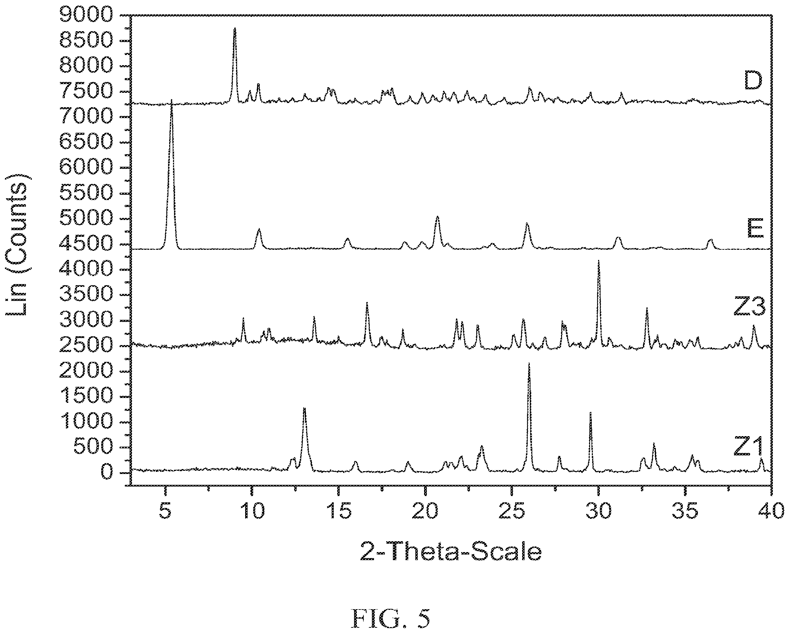

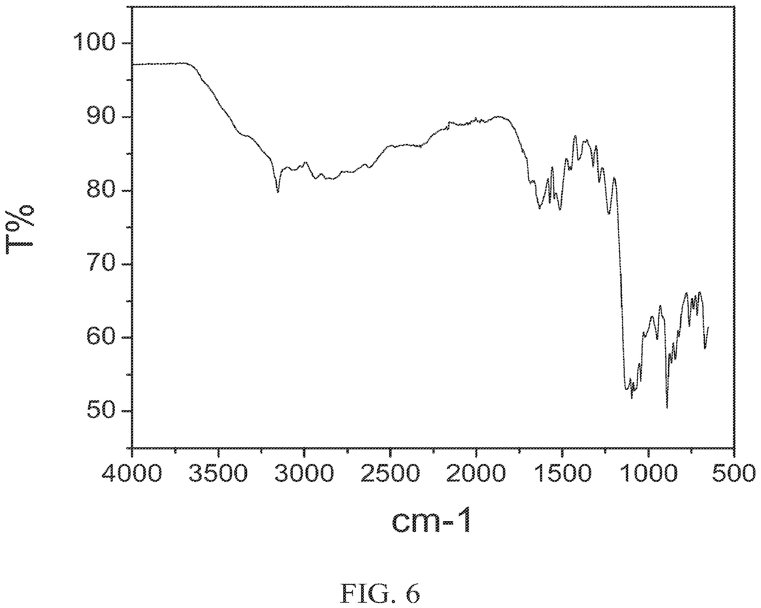

200 mg of zoledronic acid and 54 mg of L-lysine were slurried in 2 mL of tetrahydrofuran and 200 µl of water overnight. The solids gathered after filtration were dried and stored in a screw cap vials for subsequent analysis. The material was characterized by PXRD and FTIR corresponding to FIG. 5 and FIG. 6, respectively.

### Example 4: Preparation of zoledronic, DL-lysine, and water complex.

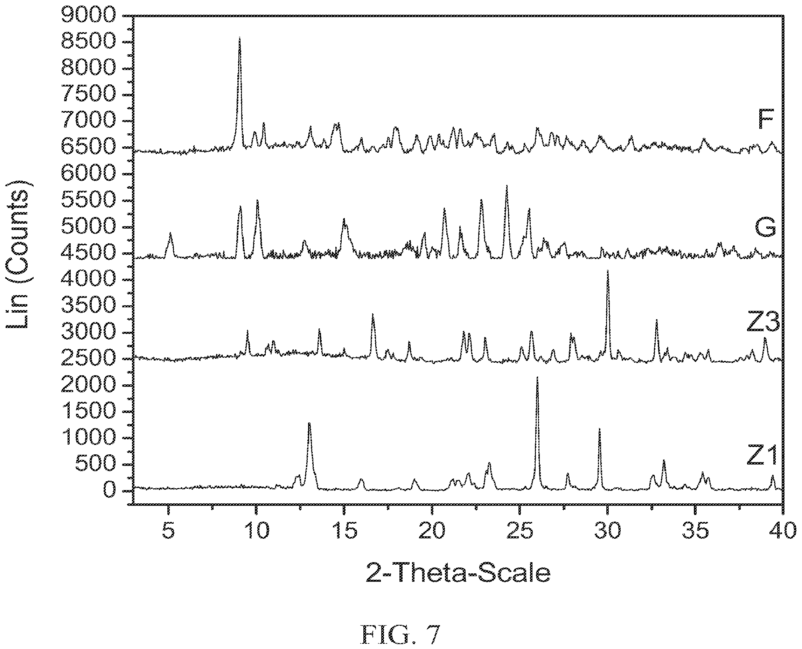

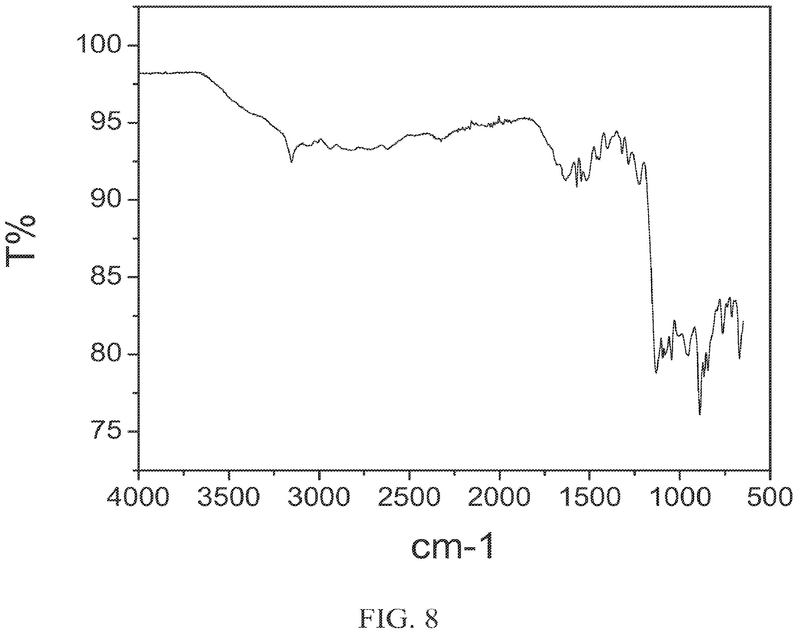

204 mg of zoledronic acid and 59 mg of DL-lysine were slurried in 2 mL of tetrahydrofuran and 200 µl of water overnight. The solids gathered after filtration were dried and stored in a screw cap vials for subsequent analysis. The material was characterized by PXRD and FTIR corresponding to FIG. 7 and FIG. 8 respectively.

### Example 5: Preparation of zoledronic acid, zoledronic, DL-lysine, ethanol, and water complex.

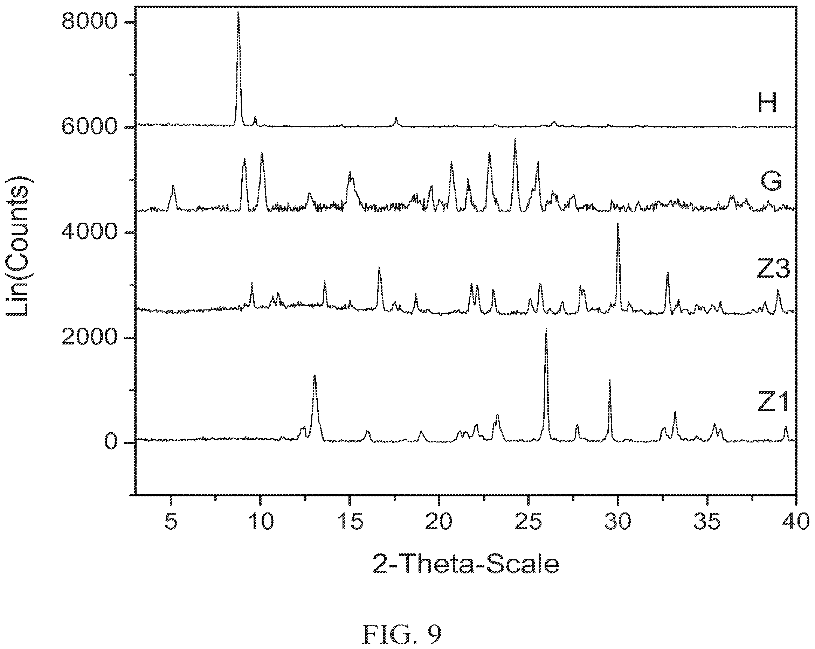

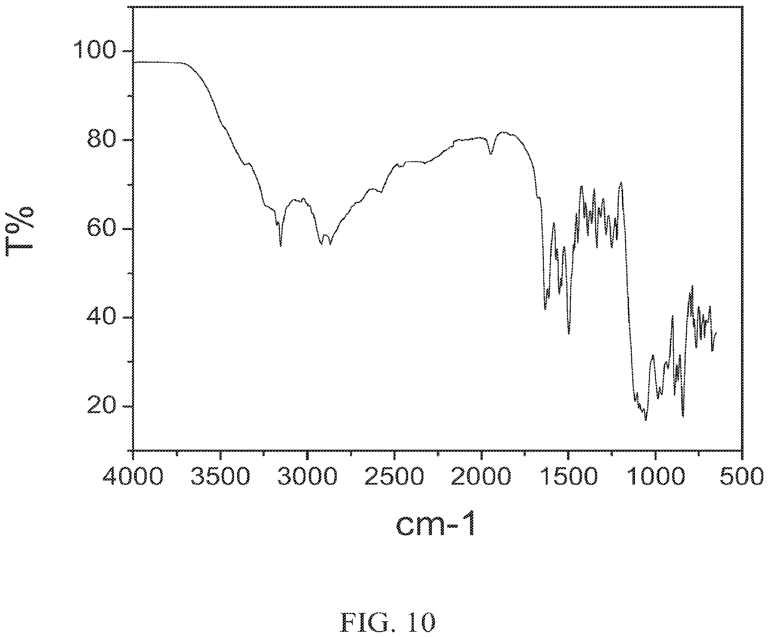

103 mg of zoledronic acid and 54 mg of DL-lysine were dissolved in 400 µl of water, capped and stirred overnight. The next day 0.25mL of ethanol was added drop wise. The vial was capped with a screw cap vial and after 1 day crystals appeared and were filtered off. The material was stored for subsequent analysis. The material was characterized by PXRD and FTIR corresponding to FIG. 9 and FIG. 10 respectively.

### Example 6: Preparation of zoledronic, nicotinamide, and water complex by solvent-drop grinding.

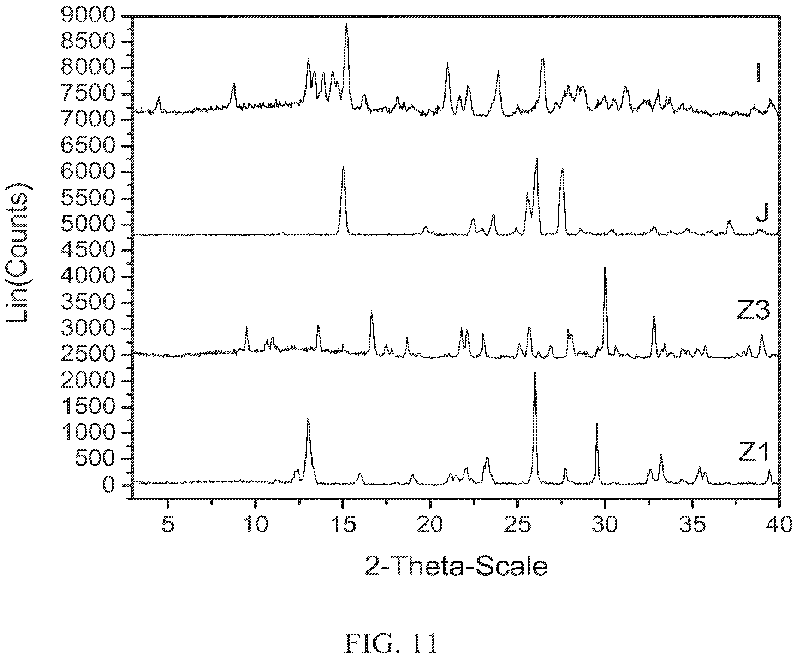

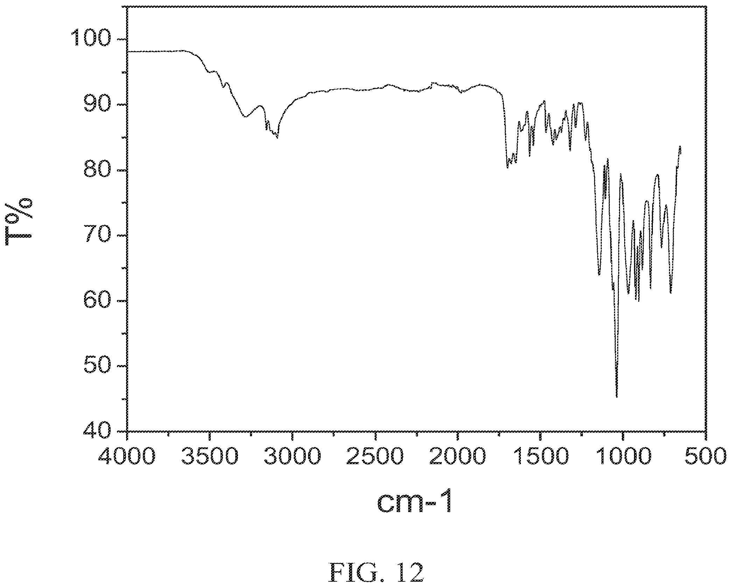

99 mg of zoledronic acid was ground with 44 mg of nicotinamide and 40 µl of water was added to the solid mixture. The solids gathered after grinding were stored in screw cap vials for subsequent analysis. The material was characterized by PXRD and FTIR corresponding to FIG. 11 and FIG. 12, respectively.

### Example 7: Preparation of zoledronic, nicotinamide, and water complex from solution crystallization.

25 mg of zoledronic acid and 138 mg of nicotinamide were dissolved in 2mL of a water:ethylacetate mix (1:1 v/v). The solution was then allowed to stand for several hours to effect the slow evaporation of solvent. The solids gathered were characterized and produced very similar PXRD and FTIR patterns to that of Example 6 product:

### Example 8: Preparation of zoledronic, adenine, and water complex by solvent-drop grinding.

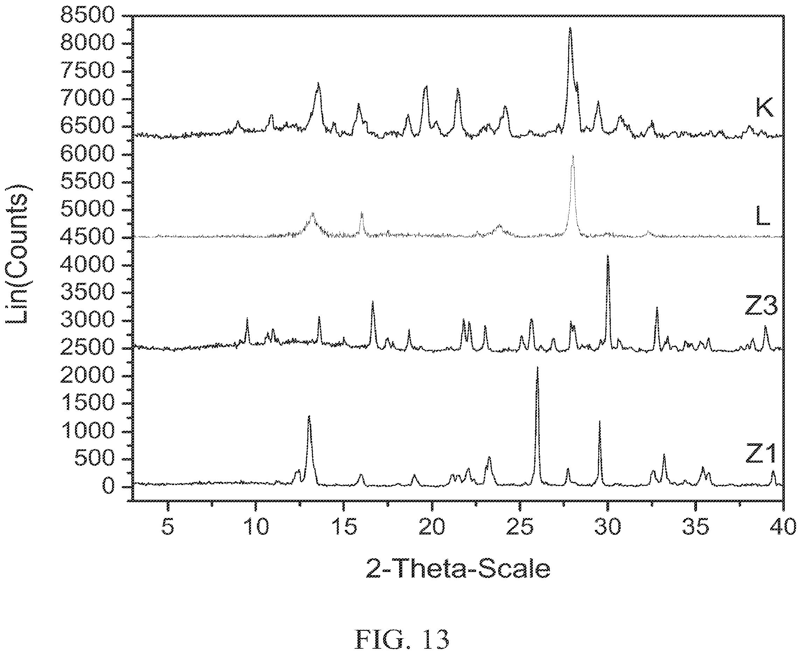

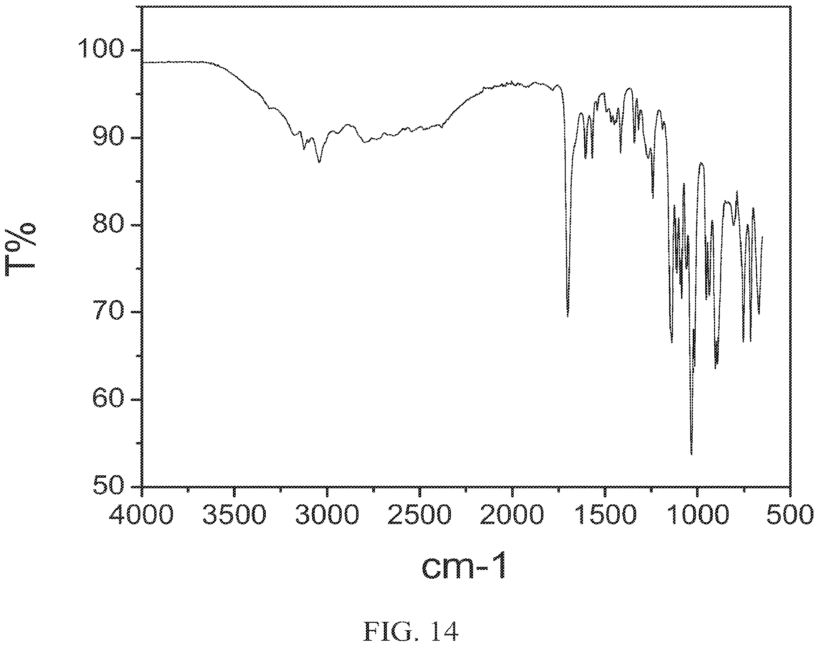

96 mg of zoledronic acid was ground with 65 mg of adenine and 60 µL of water was added to the solid mixture. The solids gathered after grinding were stored in screw cap vials for subsequent analysis. The material was characterized by PXRD and FTIR corresponding to FIG. 13 and FIG. 14, respectively.

### Example 9: Preparation of zoledronic, adenine, and water complex from solution slurry.

99 mg of zoledronic acid and 54 mg of adenine were slurried in 2 mL of a water:ethanol mix (1:1 v/v) overnight. The solids gathered after filtration were dried, characterized and produced very similar PXRD and FTIR patterns to that of Example 8 product.

### Example 10: Preparation of zoledronic and glycine complex.

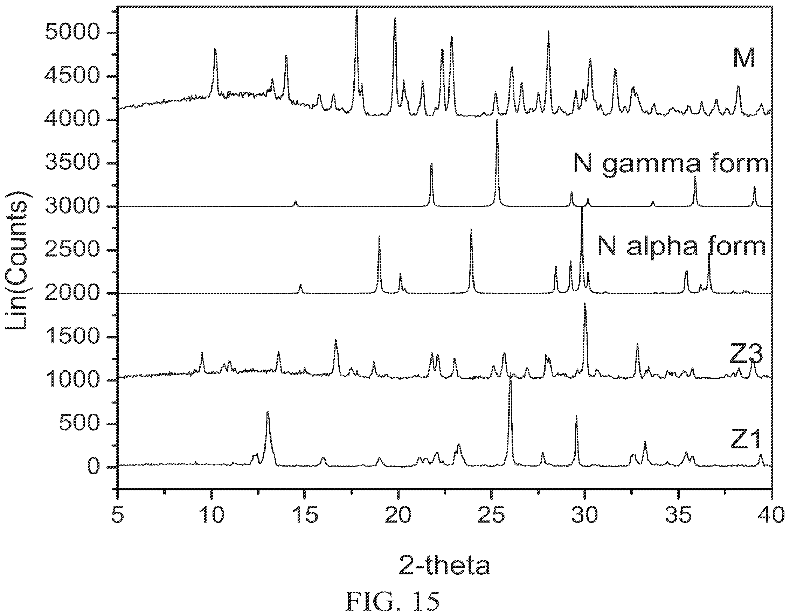

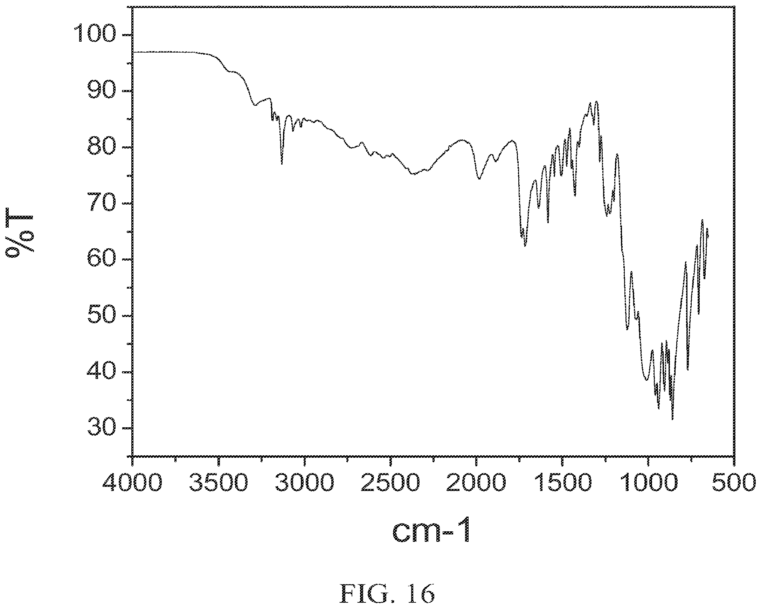

178 mg of zoledronic acid and 45 mg of glycine were slurried in 2 mL of water overnight. The solids gathered after filtration were dried and stored in a screw cap vials for subsequent analysis. The material was characterized by PXRD and FTIR corresponding to FIG. 15 and FIG. 16, respectively.

### Example 11: Preparation of zoledronic diammonia water complex.

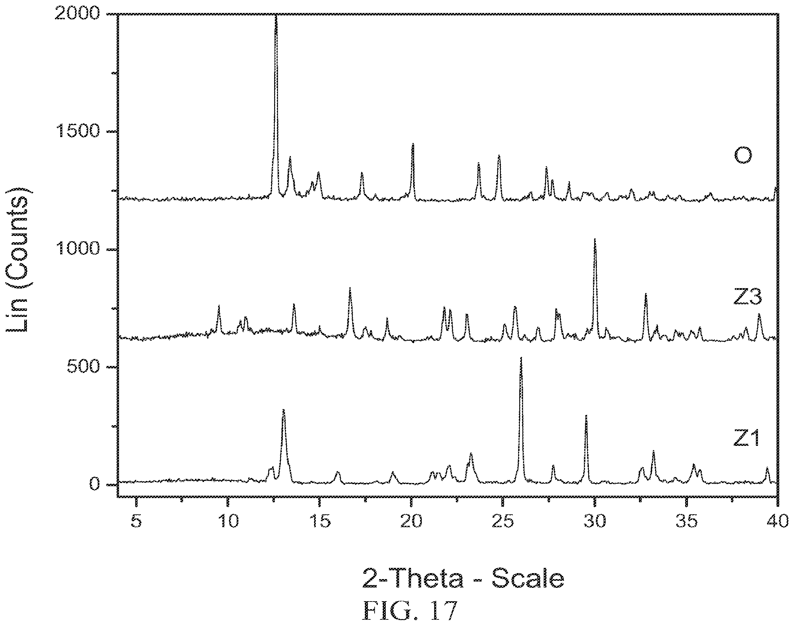

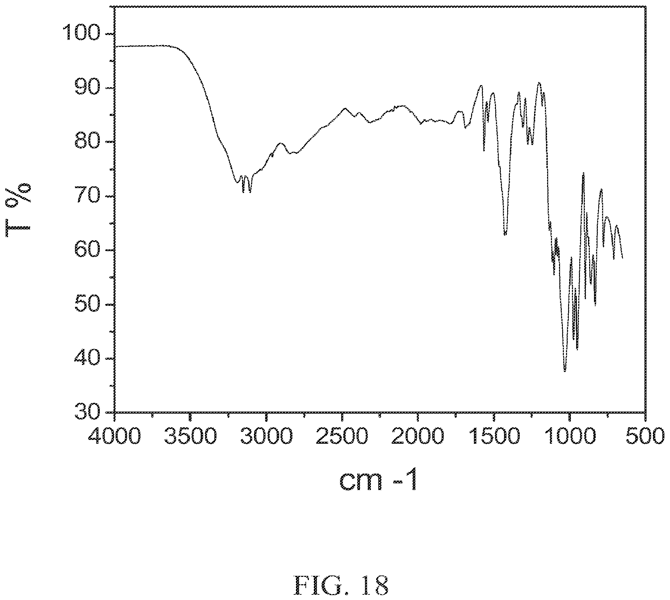

1.5 g of zoledronic acid was slurried in 7N ammonia in methanol overnight. The material was filtered and rinsed. The particulate material was dissolved in water with medium heat and left to evaporate at ambient conditions to obtain colorless blocks after 1 day. The material was characterized by PXRD and FTIR corresponding to FIG. 17 and FIG. 18, respectively.

### Example 12: Preparation of zoledronic, DL-lysine, and water complex.

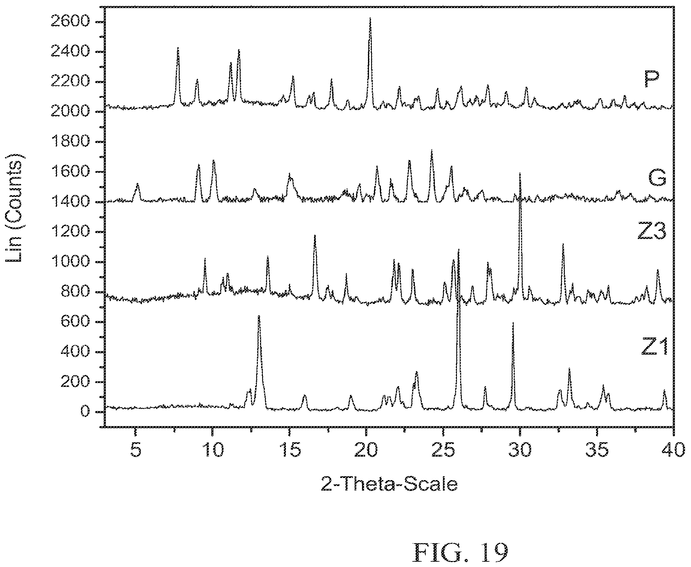

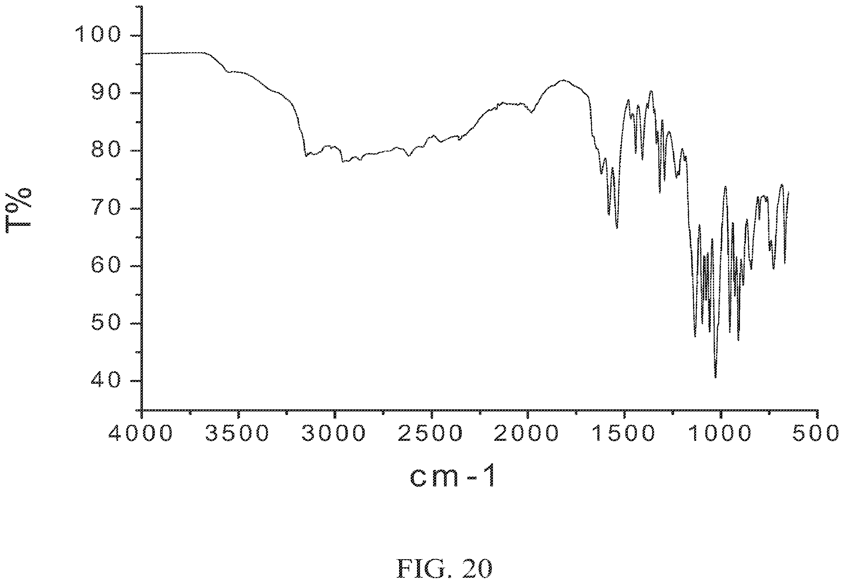

200 mg of zoledronic acid and 102 mg of DL-lysine were slurried in 2 mL of tetrahydrofuran and 400 µl of water overnight. The solids gathered after filtration were dried and stored in a screw cap vials for subsequent analysis. The material was characterized by PXRD and FTIR corresponding to FIG. 19 and FIG. 20 respectively.

### Example 13: Preparation of zoledronic, DL-lysine, and water complex.

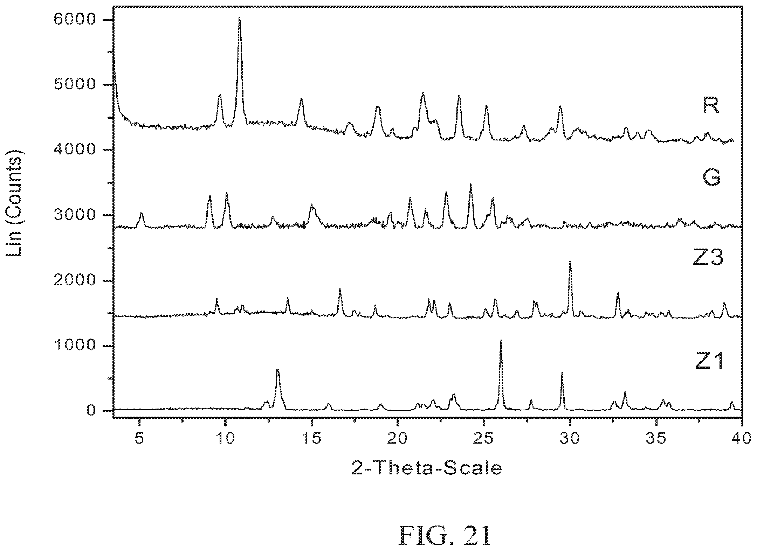

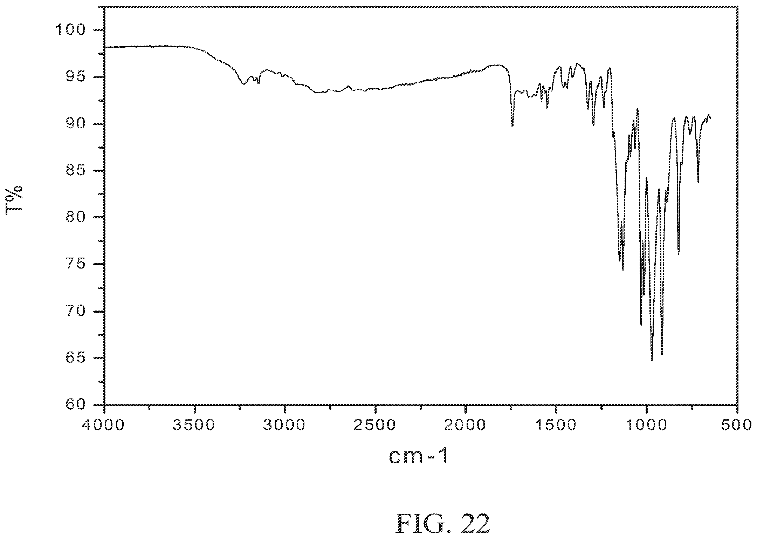

1 g of zoledronic acid and 283 mg of DL-lysine were slurried in 80 mL of tetrahydrofuran and 8 mL of water overnight. The solids gathered after filtration were dried and stored in a screw cap vials for subsequent analysis. The material was characterized by PXRD and FTIR corresponding to FIG. 21 and FIG. 22 respectively.

### Example 14: Preparation of zoledronic, DL-lysine, and water complex by antisolvent method.

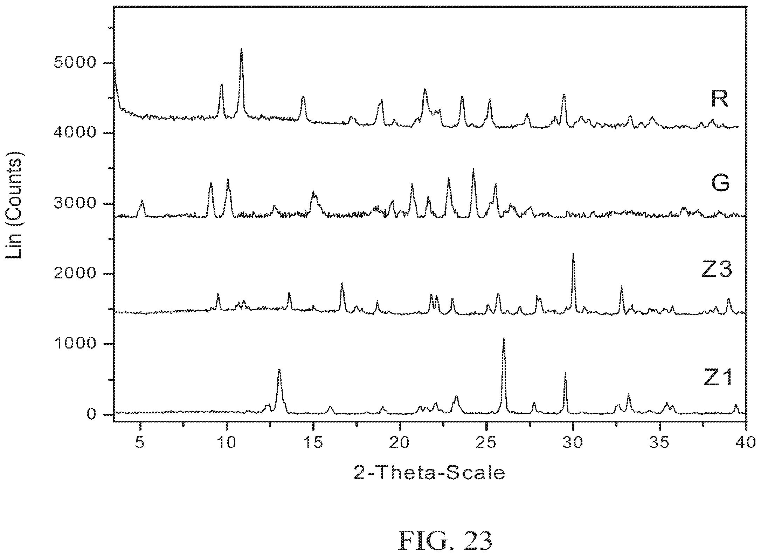

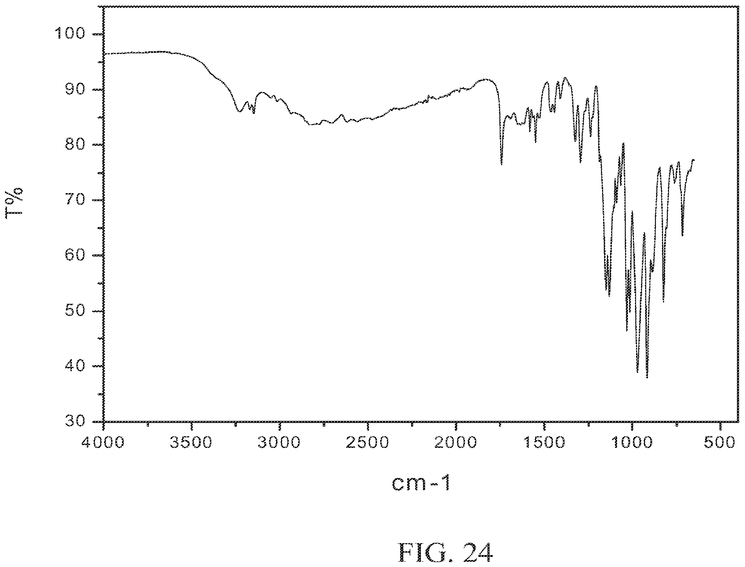

This complex can also be prepared by the antisolvent method by dissolving 1g of zoledronic acid and 283 mg of DL-lysine in 5 mL of hot water and adding 40 mL of ethanol as an antisolvent stirred overnight. Similar PXRD and FTIR profiles were obtained as shown in FIG. 23 and FIG. 24 respectively.

### Example 15: Preparation of zoledronic, L-lysine, and water complex.

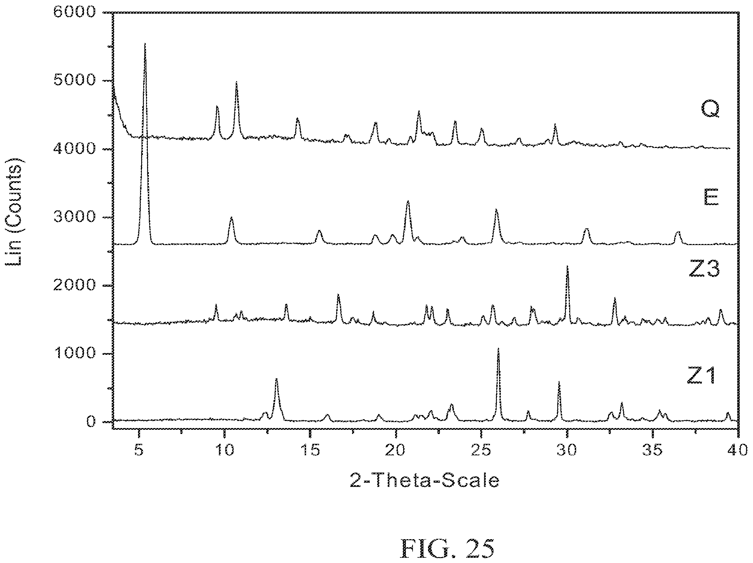

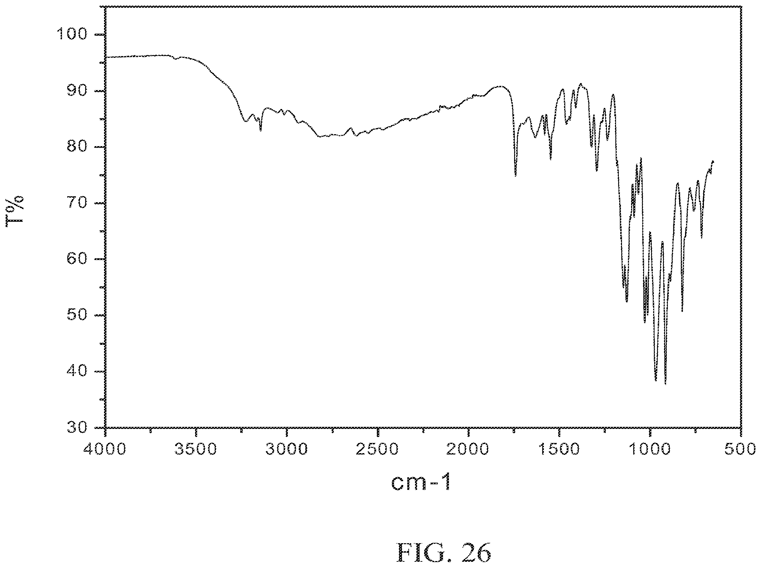

1 g of zoledronic acid and 255 mg of L-lysine were dissolved in 60 mL of hot water. 100 mL of ethanol was then added as an antisolvent. The solids gathered after filtration were dried and stored in a screw cap vials for subsequent analysis. The material was characterized by PXRD and FTIR corresponding to FIG. 25 and FIG. 26 respectively.

### Example 16: The Animal PK Studies

These studies were conducted on rats and dogs as they are suitable animal models for zoledronic acid. This can be attributed to the fact that both animals have historically been used in the safety evaluation and PK screening studies and are recommended by appropriate regulatory agencies. In addition, rats and dogs have also been established as appropriate species for assessing the absorption of bisphosphonate drugs including zoledronic acid. Pure zoledronic acid and zoledronic acid complexes prepared by the methods in this invention were delivered to the rats and dogs through IV or oral routes. Additional tests included ID administration in rats and administration of enteric coated capsules in dogs. All compounds delivered were well tolerated by the animals with no adverse events or physical abnormalities noticed.

Test Subjects: 8-week male Sprague-Dawley Rats (217-259 grams) were obtained from Hilltop Lab Animals, Scottdale, PA USA. Some animals have surgical catheters (jugular vein and intraduodenum) were implanted to the animals prior to the studies. Beagle dogs from Marshall Farms, NY, USA, weighing from (9-12 kg) were used in the studies presented herein. Surgical catheters (jugular vein) were implanted prior to the studies.

Housing: Rats were individually housed in stainless steel cages to prevent catheter exteriorization. Acclimation (Pre-dose Phase) was for 1 day. Dogs were already in the test facility (Absorption Systems Inc., USA) and did not need acclimation.

Environment: Environmental controls for the animal room were set to maintain 18 to 26 °C, a relative humidity of 30 to 70%, a minimum of 10 air changes/hour, and a 12-hour light/12-hour dark cycle. The light/dark cycle could be interrupted for study-related activities.

Diet: For rats, water and certified Rodent Diet #8728C (Harlan Teklad) were provided. For dogs, water and the standard dog chow diet were given twice daily (every 12 hours).

Fasting: All test animals were fasted overnight before IV, oral, or ID administration of zoledronic acid or zoledronic acid complexes.

Routes of Rat Dosing: Zoledronic acid and its complex formulations were administered through IV, oral and ID. The doses administered to all rats in these studies were measured as zoledronic acid, not as the complex form contained in the suspension:
i. IV Administration: the dose of zoledronic acid for IV administration was 0.5 mg/kg. The dose ofeach rat was calculated on a per rat basis (not on an average weight of all the rats in the lot).
ii. Oral gavage administration: solid suspensions were administered. The dose of each rat was calculated on a per rat basis (not on an average weight of all the rats in the lot). For solid suspensions, animals were administered 5 mg/kg of zoledronic acid or 5 mg/kg of zoledronic acid in zoledronic acid complexes contained in a suspension of PEG 400.
iii. Duodenal cannula administration: solid suspensions were administered. The dose of each rat was calculated on a per rat basis (not on an average weight of all the rats in the lot). For solid suspensions, animals were administered 5 mg/kg of zoledronic, acid or 5 mg/kg of zoledronic acid in zoledronic acid complexes contained in a suspension of PEG 400.

Routes of Dog Dosing: Zoledronic acid and its complex formulations were administered IV and orally. The doses administered to all dogs in these studies were measured as zoledronic acid in each complex, not as the complex form contained in the powder in the gelatin capsule or in solution for IV:
i. IV Administration: The dose volume of each dog was adjusted based upon the average weight of the dog.
ii. Oral administration: zoledronic acid and its equivalent of zoledronic acid complex formulations were administered through size 0 or 00 gelatin capsules based on the average weight of the dogs.
iii. Oral administration with enteric coated capsules: zoledronic acid and its equivalent of zoledronic acid complex formulations were administered through size 0 enteric coated gelatin capsules based on the average weight of the dogs.
iv. Oral administration of the molecular complexes with additional coformers: physical mixtures of zoledronic acid complexes with additional coformers were administered through size 0 or 00 or 000 or 13 gelatin capsules based on the average weight of the dogs.

Groups: Two major groups of animals were selected for the study.
Group I consists of rat studies. The rat studies were divided into four subgroups (I-IV) where the results of each data point on the PK profile was the average drug concentration in the plasma of 3 rats.
Group 2 consists of dog studies. The dog studies were divided into five groups with subgroups (A, B, C, D,E, F, G, H, J, K, L, M) where the results of each data point on the PK profile was the average drug concentration in the serum of mainly 5 dogs. The PK profile for subgroup N was the average profile of 4 dogs.

### Details of Group 1 rat dosing

**Group I (IV administration). Group members, designated IV doses are listed below**

| **Group # I** | **Designation** | **# of rats** | **Dose*** | **Dose volume** |
|---|---|---|---|---|
| G1 | Zoledronic Acid | 3 | 0.5 mg/kg | 1 mL |

IV comparator group, was conducted to calculate MAT (mean absorption time) and ka (absorption rate constant) for the oral groups.

**Group II (oral gavage): Group designations and oral doses are listed below:**

| **Group # II** | **Designation** | **# of Rats** | **Dose*** | **Dose volume mL/kg** | **Compound** |
|---|---|---|---|---|---|
| G2 | Zoledronic Acid in PEG400 | 3 | 5 mg/kg | 1 mL | Zoledronic acid |
| G3 | Solid suspension in PEG400 | 3 | 5 mg/kg equivalent | 1 mL | Zoledronic and glycine complex |
| G4 | Solid suspension in PEG400 | 3 | 5 mg/kg equivalent | 1 mL | Zoledronic, nicotinamide, and water complex |
| G5 | Solid suspension in PEG400 | 3 | 5 mg/kg equivalent | 1 mL | Zoledronic acid, sodium zoledronic salt, and water complex |
| G6 | Solid suspension in PEG400 | 3 | 5 mg/kg equivalent | 1 mL | Zoledronic, L-lysine, and water complex |
| G7 | Solid suspension in PEG400 | 3 | 5 mg/kg equivalent | 1 mL | Zoledronic, DL-lysine, and water complex |

**Group III (ID administration): Group designations and oral doses are listed below:**

| **Group # III** | **Designation** | **# of rats** | **Dose*** | **Dose volume mL/kg** | **Compound** |
|---|---|---|---|---|---|
| G8 | Zoledronic Acid in PEG400 | 3 | 5 mg/kg | 1 mL | Zoledronic acid |
| G9 | Solid suspension in PEG400 | 3 | 5 mg/kg equivalent | 1 mL | Zoledronic and glycine complex |
| G10 | Solid suspension in PEG400 | 3 | 5 mg/kg equivalent | 1 mL | Zoledronic, nicotinamide, and water complex |
| G11 | Solid suspension in PEG400 | 3 | 5 mg/kg equivalent | 1 mL | Zoledronic acid, sodium zoledronic salt, and water complex |
| G12 | Solid suspension in PEG400 | 3 | 5 mg/kg equivalent | 1 mL | Zoledronic, L-lysine, and water complex |
| G13 | Solid suspension in PEG400 | 3 | 5 mg/kg equivalent | 1 mL | Zoledronic, DL-lysine, and water complex |

**Group IV (oral gavage): Group designations and oral doses are listed below:**

| **Group # IV** | **Compound** | **# of rats** | **Dose** | **Dose volume/kg** | **Excess coformer** | **Excess coformer amount mg/kg** |
|---|---|---|---|---|---|---|
| G14 | Zoledronic and glycine complex, solid suspension in PEG400 | 3 | 5 mg/kg equivalent | 1 mL | Glycine | 45 |
| G15 | Zoledronic and glycine complex, solid suspension in PEG400 | 3 | 5 mg/kg equivalent | 1 mL | Glycine | 25 |
| G16 | Zoledronic and glycine complex, solid suspension in PEG400 | 3 | 5 mg/kg equivalent | 1 mL | Glycine | 5 |
| G17 | Zoledronic, DL-lysine, and water complex, solid suspension in PEG400 | 3 | 5 mg/kg equivalent | 1 mL | DL-lysine monohydrat e | 39.32 |
| G18 | Zoledronic, DL-lysine, and water complex, solid suspension in PEG400 | 3 | 5 mg/kg equivalent | 1 mL | DL-lysine monohydrat e | 28.08 |
| G19 | Zoledronic, DL-lysine, and water complex, solid suspension in PEG400 | 3 | 5 mg/kg equivalent | 1 mL | DL-lysine monohydrat e | 5.62 |
| G20 | Zoledronic, DL-lysine, and water complex, solid suspension in PEG400 | 3 | 5 mg/kg equivalent | 1 mL | n/a | n/a |

Rat blood sample collection, handling and analysis: Blood (approx. 300 µL per sample) samples were withdrawn from each of 3 animals in Group I (IV administration) at eight (8) time points: 5 min, 15 min, 30 min, 1 hr, 2 hr, 4 hr, 8 hr, and 24 hrs, after initial administration of zoledronic acid or its complexes, into EDTA plasma tubes. Plasma was collected after centrifugation at 13,000 rpm for 5 min at 4°C and immediately frozen and stored at -60 to -80 °C until analysis.

Samples were thawed on the day of analysis and the amount of zoledronic acid in the samples was quantified by analyzed by LC/MS/MS method.

Details of Group 2 dog dosing: Prior to dosing, all dogs received a 20 mL dose of citric acid (24 mg/mL in water) to lower the pH of their stomach. After dosing capsules or IV, all dogs received additional 6.25 mL citric acid solution (24 mg/mL in water) as a rinse.

**Group A, (IV administration). Group members, designated IV doses are listed below:**

| **Group # A** | **Designation** | **# of fasted Dogs** | **Dose*** | **Dose volume** |
|---|---|---|---|---|
| Leg I | Zoledronic Acid | 5 | 0.05 mg/kg | 1 mL/kg |

IV comparator group, was conducted to calculate MAT (mean absorption time) and ka (absorption rate constant) for the oral groups.

**Group B (oral administration): Group) designations and oral doses are listed below:**

| **Group # B** | **Compound** | **Dosing Route** | **Dose of compound in the gelatin capsules** | **# of fasted Dogs (9-12 kg)** | **Dosing Solution Conc. mg/mL** |
|---|---|---|---|---|---|
| Leg 2 | Zoledronic acid | oral | 5 mg/kg equivalent | 5 | n/a |
| Leg 3 | Zoledronic and glycine complex | oral | 5 mg/kg equivalent | 5 | n/a |
| Leg 4 | Zoledronic, DL-lysine, and water complex | oral | 5 mg/kg equivalent | 5 | n/a |
| Leg 5 | Zoledronic, L-lysine, and water complex | oral | 5 mg/kg equivalent | 5 | n/a |
| Leg 6 | Zoledronic, DL-lysine, and water complex | oral | 5 mg/kg equivalent | 5 | n/a |

**Group C (oral administration): Group designations and oral doses are listed below:**

| **Group #C** | **Compound** | **# of fasted Dogs (9-12 kg)** | **Dosing Route** | **Dose of compound in the gelatin capsules** | **Excess coformer** | **Excess coformer amount** |
|---|---|---|---|---|---|---|
| Leg 7 | Zoledronic acid monohydrate | 5 | oral | 56.0 mg; enterically coated capsules | n/a | n/a |
| Leg 8 | Zoledronic and glycine complex | 5 | oral | 67.0 mg; enterically coated capsules | n/a | n/a |
| Leg 9 | Zoledronic, DL-lysine, and water complex | 5 | oral | 87.7 mg | DL-lysine monohydr ate | 294.8 mg |
| Leg 10 | Zoledronic, DL-lysine, and water complex | 5 | oral | 87.7 mg; enterically coated capsules | DL-lysine monohydr ate | 294.8 mg |
| Leg 11 | Zoledronic, DL-lysine, and water complex | 5 | oral | 84.2 mg | DL-lysine monohydr ate | 294.8 mg |
| Leg 12 | Zoledronic, DL-lysine, and water complex | 5 | oral | 87.7 mg; enterically coated capsules | n/a | n/a |

**Group D, (15 min IV infusion): Group members, designated IV doses are listed below:**

| **Group # D** | **Designation** | **# of fasted Dogs (9-12 kg)** | **Dose*** | **Dosing solution concentration** |
|---|---|---|---|---|
| Leg 13 | Zoledronic Acid | 5 | 0.183 mg/kg IV | 0.1 mg/mL |

**Group E, (oral administration): Group members, designated IV doses are listed below:**

| **Group # E** | **Compound** | **# of fasted Dogs (9-12 kg)** | **Dosing Route** | **Dose of compound in the gelatin capsules** | **Excess coformer** | **Excess coformer amount** |
|---|---|---|---|---|---|---|
| Leg 14 | Zoledronic, DL-lysine, and water complex | 5 | oral | 35.4 mg | DL-lysine monohydrate | 123.8 mg |
| Leg 15 | Zoledronic and glycine complex | 5 | oral | 67.0 mg | DL-lysine monohydrate | 294.8 mg |
| Leg 16 | Zoledronic, L-lysine, and water complex | 5 | oral | 87.7 mg | DL-lysine monohydrate | 294.8 mg |
| Leg 17 | Zoledronic, DL-lysine, and water complex | 5 | oral | 35.4 mg | DL-lysine monohydrate | 294.8 mg |

**Group F, (15 min IV infusion): Group members, designated IV doses are listed below:**

| **Group # F** | **Designation** | **# of fasted Dogs (9-12 kg)** | **Dose*** | **Dosing solution concentration** |
|---|---|---|---|---|
| Leg 18 | Zoledronic Acid | 5 | 0.12 mg/kg IV infusion | 0.1 mg/mL |

**Group G (oral administration): Group designations and oral doses are listed below:**

| **Group #G** | **Compound** | **# of fasted Dogs (10-13 kg)** | **Dosing Route** | **Dose of compound in the gelatin capsules** | **Excess coformer** | **Excess coformer amount** |
|---|---|---|---|---|---|---|
| Leg 19 | Zoledronic acid | 5 | PO | 61.3 mg | DL-lysine monohydrate | 322.9 mg |
| Leg 20 | Zoledronic, L-lysine, and water complex | 5 | PO | 76.8 mg | L-lysine HCl | 359.2 mg |

**Group H (oral administration): Group designations and oral doses are listed below:**

| **Group # H** | **Compound** | **# of fasted Dogs (9-12 kg)** | **Dosing Route** | **Dose of compound in the gelatin capsules** | **Excess coformer** | **Excess coformer amount** |
|---|---|---|---|---|---|---|
| Leg 21 | Zoledronic, DL-lysine, and water complex | 5 | PO | 84.2 mg | L-lysine HCl | 328.0 mg |
| Leg 22 | Zoledronic, DL-lysine, and water complex | 5 | PO | 69.0 mg | DL-lysine monohydrate | 241.8 mg |
| Leg 23 | Zoledronic, L-lysine, and water complex | 5 | PO | 70.1 mg | DL-lysine monohydrate | 294.9 mg |

**Group J (oral administration): Group designations and oral doses are listed below:**

| **Group # J** | **Compound** | **# of fasted Dogs (10.5-13.5 kg)** | **Dosing Route** | **Dose of compound in the gelatin capsules** | **Excess coformer** | **Excess coformer amount** |
|---|---|---|---|---|---|---|
| Leg 24 | Zoledronic acid | 5 | PO | 64.0 mg | L-lysine HCl | 374.8 mg |
| Leg 25 | Zoledronic, L-lysine, and water complex | 5 | PO | 80.1 mg | N/A | N/A |
| Leg 26 | Zoledronic and glycine complex | 5 | PO | 76.5 mg | L-lysine HCl | 374.8 mg |

**Group K (oral administration): Group designations and oral doses are listed below:**

| **Group # K** | **Compound** | **# of fasted Dogs (8-11 kg)** | **Dosing Route** | **Dose of compound in the gelatin capsules** | **Excess coformer** | **Excess coformer amount** |
|---|---|---|---|---|---|---|
| Leg 27 | Zoledronic, DL-lysine, and water complex | 5 | PO | 32.0 mg | DL-lysine monohydr ate | 266.8 mg |
| Leg 28 | Zoledronic, DL-lysine, and water complex | 5 | PO | 76.2 mg | DL-lysine monohydr ate | 266.8 mg |

**Group L (oral administration): Group designations and oral doses are listed below:**

| **Group # L** | **Compound** | **# of fasted Dogs (8.3-11.3 kg)** | **Dosing Route** | **Dose of compound in the gelatin capsules** | **Excess coformer** | **Excess coformer amount** |
|---|---|---|---|---|---|---|
| Leg 29 | Zoledronic, DL-lysine, and water complex | 5 | PO | 64.4 mg | DL-lysine monohydrate | 275.2 mg |
| Leg 30 | Micronized Zoledronic, DL-lysine, and water complex | 5 | PO | 64.4 mg | Micronized DL-lysine monohydrate | 275.2 mg |

**Group M (oral administration): Group designations and oral doses are listed below:**

| **Group # M** | **Compound** | **# of fasted Dogs (8.4-11.4 kg)** | **Dosing Route** | **Dose of compound in the gelatin capsules** | **Excess coformer** | **Excess coformer amount** |
|---|---|---|---|---|---|---|
| Leg 31 | Zoledronic, DL-lysine, and water complex | 4 | PO | 50.8 mg | DL-lysine monohydrate | 278.0 mg |
| Leg 32 | Micronized Zoledronic, DL-lysine, and water complex | 5 | PO | 50.8 mg | Micronized DL-lysine monohydrate | 278.0 mg |

**Group N (oral administration): Group designations and oral doses are listed below:**

| **Group # N** | **Compound** | **# of fasted Dogs** | **Dosing Route** | **Dose of compound** | **Excess coformer** | **Excess coformer amount** |
|---|---|---|---|---|---|---|
| Leg 33 | Zoledronic, DL-lysine, and water complex | 4 (7.5-10.5 kg) | PO | 59.2 mg in the gelatin capsules | DL-lysine monohydrate | 112.3 mg |
| Leg 34 | Zoledronic, DL-lysine, and water complex | 4 (8.1-11.1 kg) | PO | 63.1 mg in the gelatin capsules | DL-lysine monohydrate | 280.8 mg |
| Leg 35 | Zoledronic, DL-lysine, and water complex | 4(10.1-13.1 kg) | PO | 76.3 mg in the gelatin capsules | DL-lysine monohydrate | 561.6 mg |
| Leg 36 | Zoledronic, DL-lysine, and water complex | 4 (7.5-10.5 kg) | PO | 59.2 mg in the gelatin capsules | DL-lysine monohydrate | 1123.3 mg |
| Leg 37 | Zoledronic, DL-lysine, and water complex | 4 (8.1-11.1 kg) | PO | 63.1 mg in the gelatin capsules | DL-lysine monohydrate | 1965.7 mg |
| Leg 38 | Zoledronic acid | 4(10.1-13.1 kg) | IV | 0.12 mg/kg, 15 min IV infusion | N/A | N/A |

After initial administration of zoledronic acid or its complexes, blood (approx. 2.5 mL per sample) was withdrawn from each of 5 animals in Group A (IV administration) at 15 time points: Pre-dose (0), 2, 5, 10, 15, 30, 45min, 1, 1.5, 2, 4, 6, 8, 24 and 48 hrs and at 13 time points for Group B (oral administration): Pre-dose (0), 5, 10, 15, 30, 45min, 1, 1.5, 2, 4, 6, 8, and 24 hrs. Blood samples were placed without the use of an anticoagulant and allowed to sit at room temperature for approximately 30 minutes. Samples were then centrifuged at a temperature of 4°C, at a speed of 13,000 rpm, for 5 minutes. Serum was collected and split into two aliquots and stored frozen (- 80 °C) until analysis. Samples were thawed on the day of analysis and processed using analytical procedures for zoledronic acid containing an LC/MS/MS analysis method.

### Animal PK studies results

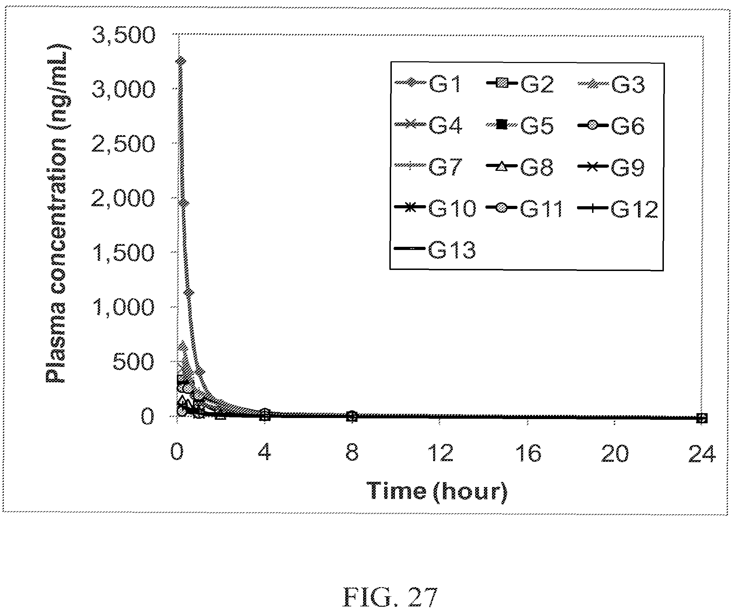

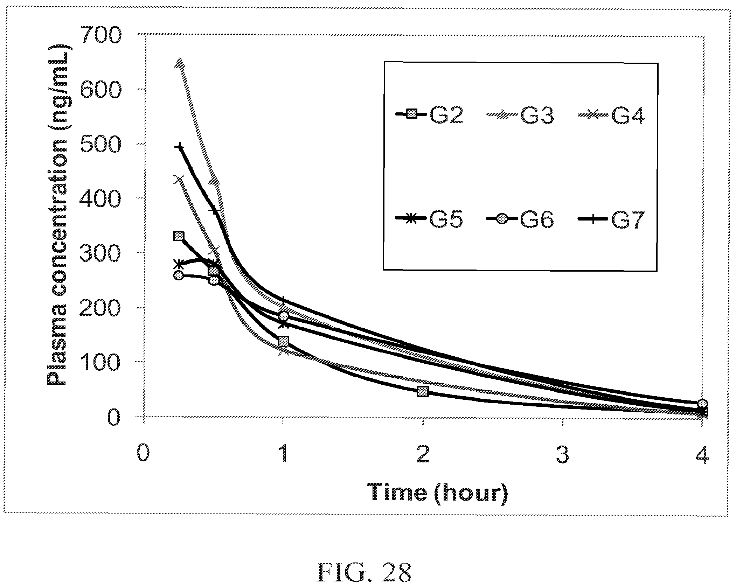

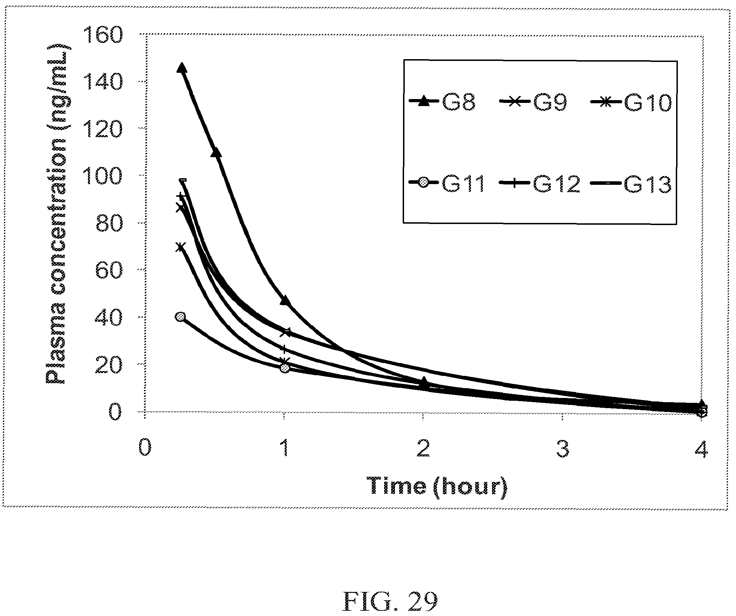

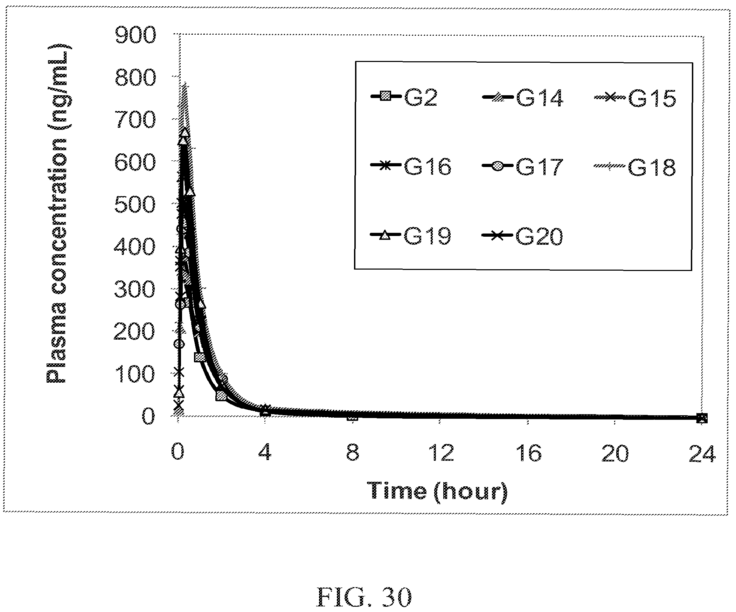

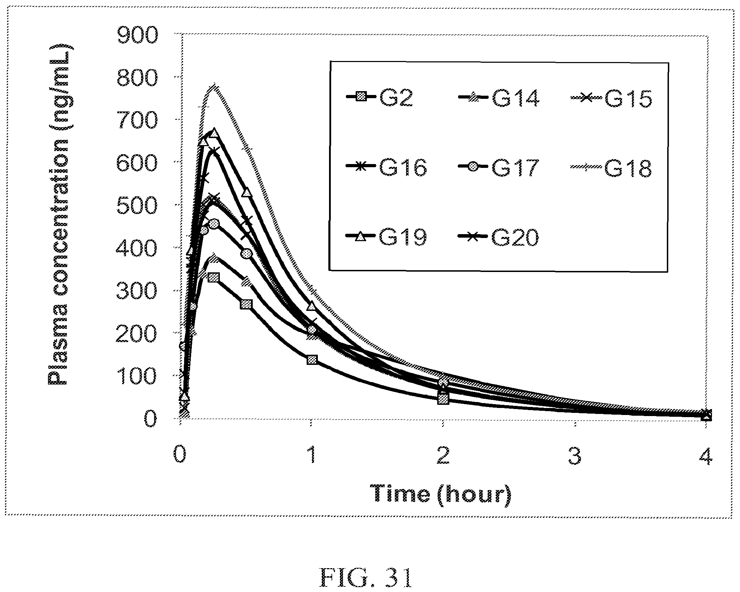

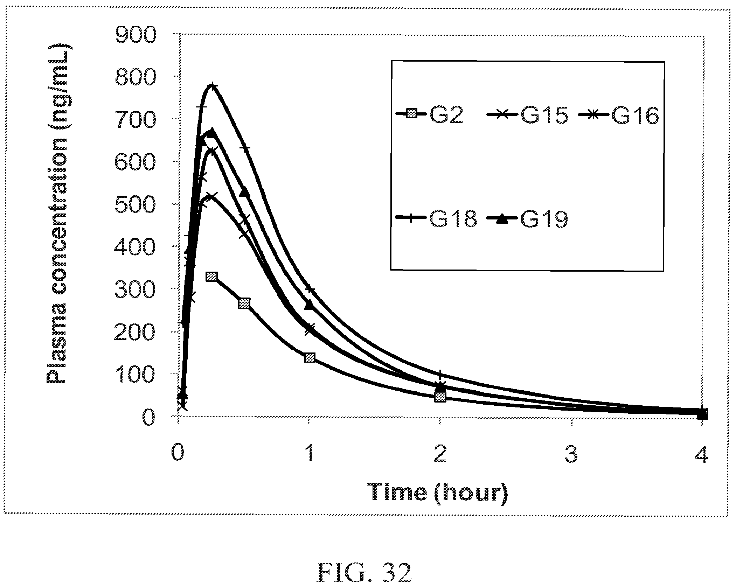

Rat study: The results of the first rat study are summarized in Table 1; the concentrations (ng/mL) of zoledronic acid in the plasma samples are the average values of the analytical results of 3 rats. In addition, the PK profiles of the IV, oral and ID groups are shown in Figure 27. The profiles of oral and ID groups are shown in Figures 28 and 29. It suggests that some zoledronic acid complexes have improved oral bioavailability compared with that of the parent zoledronic acid. The complexes with improved bioavailability were further tested in a second rat PK study in which excess coformers were added to the zoledronic acid complexes and then administered to rats by oral gavage. The results of this second study are summarized in Table 2 and their PK profiles are shown in Figures 30, 31 and 32. These figures show improved bioavailabilities of several zoledronic acid complexes with excess coformers. The effect of excess coformers with zoledronic acid complexes in improving bioavailability is not fully understood.

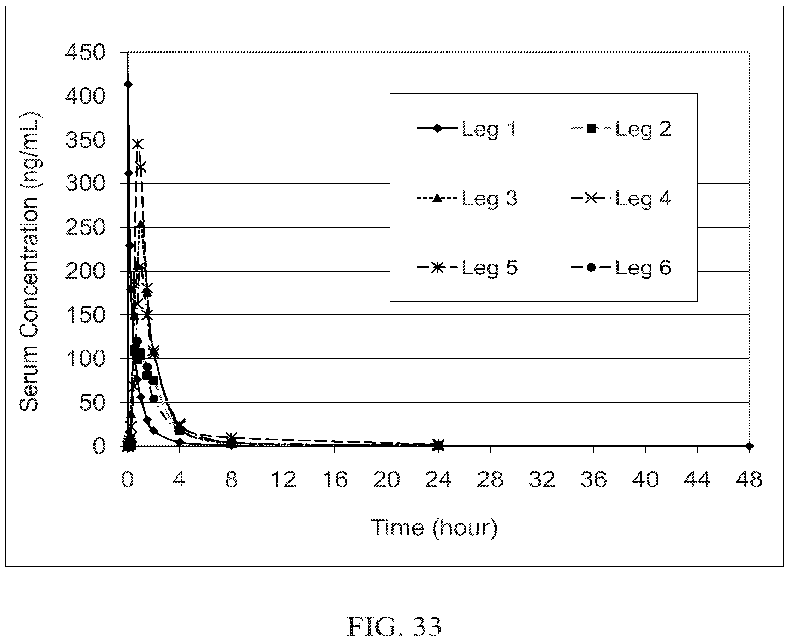

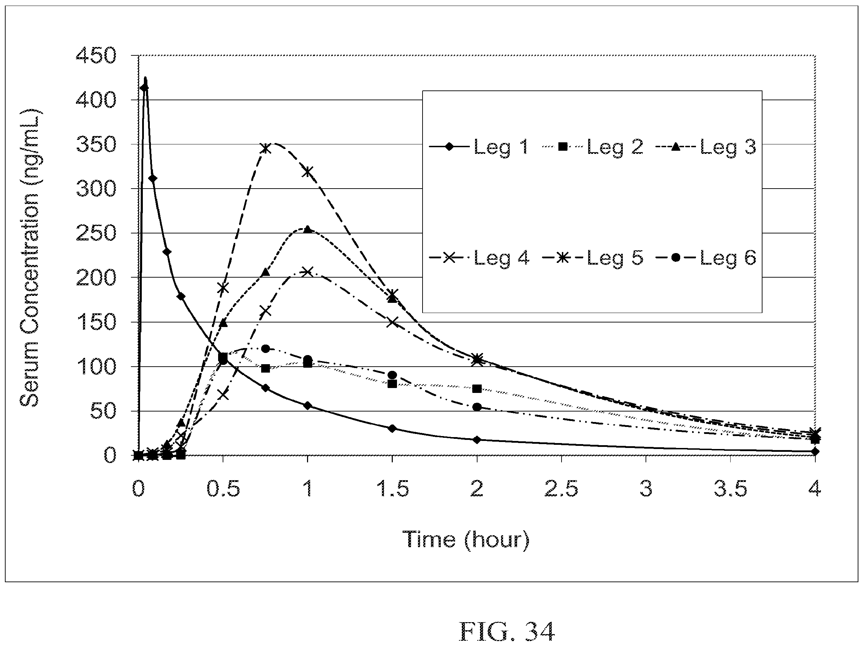

Dog study: The results of the first dog study (Legs 1-6) are summarized in Table 3. The concentrations (ng/mL) of zoledronic acid are the average values of the analytical results of 5 dogs. The PK profiles of the IV and oral groups are shown in Figures 33 and 34 which represent the first four hours of the 48hr PK profile. These results and Figure 34 suggest that most if not all zoledronic acid complexes have achieved improved oral bioavailability compared to that of the parent zoledronic acid delivered orally.

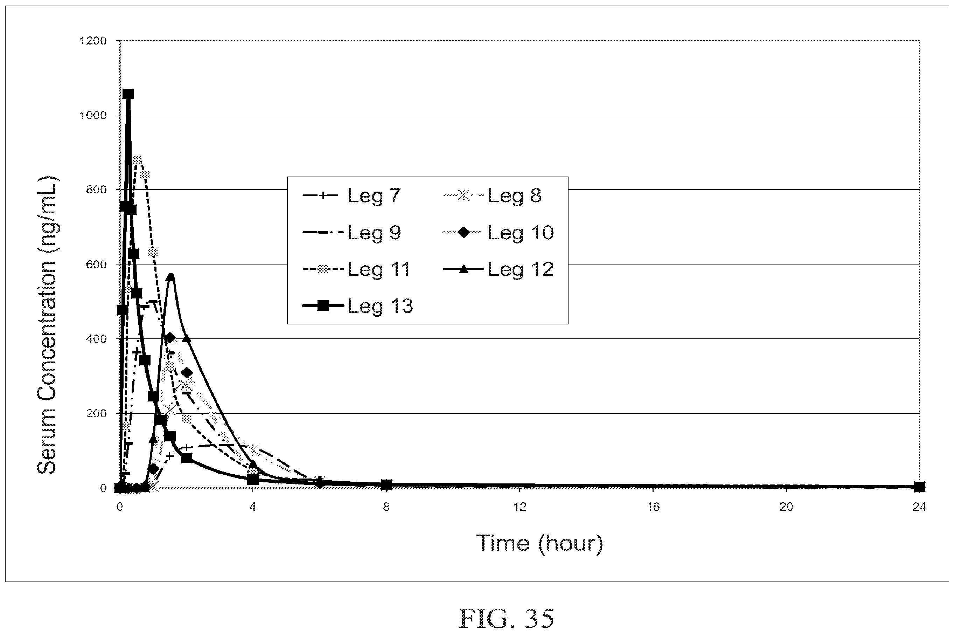

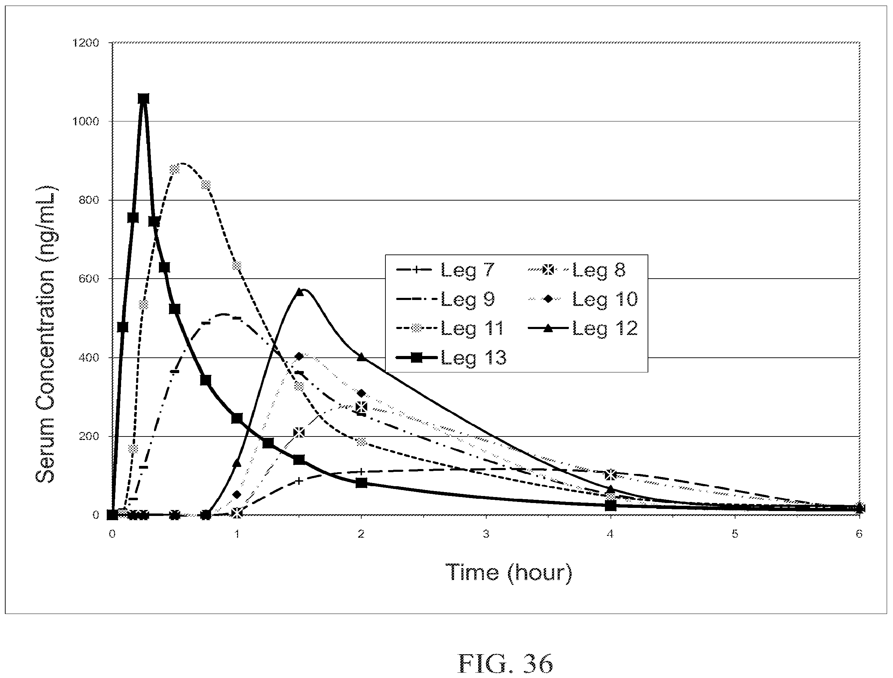

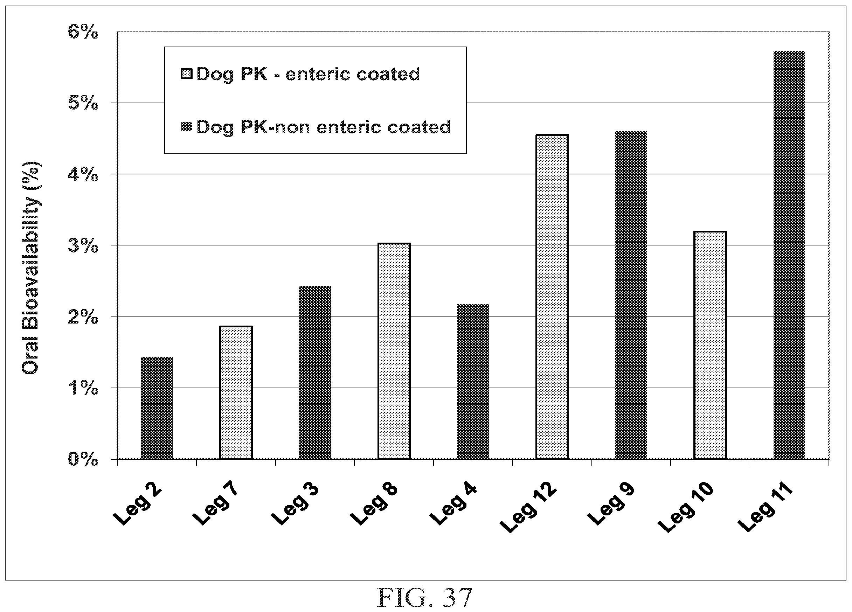

The results of another dog study (Legs 7-13) are summarized in Table 4; the concentrations (ng/mL) of zoledronic acid shown are the average values of the analytical results of 5 dogs. The PK profiles of the IV and oral groups are shown in Figures 35 and 36. Figure 36 represents the first 6 hours of the 24 hour PK profile. These results and Figure 35 suggest that most if not all zoledronic acid complexes have achieved improved oral bioavailability compared with that of the parent zoledronic acid delivered orally. Specifically, there was a significant improvement in zoledronic acid bioavailability for the novel zoledronic acid complexes with excess amino acid coformer (Leg 11, Figure 37) compared to that of the parent drug. The results have also shown that there was improvement in the bioavailability of the enterically coated capsules compared with the non-enterically coated capsules (Figure 37, Legs 7 and 2, Legs 8 and 3, Legs 12 and 4), but surprisingly the bioavailability was significantly altered when excess amino acid coformer was added to form a physical mixture inside the enterically coated capsules (Figure 37, Legs 9 and 10). The reason behind it is not fully understood.

The results have shown that there is a slight increase in the oral bioavailability of zoledronic acid from the enteric coated capsules filled with neat (i.e. with no excess coformer) zoledronic acid amino acid complex. Therefore, it is expected that the excess coformer with the novel zoledronic acid complexes would also lead to increased bioavailability when delivered in enterically coated capsules. Surprisingly, when excess coformer was added to the zoledronic acid, the bioavailability of the enterically coated capsules was lower than that of the non-enterically coated capsules. This suggests that a physical powder mixture of the molecular complex and excess coformer might decrease the bioavailability when delivered to the duodenum. The mechanism behind this surprising finding is not yet understood.

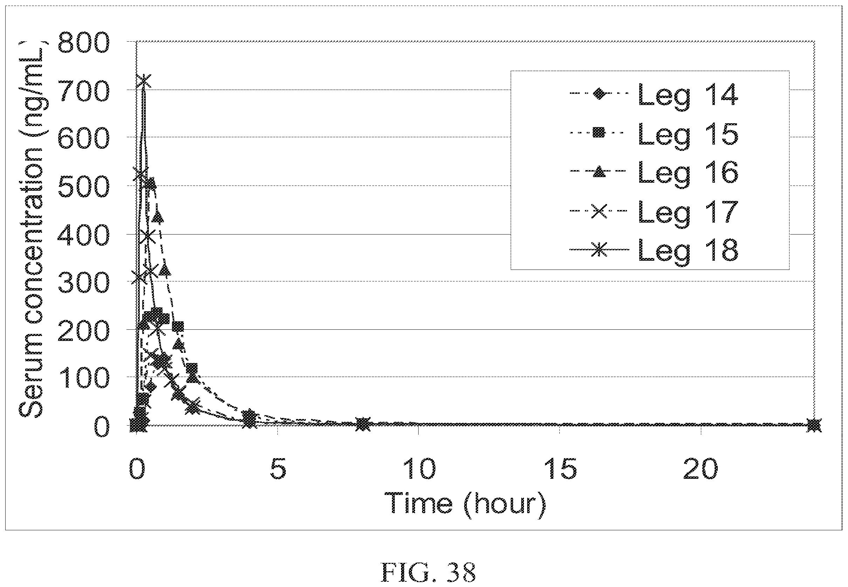

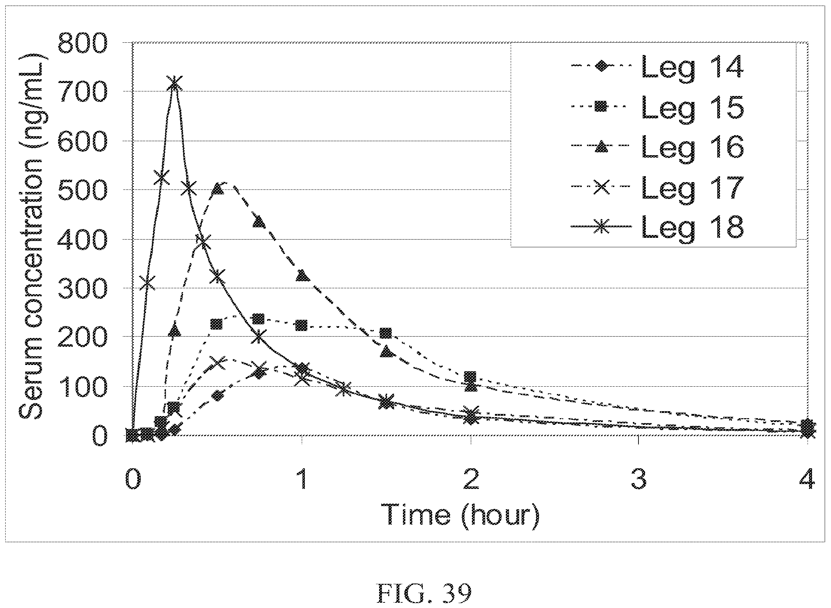

The analytical results of yet another dog study (Legs 14-18) are shown in Table 5, which contains averaged data from five dogs. The PK profiles of the IV and oral groups are shown in Figures 38 and 39. Figure 39 represents the first 4 hours of the 24 hour PK profile.

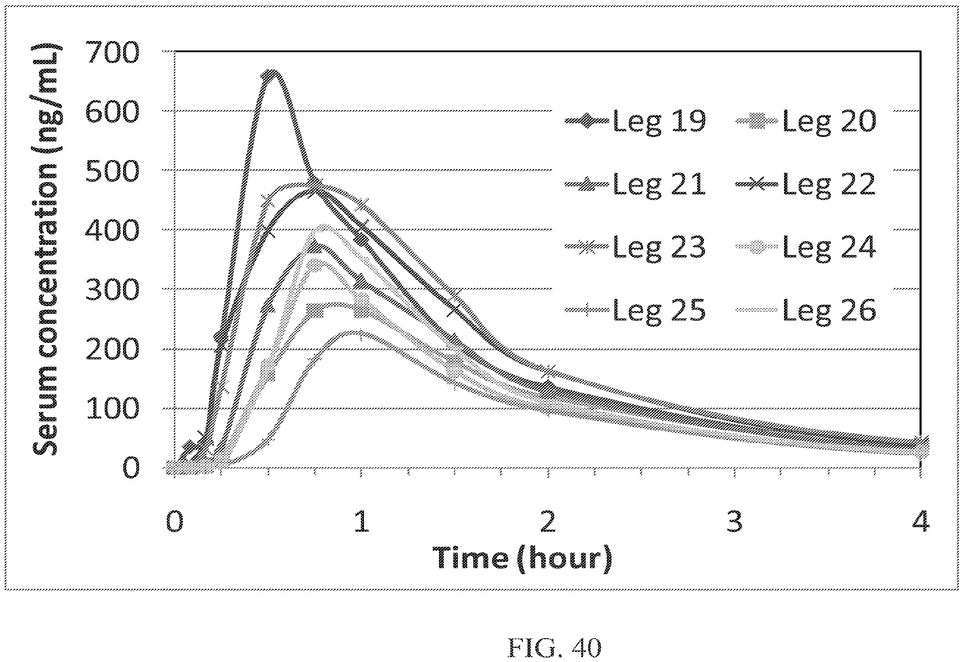

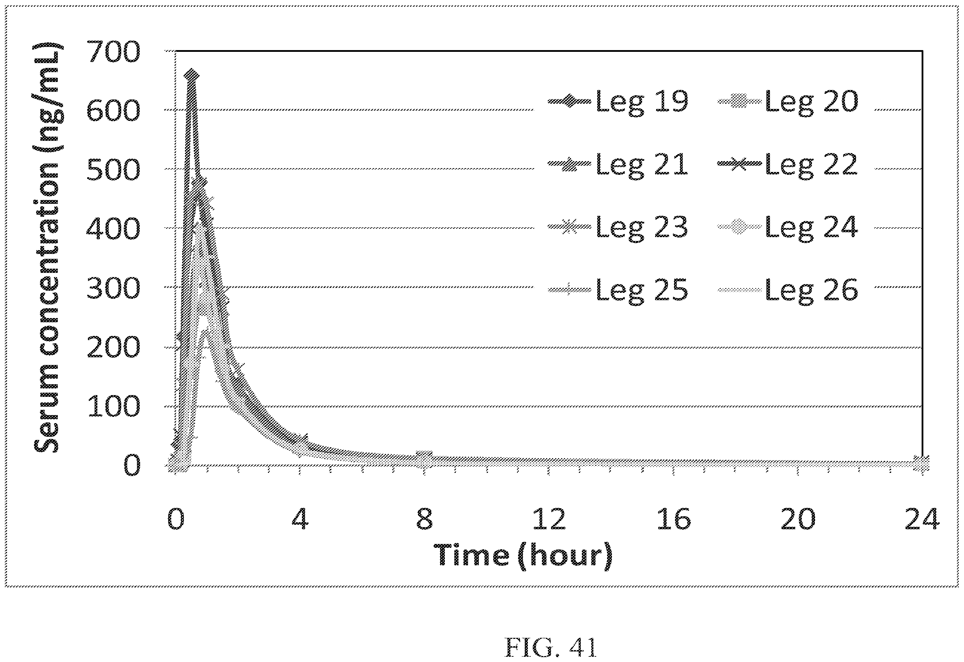

The analytical results of another dog study (Legs 19-26) are shown in Table 6, which contains averaged data from five dogs. The PK profiles of the IV and oral groups are shown in Figures 40 and 41. Figure 40 represents the first 4 hours of the 24 hour PK profile.

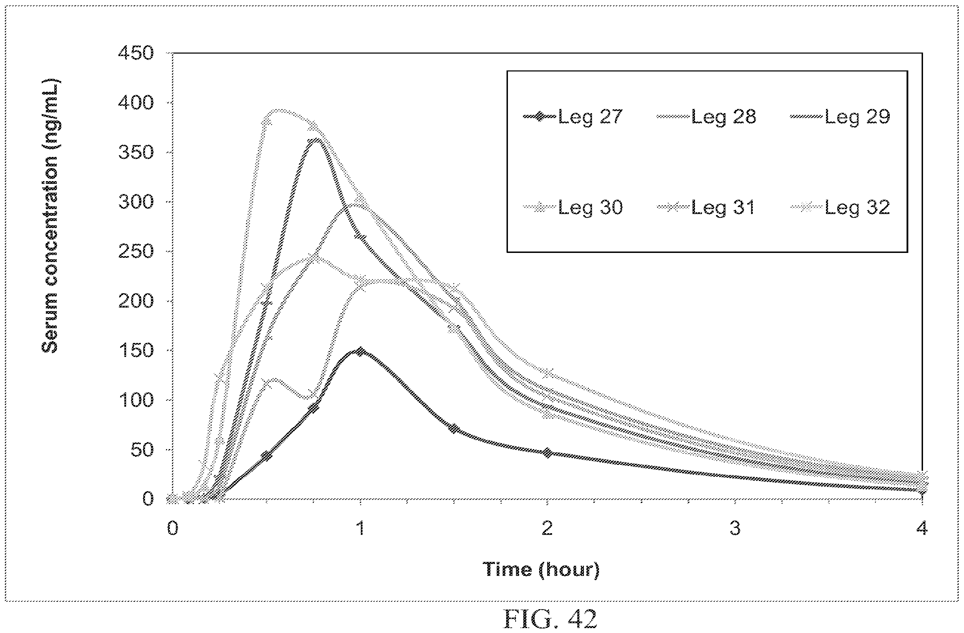

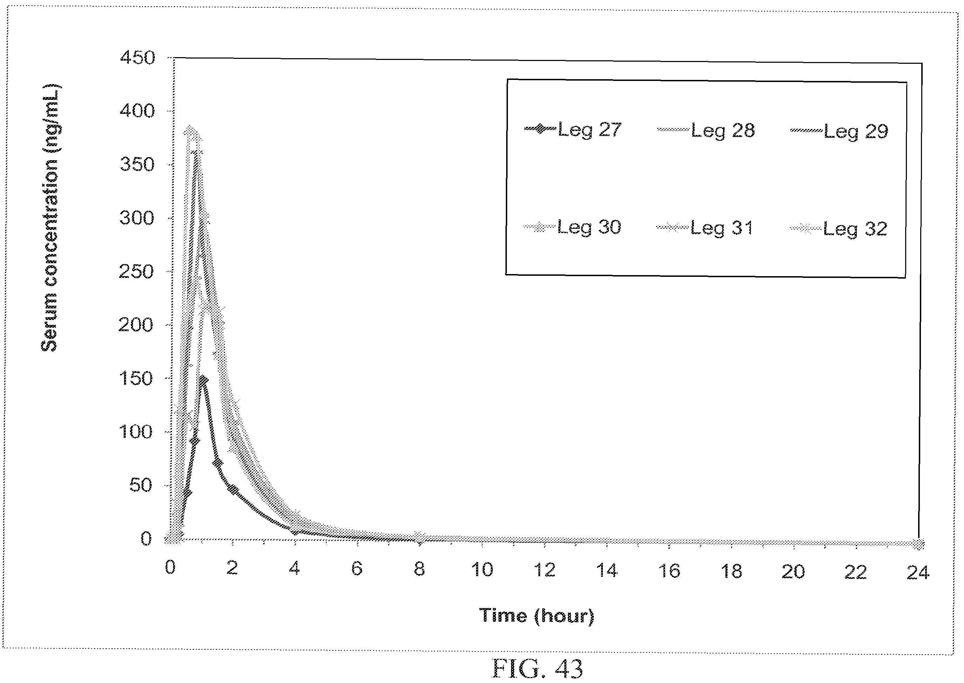

The analytical results of another dog study (Legs 27-32) are shown in Table 7, which contains the average data from 5 dogs with the exception of Leg 31 which is the average of 4 dogs. In this study, micronized materials (zoledronic:DL-lysine:water complex and pure DL-lysine) with size mean diameter of 5 micron by volume were used in some legs. Micronized materials were employed in our study to examine the possibility of increasing the Cₘₐₓ of the drug through increasing the surface area and subsequently improving its rate of dissolution that should lead to higher concentration of the drug available for absorption through the GI tract. The results are summarized in Leg 30 and 32 in Table 7. The results of the micronized materials in both legs have shown a slight increase in the bioavailability of the drug. The PK profiles of the oral groups are shown in Figures 42 and 43. Figure 42 represents the first 4 hours of the 24 hour PK profile.

The analytical results of yet another dog study (Legs 33-38) are shown in Tables 8 and 9 which contains the average data from 4 dogs. In this study, capsules of particulate materials (zoledronic:DL-lysine:water complex and excess pure DL-lysine). Prior to dosing, all dogs received a 20 ml dose of citric acid (24 mg/mL in water) to lower the pH of the stomach. After dosing capsules or IV, all dogs received additional 6.25 mL citric acid solution (24 mg/mL in water) as a rinse.

During the study, both serum and urine samples were collected from the animals. Urine samples were collected (N = 4) over five intervals, 0-4 hours, 4-8 hours, 8-12 hours, 12-24 hours and 24- 96 hours. Bioanalysis for urine excretion samples after dosing was performed. Samples were assayed for zoledronic acid using a validated LC/MS/MS method.

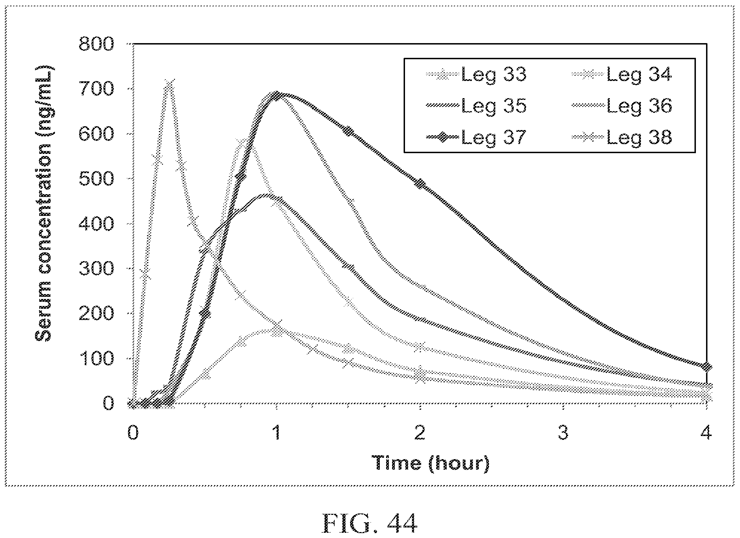

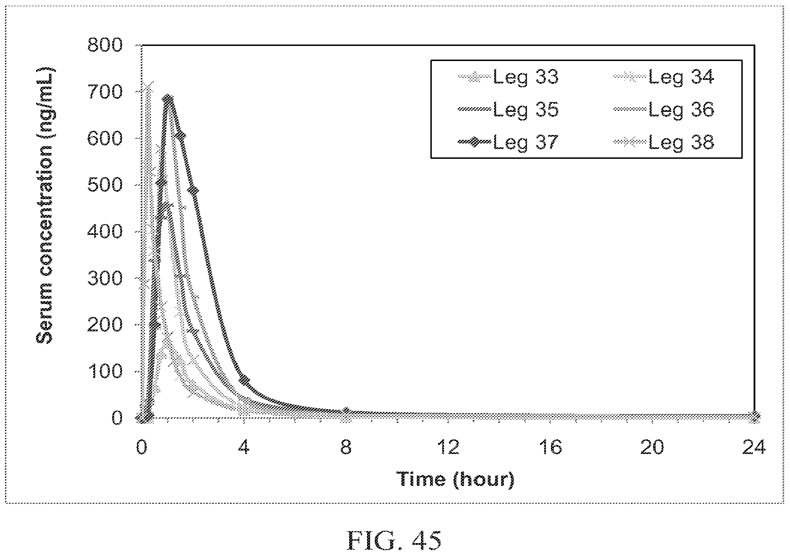

The results of Legs 33-38 are summarized in Table 8 (serum) and Table 9 (urine). The results show a significant increase in bioavailability of the bisphosphonic acid, particularly with high levels of lysine. The PK profiles are shown in Figures 44 and 45. Figure 44 represents the first 4 hours of the 24 hour PK profile.

**Table 1. Rat plasma concentrations of zoledronic acid from pure zoledronic acid and zoledronic acid complexes delivered via different routes.**

| **Group #** | **Complex** | **Dosing Route** | **Vehicle** | **Time (hour)** | **Average plasma concentration of 3 rats (ng/mL)** |
|---|---|---|---|---|---|
| G1 | Zoledronic acid | IV | Water | 0.083333 | 3254.05 |
| | | | | 0.25 | 1950.62 |
| | | | | 0.5 | 1128.75 |
| | | | | 1 | 404.28 |
| | | | | 2 | 112.68 |
| | | | | 4 | 30.46 |
| | | | | 8 | 10.66 |
| | | | | 24 | 2.98 |
| G2 | Zoledronic acid | PO | PEG 400 | 0.25 | 330.06 |
| | | | | 0.5 | 267.45 |
| | | | | 1 | 138.91 |
| | | | | 2 | 47.72 |
| | | | | 4 | 11.78 |
| | | | | 8 | 2.00 |
| | | | | 24 | 0.00 |
| G3 | Zoledronic and glycine complex | PO | PEG 400 | 0.25 | 648.01 |
| | | | | 0.5 | 435.38 |
| | | | | 1 | 200.88 |
| | | | | 4 | 12.78 |
| | | | | 8 | 1.46 |
| | | | | 24 | 0.00 |
| G4 | Zoledronic, nicotinamide, and water complex | PO | PEG 400 | 0.25 | 434.61 |
| | | | | 0.5 | 304.94 |
| | | | | 1 | 122.35 |
| | | | | 4 | 7.68 |
| | | | | 8 | 1.82 |
| | | | | 24 | 0.00 |
| G5 | Zoledronic acid, sodium zoledronic salt, and water complex | PO | PEG 400 | 0.25 | 278.47 |
| | | | | 0.5 | 280.20 |
| | | | | 1 | 171.59 |
| | | | | 4 | 13.42 |
| | | | | 8 | 1.78 |
| | | | | 24 | 0.00 |
| G6 | Zoledronic, L-lysine, and water complex | PO | PEG 400 | 0.25 | 258.43 |
| | | | | 0.5 | 249.82 |
| | | | | 1 | 184.95 |
| | | | | 4 | 28.70 |
| | | | | 8 | 3.27 |
| | | | | 24 | 0.00 |
| G7 | Zoledronic, DL-lysine, and water complex | PO | PEG 400 | 0.25 | 494.31 |
| | | | | 0.5 | 379.27 |
| | | | | 1 | 213.48 |
| | | | | 4 | 14.57 |
| | | | | 8 | 3.42 |
| | | | | 24 | 0.00 |
| G8 | Zoledronic acid | ID | PEG 400 | 0.25 | 145.67 |
| | | | | 0.5 | 109.92 |
| | | | | 1 | 47.36 |
| | | | | 2 | 12.94 |
| | | | | 4 | 3.85 |
| | | | | 8 | 0.97 |
| | | | | 24 | 0.00 |
| G9 | Zoledronic and glycine complex | ID | PEG 400 | 0.25 | 86.51 |
| | | | | 1 | 33.93 |
| | | | | 4 | 1.75 |
| | | | | 8 | 1.55 |
| | | | | 24 | 0.00 |
| G10 | Zoledronic, nicotinamide, and water complex | ID | PEG 400 | 0.25 | 69.71 |
| | | | | 1 | 21.03 |
| | | | | 4 | 0.86 |
| | | | | 8 | 0.00 |
| | | | | 24 | 0.00 |
| G11 | Zoledronic acid, sodium zoledronic salt, and water complex | ID | PEG 400 | 0.25 | 39.99 |
| | | | | 1 | 18.50 |
| | | | | 4 | 0.71 |
| | | | | 8 | 0.00 |
| | | | | 24 | 0.00 |
| G12 | Zoledronic, L-lysine, and water complex | ID | PEG 400 | 0.25 | 91.21 |
| | | | | 1 | 26.53 |
| | | | | 4 | 0.74 |
| | | | | 8 | 0.00 |
| | | | | 24 | 0.00 |
| G13 | Zoledronic, DL-lysine, and water complex | ID | PEG 400 | 0.25 | 98.25 |
| | | | | 1 | 34.61 |
| | | | | 4 | 2.65 |
| | | | | 8 | 1.02 |
| | | | | 24 | 0.80 |

**Table 2. Rat plasma concentrations of zoledronic acid from zoledronic acid complexes with excess coformers, delivered by oral gavage**

| **Group #** | **Complex** | **Dosing Route** | **Vehicle** | **Time (hour)** | **Average plasma concentration of 3 rats (ng/mL)** |
|---|---|---|---|---|---|
| G14 | Zoledronic and glycine complex and 45 mg/kg glycine | PO | PEG 400 | 0.0333333 | 14.61 |
| | | | | 0.0833333 | 206.26 |
| | | | | 0.1666667 | 340.19 |
| | | | | 0.25 | 375.99 |
| | | | | 0.5 | 321.36 |
| | | | | 1 | 197.01 |
| | | | | 4 | 17.35 |
| | | | | 24 | 0.00 |
| G15 | Zoledronic and glycine complex and 25 mg/kg glycine | PO | PEG 400 | 0.0333333 | 24.48 |
| | | | | 0.0833333 | 281.08 |
| | | | | 0.1666667 | 502.20 |
| | | | | 0.25 | 516.58 |
| | | | | 0.5 | 430.10 |
| | | | | 1 | 203.48 |
| | | | | 2 | 73.27 |
| | | | | 4 | 14.70 |
| | | | | 24 | 0.00 |
| G16 | Zoledronic and glycine complex and 5 mg/kg glycine | PO | PEG 400 | 0.0333333 | 60.03 |
| | | | | 0.0833333 | 365.23 |
| | | | | 0.1666667 | 563.83 |
| | | | | 0.25 | 625.05 |
| | | | | 0.5 | 464.34 |
| | | | | 1 | 209.65 |
| | | | | 2 | 74.28 |
| | | | | 4 | 12.17 |
| | | | | 24 | 0.00 |
| G17 | Zoledronic, DL-lysine, and water complex and 39.32 mg/kg DL-lysine monohydrate | PO | PEG 400 | 0.0333333 | 168.19 |
| | | | | 0.0833333 | 263.28 |
| | | | | 0.1666667 | 440.26 |
| | | | | 0.25 | 456.18 |
| | | | | 0.5 | 385.57 |
| | | | | 1 | 209.26 |
| | | | | 2 | 85.65 |
| | | | | 4 | 14.58 |
| | | | | 24 | 0.71 |
| G18 | Zoledronic, DL-lysine, and water complex and 28.08 mg/kg DL-lysine monohydrate | PO | PEG 400 | 0.0333333 | 219.95 |
| | | | | 0.0833333 | 427.02 |
| | | | | 0.1666667 | 729.65 |
| | | | | 0.25 | 777.54 |
| | | | | 0.5 | 632.07 |
| | | | | 1 | 300.86 |
| | | | | 2 | 100.59 |
| | | | | 4 | 21.14 |
| | | | | 24 | 0.00 |
| G19 | Zoledronic, DL-lysine, and water complex and 5.62 mg/kg DL-lysine monohydrate | PO | PEG 400 | 0.0333333 | 53.78 |
| | | | | 0.0833333 | 394.73 |
| | | | | 0.1666667 | 649.52 |
| | | | | 0.25 | 669.20 |
| | | | | 0.5 | 530.00 |
| | | | | 1 | 265.20 |
| | | | | 2 | 73.31 |
| | | | | 4 | 15.41 |
| | | | | 24 | 0.00 |
| G20 | Zoledronic, DL-lysine, and water complex | PO | PEG 400 | 0.0333333 | 103.13 |
| | | | | 0.0833333 | 352.18 |
| | | | | 0.1666667 | 475.33 |
| | | | | 0.25 | 505.48 |
| | | | | 0.5 | 431.41 |
| | | | | 1 | 224.56 |
| | | | | 2 | 69.95 |
| | | | | 4 | 14.96 |
| | | | | 24 | 0.00 |

**Table 3. Dog serum concentrations of zoledronic acid from pure zoledronic acid and zoledronic acid complexes delivered via different routes.**

| **Leg #** | **Complex** | **Dosing Route** | **Vehicle** | **Time (hour)** | **Average serum concentration of 5 dogs (ng/mL)** |
|---|---|---|---|---|---|
| | | | | 0 | 0.00 |
| | | | | 0.0333 | 413.44 |
| | | | | 0.0833 | 311.68 |
| | | | | 0.1667 | 228.97 |
| | | | | 0.25 | 178.63 |
| | | | | 0.5 | 111.11 |
| 1 | 0.05 mg/kg Zoledronic acid | IV | Saline solution | 0.75 | 75.91 |
| | | | | 1 | 56.07 |
| | | | | 1.5 | 30.35 |
| | | | | 2 | 17.61 |
| | | | | 4 | 4.29 |
| | | | | 8 | 1.13 |
| | | | | 24 | 0.00 |
| | | | | 48 | 0.00 |
| 2 | 56.0 mg Zoledronic acid monohydrate capsule | PO | n/a | 0 | 0.00 |
| | | | | 0.0833 | 0.00 |
| | | | | 0.1667 | 0.00 |
| | | | | 0.25 | 0.31 |
| | | | | 0.5 | 110.73 |
| | | | | 0.75 | 97.98 |
| | | | | 1 | 103.60 |
| | | | | 1.5 | 80.57 |
| | | | | 2 | 75.16 |
| | | | | 4 | 17.86 |
| | | | | 8 | 2.71 |
| | | | | 24 | 0.56 |
| | | | | 0 | 0.00 |
| | | | | 0.0833 | 2.45 |
| | | | | 0.1667 | 12.75 |
| | | | | 0.25 | 37.07 |
| | | | | 0.5 | 149.20 |
| 3 | 67.0 mg Zoledronic and | PO | n/a | 0.75 | 206.14 |
| | glycine complex capsule | | | 1 | 254.20 |
| | | | | 1.5 | 176.11 |
| | | | | 2 | 109.25 |
| | | | | 4 | 20.43 |
| | | | | 8 | 3.96 |
| | | | | 24 | 0.97 |
| | | | | 0 | 0.00 |
| | | | | 0.0833 | 3.11 |
| | | | | 0.1667 | 6.49 |
| | | | | 0.25 | 22.55 |
| | | | | 0.5 | 68.28 |
| | | | | 0.75 | 162.72 |
| 4 | 87.7 mg Zoledronic, DL- | PO | n/a | 1 | 206.14 |
| | lysine, and water complex capsule | | | 1.5 | 149.92 |
| | | | | 2 | 105.81 |
| | | | | 4 | 25.51 |
| | | | | 8 | 4.22 |
| | | | | 24 | 0.56 |
| | | | | 0 | 0.00 |
| | | | | 0.0833 | 0.00 |
| | | | | 0.1667 | 3.13 |
| | | | | 0.25 | 10.06 |
| | | | | 0.5 | 188.52 |
| | | | | 0.75 | 345.28 |
| 5 | 87.7 mg Zoledronic, L- | PO | n/a | 1 | 318.97 |
| | lysine, and water complex capsule | | | 1.5 | 180.77 |
| | | | | 2 | 109.23 |
| | | | | 4 | 23.11 |
| | | | | 8 | 9.73 |
| | | | | 24 | 1.93 |
| | | | | 0 | 0.00 |
| | | | | 0.0833 | 0.00 |
| | | | | 0.1667 | 0.20 |
| | | | | 0.25 | 1.92 |
| | | | | 0.5 | 106.47 |
| 6 | 84.2 mg Zoledronic, DL- | PO | n/a | 0.75 | 120.13 |
| | lysine, and water complex capsule | | | 1 | 108.13 |
| | | | | 1.5 | 90.45 |
| | | | | 2 | 54.48 |
| | | | | 4 | 18.14 |
| | | | | 8 | 4.35 |
| | | | | 24 | 1.06 |

**Table 4. Dog serum concentrations of zoledronic acid from pure zoledronic acid and zoledronic acid complexes delivered via different routes, using enteric or non-enteric coated gelatin capsules.**

| **Leg #** | **Complex** | **Dosing Route** | **Vehicle** | **Time (hour)** | **Average serum concentration of 5 dogs (ng/mL)** |
|---|---|---|---|---|---|
| 7 | 56.0 mg Zoledronic acid monohydrate enteric coated capsule | PO | n/a | 0 | 0.00 |
| | | | | 0.1667 | 0.00 |
| | | | | 0.25 | 0.00 |
| | | | | 0.5 | 0.00 |
| | | | | 0.75 | 0.00 |
| | | | | 1 | 9.84 |
| | | | | 1.5 | 86.13 |
| | | | | 2 | 109.37 |
| | | | | 4 | 107.64 |
| | | | | 6 | 14.15 |
| | | | | 8 | 4.57 |
| | | | | 24 | 0.50 |
| 8 | 67.0 mg Zoledronic and glycine complex enteric coated capsule | PO | n/a | 0 | 0.00 |
| | | | | 0.1667 | 0.00 |
| | | | | 0.25 | 0.00 |
| | | | | 0.5 | 0.00 |
| | | | | 0.75 | 0.00 |
| | | | | 1 | 4.42 |
| | | | | 1.5 | 208.97 |
| | | | | 2 | 274.53 |
| | | | | 4 | 101.20 |
| | | | | 6 | 16.71 |
| | | | | 8 | 7.14 |
| | | | | 24 | 2.17 |
| | | | | 0 | 0.00 |
| | | | | 0.0833 | 13.31 |
| | | | | 0.1667 | 39.76 |
| | | | | 0.25 | 120.41 |
| | | | | 0.5 | 364.68 |
| | 87.7 mg Zoledronic, DL-lysine, and water complex with 294.8 mg DL-lysine monohydrate capsule | | | 0.75 | 487.59 |
| 9 | | PO | n/a | 1 | 499.60 |
| | | | | 1.5 | 362.16 |
| | | | | 2 | 254.72 |
| | | | | 4 | 52.22 |
| | | | | 6 | 16.61 |
| | | | | 8 | 8.93 |
| | | | | 24 | 2.92 |
| | | | | 0 | 0.00 |
| | | | | 0.1667 | 0.00 |
| | | | | 0.25 | 0.00 |
| | | | | 0.5 | 0.00 |
| | 87.7 mg Zoledronic, DL-lysine, and water complex with 294.8 mg DL-lysine monohydrate enteric coated capsule | | | 0.75 | 3.71 |
| 10 | | PO | n/a | 1 | 51.32 |
| | | | | 1.5 | 403.15 |
| | | | | 2 | 309.08 |
| | | | | 4 | 44.83 |
| | | | | 6 | 13.15 |
| | | | | 8 | 7.09 |
| | | | | 24 | 2.66 |
| | | | | 0 | 0.22 |
| | | | | 0.1667 | 167.03 |
| | | | | 0.25 | 533.96 |
| | | | | 0.5 | 878.63 |
| | 84.2 mg Zoledronic, DL-lysine, and water complex with 294.8 mg DL-lysine monohydrate capsule | | | 0.75 | 838.82 |
| 11 | | PO | n/a | 1 | 633.50 |
| | | | | 1.5 | 326.63 |
| | | | | 2 | 185.44 |
| | | | | 4 | 46.86 |
| | | | | 6 | 20.26 |
| | | | | 8 | 11.49 |
| | | | | 24 | 5.95 |
| 12 | 87.7 mg Zoledronic, DL-lysine, and water complex enteric coated capsule | PO | n/a | 0 | 0.57 |
| | | | | 0.1667 | 0.60 |
| | | | | 0.25 | 0.59 |
| | | | | 0.5 | 0.61 |
| | | | | 0.75 | 0.40 |
| | | | | 1 | 132.15 |
| | | | | 1.5 | 566.18 |
| | | | | 2 | 402.12 |
| | | | | 4 | 65.35 |
| | | | | 6 | 21.02 |
| | | | | 8 | 12.18 |
| | | | | 24 | 4.33 |
| 13 | 0.183 mg/kg Zoledronic acid | IV | Saline solution | 0 | 0.64 |
| | | | | 0.0833 | 476.79 |
| | | | | 0.1667 | 755.68 |
| | | | | 0.25 | 1057.75 |
| | | | | 0.3333 | 745.67 |
| | | | | 0.4167 | 629.22 |
| | | | | 0.5 | 522.78 |
| | | | | 0.75 | 342.58 |
| | | | | 1 | 245.36 |
| | | | | 1.25 | 182.59 |
| | | | | 1.5 | 139.77 |
| | | | | 2 | 80.87 |
| | | | | 4 | 23.40 |
| | | | | 8 | 8.78 |
| | | | | 24 | 3.84 |

**Table 5. Dog serum concentrations of zoledronic acid from pure zoledronic acid and zoledronic acid complexes delivered via different routes.**

| **Leg #** | **Complex** | **Dosing Route** | **Vehicle** | **Time (hour)** | **Average serum concentration of 5 dogs (ng/mL)** |
|---|---|---|---|---|---|
| 14 | 35.4 mg Zoledronic, DL-lysine, and water complex, with 123.8 mg DL-lysine monohydrate gelatin capsule | PO | n/a | 0 | 0.00 |
| | | | | 0.0833 | 0.00 |
| | | | | 0.1667 | 0.72 |
| | | | | 0.25 | 11.40 |
| | | | | 0.5 | 78.95 |
| | | | | 0.75 | 126.46 |
| | | | | 1 | 137.38 |
| | | | | 1.5 | 64.73 |
| | | | | 2 | 33.38 |
| | | | | 4 | 6.14 |
| | | | | 8 | 0.89 |
| | | | | 24 | 0.00 |
| 15 | 67.0 mg Zoledronic and glycine complex, with 294.8 mg DL-lysine monohydrate gelatin capsule | PO | n/a | 0 | 0.00 |
| | | | | 0.0833 | 2.58 |
| | | | | 0.1667 | 26.13 |
| | | | | 0.25 | 55.58 |
| | | | | 0.5 | 225.41 |
| | | | | 0.75 | 234.95 |
| | | | | 1 | 221.91 |
| | | | | 1.5 | 204.90 |
| | | | | 2 | 117.22 |
| | | | | 4 | 17.79 |
| | | | | 8 | 3.34 |
| | | | | 24 | 0.77 |
| 16 | 87.7 mg Zoledronic, L-lysine, and water complex, with 294.8 mg DL-lysine monohydrate gelatin capsule | PO | n/a | 0 | 0.00 |
| | | | | 0.0833 | 3.26 |
| | | | | 0.1667 | 17.21 |
| | | | | 0.25 | 213.77 |
| | | | | 0.5 | 504.17 |
| | | | | 0.75 | 436.00 |
| | | | | 1 | 325.21 |
| | | | | 1.5 | 171.42 |
| | | | | 2 | 100.81 |
| | | | | 4 | 23.38 |
| | | | | 8 | 4.65 |
| | | | | 24 | 1.48 |
| 17 | 35.4 mg Zoledronic, DL-lysine, and water complex, with 294.8 mg | PO | n/a | 0 | 0.00 |
| | | | | 0.0833 | 0.00 |
| | | | | 0.1667 | 13.47 |
| | | | | 0.25 | 50.04 |
| | DL-lysine monohydrate gelatin capsule | | | 0.5 | 146.68 |
| | | | | 0.75 | 137.24 |
| | | | | 1 | 116.38 |
| | | | | 1.5 | 66.70 |
| | | | | 2 | 44.94 |
| | | | | 4 | 8.87 |
| | | | | 8 | 1.58 |
| | | | | 24 | 0.21 |
| | | | | 0 | 0.00 |
| | | | | 0.0833 | 309.13 |
| | | | | 0.1667 | 524.58 |
| | | | | 0.25 | 717.15 |
| | | | | 0.3333 | 501.70 |
| | 0.12 mg/kg Zoledronic acid | | Saline solution | 0.4167 | 392.35 |
| 18 | | IV | | 0.5 | 322.84 |
| | | | | 0.75 | 201.78 |
| | | | | 1 | 132.86 |
| | | | | 1.25 | 93.22 |
| | | | | 1.5 | 69.06 |
| | | | | 2 | 38.38 |
| | | | | 4 | 9.14 |
| | | | | 8 | 3.24 |
| | | | | 24 | 1.21 |

**Table 6. Dog serum concentrations of zoledronic acid from pure zoledronic acid and zoledronic acid complexes delivered orally.**

| **Leg #** | **Complex** | **Dosing Route** | **Vehicle** | **Time (hour)** | **Average serum concentration of 5 dogs (ng/mL)** |
|---|---|---|---|---|---|
| 19 | 61.3 mg Zoledronic acid, with 322.9 mg DL-lysine monohydrate gelatin capsule | PO | n/a | 0 | 0.00 |
| | | | | 0.0833 | 34.10 |
| | | | | 0.1667 | 42.74 |
| | | | | 0.25 | 219.76 |
| | | | | 0.5 | 659.25 |
| | | | | 0.75 | 478.77 |
| | | | | 1 | 383.80 |
| | | | | 1.5 | 209.87 |
| | | | | 2 | 135.97 |
| | | | | 4 | 34.22 |
| | | | | 8 | 8.53 |
| | | | | 24 | 2.07 |
| | | | | 0 | 0.20 |
| | | | | 0.0833 | 0.21 |
| | | | | 0.1667 | 4.10 |
| | | | | 0.25 | 12.03 |
| | | | | 0.5 | 156.89 |
| | 76.8 mg Zoledronic, L-lysine, and water complex, with 359.2 mg L-lysine HCl gelatin capsule | | | 0.75 | 263.80 |
| 20 | | PO | n/a | 1 | 265.48 |
| | | | | 1.5 | 178.89 |
| | | | | 2 | 118.73 |
| | | | | 4 | 36.12 |
| | | | | 8 | 12.32 |
| | | | | 24 | 2.56 |
| | | | | 0 | 0.00 |
| | | | | 0.0833 | 0.20 |
| | | | | 0.1667 | 5.77 |
| | | | | 0.25 | 32.62 |
| | | | | 0.5 | 273.09 |
| | 84.2 mg Zoledronic, DL-lysine, and water complex, with 328.0 mg L-lysine HCl gelatin capsule | | | 0.75 | 373.00 |
| 21 | | PO | n/a | 1 | 314.46 |
| | | | | 1.5 | 214.18 |
| | | | | 2 | 128.08 |
| | | | | 4 | 30.87 |
| | | | | 8 | 6.80 |
| | | | | 24 | 2.12 |
| | | | | 0 | 0.00 |
| | | | | 0.0833 | 7.35 |
| | | | | 0.1667 | 48.84 |
| | | | | 0.25 | 204.61 |
| | | | | 0.5 | 398.98 |
| | 69.0 mg Zoledronic, DL-lysine, and water complex, with 241.8 mg DL-lysine monohydrate gelatin capsule | | | 0.75 | 465.56 |
| 22 | | PO | n/a | 1 | 406.10 |
| | | | | 1.5 | 265.75 |
| | | | | 2 | 161.63 |
| | | | | 4 | 36.68 |
| | | | | 8 | 9.66 |
| | | | | 24 | 3.45 |
| 23 | 70.1 mg Zoledronic, L-lysine, and water complex, with 294.9 mg DL-lysine monohydrate gelatin capsule | PO | n/a | 0 | 0.52 |
| | | | | 0.0833 | 1.99 |
| | | | | 0.1667 | 31.45 |
| | | | | 0.25 | 135.92 |
| | | | | 0.5 | 449.28 |
| | | | | 0.75 | 474.97 |
| | | | | 1 | 442.86 |
| | | | | 1.5 | 290.01 |
| | | | | 2 | 162.59 |
| | | | | 4 | 42.25 |
| | | | | 8 | 10.77 |
| | | | | 24 | 3.28 |
| 24 | 64.0 mg Zoledronic acid, with 374.8 mg L-lysine HCl gelatin capsule | PO | n/a | 0 | 0.00 |
| | | | | 0.0833 | 0.00 |
| | | | | 0.1667 | 1.20 |
| | | | | 0.25 | 14.11 |
| | | | | 0.5 | 171.59 |
| | | | | 0.75 | 340.09 |
| | | | | 1 | 283.01 |
| | | | | 1.5 | 162.59 |
| | | | | 2 | 99.96 |
| | | | | 4 | 26.27 |
| | | | | 8 | 4.56 |
| | | | | 24 | 0.89 |
| 25 | 80.1 mg Zoledronic, L-lysine, and water complex gelatin capsule | PO | n/a | 0 | 0.00 |
| | | | | 0.0833 | 0.00 |
| | | | | 0.1667 | 0.32 |
| | | | | 0.25 | 2.16 |
| | | | | 0.5 | 47.70 |
| | | | | 0.75 | 181.00 |
| | | | | 1 | 224.61 |
| | | | | 1.5 | 142.02 |
| | | | | 2 | 95.10 |
| | | | | 4 | 23.06 |
| | | | | 8 | 3.97 |
| | | | | 24 | 1.20 |
| 26 | 76.5 mg Zoledronic and glycine complex, with 374.8 mg L-lysine HCl gelatin capsule | PO | n/a | 0 | 0.00 |
| | | | | 0.0833 | 0.00 |
| | | | | 0.1667 | 0.85 |
| | | | | 0.25 | 3.18 |
| | | | | 0.5 | 169.29 |
| | | | | 0.75 | 397.95 |
| | | | | 1 | 352.39 |
| | | | | 1.5 | 200.22 |
| | | | | 2 | 109.96 |
| | | | | 4 | 25.15 |
| | | | | 8 | 4.34 |
| | | | | 24 | 1.66 |

**Table 7. Dog serum concentrations of zoledronic acid from pure zoledronic acid and zoledronic acid complexes delivered orally.**

| **Leg #** | **Complex** | **Dosing Route** | **Vehicle** | **Time (hour)** | **Average serum concentration of 5 dogs (ng/mL)** |
|---|---|---|---|---|---|
| | | | | 0 | 0.00 |
| | | | | 0.0833 | 0.00 |
| | | | | 0.1667 | 0.52 |
| | | | | 0.25 | 4.25 |
| | 32.0 mg Zoledronic, DL- | | | 0.5 | 43.64 |
| | lysine, and water | | | 0.75 | 91.85 |
| 27 | complex, with 266.8 mg | PO | n/a | 1 | 148.71 |
| | DL-lysine monohydrate | | | 1.5 | 71.25 |
| | gelatin capsule | | | 2 | 46.68 |
| | | | | 4 | 8.83 |
| | | | | 8 | 1.02 |
| | | | | 24 | 0.00 |
| | | | | 0 | 0.00 |
| | | | | 0.0833 | 0.37 |
| | | | | 0.1667 | 3.48 |
| | | | | 0.25 | 12.59 |
| | 76.2 mg Zoledronic, DL- | | | 0.5 | 162.37 |
| | lysine, and water | | | 0.75 | 244.28 |
| 28 | complex, with 266.8 mg | PO | n/a | 1 | 295.79 |
| | DL-lysine monohydrate | | | 1.5 | 202.36 |
| | gelatin capsule | | | 2 | 110.16 |
| | | | | 4 | 21.43 |
| | | | | 8 | 3.16 |
| | | | | 24 | 0.81 |
| | | | | 0 | 0.00 |
| | | | | 0.0833 | 0.00 |
| | | | | 0.1667 | 2.10 |
| | | | | 0.25 | 23.08 |
| | 64.4 mg Zoledronic, DL- | | | 0.5 | 197.71 |
| | lysine, and water | | | 0.75 | 361.80 |
| 29 | complex, with 275.2 mg | PO | n/a | 1 | 264.70 |
| | DL-lysine monohydrate | | | 1.5 | 173.72 |
| | gelatin capsule | | | 2 | 93.35 |
| | | | | 4 | 15.54 |
| | | | | 8 | 2.97 |
| | | | | 24 | 0.71 |
| | | | | 0 | 0.00 |
| | | | | 0.0833 | 2.95 |
| | | | | 0.1667 | 13.08 |
| | | | | 0.25 | 61.19 |
| 30 | 64.4 mg micronized | PO | n/a | 0.5 | 383.13 |
| | Zoledronic, DL-lysine, | | | 0.75 | 377.27 |
| | and water complex, with | | | 1 | 305.30 |
| | 275.2 mg micronized DL- | | | 1.5 | 172.67 |
| | lysine monohydrate | | | 2 | 86.54 |
| | gelatin capsule | | | 4 | 13.56 |
| | | | | 8 | 3.52 |
| | | | | 24 | 0.87 |
| | | | | 0 | 0.00 |
| | | | | 0.0833 | 0.00 |
| | | | | 0.1667 | 0.00 |
| | | | | 0.25 | 1.50 |
| | 50.8 mg Zoledronic, DL- | | | 0.5 | 116.12 |
| | lysine, and water | | | 0.75 | 105.85 |
| 31 | complex, with 278.0 mg | PO | n/a | 1 | 214.29 |
| | DL-lysine monohydrate | | | 1.5 | 193.10 |
| | gelatin capsule | | | 2 | 103.50 |
| | | | | 4 | 18.42 |
| | | | | 8 | 2.57 |
| | | | | 24 | 0.31 |
| | | | | 0 | 0.00 |
| | | | | 0.0833 | 2.42 |
| | | | | 0.1667 | 33.98 |
| | | | | 0.25 | 121.95 |
| | 50.8 mg micronized | | | 0.5 | 212.75 |
| | Zoledronic, DL-lysine, | | | 0.75 | 242.80 |
| 32 | and water complex, with | PO | n/a | 1 | 221.71 |
| | 278.0 mg micronized DL- | | | 1.5 | 212.75 |
| | lysine monohydrate | | | 2 | 126.93 |
| | gelatin capsule | | | 4 | 23.77 |
| | | | | 8 | 3.64 |
| | | | | 24 | 0.80 |

**Table 8. Dog serum concentrations of zoledronic acid from pure zoledronic acid and zoledronic acid complexes delivered via different routes.**

| **Leg #** | **Complex** | **Dosing Route** | **Vehicle** | **Time (hour)** | **Average serum concentration of 4 dogs (ng/mL)** |
|---|---|---|---|---|---|
| 33 | 59.2 mg Zoledronic, DL-lysine, and water complex, with 112.3 mg | PO | n/a | 0 | 0.00 |
| | | | | 0.0833 | 0.00 |
| | | | | 0.1667 | 0.00 |
| | DL-lysine monohydrate gelatin capsule | | | 0.25 | 0.00 |
| | | | | 0.5 | 66.80 |
| | | | | 0.75 | 139.37 |
| | | | | 1 | 161.23 |
| | | | | 1.5 | 124.08 |
| | | | | 2 | 72.53 |
| | | | | 4 | 16.99 |
| | | | | 8 | 2.30 |
| | | | | 24 | 0.00 |
| | | | | 0 | 0.00 |
| | | | | 0.0833 | 0.00 |
| | | | | 0.1667 | 0.00 |
| | | | | 0.25 | 0.00 |
| | 63.1 mg Zoledronic, DL- | | | 0.5 | 206.30 |
| | lysine, and water | | | 0.75 | 577.25 |
| 34 | complex, with 280.8 mg | PO | n/a | 1 | 449.00 |
| | DL-lysine monohydrate | | | 1.5 | 226.50 |
| | gelatin capsule | | | 2 | 125.33 |
| | | | | 4 | 23.45 |
| | | | | 8 | 6.00 |
| | | | | 24 | 1.37 |
| | | | | 0 | 0.00 |
| | | | | 0.0833 | 0.00 |
| | | | | 0.1667 | 24.88 |
| | | | | 0.25 | 38.21 |
| | 76.3 mg Zoledronic, DL- | | | 0.5 | 338.33 |
| | lysine, and water | | | 0.75 | 429.38 |
| 35 | complex, with 561.6 mg | PO | n/a | 1 | 456.23 |
| | DL-lysine monohydrate | | | 1.5 | 304.78 |
| | gelatin capsule | | | 2 | 186.70 |
| | | | | 4 | 41.48 |
| | | | | 8 | 11.11 |
| | | | | 24 | 2.35 |
| | | | | 0 | 0.00 |
| | | | | 0.0833 | 0.00 |
| | | | | 0.1667 | 0.31 |
| | | | | 0.25 | 29.50 |
| | | | | 0.5 | 192.57 |
| | 59.2 mg Zoledronic, DL- | | | 0.75 | 517.75 |
| | lysine, and water | | | 1 | 688.50 |
| 36 | complex, with 1123.3 mg | PO | n/a | 1.5 | 451.50 |
| | DL-lysine monohydrate | | | 2 | 259.75 |
| | gelatin capsule | | | 4 | 37.05 |
| | | | | 8 | 6.95 |
| | | | | 24 | 2.62 |
| 37 | 63.1 mg Zoledronic, DL- | PO | n/a | 0 | 0.00 |
| | lysine, and water | | | 0.0833 | 0.00 |
| | complex, with 1965.7 mg | | | 0.1667 | 0.00 |
| | DL-lysine monohydrate | | | 0.25 | 5.55 |
| | gelatin capsule | | | 0.5 | 200.00 |
| | | | | 0.75 | 504.73 |
| | | | | 1 | 683.50 |
| | | | | 1.5 | 606.00 |
| | | | | 2 | 488.03 |
| | | | | 4 | 81.28 |
| | | | | 8 | 12.34 |
| | | | | 24 | 4.07 |
| | | | | 0 | 0.00 |
| | | | | 0.0833 | 287.75 |
| | | | | 0.1667 | 541.50 |
| | | | | 0.25 | 710.75 |
| | | | | 0.3333 | 528.75 |
| | | | | 0.4167 | 405.50 |
| | | | | 0.5 | 358.25 |
| 38 | 0.12 mg/kg Zoledronic acid | IV | Saline solution | 0.75 | 239.50 |
| | | | | 1 | 174.00 |
| | | | | 1.25 | 121.38 |
| | | | | 1.5 | 90.58 |
| | | | | 2 | 55.68 |
| | | | | 4 | 15.13 |
| | | | | 8 | 5.74 |
| | | | | 24 | 2.49 |

**Table 9. Quantity of zoledronic acid in dog urine from zoledronic acid, DL-lysine and water complex and excess coformer delivered via different routes at different doses. During the study, urine samples were collected from the animals (N = 4) over five intervals, 0-4 hours, 4-8 hours, 8-12 hours, 12-24 hours and 24- 96 hours. Bioanalysis for urine excretion samples after dosing has been performed. Samples were assayed for zoledronic acid using a validated LC/MS/MS method.**

| **Leg #** | **Complex** | **Dosing Route** | **Vehicle** | **Time interval (hour)** | **Average quantity of zoledronic acid in urine excretion of 4 dogs (ng)** |
|---|---|---|---|---|---|
| 33 | 59.2 mg Zoledronic, DL-lysine, and water complex, with 1 12.3 mg DL-lysine monohydrate gelatin capsule | PO | n/a | 0 - 4 | 43251 |
| | | | | 4 - 8 | 548 |
| | | | | 8 - 12 | 102750 |
| | | | | 12 - 24 | 147710 |
| | | | | 24 - 96 | 20571 |
| 34 | 63.1 mg Zoledronic, DL-lysine, and water complex, with 280.8 mg DL-lysine monohydrate gelatin capsule | PO | n/a | 0 - 4 | 121045 |
| | | | | 4 - 8 | 1393 |
| | | | | 8 - 12 | 228375 |
| | | | | 12 - 24 | 204485 |
| | | | | 24 - 96 | 98205 |
| 35 | 76.3 mg Zoledronic, DL-lysine, and water complex, with 561.6 mg DL-lysine monohydrate gelatin capsule | PO | n/a | 0 - 4 | 440062 |
| | | | | 4 - 8 | 16970 |
| | | | | 8 - 12 | 285490 |
| | | | | 12 - 24 | 287863 |
| | | | | 24 - 96 | 97306 |
| 36 | 59.2 mg Zoledronic, DL-lysine, and water complex, with 1123.3 mg DL-lysine monohydrate gelatin capsule | PO | n/a | 0 - 4 | 311764 |
| | | | | 4 - 8 | 24 |
| | | | | 8 - 12 | 385625 |
| | | | | 12 - 24 | 456538 |
| | | | | 24 - 96 | 105767 |
| 37 | 63.1 mg Zoledronic, DL-lysine, and water complex, with 1965.7 mg DL-lysine monohydrate gelatin capsule | PO | n/a | 0 - 4 | 234333 |
| | | | | 4 - 8 | 178950 |
| | | | | 8 - 12 | 888750 |
| | | | | 12 - 24 | 117100 |
| | | | | 24 - 96 | 186090 |
| 38 | 0.12 mg/kg Zoledronic acid | IV | Saline solution | 0 - 4 | 242050 |
| | | | | 4 - 8 | 21165 |
| | | | | 8 - 12 | 10925 |
| | | | | 12 - 24 | 43700 |
| | | | | 24 - 96 | 263151 |

**Table 10. Aqueous solubility of zoledronic acid (ZA) and novel zoledronic acid complexes at room temperature.**

| ***Compound*** | ***Conc. mg*/*mL*** | ***mMol*/*L (complex)*** |
|---|---|---|
| ZA monohydrate | 1.57 | 5.41 |
| ZA : Glycine | 11.89 | 34.25 |
| ZA : L-Lysine dihydrate | 8.22 | 18.09 |
| ZA : DL-Lysine dihydrate | 6.85 | 15.08 |
| ZA : DL-Lysine monohydrate | 13.9 | 31.86 |

**Table 11. Particular unit dose pharmaceutical compositions. As indicated in the columns 1-3 and 4-6 above, the compositions comprise an API and coformer, wherein the coformer is either present as a molecular complex coformer, an additional coformer or both a molecular complex coformer and additional coformer, with the total amount of coformer present in the unit dose indicated. Each three cell combination of API, coformer and amount of coformer represent an individual unit dose pharmaceutical composition.**

| **API** | **Amino Acid** | **Amount of Amino Acid per Unit Dose of API** | **API** | **Amino Acid** | **Amount of Amino Acid per Unit Dose of API** |
|---|---|---|---|---|---|
| abacavir | lysine | ≥100mg | abacavir | lysine | ≥3g |
| acarbose | lysine | ≥100mg | acarbose | lysine | ≥3g |
| acetazolamide | lysine | ≥100mg | acetazolamide | lysine | ≥3g |
| acyclovir | lysine | ≥100mg | acyclovir | lysine | ≥3g |
| albuterol (salbutamol) | lysine | ≥100mg | albuterol (salbutamol) | lysine | ≥3g |
| allopurinol | lysine | ≥100mg | allopurinol | lysine | ≥3g |
| amiloride | lysine | ≥100mg | amiloride | lysine | ≥3g |
| amisulpride | lysine | ≥100mg | amisulpride | lysine | ≥3g |
| amlodipine | lysine | ≥100mg | amlodipine | lysine | ≥3g |
| amoxicillin | lysine | ≥100mg | amoxicillin | lysine | ≥3g |
| amphetamine | lysine | ≥100mg | amphetamine | lysine | ≥3g |
| atenolol | lysine | ≥100mg | atenolol | lysine | ≥3g |
| atropine | lysine | ≥100mg | atropine | lysine | ≥3g |
| azathioprine | lysine | ≥100mg | azathioprine | lysine | ≥3g |
| benserazide | lysine | ≥100mg | benserazide | lysine | ≥3g |
| benznidazole | lysine | ≥100mg | benznidazole | lysine | ≥3g |
| camostat | lysine | ≥100mg | camostat | lysine | ≥3g |
| captopril | lysine | ≥100mg | captopril | lysine | ≥3g |
| cefdinir | lysine | ≥100mg | cefdinir | lysine | ≥3g |
| cefotiam hexetil hydrochloride | lysine | ≥100mg | cefotiam hexetil hydrochloride | lysine | ≥3g |
| cefprozil | lysine | ≥100mg | cefprozil | lysine | ≥3g |
| cefuroxime axetil | lysine | ≥100mg | cefuroxime axetil | lysine | ≥3g |
| chloramphenicol | lysine | ≥100mg | chloramphenicol | lysine | ≥3g |
| cimetidine | lysine | ≥100mg | cimetidine | lysine | ≥3g |
| ciprofloxacin | lysine | ≥100mg | ciprofloxacin | lysine | ≥3g |
| codeine | lysine | ≥100mg | codeine | lysine | ≥3g |
| colchicine | lysine | ≥100mg | colchicine | lysine | ≥3g |
| cyclophosphamide | lysine | ≥100mg | cyclophosphamide | lysine | ≥3g |
| dapsone | lysine | ≥100mg | dapsone | lysine | ≥3g |
| dexamethasone | lysine | ≥100mg | dexamethasone | lysine | ≥3g |
| didanosine | lysine | ≥100mg | didanosine | lysine | ≥3g |
| diethylcarbamazine | lysine | ≥100mg | diethylcarbamazine | lysine | ≥3g |
| methionine | lysine | ≥100mg | methionine | lysine | ≥3g |
| dolasetron | lysine | ≥100mg | dolasetron | lysine | ≥3g |
| doxifluridine | lysine | ≥100mg | doxifluridine | lysine | ≥3g |
| doxycycline | lysine | ≥100mg | doxycycline | lysine | ≥3g |
| ergonovine | lysine | ≥100mg | ergonovine | lysine | ≥3g |
| erythromycin ethylsuccinate | lysine | ≥100mg | erythromycin ethylsuccinate | lysine | ≥3g |
| ethambutol | lysine | ≥100mg | ethambutol | lysine | ≥3g |
| ethosuximide | lysine | ≥100mg | ethosuximide | lysine | ≥3g |
| famotidine | lysine | ≥100mg | famotidine | lysine | ≥3g |
| fluconazole | lysine | ≥100mg | fluconazole | lysine | ≥3g |
| folic acid | lysine | ≥100mg | folic acid | lysine | ≥3g |
| furosemide | lysine | ≥100mg | furosemide | lysine | ≥3g |
| fursultiamine | lysine | ≥100mg | fursultiamine | lysine | ≥3g |
| gabapentin | lysine | ≥100mg | gabapentin | lysine | ≥3g |
| glipizide | lysine | ≥100mg | glipizide | lysine | ≥3g |
| granisetron | lysine | ≥100mg | granisetron | lysine | ≥3g |
| griseofulvin | lysine | ≥100mg | griseofulvin | lysine | ≥3g |
| hydralazine | lysine | ≥100mg | hydralazine | lysine | ≥3g |
| hydrochlorothiazide | lysine | >100mg | hydrochlorothiazide | lysine | ≥3g |
| imidapril | lysine | ≥100mg | imidapril | lysine | ≥3g |
| isoniazid | lysine | ≥100mg | isoniazid | lysine | ≥3g |
| lamivudine | lysine | ≥100mg | lamivudine | lysine | ≥3g |
| 1-carbocysteine | lysine | ≥100mg | 1-carbocysteine | lysine | ≥3g |
| levetiracetam | lysine | ≥100mg | levetiracetam | lysine | ≥3g |
| levofloxacin | lysine | ≥100mg | levofloxacin | lysine | ≥3g |
| linezolid | lysine | ≥100mg | linezolid | lysine | ≥3g |
| lisinopril | lysine | ≥100mg | lisinopril | lysine | ≥3g |
| losartan | lysine | ≥100mg | losartan | lysine | ≥3g |
| methotrexate | lysine | ≥100mg | methotrexate | lysine | ≥3g |
| methyldopa | lysine | ≥100mg | methyldopa | lysine | ≥3g |
| s-methylmethionine | lysine | ≥100mg | s-methylmethionine | lysine | ≥3g |
| metoclopramide | lysine | ≥100mg | metoclopramide | lysine | ≥3g |
| metronidazole | lysine | ≥100mg | metronidazole | lysine | ≥3g |
| moxifloxacin | lysine | ≥100mg | moxifloxacin | lysine | ≥3g |
| nalidixic acid | lysine | ≥100mg | nalidixic acid | lysine | ≥3g |
| nicorandil | lysine | ≥100mg | nicorandil | lysine | ≥3g |
| nifurtimox | lysine | ≥100mg | nifurtimox | lysine | ≥3g |
| nitrofurantoin | lysine | ≥100mg | nitrofurantoin | lysine | ≥3g |
| nizatidine | lysine | ≥100mg | nizatidine | lysine | ≥3g |
| nystatin | lysine | ≥100mg | nystatin | lysine | ≥3g |
| ondansetron | lysine | ≥100mg | ondansetron | lysine | ≥3g |
| oseltamivir | lysine | ≥100mg | oseltamivir | lysine | ≥3g |
| oxcarbazepine | lysine | ≥100mg | oxcarbazepine | lysine | ≥3g |
| penicillamine | lysine | ≥100mg | penicillamine | lysine | ≥3g |
| perindopril | lysine | ≥100mg | perindopril | lysine | ≥3g |
| phenobarbital | lysine | >100mg | phenobarbital | lysine | ≥3g |
| phenoxymethylpenicill in | lysine | >100mg | phenoxymethylpenicill in | lysine | ≥3g |
| pravastatin sodium | lysine | ≥100mg | pravastatin sodium | lysine | ≥3g |
| prednisolone | lysine | ≥100mg | prednisolone | lysine | ≥3g |
| primaquine | lysine | ≥100mg | primaquine | lysine | ≥3g |
| procaterol | lysine | ≥100mg | procaterol | lysine | ≥3g |
| propylthiouracil | lysine | ≥100mg | propylthiouracil | lysine | ≥3g |
| pseudoephedrine | lysine | ≥100mg | pseudoephedrine | lysine | ≥3g |
| pyrazinamide | lysine | ≥100mg | pyrazinamide | lysine | ≥3g |
| pyridostigmine bromide | lysine | ≥100mg | pyridostigmine bromide | lysine | ≥3g |
| pyridoxine hydrochloride | lysine | ≥100mg | pyridoxine hydrochloride | lysine | ≥3g |
| ranitidine | lysine | ≥100mg | ranitidine | lysine | ≥3g |
| ribavirin | lysine | ≥100mg | ribavirin | lysine | ≥3g |
| riboflavin | lysine | ≥100mg | riboflavin | lysine | ≥3g |
| rizatriptan | lysine | ≥100mg | rizatriptan | lysine | ≥3g |
| stavudine | lysine | ≥100mg | stavudine | lysine | ≥3g |
| sulfadiazine | lysine | ≥100mg | sulfadiazine | lysine | ≥3g |
| sulfamethoxazole | lysine | ≥100mg | sulfamethoxazole | lysine | ≥3g |
| sultamicillin | lysine | ≥100mg | sultamicillin | lysine | ≥3g |
| sumatriptan | lysine | ≥100mg | sumatriptan | lysine | ≥3g |
| taltirelin | lysine | ≥100mg | taltirelin | lysine | ≥3g |
| tegafur | lysine | ≥100mg | tegafur | lysine | ≥3g |
| tenofovir disoproxil | lysine | ≥100mg | tenofovir disoproxil | lysine | ≥3g |
| theophylline | lysine | ≥100mg | theophylline | lysine | ≥3g |
| thiamine | lysine | ≥100mg | thiamine | lysine | ≥3g |
| trimetazidine | lysine | ≥100mg | trimetazidine | lysine | ≥3g |
| trimethoprim | lysine | ≥100mg | trimethoprim | lysine | ≥3g |
| voglibose | lysine | ≥100mg | voglibose | lysine | ≥3g |
| zidovudine | lysine | ≥100mg | zidovudine | lysine | ≥3g |
| zolmitriptan | lysine | ≥100mg | zolmitriptan | lysine | ≥3g |
| acetylcarnitine | lysine | ≥100mg | acetylcarnitine | lysine | ≥3g |
| capecitabine | lysine | ≥100mg | capecitabine | lysine | ≥3g |
| cefaclor | lysine | ≥100mg | cefaclor | lysine | ≥3g |
| cefixime | lysine | ≥100mg | cefixime | lysine | ≥3g |
| cefmetazole | lysine | ≥100mg | cefmetazole | lysine | ≥3g |
| cefpodoxime proxetil | lysine | ≥100mg | cefpodoxime proxetil | lysine | ≥3g |
| cefroxadine | lysine | ≥100mg | cefroxadine | lysine | ≥3g |
| al foscerate | lysine | ≥100mg | alfoscerate | lysine | ≥3g |
| cilazapril | lysine | ≥100mg | cilazapril | lysine | ≥3g |
| cimetropium bromide | lysine | ≥100mg | cimetropium bromide | lysine | ≥3g |
| diacerein | lysine | ≥100mg | diacerein | lysine | ≥3g |
| erdosteine | lysine | ≥100mg | erdosteine | lysine | ≥3g |
| famciclovir | lysine | ≥100mg | famciclovir | lysine | ≥3g |
| gemifloxacin | lysine | ≥100mg | gemifloxacin | lysine | ≥3g |
| levosulpiride | lysine | ≥100mg | levosulpiride | lysine | ≥3g |
| nabumetone | lysine | ≥100mg | nabumetone | lysine | ≥3g |
| oxiracetam | lysine | ≥100mg | oxiracetam | lysine | ≥3g |
| phendimetrazine | lysine | ≥100mg | phendimetrazine | lysine | ≥3g |
| rabeprazole | lysine | ≥100mg | rabeprazole | lysine | ≥3g |
| roxatidine acetate | lysine | ≥100mg | roxatidine acetate | lysine | ≥3g |
| tamsulosin | lysine | ≥100mg | tamsulosin | lysine | ≥3g |
| terazosin | lysine | ≥100mg | terazosin | lysine | ≥3g |
| thioctic | lysine | ≥100mg | thioctic | lysine | ≥3g |
| tosufloxacin | lysine | ≥100mg | tosufloxacin | lysine | ≥3g |
| triflusal | lysine | ≥100mg | triflusal | lysine | ≥3g |
| zaltoprofen | lysine | ≥100mg | zaltoprofen | lysine | ≥3g |
| etidronic acid | lysine | ≥100mg | etidronic acid | lysine | ≥3g |
| zoledronic acid | lysine | ≥100mg | zoledronic acid | lysine | ≥3g |
| clodronic acid | lysine | ≥100mg | clodronic acid | lysine | ≥3g |
| tiludronic acid | lysine | ≥100mg | tiludronic acid | lysine | ≥3g |
| pamidronic acid | lysine | ≥100mg | pamidronic acid | lysine | ≥3g |
| alendronic acid | lysine | ≥100mg | alendronic acid | lysine | ≥3g |
| risedronic acid | lysine | ≥100mg | risedronic acid | lysine | ≥3g |
| ibandronic acid | lysine | ≥100mg | ibandronic acid | lysine | ≥3g |
| abacavir | glycine | ≥100mg | abacavir | glycine | ≥3g |
| acarbose | glycine | ≥100mg | acarbose | glycine | ≥3g |
| acetazolamide | glycine | ≥100mg | acetazolamide | glycine | ≥3g |
| acyclovir | glycine | ≥100mg | acyclovir | glycine | ≥3g |
| albuterol (salbutamol) | glycine | ≥100mg | albuterol (salbutamol) | glycine | ≥3g |
| allopurinol | glycine | ≥100mg | allopurinol | glycine | ≥3g |
| amiloride | glycine | ≥100mg | amiloride | glycine | ≥3g |
| amisulpride | glycine | ≥100mg | amisulpride | glycine | ≥3g |
| amlodipine | glycine | ≥100mg | amlodipine | glycine | ≥3g |
| amoxicillin | glycine | ≥100mg | amoxicillin | glycine | ≥3g |
| amphetamine | glycine | ≥100mg | amphetamine | glycine | ≥3g |
| atenolol | glycine | ≥100mg | atenolol | glycine | ≥3g |
| atropine | glycine | ≥100mg | atropine | glycine | ≥3g |
| azathioprine | glycine | ≥100mg | azathioprine | glycine | ≥3g |
| benserazide | glycine | ≥100mg | benserazide | glycine | ≥3g |
| benznidazole | glycine | ≥100mg | benznidazole | glycine | ≥3g |
| camostat | glycine | ≥100mg | camostat | glycine | ≥3g |
| captopril | glycine | ≥100mg | captopril | glycine | ≥3g |
| cefdinir | glycine | ≥100mg | cefdinir | glycine | ≥3g |
| cefotiam hexetil hydrochloride | glycine | ≥100mg | cefotiam hexetil hydrochloride | glycine | ≥3g |
| cefprozil | glycine | ≥100mg | cefprozil | glycine | ≥3g |
| cefuroxime axetil | glycine | ≥100mg | cefuroxime axetil | glycine | ≥3g |
| chloramphenicol | giycine | ≥100mg | chloramphenicol | glycine | ≥3g |
| cimetidine | glycine | ≥100mg | cimetidine | glycine | ≥3g |
| ciprofloxacin | glycine | ≥100mg | ciprofloxacin | glycine | ≥3g |
| codeine | glycine | ≥100mg | codeine | glycine | ≥3g |
| colchicine | glycine | ≥100mg | colchicine | glycine | ≥3g |
| cyclophosphamide | glycine | ≥100mg | cyclophosphamide | glycine | ≥3g |
| dapsone | glycine | ≥100mg | dapsone | glycine | ≥3g |
| dexamethasone | glycine | ≥100mg | dexamethasone | glycine | ≥3g |
| didanosine | glycine | ≥100mg | didanosine | glycine | ≥3g |
| diethylcarbamazine | glycine | ≥100mg | diethylcarbamazine | glycine | ≥3g |
| methionine | glycine | ≥100mg | methionine | glycine | ≥3g |
| dolasetron | glycine | ≥100mg | dolasetron | glycine | >3g |
| doxifluridine | glycine | ≥100mg | doxifluridine | glycine | ≥3g |
| doxycycline | glycine | ≥100mg | doxycycline | glycine | ≥3g |
| ergonovine | glycine | ≥100mg | ergonovine | glycine | ≥3g |
| erythromycin ethylsuccinate | glycine | ≥100mg | erythromycin ethylsuccinate | glycine | ≥3g |
| ethambutol | glycine | ≥100mg | ethambutol | glycine | ≥3g |
| ethosuximide | glycine | ≥100mg | ethosuximide | glycine | ≥3g |
| famotidine | glycine | ≥100mg | famotidine | glycine | >3g |
| fluconazole | glycine | ≥100mg | fluconazole | glycine | ≥3g |
| folic acid | glycine | ≥100mg | folic acid | glycine | ≥3g |
| furosemide | glycine | ≥100mg | furosemide | glycine | ≥3g |
| fursultiamine | glycine | ≥100mg | fursultiamine | glycine | ≥3g |
| gabapentin | glycine | ≥100mg | gabapentin | glycine | ≥3g |
| glipizide | glycine | ≥100mg | glipizide | glycine | ≥3g |
| granisetron | glycine | ≥100mg | granisetron | glycine | ≥3g |
| griseofulvin | glycine | ≥100mg | griseofulvin | glycine | ≥3g |
| hydralazine | glycine | ≥100mg | hydralazine | glycine | ≥3g |
| hydrochlorothiazide | glycine | ≥100mg | hydrochlorothiazide | glycine | ≥3g |
| imidapril | glycine | ≥100mg | imidapril | glycine | ≥3g |
| isoniazid | glycine | ≥100mg | isoniazid | glycine | ≥3g |
| lamivudine | glycine | ≥100mg | lamivudine | glycine | ≥3g |
| l-carbocysteine | glycine | ≥100mg | l-carbocysteine | glycine | ≥3g |
| levetiracetam | glycine | ≥100mg | levetiracetam | glycine | ≥3g |
| levofloxacin | glycine | ≥100mg | levofloxacin | glycine | ≥3g |
| linezolid | glycine | ≥100mg | linezolid | glycine | ≥3g |
| lisinopril | glycine | ≥100mg | lisinopril | glycine | ≥3g |
| losartan | glycine | ≥100mg | losartan | glycine | ≥3g |
| methotrexate | glycine | ≥100mg | methotrexate | glycine | ≥3g |
| methyldopa | glycine | ≥100mg | methyldopa | glycine | ≥3g |
| s-methylmethionine | glycine | ≥100mg | s-methylmethionine | glycine | ≥3g |
| metoclopramide | glycine | ≥100mg | metoclopramide | glycine | ≥3g |
| metronidazole | glycine | ≥100mg | metronidazole | glycine | ≥3g |
| moxifloxacin | glycine | ≥100mg | moxifloxacin | glycine | ≥3g |
| nalidixic acid | glycine | ≥100mg | nalidixic acid | glycine | ≥3g |
| nicorandil | glycine | ≥100mg | nicorandil | glycine | ≥3g |
| nifurtimox | glycine | ≥100mg | nifurtimox | glycine | ≥3g |
| nitrofurantoin | glycine | ≥100mg | nitrofurantoin | glycine | ≥3g |
| nizatidine | glycine | ≥100mg | nizatidine | glycine | ≥3g |
| nystatin | glycine | ≥100mg | nystatin | glycine | ≥3g |
| ondansetron | glycine | ≥100mg | ondansetron | glycine | ≥3g |
| oseltamivir | glycine | ≥100mg | oseltamivir | glycine | ≥3g |
| oxcarbazepine | glycine | ≥100mg | oxcarbazepine | glycine | ≥3g |
| penicillamine | glycine | ≥100mg | penicillamine | glycine | ≥3g |
| perindopril | glycine | ≥100mg | perindopril | glycine | ≥3g |
| phenobarbital | glycine | ≥100mg | phenobarbital | glycine | ≥3g |
| phenoxymethylpenicill in | glycine | ≥100mg | phenoxymethylpenicill in | glycine | ≥3g |
| pravastatin sodium | glycine | ≥100mg | pravastatin sodium | glycine | ≥3g |
| prednisolone | glycine | ≥100mg | prednisolone | glycine | ≥3g |
| primaquine | glycine | ≥100mg | primaquine | glycine | ≥3g |
| procaterol | glycine | ≥100mg | procaterol | glycine | ≥3g |
| propylthiouracil | glycine | ≥100mg | propylthiouracil | glycine | ≥3g |
| pseudoephedrine | glycine | ≥100mg | pseudoephedrine | glycine | ≥3g |
| pyrazinamide | glycine | ≥100mg | pyrazinamide | glycine | ≥3g |
| pyridostigmine bromide | glycine | ≥100mg | pyridostigmine bromide | glycine | ≥3g |
| pyridoxine hydrochloride | glycine | ≥100mg | pyridoxine hydrochloride | glycine | ≥3g |
| ranitidine | glycine | ≥100mg | ranitidine | glycine | ≥3g |
| ribavirin | glycine | ≥100mg | ribavirin | glycine | ≥3g |
| riboflavin | glycine | ≥100mg | riboflavin | glycine | ≥3g |
| rizatriptan | glycine | ≥100mg | rizatriptan | glycine | ≥3g |
| stavudine | glycine | ≥100mg | stavudine | glycine | ≥3g |
| sulfadiazine | glycine | ≥100mg | sulfadiazine | glycine | ≥3g |
| sulfamethoxazole | glycine | ≥100mg | sulfamethoxazole | glycine | ≥3g |
| sultamicillin | glycine | ≥100mg | sultamicillin | glycine | ≥3g |
| sumatriptan | glycine | ≥100mg | sumatriptan | glycine | ≥3g |
| taltirelin | glycine | ≥100mg | taltirelin | glycine | ≥3g |
| tegafur | glycine | ≥100mg | tegafur | glycine | ≥3g |
| tenofovir disoproxil | glycine | ≥100mg | tenofovir disoproxil | glycine | ≥3g |
| theophylline | glycine | ≥100mg | theophylline | glycine | ≥3g |
| thiamine | glycine | ≥100mg | thiamine | glycine | ≥3g |
| trimetazidine | glycine | ≥100mg | trimetazidine | glycine | ≥3g |
| trimethoprim | glycine | ≥100mg | trimethoprim | glycine | ≥3g |
| voglibose | glycine | ≥100mg | voglibose | glycine | ≥3g |
| zidovudine | glycine | ≥100mg | zidovudine | glycine | ≥3g |
| zolmitriptan | glycine | ≥100mg | zolmitriptan | glycine | ≥3g |
| acetylcarnitine | glycine | ≥100mg | acetylcarnitine | glycine | ≥3g |
| capecitabine | glycine | ≥100mg | capecitabine | glycine | ≥3g |
| cefaclor | glycine | ≥100mg | cefaclor | glycine | ≥3g |
| cefixime | glycine | ≥100mg | cefixime | glycine | ≥3g |
| cefmetazole | glycine | ≥100mg | cefmetazole | glycine | ≥3g |
| cefpodoxime proxetil | glycine | ≥100mg | cefpodoxime proxetil | glycine | ≥3g |
| cefroxadine | glycine | ≥100mg | cefroxadine | glycine | ≥3g |
| alfoscerate | glycine | ≥100mg | alfoscerate | glycine | ≥3g |
| cilazapril | glycine | ≥100mg | cilazapril | glycine | ≥3g |
| cimetropium bromide | glycine | ≥100mg | cimetropium bromide | glycine | ≥3g |
| diacerein | glycine | ≥100mg | diacerein | glycine | ≥3g |
| erdosteine | glycine | ≥100mg | erdosteine | glycine | ≥3g |
| famciclovir | glycine | ≥100mg | famciclovir | glycine | ≥3g |
| gemifloxacin | glycine | ≥100mg | gemifloxacin | glycine | ≥3g |
| levosulpiride | glycine | ≥100mg | levosulpiride | glycine | ≥3g |
| nabumetone | glycine | ≥100mg | nabumetone | glycine | ≥3g |
| oxiracetam | glycine | ≥100mg | oxiracetam | glycine | ≥3g |
| phendimetrazine | glycine | ≥100mg | phendimetrazine | glycine | ≥3g |
| rabeprazole | glycine | ≥100mg | rabeprazole | glycine | ≥3g |
| roxatidine acetate | glycine | ≥100mg | roxatidine acetate | glycine | ≥3g |
| tamsulosin | glycine | ≥100mg | tamsulosin | glycine | ≥3g |
| terazosin | glycine | ≥100mg | terazosin | glycine | ≥3g |
| thioctic | glycine | ≥100mg | thioctic | glycine | ≥3g |
| tosufloxacin | glycine | ≥100mg | tosufloxacin | glycine | ≥3g |
| triflusal | glycine | ≥100mg | triflusal | glycine | ≥3g |
| zaltoprofen | glycine | ≥100mg | zaltoprofen | glycine | ≥3g |
| etidronic acid | glycine | ≥100mg | etidronic acid | glycine | ≥3g |
| zoledronic acid | glycine | ≥100mg | zoledronic acid | glycine | ≥3g |
| clodronic acid | glycine | ≥100mg | clodronic acid | glycine | ≥3g |
| tiludronic acid | glycine | ≥100mg | tiludronic acid | glycine | ≥3g |
| pamidronic acid | glycine | ≥100mg | pamidronic acid | glycine | ≥3g |
| alendronic acid | glycine | ≥100mg | alendronic acid | glycine | ≥3g |
| risedronic acid | glycine | ≥100mg | risedronic acid | glycine | ≥3g |
| ibandronic acid | glycine | ≥100mg | ibandronic acid | glycine | ≥3g |
| ibandronic acid | glycine | ≥100mg | abacavir | lysine | ≥5g |
| abacavir | lysine | ≥500mg | acarbose | lysine | ≥5g |
| acarbose | lysine | ≥500mg | acetazolamide | lysine | ≥5g |
| acetazolamide | lysine | ≥500mg | acyclovir | lysine | ≥5g |
| acyclovir | lysine | ≥500mg | albuterol (salbutamol) | lysine | ≥5g |
| albuterol (salbutamol) | lysine | ≥500mg | allopurinol | lysine | ≥5g |
| allopurinol | lysine | ≥500mg | amiloride | lysine | ≥5g |
| amiloride | lysine | ≥500mg | amisulpride | lysine | ≥5g |
| amisulpride | lysine | ≥500mg | amlodipine | lysine | ≥5g |
| amlodipine | lysine | ≥500mg | amoxicillin | lysine | ≥5g |
| amoxicillin | lysine | ≥500mg | amphetamine | lysine | ≥5g |
| amphetamine | lysine | ≥500mg | atenolol | lysine | ≥5g |
| atenolol | lysine | ≥500mg | atropine | lysine | ≥5g |
| atropine | lysine | ≥500mg | azathioprine | lysine | ≥5g |
| azathioprine | lysine | ≥500mg | benserazide | lysine | ≥5g |
| benserazide | lysine | ≥500mg | benznidazole | lysine | ≥5g |
| benznidazole | lysine | ≥500mg | camostat | lysine | ≥5g |
| camostat | lysine | ≥500mg | captopril | lysine | ≥5g |
| captopril | lysine | ≥500mg | cefdinir | lysine | ≥5g |
| cefdinir | lysine | ≥500mg | cefotiam hexetil hydrochloride | lysine | ≥5g |
| cefotiam hexetil hydrochloride | lysine | ≥500mg | cefprozil | lysine | ≥5g |
| cefprozil | lysine | ≥500mg | cefuroxime axetil | lysine | ≥5g |
| cefuroxime axetil | lysine | ≥500mg | chloramphenicol | lysine | ≥5g |
| chloramphenicol | lysine | ≥500mg | cimetidine | lysine | ≥5g |
| cimetidine | lysine | ≥500mg | ciprofloxacin | lysine | ≥5g |
| ciprofloxacin | lysine | ≥500mg | codeine | lysine | ≥5g |
| codeine | lysine | ≥500mg | colchicine | lysine | ≥5g |
| colchicines | lysine | ≥500mg | cyclophosphamide | lysine | ≥5g |
| cyclophosphamide | lysine | ≥500mg | dapsone | lysine | ≥5g |
| dapsone | lysine | ≥500mg. | dexamethasone | lysine | ≥5g |
| dexamethasone | lysine | ≥500mg | didanosine | lysine | ≥5g |
| didanosine | lysine | ≥500mg | diethylcarbamazine | lysine | ≥5g |
| diethylcarbamazine | lysine | ≥500mg | methionine | lysine | ≥5g |
| methionine | lysine | ≥500mg | dolasetron | lysine | ≥5g |
| dolasetron | lysine | ≥500mg | doxifluridine | lysine | ≥5g |
| doxifluridine | lysine | ≥500mg | doxycycline | lysine | ≥5g |
| doxycycline | lysine | ≥500mg | ergonovine | lysine | ≥5g |
| ergonovine | lysine | ≥500mg | erythromycin ethylsuccinate | lysine | ≥5g |
| erythromycin ethylsuccinate | lysine | ≥500mg | ethambutol | lysine | ≥5g |
| ethambutol | lysine | ≥500mg | ethosuximide | lysine | ≥5g |
| ethosuximide | lysine | ≥500mg | famotidine | lysine | ≥5g |
| famotidine | lysine | ≥500mg | fluconazole | lysine | ≥5g |
| fluconazole | lysine | ≥500mg | folic acid | lysine | ≥5g |
| folic acid | lysine | ≥500mg | furosemide | lysine | >5g |
| furosemide | lysine | ≥500mg | fursultiamine | lysine | ≥5g |
| fursultiamine | lysine | ≥500mg | gabapentin | lysine | ≥5g |
| gabapentin | lysine | ≥500mg | glipizide | lysine | ≥5g |
| glipizide | lysine | ≥500mg | granisetron | lysine | ≥5g |
| granisetron | lysine | ≥500mg | griseofulvin | lysine | ≥5g |
| griseofulvin | lysine | ≥500mg | hydralazine | lysine | ≥5g |
| hydralazine | lysine | ≥500mg | hydrochlorothiazide | lysine | ≥5g |
| hydrochlorothiazide | lysine | ≥500mg | imidapril | lysine | ≥5g |
| imidapril | lysine | ≥500mg | isoniazid | lysine | ≥5g |
| isoniazid | lysine | ≥500mg | lamivudine | lysine | ≥5g |
| lamivudine | lysine | ≥500mg | 1-carbocysteine | lysine | ≥5g |
| 1-carbocysteine | lysine | ≥500mg | levetiracetam | lysine | ≥5g |
| levetiracetam | lysine | ≥500mg | levofloxacin | lysine | ≥5g |
| levofloxacin | lysine | ≥500mg | linezolid | lysine | ≥5g |
| linezolid | lysine | ≥500mg | lisinopril | lysine | ≥5g |
| lisinopril | lysine | ≥500mg | losartan | lysine | ≥5g |
| losartan | lysine | ≥500mg | methotrexate | lysine | ≥5g |
| methotrexate | lysine | ≥500mg | methyldopa | lysine | ≥5g |
| methyldopa | lysine | ≥500mg | s-methylmethionine | lysine | ≥5g |
| s-methylmethionine | lysine | ≥500mg | metoclopramide | lysine | ≥5g |
| metoclopramide | lysine | ≥500mg | metronidazole | lysine | ≥5g |
| metronidazole | lysine | ≥500mg | moxifloxacin | lysine | ≥5g |
| moxifloxacin | lysine | ≥500mg | nalidixic acid | lysine | ≥5g |
| nalidixic acid | lysine | ≥500mg | nicorandil | lysine | ≥5g |
| nicorandil | lysine | ≥500mg | nifurtimox | lysine | ≥5g |
| nifurtimox | lysine | ≥500mg | nitrofurantoin | lysine | ≥5g |
| nitrofurantoin | lysine | ≥500mg | nizatidine | lysine | ≥5g |
| nizatidine | lysine | ≥500mg | nystatin | lysine | ≥5g |
| nystatin | lysine | ≥500mg | ondansetron | lysine | ≥5g |
| ondansetron | lysine | ≥500mg | oseltamivir | lysine | ≥5g |
| oseltamivir | lysine | ≥500mg | oxcarbazepine | lysine | ≥5g |
| oxcarbazepine | lysine | ≥500mg | penicillamine | lysine | ≥5g |
| penicillamine | lysine | ≥500mg | perindopril | lysine | ≥5g |
| perindopril | lysine | ≥500mg | phenobarbital | lysine | ≥5g |
| phenobarbital | lysine | ≥500mg | phenoxymethylpenicill in | lysine | ≥5g |
| phenoxymethylpenicill in | lysine | ≥500mg | pravastatin sodium | lysine | ≥5g |
| pravastatin sodium | lysine | ≥500mg | prednisolone | lysine | ≥5g |
| prednisolone | lysine | ≥500mg | primaquine | lysine | ≥5g |
| primaquine | lysine | ≥500mg | procaterol | lysine | ≥5g |
| procaterol | lysine | ≥500mg | propylthiouracil | lysine | ≥5g |
| propylthiouracil | lysine | ≥500mg | pseudoephedrine | lysine | ≥5g |
| pseudoephedrine | lysine | ≥500mg | pyrazinamide | lysine | ≥5g |
| pyrazinamide | lysine | ≥500mg | pyridostigmine bromide | lysine | ≥5g |
| pyridostigmine bromide | lysine | ≥500mg | pyridoxine hydrochloride | lysine | ≥5g |
| pyridoxine hydrochloride | lysine | ≥500mg | ranitidine | lysine | ≥5g |
| ranitidine | lysine | ≥500mg | ribavirin | lysine | ≥5g |
| ribavirin | lysine | ≥500mg | riboflavin | lysine | ≥5g |
| riboflavin | lysine | ≥500mg | rizatriptan | lysine | ≥5g |
| rizatriptan | lysine | ≥500mg | stavudine | lysine | ≥5g |
| stavudine | lysine | ≥500mg | sulfadiazine | lysine | ≥5g |
| sulfadiazine | lysine | ≥500mg | sulfamethoxazole | lysine | ≥5g |
| sulfamethoxazole | lysine | ≥500mg | sultamicillin | lysine | ≥5g |
| sultamicillin | lysine | ≥500mg | sumatriptan | lysine | ≥5g |
| sumatriptan | lysine | ≥500mg | taltirelin | lysine | ≥5g |
| taltirelin | lysine | ≥500mg | tegafur | lysine | ≥5g |
| tegafur | lysine | ≥500mg | tenofovir disoproxil | lysine | ≥5g |
| tenofovir disoproxil | lysine | ≥500mg | theophylline | lysine | ≥5g |
| theophylline | lysine | ≥500mg | thiamine | lysine | ≥5g |
| thiamine | lysine | ≥500mg | trimetazidine | lysine | ≥5g |
| trimetazidine | lysine | ≥500mg | trimethoprim | lysine | ≥5g |
| trimethoprim | lysine | ≥500mg | voglibose | lysine | ≥5g |
| voglibose | lysine | ≥500mg | zidovudine | lysine | ≥5g |
| zidovudine | lysine | ≥500mg | zolmitriptan | lysine | ≥5g |
| zolmitriptan | lysine | ≥500mg | acetylcarnitine | lysine | ≥5g |
| acetylcarnitine | lysine | ≥500mg | capecitabine | lysine | ≥5g |
| capecitabine | lysine | ≥500mg | cefaclor | lysine | ≥5g |
| cefaclor | lysine | ≥500mg | cefixime | lysine | ≥5g |
| cefixime | lysine | ≥500mg | cefmetazole | lysine | ≥5g |
| cefmetazole | lysine | ≥500mg | cefpodoxime proxetil | lysine | ≥5g |
| cefpodoxime proxetil | lysine | ≥500mg | cefroxadine | lysine | ≥5g |
| cefroxadine | lysine | ≥500mg | alfoscerate | lysine | ≥5g |
| alfoscerate | lysine | ≥500mg | cilazapril | lysine | ≥5g |
| cilazapril | lysine | ≥500mg | cimetropium bromide | lysine | ≥5g |
| cimetropium bromide | lysine | ≥500mg | diacerein | lysine | ≥5g |
| diacerein | lysine | ≥500mg | erdosteine | lysine | ≥5g |
| erdosteine | lysine | ≥500mg | famciclovir | lysine | ≥5g |
| famciclovir | lysine | ≥500mg | gemifloxacin | lysine | ≥5g |
| gemifloxacin | lysine | ≥500mg | levosulpiride | lysine | ≥5g |
| levosulpiride | lysine | ≥500mg | nabumetone | lysine | ≥5g |
| nabumetone | lysine | ≥500mg | oxiracetam | lysine | ≥5g |
| oxiracetam | lysine | ≥500mg | phendimetrazine | lysine | ≥5g |
| phendimetrazine | lysine | ≥500mg | rabeprazole | lysine | ≥5g |
| rabeprazole | lysine | ≥500mg | roxatidine acetate | lysine | ≥5g |
| roxatidine acetate | lysine | ≥500mg | tamsulosin | lysine | ≥5g |
| tamsulosin | lysine | ≥500mg | terazosin | lysine | ≥5g |
| terazosin | lysine | ≥500mg | thioctic | lysine | ≥5g |
| thioctic | lysine | ≥500mg | tosufloxacin | lysine | ≥5g |
| tosufloxacin | lysine | ≥500mg | triflusal | lysine | ≥5g |
| triflusal | lysine | ≥500mg | zaltoprofen | lysine | ≥5g |
| zaltoprofen | lysine | ≥500mg | etidronic acid | lysine | ≥5g |
| etidronic acid | lysine | ≥500mg | zoledronic acid | lysine | ≥5g |
| zoledronic acid | lysine | ≥500mg | clodronic acid | lysine | ≥5g |
| clodronic acid | lysine | ≥500mg | tiludronic acid | lysine | ≥5g |
| tiludronic acid | lysine | ≥500mg | pamidronic acid | lysine | ≥5g |
| pamidronic acid | lysine | ≥500mg | alendronic acid | lysine | ≥5g |
| alendronic acid | lysine | ≥500mg | risedronic acid | lysine | ≥5g |
| risedronic acid | lysine | ≥500mg | ibandronic acid | lysine | ≥5g |
| ibandronic acid | lysine | ≥500mg | abacavir | glycine | ≥5g |
| abacavir | glycine | ≥500mg | acarbose | glycine | ≥5g |
| acarbose | glycine | ≥500mg | acetazolamide | glycine | ≥5g |
| acetazolamide | glycine | ≥500mg | acyclovir | glycine | ≥5g |
| acyclovir | glycine | ≥500mg | albuterol (salbutamol) | glycine | ≥5g |
| albuterol (salbutamol) | glycine | ≥500mg | allopurinol | glycine | ≥5g |
| allopurinol | glycine | ≥500mg | amiloride | glycine | ≥5g |
| amiloride | glycine | ≥500mg | amisulpride | glycine | ≥5g |
| amisulpride | glycine | ≥500mg | amlodipine | glycine | ≥5g |
| amlodipine | glycine | ≥500mg | amoxicillin | glycine | ≥5g |
| amoxicillin | glycine | ≥500mg | amphetamine | glycine | ≥5g |
| amphetamine | glycine | ≥500mg | atenolol | glycine | ≥5g |
| atenolol | glycine | ≥500mg | atropine | glycine | ≥5g |
| atropine | glycine | ≥500mg | azathioprine | glycine | ≥5g |
| azathioprine | glycine | ≥500mg | benserazide | glycine | ≥5g |
| benserazide | glycine | ≥500mg | benznidazole | glycine | ≥5g |
| benznidazole | glycine | ≥500mg | camostat | glycine | ≥5g |
| camostat | glycine | ≥500mg | captopril | glycine | ≥5g |
| captopril | glycine | ≥500mg | cefdinir | glycine | ≥5g |
| cefdinir | glycine | ≥500mg | cefotiam hexetil hydrochloride | glycine | ≥5g |
| cefotiam hexetil hydrochloride | glycine | ≥500mg | cefprozil | glycine | ≥5g |
| cefprozil | glycine | ≥500mg | cefuroxime axetil | glycine | ≥5g |
| cefuroxime axetil | glycine | ≥500mg | chloramphenicol | glycine | ≥5g |
| chloramphenicol | glycine | ≥500mg | cimetidine | glycine | ≥5g |
| cimetidine | glycine | ≥500mg | ciprofloxacin | glycine | ≥5g |
| ciprofloxacin | glycine | ≥500mg | codeine | glycine | ≥5g |
| codeine | glycine | ≥500mg | colchicine | glycine | ≥5g |
| colchicines | glycine | ≥500mg | cyclophosphamide | glycine | ≥5g |
| cyclophosphamide | glycine | ≥500mg | dapsone | glycine | ≥5g |
| dapsone | glycine | ≥500mg | dexamethasone | glycine | ≥5g |
| dexamethasone | glycine | ≥500mg | didanosine | glycine | ≥5g |
| didanosine | glycine | ≥500mg | diethylcarbamazine | glycine | ≥5g |
| diethylcarbamazine | glycine | ≥500mg | methionine | glycine | ≥5g |
| methionine | glycine | ≥500mg | dolasetron | glycine | ≥5g |
| dolasetron | glycine | ≥500mg | doxifluridine | glycine | ≥5g |
| doxifluridine | glycine | ≥500mg | doxycycline | glycine | ≥5g |
| doxycycline | glycine | ≥500mg | ergonovine | glycine | ≥5g |
| ergonovine | glycine | ≥500mg | erythromycin ethylsuccinate | glycine | ≥5g |
| erythromycin ethylsuccinate | glycine | ≥500mg | ethambutol | glycine | ≥5g |
| ethambutol | glycine | ≥500mg | ethosuximide | glycine | ≥5g |
| ethosuximide | glycine | ≥500mg | famotidine | glycine | ≥5g |
| famotidine | glycine | ≥500mg | fluconazole | glycine | ≥5g |
| fluconazole | glycine | ≥500mg | folic acid | glycine | ≥5g |
| folic acid | glycine | ≥500mg | furosemide | glycine | ≥5g |
| furosemide | glycine | ≥500mg | fursultiamine | glycine | ≥5g |
| fursultiamine | glycine | ≥500mg | gabapentin | glycine | ≥5g |
| gabapentin | glycine | ≥500mg | glipizide | glycine | ≥5g |
| glipizide | glycine | ≥500mg | granisetron | glycine | ≥5g |
| granisetron | glycine | ≥500mg | griseofulvin | glycine | ≥5g |
| griseofulvin | glycine | ≥500mg | hydralazine | glycine | ≥5g |
| hydralazine | glycine | ≥500mg | hydrochlorothiazide | glycine | ≥5g |
| hydrochlorothiazide | glycine | ≥500mg | imidapril | glycine | ≥5g |
| imidapril | glycine | ≥500mg | isoniazid | glycine | ≥5g |
| isoniazid | glycine | ≥500mg | lamivudine | glycine | ≥5g |
| lamivudine | glycine | ≥500mg | 1-carbocysteine | glycine | ≥5g |
| 1-carbocysteine | glycine | ≥500mg | levetiracetam | glycine | ≥5g |
| levetiracetam | glycine | ≥500mg | levofloxacin | glycine | ≥5g |
| levofloxacin | glycine | ≥500mg | linezolid | glycine | ≥5g |
| linezolid | glycine | ≥500mg | lisinopril | glycine | ≥5g |
| lisinopril | glycine | ≥500mg | losartan | glycine | ≥5g |
| losartan | glycine | ≥500mg | methotrexate | glycine | ≥5g |
| methotrexate | glycine | ≥500mg | methyldopa | glycine | ≥5g |
| methyldopa | glycine | ≥500mg | s-methylmethionine | glycine | ≥5g |
| s-methylmethionine | glycine | ≥500mg | metoclopramide | glycine | ≥5g |
| metoclopramide | glycine | ≥500mg | metronidazole | glycine | ≥5g |
| metronidazole | glycine | ≥500mg | moxifloxacin | glycine | ≥5g |
| moxifloxacin | glycine | ≥500mg | nalidixic acid | glycine | ≥5g |
| nalidixic acid | glycine | ≥500mg | nicorandil | glycine | ≥5g |
| nicorandil | glycine | ≥500mg | nifurtimox | glycine | ≥5g |
| nifurtimox | glycine | ≥500mg | nitrofurantoin | glycine | ≥5g |
| nitrofurantoin | glycine | ≥500mg | nizatidine | glycine | ≥5g |
| nizatidine | glycine | ≥500mg | nystatin | glycine | ≥5g |
| nystatin | glycine | ≥500mg | ondansetron | glycine | ≥5g |
| ondansetron | glycine | ≥500mg | oseltamivir | glycine | ≥5g |
| oseltamivir | glycine | ≥500mg | oxcarbazepine | glycine | ≥5g |
| oxcarbazepine | glycine | ≥500mg | penicillamine | glycine | ≥5g |
| penicillamine | glycine | ≥500mg | perindopril | glycine | ≥5g |
| perindopril | glycine | ≥500mg | phenobarbital | glycine | ≥5g |
| phenobarbital | glycine | ≥500mg | phenoxymethylpenicillin | glycine | ≥5g |
| phenoxymethylpenicillin | glycine | ≥500mg | pravastatin sodium | glycine | ≥5g |
| pravastatin sodium | glycine | ≥500mg | prednisolone | glycine | ≥5g |
| prednisolone | glycine | ≥500mg | primaquine | glycine | ≥5g |
| primaquine | glycine | ≥500mg | procaterol | glycine | ≥5g |
| procaterol | glycine | ≥500mg | propylthiouracil | glycine | ≥5g |
| propylthiouracil | glycine | ≥500mg | pseudoephedrine | glycine | ≥5g |
| pseudoephedrine | glycine | ≥500mg | pyrazinamide | glycine | ≥5g |
| pyrazinamide | glycine | ≥500mg | pyridostigmine bromide | glycine | ≥5g |
| pyridostigmine bromide | glycine | ≥500mg | pyridoxine hydrochloride | glycine | ≥5g |
| pyridoxine hydrochloride | glycine | ≥500mg | ranitidine | glycine | ≥5g |
| ranitidine | glycine | ≥500mg | ribavirin | glycine | ≥5g |
| ribavirin | glycine | ≥500mg | riboflavin | glycine | ≥5g |
| riboflavin | glycine | ≥500mg | rizatriptan | glycine | ≥5g |
| rizatriptan | glycine | ≥500mg | stavudine | glycine | ≥5g |
| stavudine | glycine | ≥500mg | sulfadiazine | glycine | ≥5g |
| sulfadiazine | glycine | ≥500mg | sulfamethoxazole | glycine | ≥5g |
| sulfamethoxazole | glycine | ≥500mg | sultamicillin | glycine | ≥5g |
| sultamicillin | glycine | ≥500mg | sumatriptan | glycine | ≥5g |
| sumatriptan | glycine | ≥500mg | taltirelin | glycine | ≥5g |
| taltirelin | glycine | ≥500mg | tegafur | glycine | ≥5g |
| tegafur | glycine | ≥500mg | tenofovir disoproxil | glycine | ≥5g |
| tenofovir disoproxil | glycine | ≥500mg | theophylline | glycine | ≥5g |
| theophylline | glycine | ≥500mg | thiamine | glycine | ≥5g |
| thiamine | glycine | ≥500mg | trimetazidine | glycine | ≥5g |
| trimetazidine | glycine | ≥500mg | trimethoprim | glycine | ≥5g |
| trimethoprim | glycine | ≥500mg | voglibose | glycine | ≥5g |
| voglibose | glycine | ≥500mg | zidovudine | glycine | ≥5g |
| zidovudine | glycine | ≥500mg | zolmitriptan | glycine | ≥5g |
| zolmitriptan | glycine | ≥500mg | acetylcarnitine | glycine | ≥5g |
| acetylcarnitine | glycine | ≥500mg | capecitabine | glycine | ≥5g |
| capecitabine | glycine | ≥500mg | cefaclor | glycine | ≥5g |
| cefaclor | glycine | ≥500mg | cefixime | glycine | ≥5g |
| cefixime | glycine | ≥500mg | cefmetazole | glycine | ≥5g |
| cefmetazole | glycine | ≥500mg | cefpodoxime proxetil | glycine | ≥5g |
| cefpodoxime proxetil | glycine | ≥500mg | cefroxadine | glycine | ≥5g |
| cefroxadine | glycine | ≥500mg | alfoscerate | glycine | ≥5g |
| alfoscerate | glycine | ≥500mg | cilazapril | glycine | ≥5g |
| cilazapril | glycine | ≥500mg | cimetropium bromide | glycine | ≥5g |
| cimetropium bromide | glycine | ≥500mg | diacerein | glycine | ≥5g |
| diacerein | glycine | ≥500mg | erdosteine | glycine | ≥5g |
| erdosteine | glycine | ≥500mg | famciclovir | glycine | ≥5g |
| famciclovir | glycine | ≥500mg | gemifloxacin | glycine | ≥5g |
| gemifloxacin | glycine | ≥500mg | levosulpiride | glycine | ≥5g |
| levosulpiride | glycine | ≥500mg | nabumetone | glycine | ≥5g |
| nabumetone | glycine | ≥500mg | oxiracetam | glycine | ≥5g |
| oxiracetam | glycine | ≥500mg | phendimetrazine | glycine | ≥5g |
| phendimetrazine | glycine | ≥500mg | rabeprazole | glycine | ≥5g |
| rabeprazole | glycine | ≥500mg | roxatidine acetate | glycine | ≥5g |
| roxatidine acetate | glycine | ≥500mg | tamsulosin | glycine | ≥5g |
| tamsulosin | glycine | ≥500mg | terazosin | glycine | ≥5g |
| terazosin | glycine | ≥500mg | thioctic | glycine | ≥5g |
| thioctic | glycine | ≥500mg | tosufloxacin | glycine | ≥5g |
| tosufloxacin | glycine | ≥500mg | triflusal | glycine | ≥5g |
| triflusal | glycine | ≥500mg | zaltoprofen | glycine | ≥5g |
| zaltoprofen | glycine | ≥500mg | etidronic acid | glycine | ≥5g |
| etidronic acid | glycine | ≥500mg | zoledronic acid | glycine | ≥5g |
| zoledronic acid | glycine | ≥500mg | clodronic acid | glycine | ≥5g |
| clodronic acid | glycine | ≥500mg | tiludronic acid | glycine | ≥5g |
| tiludronic acid | glycine | ≥500mg | pamidronic acid | glycine | ≥5g |
| pamidronic acid | glycine | ≥500mg | alendronic acid | glycine | ≥5g |
| alendronic acid | glycine | ≥500mg | risedronic acid | glycine | ≥5g |
| risedronic acid | glycine | ≥500mg | ibandronic acid | glycine | ≥5g |
| ibandronic acid | glycine | ≥500mg | abacavir | lysine | ≥7.5g |
| ibandronic acid | glycine | ≥500mg | acarbose | lysine | ≥7.5g |
| abacavir | lysine | ≥1.25g | acetazolamide | lysine | ≥7.5g |
| acarbose | lysine | ≥1.25g | acyclovir | lysine | ≥7.5g |
| acetazolamide | lysine | ≥1.25g | albuterol (salbutamol) | lysine | ≥7.5g |
| acyclovir | lysine | ≥1.25g | allopurinol | lysine | ≥7.5g |
| albuterol (salbutamol) | lysine | ≥1.25g | amiloride | lysine | ≥7.5g |
| allopurinol | lysine | ≥1.25g | amisulpride | lysine | ≥7.5g |
| amiloride | lysine | ≥1.25g | amlodipine | lysine | ≥7.5g |
| amisulpride | lysine | ≥1.25g | amoxicillin | lysine | ≥7.5g |
| amlodipine | lysine | ≥1.25g | amphetamine | lysine | ≥7.5g |
| amoxicillin | lysine | ≥1.25g | atenolol | lysine | ≥7.5g |
| amphetamine | lysine | ≥1.25g | atropine | lysine | ≥7.5g |
| atenolol | lysine | ≥1.25g | azathioprine | lysine | ≥7.5g |
| atropine | lysine | ≥1.25g | benserazide | lysine | ≥7.5g |
| azathioprine | lysine | ≥1.25g | benznidazole | lysine | ≥7.5g |
| benserazide | lysine | ≥1.25g | camostat | lysine | ≥7.5g |
| benznidazole | lysine | ≥1.25g | captopril | lysine | ≥7.5g |
| camostat | lysine | ≥1.25g | cefdinir | lysine | ≥7.5g |
| captopril | lysine | ≥1.25g | cefotiam hexetil hydrochloride | lysine | ≥7.5g |
| cefdinir | lysine | ≥1.25g | cefprozil | lysine | ≥7.5g |
| cefotiam hexetil hydrochloride | lysine | ≥1.25g | cefuroxime axetil | lysine | ≥7.5g |
| cefprozil | lysine | ≥1.25g | chloramphenicol | lysine | ≥7.5g |
| cefuroxime axetil | lysine | ≥1.25g | cimetidine | lysine | ≥7.5g |
| chloramphenicol | lysine | ≥1.25g | ciprofloxacin | lysine | ≥7.5g |
| cimetidine | lysine | ≥1.25g | codeine | lysine | ≥7.5g |
| ciprofloxacin | lysine | ≥1.25g | colchicine | lysine | ≥7.5g |
| codeine | lysine | ≥1.25g | cyclophosphamide | lysine | ≥7.5g |
| colchicine | lysine | ≥1.25g | dapsone | lysine | ≥7.5g |
| cyclophosphamide | lysine | ≥1.25g | dexamethasone | lysine | ≥7.5g |
| dapsone | lysine | ≥1.25g | didanosine | lysine | ≥7.5g |
| dexamethasone | lysine | ≥1.25g | diethylcarbamazine | lysine | ≥7.5g |
| didanosine | lysine | ≥1.25g | methionine | lysine | ≥7.5g |
| diethylcarbamazine | lysine | ≥1.25g | dolasetron | lysine | ≥7.5g |
| methionine | lysine | ≥1.25g | doxifluridine | lysine | ≥7.5g |
| dolasetron | lysine | ≥1.25g | doxycycline | lysine | ≥7.5g |
| doxifluridine | lysine | ≥1.25g | ergonovine | lysine | ≥7.5g |
| doxycycline | lysine | ≥1.25g | erythromycin ethylsuccinate | lysine | ≥7.5g |
| ergonovine | lysine | ≥1.25g | ethambutol | lysine | ≥7.5g |
| erythromycin ethylsuccinate | lysine | ≥1.25g | ethosuximide | lysine | ≥7.5g |
| ethambutol | lysine | ≥1.25g | famotidine | lysine | ≥7.5g |
| ethosuximide | lysine | ≥1.25g | fluconazole | lysine | ≥7.5g |
| famotidine | lysine | ≥1.25g | folic acid | lysine | ≥7.5g |
| fluconazole | lysine | ≥1.25g | furosemide | lysine | ≥7.5g |
| folic acid | lysine | ≥1.25g | fursultiamine | lysine | ≥7.5g |
| furosemide | lysine | ≥1.25g | gabapentin | lysine | ≥7.5g |
| fursultiamine | lysine | ≥1.25g | glipizide | lysine | ≥7.5g |
| gabapentin | lysine | ≥1.25g | granisetron | lysine | ≥7.5g |
| glipizide | lysine | ≥1.25g | griseofulvin | lysine | ≥7.5g |
| granisetron | lysine | ≥1.25g | hydralazine | lysine | ≥7.5g |
| griseofulvin | lysine | ≥1.25g | hydrochlorothiazide | lysine | ≥7.5g |
| hydralazine | lysine | ≥1.25g | imidapril | lysine | ≥7.5g |
| hydrochlorothiazide | lysine | ≥1.25g | isoniazid | lysine | ≥7.5g |
| imidapril | lysine | ≥1.25g | lamivudine | lysine | ≥7.5g |
| isoniazid | lysine | ≥1.25g | 1-carbocysteine | lysine | ≥7.5g |
| lamivudine | lysine | ≥1.25g | levetiracetam | lysine | ≥7.5g |
| 1-carbocysteine | lysine | ≥1.25g | levofloxacin | lysine | ≥7.5g |
| levetiracetam | lysine | ≥1.25g | linezolid | lysine | ≥7.5g |
| levofloxacin | lysine | ≥1.25g | lisinopril | lysine | ≥7.5g |
| linezolid | lysine | ≥1.25g | losartan | lysine | ≥7.5g |
| lisinopril | lysine | ≥1.25g | methotrexate | lysine | ≥7.5g |
| losartan | lysine | ≥1.25g | methyldopa | lysine | ≥7.5g |
| methotrexate | lysine | ≥1.25g | s-methylmethionine | lysine | ≥7.5g |
| methyldopa | lysine | ≥1.25g | metoclopramide | lysine | ≥7.5g |
| s-methylmethionine | lysine | ≥1.25g | metronidazole | lysine | ≥7.5g |
| metoclopramide | lysine | ≥1.25g | moxifloxacin | lysine | ≥7.5g |
| metronidazole | lysine | ≥1.25g | nalidixic acid | lysine | ≥7.5g |
| moxifloxacin | lysine | ≥1.25g | nicorandil | lysine | ≥7.5g |
| nalidixic acid | lysine | ≥1.25g | nifurtimox | lysine | ≥7.5g |
| nicorandil | lysine | ≥1.25g | nitrofurantoin | lysine | ≥7.5g |
| nifurtimox | lysine | ≥1.25g | nizatidine | lysine | ≥7.5g |
| nitrofurantoin | lysine | ≥1.25g | nystatin | lysine | ≥7.5g |
| nizatidine | lysine | ≥1.25g | ondansetron | lysine | ≥7.5g |
| nystatin | lysine | ≥1.25g | oseltamivir | lysine | ≥7.5g |
| ondansetron | lysine | ≥1.25g | oxcarbazepine | lysine | ≥7.5g |
| oseltamivir | lysine | ≥1.25g | penicillamine | lysine | ≥7.5g |
| oxcarbazepine | lysine | ≥1.25g | perindopril | lysine | ≥7.5g |
| penicillamine | lysine | ≥1.25g | phenobarbital | lysine | ≥7.5g |
| perindopril | lysine | ≥1.25g | phenoxymethylpenicillin | lysine | ≥7.5g |
| phenobarbital | lysine | ≥1.25g | pravastatin sodium | lysine | ≥7.5g |
| phenoxymethylpenicill in | lysine | ≥1.25g | prednisolone | lysine | ≥7.5g |
| pravastatin sodium | lysine | ≥1.25g | primaquine | lysine | ≥7.5g |
| prednisolone | lysine | ≥1.25g | procaterol | lysine | ≥7.5g |
| primaquine | lysine | ≥1.25g | propylthiouracil | lysine | ≥7.5g |
| procaterol | lysine | ≥1.25g | pseudoephedrine | lysine | ≥7.5g |
| propylthiouracil | lysine | ≥1.25g | pyrazinamide | lysine | ≥7.5g |
| pseudoephedrine | lysine | ≥1.25g | pyridostigmine bromide | lysine | ≥7.5g |
| pyrazinamide | lysine | ≥1.25g | pyridoxine hydrochloride | lysine | ≥7.5g |
| pyridostigmine bromide | lysine | ≥1.25g | ranitidine | lysine | ≥7.5g |
| pyridoxine hydrochloride | lysine | ≥1.25g | ribavirin | lysine | ≥7.5g |
| ranitidine | lysine | ≥1.25g | riboflavin | lysine | ≥7.5g |
| ribavirin | lysine | ≥1.25g | rizatriptan | lysine | ≥7.5g |
| riboflavin | lysine | ≥1.25g | stavudine | lysine | ≥7.5g |
| rizatriptan | lysine | ≥1.25g | sulfadiazine | lysine | ≥7.5g |
| stavudine | lysine | ≥1.25g | sulfamethoxazole | lysine | ≥7.5g |
| sulfadiazine | lysine | ≥1.25g | sultamicillin | lysine | ≥7.5g |
| sulfamethoxazole | lysine | ≥1.25g | sumatriptan | lysine | ≥7.5g |
| sultamicillin | lysine | ≥1.25g | taltirelin | lysine | ≥7.5g |
| sumatriptan | lysine | ≥1.25g | tegafur | lysine | ≥7.5g |
| taltirelin | lysine | ≥1.25g | tenofovir disoproxil | lysine | ≥7.5g |
| tegafur | lysine | ≥1.25g | theophylline | lysine | ≥7.5g |
| tenofovir disoproxil | lysine | ≥1.25g | thiamine | lysine | ≥7.5g |
| theophylline | lysine | ≥1.25g | trimetazidine | lysine | ≥7.5g |
| thiamine | lysine | ≥1.25g | trimethoprim | lysine | ≥7.5g |
| trimetazidine | lysine | ≥1.25g | voglibose | lysine | ≥7.5g |
| trimethoprim | lysine | ≥1.25g | zidovudine | lysine | ≥7.5g |
| voglibose | lysine | ≥1.25g | zolmitriptan | lysine | ≥7.5g |
| zidovudine | lysine | ≥1.25g | acetylcarnitine | lysine | ≥7.5g |
| zolmitriptan | lysine | ≥1.25g | capecitabine | lysine | ≥7.5g |
| acetylcarnitine | lysine | ≥1.25g | cefaclor | lysine | ≥7.5g |
| capecitabine | lysine | ≥1.25g | cefixime | lysine | ≥7.5g |
| cefaclor | lysine | ≥1.25g | cefmetazole | lysine | ≥7.5g |
| cefixime | lysine | ≥1.25g | cefpodoxime proxetil | lysine | ≥7.5g |
| cefmetazole | lysine | ≥1.25g | cefroxadine | lysine | ≥7.5g |
| cefpodoxime proxetil | lysine | ≥1.25g | alfoscerate | lysine | ≥7.5g |
| cefroxadine | lysine | ≥1.25g | cilazapril | lysine | ≥7.5g |
| alfoscerate | lysine | ≥1.25g | cimetropium bromide | lysine | ≥7.5g |
| cilazapril | lysine | ≥1.25g | diacerein | lysine | ≥7.5g |
| cimetropium bromide | lysine | ≥1.25g | erdosteine | lysine | ≥7.5g |
| diacerein | lysine | ≥1.25g | famciclovir | lysine | ≥7.5g |
| erdosteine | lysine | ≥1.25g | gemifloxacin | lysine | ≥7.5g |
| famciclovir | lysine | ≥1.25g | levosulpiride | lysine | ≥7.5g |
| gemifloxacin | lysine | ≥1.25g | nabumetone | lysine | ≥7.5g |
| levosulpiride | lysine | ≥1.25g | oxiracetam | lysine | ≥7.5g |
| nabumetone | lysine | ≥1.25g | phendimetrazine | lysine | ≥7.5g |
| oxiracetam | lysine | ≥1.25g | rabeprazole | lysine | ≥7.5g |
| phendimetrazine | lysine | ≥1.25g | roxatidine acetate | lysine | ≥7.5g |
| rabeprazole | lysine | ≥1.25g | tamsulosin | lysine | ≥7.5g |
| roxatidine acetate | lysine | ≥1.25g | terazosin | lysine | ≥7.5g |
| tamsulosin | lysine | ≥1.25g | thioctic | lysine | ≥7.5g |
| terazosin | lysine | ≥1.25g | tosufloxacin | lysine | ≥7.5g |
| thioctic | lysine | ≥1.25g | triflusal | lysine | ≥7.5g |
| tosufloxacin | lysine | ≥1.25g | zaltoprofen | lysine | ≥7.5g |
| triflusal | lysine | ≥1.25g | etidronic acid | lysine | ≥7.5g |
| zaltoprofen | lysine | ≥1.25g | zoledronic acid | lysine | ≥7.5g |
| etidronic acid | lysine | ≥1.25g | clodronic acid | lysine | ≥7.5g |
| zoledronic acid | lysine | ≥1.25g | tiludronic acid | lysine | ≥7.5g |
| clodronic acid | lysine | ≥1.25g | pamidronic acid | lysine | ≥7.5g |
| tiludronic acid | lysine | ≥1.25g | alendronic acid | lysine | ≥7.5g |
| pamidronic acid | lysine | ≥1.25g | risedronic acid | lysine | ≥7.5g |
| alendronic acid | lysine | ≥1.25g | ibandronic acid | lysine | ≥7.5g |
| risedronic acid | lysine | ≥1.25g | abacavir | glycine | ≥7.5g |
| ibandronic acid | lysine | ≥1.25g | acarbose | glycine | ≥7.5g |
| abacavir | glycine | ≥1.25g | acetazolamide | glycine | ≥7.5g |
| acarbose | glycine | ≥1.25g | acyclovir | glycine | ≥7.5g |
| acetazolamide | glycine | ≥1.25g | albuterol (salbutamol) | glycine | ≥7.5g |
| acyclovir | glycine | ≥1.25g | allopurinol | glycine | ≥7.5g |
| albuterol (salbutamol) | glycine | ≥1.25g | amiloride | glycine | ≥7.5g |
| allopurinol | glycine | ≥1.25g | amisulpride | glycine | ≥7.5g |
| amiloride | glycine | ≥1.25g | amlodipine | glycine | ≥7.5g |
| amisulpride | glycine | ≥1.25g | amoxicillin | glycine | ≥7.5g |
| amlodipine | glycine | ≥1.25g | amphetamine | glycine | ≥7.5g |
| amoxicillin | glycine | ≥1.25g | atenolol | glycine | ≥7.5g |
| amphetamine | glycine | ≥1.25g | atropine | glycine | ≥7.5g |
| atenolol | glycine | ≥1.25g | azathioprine | glycine | ≥7.5g |
| atropine | glycine | ≥1.25g | benserazide | glycine | ≥7.5g |
| azathioprine | glycine | ≥1.25g | benznidazole | glycine | ≥7.5g |
| benserazide | glycine | ≥1.25g | camostat | glycine | ≥7.5g |
| benznidazole | glycine | ≥1.25g | captopril | glycine | ≥7.5g |
| camostat | glycine | ≥1.25g | cefdinir | glycine | ≥7.5g |
| captopril | glycine | ≥1.25g | cefotiam hexetil hydrochloride | glycine | ≥7.5g |
| cefdinir | glycine | ≥1.25g | cefprozil | glycine | ≥7.5g |
| cefotiam hexetil hydrochloride | glycine | ≥1.25g | cefuroxime axetil | glycine | ≥7.5g |
| cefprozil | glycine | ≥1.25g | chloramphenicol | glycine | ≥7.5g |
| cefuroxime axetil | glycine | ≥1.25g | cimetidine | glycine | ≥7.5g |
| chloramphenicol | glycine | ≥1.25g | ciprofloxacin | glycine | ≥7.5g |
| cimetidine | glycine | ≥1.25g | codeine | glycine | ≥7.5g |
| ciprofloxacin | glycine | ≥1.25g | colchicine | glycine | ≥7.5g |
| codeine | glycine | ≥1.25g | cyclophosphamide | glycine | ≥7.5g |
| colchicine | glycine | ≥1.25g | dapsone | glycine | ≥7.5g |
| cyclophosphamide | glycine | ≥1.25g | dexamethasone | glycine | ≥7.5g |
| dapsone | glycine | ≥1.25g | didanosine | glycine | ≥7.5g |
| dexamethasone | glycine | ≥1.25g | diethylcarbamazine | glycine | ≥7.5g |
| didanosine | glycine | ≥1.25g | methionine | glycine | ≥7.5g |
| diethylcarbamazine | glycine | ≥1.25g | dolasetron | glycine | ≥7.5g |
| methionine | glycine | ≥1.25g | doxifluridine | glycine | ≥7.5g |
| dolasetron | glycine | ≥1.25g | doxycycline | glycine | ≥7.5g |
| doxifluridine | glycine | ≥1.25g | ergonovine | glycine | ≥7.5g |
| doxycycline | glycine | ≥1.25g | erythromycin ethylsuccinate | glycine | ≥7.5g |
| ergonovine | glycine | ≥1.25g | ethambutol | glycine | ≥7.5g |
| erythromycin ethylsuccinate | glycine | ≥1.25g | ethosuximide | glycine | ≥7.5g |
| ethambutol | glycine | ≥1.25g | famotidine | glycine | ≥7.5g |
| ethosuximide | glycine | ≥1.25g | fluconazole | glycine | ≥7.5g |
| famotidine | glycine | ≥1.25g | folic acid | glycine | ≥7.5g |
| fluconazole | glycine | ≥1.25g | furosemide | glycine | ≥7.5g |
| folic acid | glycine | ≥1.25g | fursultiamine | glycine | ≥7.5g |
| furosemide | glycine | ≥1.25g | gabapentin | glycine | ≥7.5g |
| fursultiamine | glycine | ≥1.25g | glipizide | glycine | ≥7.5g |
| gabapentin | glycine | ≥1.25g | granisetron | glycine | ≥7.5g |
| glipizide | glycine | ≥1.25g | griseofulvin | glycine | ≥7.5g |
| granisetron | glycine | ≥1.25g | hydralazine | glycine | ≥7.5g |
| griseofulvin | glycine | ≥1.25g | hydrochlorothiazide | glycine | ≥7.5g |
| hydralazine | glycine | ≥1.25g | imidapril | glycine | ≥7.5g |
| hydrochlorothiazide | glycine | ≥1.25g | isoniazid | glycine | ≥7.5g |
| imidapril | glycine | ≥1.25g | lamivudine | glycine | ≥7.5g |
| isoniazid | glycine | ≥1.25g | 1-carbocysteine | glycine | ≥7.5g |
| lamivudine | glycine | ≥1.25g | levetiracetam | glycine | ≥7.5g |
| 1-carbocysteine | glycine | ≥1.25g | levofloxacin | glycine | ≥7.5g |
| levetiracetam | glycine | ≥1.25g | linezolid | glycine | ≥7.5g |
| levofloxacin | glycine | ≥1.25g | lisinopril | glycine | ≥7.5g |
| linezolid | glycine | ≥1.25g | losartan | glycine | ≥7.5g |
| lisinopril | glycine | ≥1.25g | methotrexate | glycine | ≥7.5g |
| losartan | glycine | ≥1.25g | methyldopa | glycine | ≥7.5g |
| methotrexate | glycine | ≥1.25g | s-methylmethionine | glycine | ≥7.5g |
| methyldopa | glycine | ≥1.25g | metoclopramide | glycine | ≥7.5g |
| s-methylmethionine | glycine | ≥1.25g | metronidazole | glycine | ≥7.5g |
| metoclopramide | glycine | ≥1.25g | moxifloxacin | glycine | ≥7.5g |
| metronidazole | glycine | ≥1.25g | nalidixic acid | glycine | ≥7.5g |
| moxifloxacin | glycine | ≥1.25g | nicorandil | glycine | ≥7.5g |
| nalidixic acid | glycine | ≥1.25g | nifurtimox | glycine | ≥7.5g |
| nicorandil | glycine | ≥1.25g | nitrofurantoin | glycine | ≥7.5g |
| nifurtimox | glycine | >1.25g | nizatidine | glycine | ≥7.5g |
| nitrofurantoin | glycine | ≥1.25g | nystatin | glycine | ≥7.5g |
| nizatidine | glycine | ≥1.25g | ondansetron | glycine | ≥7.5g |
| nystatin | glycine | ≥1.25g | oseltamivir | glycine | ≥7.5g |
| ondansetron | glycine | ≥1.25g | oxcarbazepine | glycine | ≥7.5g |
| oseltamivir | glycine | ≥1.25g | penicillamine | glycine | ≥7.5g |
| oxcarbazepine | glycine | ≥1.25g | perindopril | glycine | ≥7.5g |
| penicillamine | glycine | ≥1.25g | phenobarbital | glycine | ≥7.5g |
| perindopril | glycine | ≥1.25g | phenoxymethylpenicillin | glycine | ≥7.5g |
| phenobarbital | glycine | ≥1.25g | pravastatin sodium | glycine | ≥7.5g |
| phenoxymethylpenicillin | glycine | ≥1.25g | prednisolone | glycine | ≥7.5g |
| pravastatin sodium | glycine | ≥1.25g | primaquine | glycine | ≥7.5g |
| prednisolone | glycine | ≥1.25g | procaterol | glycine | ≥7.5g |
| primaquine | glycine | ≥1.25g | propylthiouracil | glycine | ≥7.5g |
| procaterol | glycine | ≥1.25g | pseudoephedrine | glycine | ≥7.5g |
| propylthiouracil | glycine | ≥1.25g | pyrazinamide | glycine | ≥7.5g |
| pseudoephedrine | glycine | ≥1.25g | pyridostigmine bromide | glycine | ≥7.5g |
| pyrazinamide | glycine | ≥1.25g | pyridoxine hydrochloride | glycine | ≥7.5g |
| pyridostigmine bromide | glycine | ≥1.25g | ranitidine | glycine | ≥7.5g |
| pyridoxine hydrochloride | glycine | ≥1.25g | ribavirin | glycine | ≥7.5g |
| ranitidine | glycine | ≥1.25g | riboflavin | glycine | ≥7.5g |
| ribavirin | glycine | ≥1.25g | rizatriptan | glycine | ≥7.5g |
| riboflavin | glycine | ≥1.25g | stavudine | glycine | ≥7.5g |
| rizatriptan | glycine | ≥1.25g | sulfadiazine | glycine | ≥7.5g |
| stavudine | glycine | ≥1.25g | sulfamethoxazole | glycine | ≥7.5g |
| sulfadiazine | glycine | ≥1.25g | sultamicillin | glycine | ≥7.5g |
| sulfamethoxazole | glycine | ≥1.25g | sumatriptan | glycine | ≥7.5g |
| sultamicillin | glycine | ≥1.25g | taltirelin | glycine | ≥7.5g |
| sumatriptan | glycine | ≥1.25g | tegafur | glycine | ≥7.5g |
| taltirelin | glycine | ≥1.25g | tenofovir disoproxil | glycine | ≥7.5g |
| tegafur | glycine | ≥1.25g | theophylline | glycine | ≥7.5g |
| tenofovir disoproxil | glycine | ≥1.25g | thiamine | glycine | ≥7.5g |
| theophylline | glycine | ≥1.25g | trimetazidine | glycine | ≥7.5g |
| thiamine | glycine | ≥1.25g | trimethoprim | glycine | ≥7.5g |
| trimetazidine | glycine | ≥1.25g | voglibose | glycine | ≥7.5g |
| trimethoprim | glycine | ≥1.25g | zidovudine | glycine | ≥7.5g |
| voglibose | glycine | ≥1.25g | zolmitriptan | glycine | ≥7.5g |
| zidovudine | glycine | ≥1.25g | acetylcarnitine | glycine | ≥7.5g |
| zolmitriptan | glycine | ≥1.25g | capecitabine | glycine | ≥7.5g |
| acetylcarnitine | glycine | ≥1.25g | cefaclor | glycine | ≥7.5g |
| capecitabine | glycine | ≥1.25g | cefixime | glycine | ≥7.5g |
| cefaclor | glycine | ≥1.25g | cefmetazole | glycine | ≥7.5g |
| cefixime | glycine | ≥1.25g | cefpodoxime proxetil | glycine | ≥7.5g |
| cefmetazole | glycine | ≥1.25g | cefroxadine | glycine | ≥7.5g |
| cefpodoxime proxetil | glycine | ≥1.25g | alfoscerate | glycine | ≥7.5g |
| cefroxadine | glycine | ≥1.25g | cilazapril | glycine | ≥7.5g |
| alfoscerate | glycine | ≥1.25g | cimetropium bromide | glycine | ≥7.5g |
| cilazapril | glycine | ≥1.25g | diacerein | glycine | ≥7.5g |
| cimetropium bromide | glycine | ≥1.25g | erdosteine | glycine | ≥7.5g |
| diacerein | glycine | ≥1.25g | famciclovir | glycine | ≥7.5g |
| erdosteine | glycine | ≥1.25g | gemifloxacin | glycine | ≥7.5g |
| famciclovir | glycine | ≥1.25g | levosulpiride | glycine | ≥7.5g |
| gemifloxacin | glycine | ≥1.25g | nabumetone | glycine | ≥7.5g |
| levosulpiride | glycine | ≥1.25g | oxiracetam | glycine | ≥7.5g |
| nabumetone | glycine | ≥1.25g | phendimetrazine | glycine | ≥7.5g |
| oxiracetam | glycine | ≥1.25g | rabeprazole | glycine | ≥7.5g |
| phendimetrazine | glycine | ≥1.25g | roxatidine acetate | glycine | ≥7.5g |
| rabeprazole | glycine | ≥1.25g | tamsulosin | glycine | ≥7.5g |
| roxatidine acetate | glycine | ≥1.25g | terazosin | glycine | ≥7.5g |
| tamsulosin | glycine | ≥1.25g | thioctic | glycine | ≥7.5g |
| terazosin | glycine | ≥1.25g | tosufloxacin | glycine | ≥7.5g |
| thioctic | glycine | ≥1.25g | triflusal | glycine | ≥7.5g |
| tosufloxacin | glycine | ≥1.25g | zaltoprofen | glycine | ≥7.5g |
| triflusal | glycine | ≥1.25g | etidronic acid | glycine | ≥7.5g |
| zaltoprofen | glycine | ≥1.25g | zoledronic acid | glycine | ≥7.5g |
| etidronic acid | glycine | ≥1.25g | clodronic acid | glycine | ≥7.5g |
| zoledronic acid | glycine | ≥1.25g | tiludronic acid | glycine | ≥7.5g |
| clodronic acid | glycine | ≥1.25g | pamidronic acid | glycine | ≥7.5g |
| tiludronic acid | glycine | ≥1.25g | alendronic acid | glycine | ≥7.5g |
| pamidronic acid | glycine | ≥1.25g | risedronic acid | glycine | ≥7.5g |
| alendronic acid | glycine | ≥1.25g | ibandronic acid | glycine | ≥7.5g |
| risedronic acid | glycine | ≥1.25g | abacavir | lysine | ≥10g |
| ibandronic acid | glycine | ≥1.25g | acarbose | lysine | ≥10g |
| abacavir | lysine | ≥1.5g | acetazolamide | lysine | ≥10g |
| acarbose | lysine | ≥1.5g | acyclovir | lysine | ≥10g |
| acetazolamide | lysine | ≥1.5g | albuterol (salbutamol) | lysine | ≥10g |
| acyclovir | lysine | ≥1.5g | allopurinol | lysine | ≥10g |
| albuterol (salbutamol) | lysine | ≥1.5g | amiloride | lysine | ≥10g |
| allopurinol | lysine | ≥1.5g | amisulpride | lysine | ≥10g |
| amiloride | lysine | ≥1.5g | amlodipine | lysine | ≥10g |
| amisulpride | lysine | ≥1.5g | amoxicillin | lysine | ≥10g |
| amlodipine | lysine | ≥1.5g | amphetamine | lysine | ≥10g |
| amoxicillin | lysine | ≥1.5g | atenolol | lysine | ≥10g |
| amphetamine | lysine | ≥1.5g | atropine | lysine | ≥10g |
| atenolol | lysine | ≥1.5g | azathioprine | lysine | ≥10g |
| atropine | lysine | ≥1.5g | benserazide | lysine | ≥10g |
| azathioprine | lysine | ≥1.5g | benznidazole | lysine | ≥10g |
| benserazide | lysine | ≥1.5g | camostat | lysine | ≥10g |
| benznidazole | lysine | ≥1.5g | captopril | lysine | ≥10g |
| camostat | lysine | ≥1.5g | cefdinir | lysine | ≥10g |
| captopril | lysine | ≥1.5g | cefotiam hexetil hydrochloride | lysine | ≥10g |
| cefdinir | lysine | ≥1.5g | cefprozil | lysine | ≥10g |
| cefotiam hexetil hydrochloride | lysine | ≥1.5g | cefuroxime axetil | lysine | ≥10g |
| cefprozil | lysine | ≥1.5g | chloramphenicol | lysine | ≥10g |
| cefuroxime axetil | lysine | ≥1.5g | cimetidine | lysine | ≥10g |
| chloramphenicol | lysine | ≥1.5g | ciprofloxacin | lysine | ≥10g |
| cimetidine | lysine | ≥1.5g | codeine | lysine | ≥10g |
| ciprofloxacin | lysine | ≥1.5g | colchicine | lysine | ≥10g |
| codeine | lysine | ≥1.5g | cyclophosphamide | lysine | ≥10g |
| colchicine | lysine | ≥1.5g | dapsone | lysine | ≥10g |
| cyclophosphamide | lysine | ≥1.5g | dexamethasone | lysine | ≥10g |
| dapsone | lysine | ≥1.5g | didanosine | lysine | ≥10g |
| dexamethasone | lysine | ≥1.5g | diethylcarbamazine | lysine | ≥10g |
| didanosine | lysine | ≥1.5g | methionine | lysine | ≥10g |
| diethylcarbamazine | lysine | ≥1.5g | dolasetron | lysine | ≥10g |
| methionine | lysine | ≥1.5g | doxifluridine | lysine | ≥10g |
| dolasetron | lysine | ≥1.5g | doxycycline | lysine | ≥10g |
| doxifluridine | lysine | ≥1.5g | ergonovine | lysine | ≥10g |
| doxycycline | lysine | ≥1.5g | erythromycin ethylsuccinate | lysine | ≥10g |
| ergonovine | lysine | ≥1.5g | ethambutol | lysine | ≥10g |
| erythromycin ethylsuccinate | lysine | ≥1.5g | ethosuximide | lysine | ≥10g |
| ethambutol | lysine | ≥1.5g | famotidine | lysine | ≥10g |
| ethosuximide | lysine | ≥1.5g | fluconazole | lysine | ≥10g |
| famotidine | lysine | ≥1.5g | folic acid | lysine | ≥10g |
| fluconazole | lysine | ≥1.5g | furosemide | lysine | ≥10g |
| folic acid | lysine | ≥1.5g | fursultiamine | lysine | ≥10g |
| furosemide | lysine | ≥1.5g | gabapentin | lysine | ≥10g |
| fursultiamine | lysine | ≥1.5g | glipizide | lysine | ≥10g |
| gabapentin | lysine | ≥1.5g | granisetron | lysine | ≥10g |
| glipizide | lysine | ≥1.5g | griseofulvin | lysine | ≥10g |
| granisetron | lysine | ≥1.5g | hydralazine | lysine | ≥10g |
| griseofulvin | lysine | ≥1.5g | hydrochlorothiazide | lysine | ≥10g |
| hydralazine | lysine | ≥1.5g | imidapril | lysine | ≥10g |
| hydrochlorothiazide | lysine | ≥1.5g | isoniazid | lysine | ≥10g |
| imidapril | lysine | ≥1.5g | lamivudine | lysine | ≥10g |
| isoniazid | lysine | ≥1.5g | 1-carbocysteine | lysine | ≥10g |
| lamivudine | lysine | ≥1.5g | levetiracetam | lysine | ≥10g |
| 1-carbocysteine | lysine | ≥1.5g | levofloxacin | lysine | ≥10g |
| levetiracetam | lysine | ≥1.5g | linezolid | lysine | ≥10g |
| levofloxacin | lysine | ≥1.5g | lisinopril | lysine | ≥10g |
| linezolid | lysine | ≥1.5g | losartan | lysine | ≥10g |
| lisinopril | lysine | ≥1.5g | methotrexate | lysine | ≥10g |
| losartan | lysine | ≥1.5g | methyldopa | lysine | ≥10g |
| methotrexate | lysine | ≥1.5g | s-methylmethionine | lysine | ≥10g |
| methyldopa | lysine | ≥1.5g | metoclopramide | lysine | ≥10g |
| s-methylmethionine | lysine | ≥1.5g | metronidazole | lysine | ≥10g |
| metoclopramide | lysine | ≥1.5g | moxifloxacin | lysine | ≥10g |
| metronidazole | lysine | ≥1.5g | nalidixic acid | lysine | ≥10g |
| moxifloxacin | lysine | ≥1.5g | nicorandil | lysine | ≥10g |
| nalidixic acid | lysine | ≥1.5g | nifurtimox | lysine | ≥10g |
| nicorandil | lysine | ≥1.5g | nitrofurantoin | lysine | ≥10g |
| nifurtimox | lysine | ≥1.5g | nizatidine | lysine | ≥10g |
| nitrofurantoin | lysine | ≥1.5g | nystatin | lysine | ≥10g |
| nizatidine | lysine | ≥1.5g | ondansetron | lysine | ≥10g |
| nystatin | lysine | ≥1.5g | oseltamivir | lysine | ≥10g |
| ondansetron | lysine | ≥1.5g | oxcarbazepine | lysine | ≥10g |
| oseltamivir | lysine | ≥1.5g | penicillamine | lysine | ≥10g |
| oxcarbazepine | lysine | ≥1.5g | perindopril | lysine | ≥10g |
| penicillamine | lysine | ≥1.5g | phenobarbital | lysine | ≥10g |
| perindopril | lysine | ≥1.5g | phenoxymethylpenicill in | lysine | ≥10g |
| phenobarbital | lysine | ≥1.5g | pravastatin sodium | lysine | ≥10g |
| phenoxymethylpenicill in | lysine | ≥1.5g | prednisolone | lysine | ≥10g |
| pravastatin sodium | lysine | ≥1.5g | primaquine | lysine | ≥10g |
| prednisolone | lysine | ≥1.5g | procaterol | lysine | ≥10g |
| primaquine | lysine | ≥1.5g | propylthiouracil | lysine | ≥10g |
| procaterol | lysine | ≥1.5g | pseudoephedrine | lysine | ≥10g |
| propylthiouracil | lysine | ≥1.5g | pyrazinamide | lysine | ≥10g |
| pseudoephedrine | lysine | ≥1.5g | pyridostigmine bromide | lysine | ≥10g |
| pyrazinamide | lysine | ≥1.5g | pyridoxine hydrochloride | lysine | ≥10g |
| pyridostigmine bromide | lysine | ≥1.5g | ranitidine | lysine | ≥10g |
| pyridoxine hydrochloride | lysine | ≥1.5g | ribavirin | lysine | ≥10g |
| ranitidine | lysine | ≥1.5g | riboflavin | lysine | ≥10g |
| ribavirin | lysine | ≥1.5g | rizatriptan | lysine | ≥10g |
| riboflavin | lysine | ≥1.5g | stavudine | lysine | ≥10g |
| rizatriptan | lysine | ≥1.5g | sulfadiazine | lysine | ≥10g |
| stavudine | lysine | ≥1.5g | sulfamethoxazole | lysine | ≥10g |
| sulfadiazine | lysine | ≥1.5g | sultamicillin | lysine | ≥10g |
| sulfamethoxazole | lysine | ≥1.5g | sumatriptan | lysine | ≥10g |
| sultamicillin | lysine | ≥1.5g | taltirelin | lysine | ≥10g |
| sumatriptan | lysine | ≥1.5g | tegafur | lysine | ≥10g |
| taltirelin | lysine | ≥1.5g | tenofovir disoproxil | lysine | ≥10g |
| tegafur | lysine | ≥1.5g | theophylline | lysine | ≥10g |
| tenofovir disoproxil | lysine | ≥1.5g | thiamine | lysine | ≥10g |
| theophylline | lysine | ≥1.5g | trimetazidine | lysine | ≥10g |
| thiamine | lysine | ≥1.5g | trimethoprim | lysine | ≥10g |
| trimetazidine | lysine | ≥1.5g | voglibose | lysine | ≥10g |
| trimethoprim | lysine | ≥1.5g | zidovudine | lysine | ≥10g |
| voglibose | lysine | ≥1.5g | zolmitriptan | lysine | ≥10g |
| zidovudine | lysine | ≥1.5g | acetylcarnitine | lysine | ≥10g |
| zolmitriptan | lysine | ≥1.5g | capecitabine | lysine | ≥10g |
| acetylcarnitine | lysine | ≥1.5g | cefaclor | lysine | ≥10g |
| capecitabine | lysine | ≥1.5g | cefixime | lysine | ≥10g |
| cefaclor | lysine | ≥1.5g | cefmetazole | lysine | ≥10g |
| cefixime | lysine | ≥1.5g | cefpodoxime proxetil | lysine | ≥10g |
| cefmetazole | lysine | ≥1.5g | cefroxadine | lysine | ≥10g |
| cefpodoxime proxetil | lysine | ≥1.5g | alfoscerate | lysine | ≥10g |
| cefroxadine | lysine | ≥1.5g | cilazapril | lysine | ≥10g |
| alfoscerate | lysine | ≥1.5g | cimetropium bromide | lysine | ≥10g |
| cilazapril | lysine | ≥1.5g | diacerein | lysine | ≥10g |
| cimetropium bromide | lysine | ≥1.5g | erdosteine | lysine | ≥10g |
| diacerein | lysine | ≥1.5g | famciclovir | lysine | ≥10g |
| erdosteine | lysine | ≥1.5g | gemifloxacin | lysine | ≥10g |
| famciclovir | lysine | ≥1.5g | levosulpiride | lysine | ≥10g |
| gemifloxacin | lysine | ≥1.5g | nabumetone | lysine | ≥10g |
| levosulpiride | lysine | ≥1.5g | oxiracetam | lysine | ≥10g |
| nabumetone | lysine | ≥1.5g | phendimetrazine | lysine | ≥10g |
| oxiracetam | lysine | ≥1.5g | rabeprazole | lysine | ≥10g |
| phendimetrazine | lysine | ≥1.5g | roxatidine acetate | lysine | ≥10g |
| rabeprazole | lysine | ≥1.5g | tamsulosin | lysine | ≥10g |
| roxatidine acetate | lysine | >1.5g | terazosin | lysine | >10g |
| tamsulosin | lysine | ≥1.5g | thioctic | lysine | ≥10g |
| terazosin | lysine | ≥1.5g | tosufloxacin | lysine | ≥10g |
| thioctic | lysine | ≥1.5g | triflusal | lysine | ≥10g |
| tosufloxacin | lysine | ≥1.5g | zaltoprofen | lysine | ≥10g |
| triflusal | lysine | ≥1.5g | etidronic acid | lysine | ≥10g |
| zaltoprofen | lysine | ≥1.5g | zoledronic acid | lysine | ≥10g |
| etidronic acid | lysine | ≥1.5g | clodronic acid | lysine | ≥10g |
| zoledronic acid | lysine | ≥1.5g | tiludronic acid | lysine | ≥10g |
| clodronic acid | lysine | ≥1.5g | pamidronic acid | lysine | ≥10g |
| tiludronic acid | lysine | ≥1.5g | alendronic acid | lysine | ≥10g |
| pamidronic acid | lysine | ≥1.5g | risedronic acid | lysine | ≥10g |
| alendronic acid | lysine | ≥1.5g | ibandronic acid | lysine | ≥10g |
| risedronic acid | lysine | ≥1.5g | abacavir | glycine | ≥10g |
| ibandronic acid | lysine | ≥1.5g | acarbose | glycine | ≥10g |
| abacavir | glycine | ≥1.5g | acetazolamide | glycine | ≥10g |
| acarbose | glycine | ≥1.5g | acyclovir | glycine | ≥10g |
| acetazolamide | glycine | ≥1.5g | albuterol (salbutamol) | glycine | ≥10g |
| acyclovir | glycine | ≥1.5g | allopurinol | glycine | ≥10g |
| albuterol (salbutamol) | glycine | ≥1.5g | amiloride | glycine | ≥10g |
| allopurinol | glycine | ≥1.5g | amisulpride | glycine | ≥10g |
| amiloride | glycine | ≥1.5g | amlodipine | glycine | ≥10g |
| amisulpride | glycine | ≥1.5g | amoxicillin | glycine | ≥10g |
| amlodipine | glycine | ≥1.5g | amphetamine | glycine | ≥10g |
| amoxicillin | glycine | ≥1.5g | atenolol | glycine | ≥10g |
| amphetamine | glycine | ≥1.5g | atropine | glycine | ≥10g |
| atenolol | glycine | ≥1.5g | azathioprine | glycine | ≥10g |
| atropine | glycine | ≥1.5g | benserazide | glycine | ≥10g |
| azathioprine | glycine | ≥1.5g | benznidazole | glycine | ≥10g |
| benserazide | glycine | ≥1.5g | camostat | glycine | ≥10g |
| benznidazole | glycine | ≥1.5g | captopril | glycine | ≥10g |
| camostat | glycine | ≥1.5g | cefdinir | glycine | ≥10g |
| captopril | glycine | ≥1.5g | cefotiam hexetil hydrochloride | glycine | ≥10g |
| cefdinir | glycine | ≥1.5g | cefprozil | glycine | ≥10g |
| cefotiam hexetil hydrochloride | glycine | ≥1.5g | cefuroxime axetil | glycine | ≥10g |
| cefprozil | glycine | ≥1.5g | chloramphenicol | glycine | ≥10g |
| cefuroxime axetil | glycine | ≥1.5g | cimetidine | glycine | ≥10g |
| chloramphenicol | glycine | ≥1.5g | ciprofloxacin | glycine | ≥10g |
| cimetidine | glycine | ≥1.5g | codeine | glycine | ≥10g |
| ciprofloxacin | glycine | ≥1.5g | colchicine | glycine | ≥10g |
| codeine | glycine | ≥1.5g | cyclophosphamide | glycine | ≥10g |
| colchicine | glycine | ≥1.5g | dapsone | glycine | ≥10g |
| cyclophosphamide | glycine | ≥1.5g | dexamethasone | glycine | ≥10g |
| dapsone | glycine | ≥1.5g | didanosine | glycine | ≥10g |
| dexamethasone | glycine | ≥1.5g | diethylcarbamazine | glycine | ≥10g |
| didanosine | glycine | ≥1.5g | methionine | glycine | ≥10g |
| diethylcarbamazine | glycine | ≥15g | dolasetron | glycine | ≥10g |
| methionine | glycine | ≥1.5g | doxifluridine | glycine | ≥10g |
| dolasetron | glycine | ≥1.5g | doxycycline | glycine | ≥10g |
| doxifluridine | glycine | ≥1.5g | ergonovine | glycine | ≥10g |
| doxycycline | glycine | ≥1.5g | erythromycin ethylsuccinate | glycine | ≥10g |
| ergonovine | glycine | ≥1.5g | ethambutol | glycine | ≥10g |
| erythromycin ethylsuccinate | glycine | ≥1.5g | ethosuximide | glycine | ≥10g |
| ethambutol | glycine | ≥1.5g | famotidine | glycine | ≥10g |
| ethosuximide | glycine | ≥1.5g | fluconazole | glycine | ≥10g |
| famotidine | glycine | ≥1.5g | folic acid | glycine | ≥10g |
| fluconazole | glycine | ≥1.5g | furosemide | glycine | ≥10g |
| folic acid | glycine | ≥1.5g | fursultiamine | glycine | ≥10g |
| furosemide | glycine | ≥1.5g | gabapentin | glycine | ≥10g |
| fursultiamine | glycine | ≥1.5g | glipizide | glycine | ≥10g |
| gabapentin | glycine | ≥1.5g | granisetron | glycine | ≥10g |
| glipizide | glycine | ≥1.5g | griseofulvin | glycine | ≥10g |
| granisetron | glycine | ≥1.5g | hydralazine | glycine | ≥10g |
| griseofulvin | glycine | ≥1.5g | hydrochlorothiazide | glycine | ≥10g |
| hydralazine | glycine | ≥1.5g | imidapril | glycine | ≥10g |
| hydrochlorothiazide | glycine | ≥1.5g | isoniazid | glycine | ≥10g |
| imidapril | glycine | ≥1.5g | lamivudine | glycine | ≥10g |
| isoniazid | glycine | ≥1.5g | 1-carbocysteine | glycine | ≥10g |
| lamivudine | glycine | ≥1.5g | levetiracetam | glycine | ≥10g |
| l-carbocysteine | glycine | ≥1.5g | levofloxacin | glycine | ≥10g |
| levetiracetam | glycine | ≥1.5g | linezolid | glycine | ≥10g |
| levofloxacin | glycine | ≥1.5g | lisinopril | glycine | ≥10g |
| linezolid | glycine | ≥1.5g | losartan | glycine | ≥10g |
| lisinopril | glycine | ≥1.5g | methotrexate | glycine | ≥10g |
| losartan | glycine | ≥1.5g | methyldopa | glycine | ≥10g |
| methotrexate | glycine | ≥1.5g | s-methylmethionine | glycine | ≥10g |
| methyldopa | glycine | ≥1.5g | metoclopramide | glycine | ≥10g |
| s-methylmethionine | glycine | ≥1.5g | metronidazole | glycine | ≥10g |
| metoclopramide | glycine | ≥1.5g | moxifloxacin | glycine | ≥10g |
| metronidazole | glycine | ≥1.5g | nalidixic acid | glycine | ≥10g |
| moxitloxacin | glycine | ≥1.5g | nicorandil | glycine | ≥10g |
| nalidixic acid | glycine | ≥1.5g | nifurtimox | glycine | ≥10g |
| nicorandil | glycine | ≥1.5g | nitrofurantoin | glycine | ≥10g |
| nifurtimox | glycine | ≥1.5g | nizatidine | glycine | ≥10g |
| nitrofurantoin | glycine | ≥1.5g | nystatin | glycine | ≥10g |
| nizatidine | glycine | ≥1.5g | ondansetron | glycine | ≥10g |
| nystatin | glycine | ≥1.5g | oseltamivir | glycine | ≥10g |
| ondansetron | glycine | ≥1.5g | oxcarbazepine | glycine | ≥10g |
| oseltamivir | glycine | ≥1.5g | penicillamine | glycine | ≥10g |
| oxcarbazepine | glycine | ≥1.5g | perindopril | glycine | ≥10g |
| penicillamine | glycine | ≥1.5g | phenobarbital | glycine | ≥10g |
| perindopril | glycine | ≥1.5g | phenoxymethylpenicillin | glycine | ≥10g |
| phenobarbital | glycine | ≥1.5g | pravastatin sodium | glycine | ≥10g |
| phenoxymethylpenicillin | glycine | ≥1.5g | prednisolone | glycine | ≥10g |
| pravastatin sodium | glycine | ≥1.5g | primaquine | glycine | ≥10g |
| prednisolone | glycine | ≥1.5g | procaterol | glycine | ≥10g |
| primaquine | glycine | ≥1.5g | propylthiouracil | glycine | ≥10g |
| procaterol | glycine | ≥1.5g | pseudoephedrine | glycine | ≥10g |
| propylthiouracil | glycine | ≥1.5g | pyrazinamide | glycine | ≥10g |
| pseudoephedrine | glycine | ≥1.5g | pyridostigmine bromide | glycine | ≥10g |
| pyrazinamide | glycine | ≥1.5g | pyridoxine hydrochloride | glycine | ≥10g |
| pyridostigmine bromide | glycine | ≥1.5g | ranitidine | glycine | ≥10g |
| pyridoxine hydrochloride | glycine | ≥1.5g | ribavirin | glycine | ≥10g |
| ranitidine | glycine | ≥1.5g | riboflavin | glycine | ≥10g |
| ribavirin | glycine | ≥1. g | rizatriptan | glycine | ≥10g |
| riboflavin | glycine | ≥1.5g | stavudine | glycine | ≥10g |
| rizatriptan | glycine | ≥1.5g | sulfadiazine | glycine | ≥10g |
| stavudine | glycine | ≥1.5g | sulfamethoxazole | glycine | ≥10g |
| sulfadiazine | glycine | ≥1.5g | sultamicillin | glycine | ≥10g |
| sulfamethoxazole | gglycine | ≥10g | sumatriptan | glycine | ≥10g |
| sultamicillin | glycine | ≥1.5g | taltirelin | glycine | ≥10g |
| sumatriptan | glycine | ≥1.5g | tegafur | glycine | ≥10g |
| taltirelin | glycine | ≥1.5g | tenofovir disoproxil | glycine | ≥10g |
| tegafur | glycine | ≥1.5g | theophylline | glycine | ≥10g |
| tenofovir disoproxil | glycine | ≥1.5g | thiamine | glycine | ≥10g |
| theophylline | glycine | ≥1.5g | trimetazidine | glycine | ≥10g |
| thiamine | glycine | ≥1.5g | trimethoprim | glycine | ≥10g |
| trimetazidine | glycine | ≥1.5g | voglibose | glycine | ≥10g |
| trimethoprim | glycine | ≥1.5g | zidovudine | glycine | ≥10g |
| voglibose | glycine | ≥1.5g | zolmitriptan | glycine | ≥10g |
| zidovudine | glycine | ≥1.5g | acetylcarnitine | glycine | ≥10g |
| zolmitriptan | glycine | ≥1.5g | capecitabine | glycine | ≥10g |
| gacetylcarnitine | glycine | ≥1.5g | cefaclor | glycine | ≥10g |
| capecitabine | glycine | ≥1.5g | cefixime | glycine | ≥10g |
| cefaclor | glycine | ≥1.5g | cefmetazole | glycine | ≥10g |
| cefixime | glycine | ≥1.5g | cefpodoxime proxetil | glycine | ≥10g |
| cefmetazole | glycine | ≥1.5g | cefroxadine | glycine | ≥10g |
| cefpodoxime proxetil | glycine | ≥1.5g | alfoscerate | glycine | ≥10g |
| cefroxadine | glycine | ≥1.5g | cilazapril | glycine | ≥10g |
| alfoscerate | glycine | ≥1.5g | cimetropium bromide | glycine | ≥10g |
| cilazapril | glycine | ≥1.5g | diacerein | glycine | ≥10g |
| cimetropium bromide | glycine | ≥1.5g | erdosteine | glycine | ≥10g |
| diacerein | glycine | ≥1.5g | famciclovir | glycine | ≥10g |
| erdosteine | glycine | ≥1.5g | gemifloxacin | glycine | ≥10g |
| famciclovir | glycine | ≥1.5g | levosulpiride | glycine | ≥10g |
| gemifloxacin | glycine | ≥1.5g | nabumetone | glycine | ≥10g |
| levosulpiride | glycinc | ≥1.5g | oxiracetam | glycine | ≥10g |
| nabumetone | glycine | ≥1.5g | phendimetrazine | glycine | ≥10g |
| oxiracetam | glycine | ≥1.5g | rabeprazole | glycine | ≥10g |
| phendimetrazine | glycine | ≥1.5g | roxatidine acetate | glycine | ≥10g |
| rabeprazole | glycine | ≥1.5g | tamsulosin | glycine | ≥10g |
| roxatidine acetate | glycine | ≥1.5g | terazosin | glycine | ≥10g |
| tamsulosin | glycine | ≥1.5g | thioctic | glycine | ≥10g |
| terazosin | glycine | ≥1.5g | tosufloxacin | glycine | ≥10g |
| thioctic | glycine | ≥1.5g | triflusal | glycine | ≥10g |
| tosufloxacin | glycine | ≥1.5g | zaltoprofen | glycine | ≥10g |
| triflusal | glycine | ≥1.5g | etidronic acid | glycine | ≥10g |
| zaltoprofen | glycine | ≥1.5g | zoledronic acid | glycine | ≥10g |
| etidronic acid | glycine | ≥1.5g | clodronic acid | glycine | ≥10g |
| zoledronic acid | glycine | ≥1.5g | tiludronic acid | glycine | ≥10g |
| clodronic acid | glycine | ≥1.5g | pamidronic acid | glycine | ≥10g |
| tiludronic acid | glycine | ≥1.5g | alendronic acid | glycine | ≥10g |
| pamidronic acid | glycine | ≥1.5g | risedronic acid | glycine | ≥10g |
| alendronic acid | glycine | ≥10g | ibandronic acid | glycine | ≥10g |
| risedronic acid | glycine | ≥1.5g | abacavir | lysine | ≥15g |
| ibandronic acid | glycine | ≥1.5g | acarbose | lysine | ≥15g |
| abacavir | lysine | ≥1.75g | acetazolamide | lysine | ≥15g |
| acarbose | lysine | ≥1.75g | acyclovir | lysine | ≥15g |
| acetazolamide | lysine | ≥1.75g | albuterol (salbutamol) | lysine | ≥15g |
| acyclovir | lysine | ≥1.75g | allopurinol | lysine | ≥15g |
| albuterol (salbutamol) | lysine | ≥1.75g | amiloride | lysine | ≥15g |
| allopurinol | lysine | ≥1.75g | amisulpride | lysine | ≥15g |
| amiloride | lysine | ≥1.75g | amlodipine | lysine | ≥15g |
| amisulpride | lysine | ≥1.75g | amoxicillin | lysine | ≥15g |
| amlodipine | lysine | ≥1.75g | amphetamine | lysine | ≥15g |
| amoxicillin | lysine | ≥1.75g | atenolol | lysine | ≥15g |
| amphetamine | lysine | ≥1.75g | atropine | lysine | ≥15g |
| atenolol | lysine | ≥1.75g | azathioprine | lysine | ≥15g |
| atropine | lysine | ≥1.75g | benserazide | lysine | ≥15g |
| azathioprine | lysine | ≥1.75g | benznidazole | lysine | ≥15g |
| benserazide | lysine | ≥1.75g | camostat | lysine | ≥1 |
| benznidazole | lysine, | ≥1.75g | captopril | lysine | ≥15g |
| camostat | lysine | ≥1.75g | cefdinir | lysine | ≥15g |
| captopril | lysine | ≥1.75g | cefotiam hexetil hydrochloride | lysine | ≥15g |
| cefdinir | lysine | ≥1.75g | cefprozil | lysine | ≥15g |
| cefotiam hexetil hydrochloride | lysine | ≥1.75g | cefuroxime axetil | lysine | ≥15g |
| cefprozil | lysine | ≥1.75g | chloramphenicol | lysine | ≥15g |
| cefuroxime axetil | lysine | ≥1.75g | cimetidine | lysine | ≥15g |
| chloramphenicol | lysine | ≥1.75g | ciprofloxacin | lysine | ≥15g |
| cimetidine | lysine | ≥1.75g | codeine | lysine | ≥15g |
| ciprofloxacin | lysine | ≥1.75g | colchicine | lysine | ≥15g |
| codeine | lysine | ≥1.75g | cyclophosphamide | lysine | ≥15g |
| colchicine | lysine | ≥1.75g | dapsone | lysine | ≥15g |
| cyclophosphamide | lysine | ≥1.75g | dexamethasone | lysine | ≥15g |
| dapsone | lysine | ≥1.75g | didanosine | lysine | ≥15g |
| dexamethasone | lysine | ≥1.75g | diethylcarbamazine | lysine | ≥15g |
| didanosine | lysine | ≥1.75g | methionine | lysine | ≥15g |
| diethylcarbamazine | lysine | ≥1.75g | dolasetron | lysine | ≥15g |
| methionine | lysine | ≥1.75g | doxifluridine | lysine | ≥15g |
| dolasetron | lysine | ≥1.75g | doxycycline | lysine | ≥15g |
| doxifluridine | lysine | ≥1.75g | ergonovine | lysine | ≥15g |
| doxycycline | lysine | ≥1.75g | erythromycin ethylsuccinate | lysine | ≥15g |
| ergonovine | lysine | ≥1.75g | ethambutol | lysine | ≥15g |
| erythromycin ethylsuccinate | lysine | ≥1.75g | ethosuximide | lysine | ≥15g |
| ethambutol | lysine | ≥1.75g | famotidine | lysine | ≥15g |
| ethosuximide | lysine | ≥1.75g | fluconazole | lysine | ≥15g |
| famotidine | lysine | ≥1.75g | folic acid | lysine | ≥15g |
| fluconazole | lysine | ≥1.75g | furosemide | lysine | ≥15g |
| folic acid | lysine | ≥1.75g | fursultiamine | lysine | ≥15g |
| furosemide | lysine | ≥1.75g | gabapentin | lysine | ≥15g |
| fursultiamine | lysine | ≥1.75g | glipizide | lysine | ≥15g |
| gabapentin | lysine | ≥1.75g | granisetron | lysine | ≥15g |
| glipizide | lysine | ≥1.75g | griseofulvin | lysine | ≥15g |
| granisetron | lysine | ≥1.75g | hydralazine | lysine | ≥15g |
| griseofulvin | lysine | ≥1.75g | hydrochlorothiazide | lysine | ≥15g |
| hydralazine | lysine | ≥1.75g | imidapril | lysine | ≥15g |
| hydrochlorothiazide | lysine | ≥1.75g | isoniazid | lysine | ≥15g |
| imidapril | lysine | ≥1.75g | lamivudine | lysine | ≥15g |
| isoniazid | lysine | ≥1.75g | 1-carbocysteine | lysine | ≥15g |
| lamivudine | lysine | ≥1.75g | levetiracetam | lysine | ≥15g |
| l-carbocysteine | lysine | ≥1.75g | levofloxacin | lysine | ≥15g |
| levetiracetam | lysine | ≥1.75g | linezolid | lysine | ≥15g |
| levofloxacin | lysine | ≥1.75g | lisinopril | lysine | ≥15g |
| linezolid | lysine | ≥1.75g | losartan | lysine | ≥15g |
| lisinopril | lysine | ≥1.75g | methotrexate | lysine | ≥15g |
| losartan | lysine | ≥1.75g | methyldopa | lysine | ≥15g |
| methotrexate | lysine | ≥1.75g | s-methylmethionine | lysine | ≥15g |
| methyldopa | lysine | ≥1.75g | metoclopramide | lysine | ≥15g |
| s-methylmethionine | lysine | ≥1.75g | metronidazole | lysine | ≥15g |
| metoclopramide | lysine | ≥1.75g | moxifloxacin | lysine | ≥15g |
| metronidazole | lysine | ≥1.75g | nalidixic acid | lysine | ≥15g |
| moxifloxacin | lysine | ≥1.75g | nicorandil | lysine | ≥15g |
| nalidixic acid | lysine | ≥1.75g | nifurtimox | lysine | ≥15g |
| nicorandil | lysine | ≥1.75g | nitrofurantoin | lysine | ≥15g |
| nifurtimox | lysine | ≥1.75g | nizatidine | lysine | ≥15g |
| nitrofurantoin | lysine | ≥1.75g | nystatin | lysine | ≥15g |
| nizatidine | lysine | ≥1.75g | ondansetron | lysine | ≥15g |
| nystatin | lysine | ≥1.75g | oseltamivir | lysine | ≥15g |
| ondansetron | lysine | ≥1.75g | oxcarbazepine | lysine | ≥15g |
| oseltamivir | lysine | ≥1.75g | penicillamine | lysine | ≥15g |
| oxcarbazepine | lysine | ≥1.75g | perindopril | lysine | ≥15g |
| penicillamine | lysine | ≥1.75g | phenobarbital | lysine | ≥15g |
| perindopril | lysine | ≥1.75g | phenoxymethylpenicillin | lysine | ≥15g. |
| phenobarbital | lysine | ≥1.75g | pravastatin sodium | lysine | ≥15g |
| phenoxymethylpenicillin | lysine | ≥1.75g | prednisolone | lysine | ≥15g |
| pravastatin sodium | lysine | ≥1.75g | primaquine | lysine | ≥15g |
| prednisolone | lysine | ≥1.75g | procaterol | lysine | ≥15g |
| primaquine | lysine | ≥1.75g | propylthiouracil | lysine | ≥15g |
| procaterol | lysine | ≥1.75g | pseudoephedrine | lysine | ≥15g |
| propylthiouracil | lysine | ≥1.75g | pyrazinamide | lysine | ≥15g |
| pseudoephedrine | lysine | ≥1.75g | pyridostigmine bromide | lysine | ≥15g |
| pyrazinamide | lysine | ≥1.75g | pyridoxine hydrochloride | lysine | ≥15g |
| pyridostigmine bromide | lysine | ≥1.75g | ranitidine | lysine | ≥15g |
| pyridoxine hydrochloride | lysine | ≥1.75g | ribavirin | lysine | ≥15g |
| ranitidine | lysine | ≥1.75g | riboflavin | lysine | ≥15g |
| ribavirin | lysine | ≥1.75g | rizatriptan | lysine | ≥15g |
| riboflavin | lysine | ≥1.75g | stavudine | lysine | ≥15g |
| rizatriptan | lysine | ≥1.75g | sulfadiazine | lysine | ≥15g |
| stavudine | lysine | ≥1.75g | sulfamethoxazole | lysine | ≥15g |
| sulfadiazine | lysine | ≥1.75g | sultamicillin | lysine | ≥15g |
| sulfamethoxazole | lysine | ≥1.75g | sumatriptan | lysine | ≥15g |
| sultamicillin | lysine | ≥1.75g | taltirelin | lysine | ≥15g |
| sumatriptan | lysine | ≥1.75g | tegafur | lysine | ≥15g |
| taltirelin | lysine | ≥1.75g | tenofovir disoproxil | lysine | ≥15g |
| tegafur | lysine | ≥1.75g | theophylline | lysine | ≥15g |
| tenofovir disoproxil | lysine | ≥1.75g | thiamine | lysine | ≥15g |
| theophylline | lysine | ≥1.75g | trimetazidine | lysine | ≥15g |
| thiamine | lysine | ≥1.75g | trimethoprim | lysine | ≥15g |
| trimetazidine | lysine | ≥1.75g | voglibose | lysine | ≥15g |
| trimethoprim | lysine | ≥1.75g | zidovudine | lysine | ≥15g |
| voglibose | lysine | ≥1.75g | zolmitriptan | lysine | ≥15g |
| zidovudine | lysine | ≥1.75g | acetylcarnitine | lysine | ≥15g |
| zolmitriptan | lysine | ≥1.75g | capecitabine | lysine | ≥15g |
| acetylcarnitine | lysine | ≥1.75g | cefaclor | lysine | ≥15g |
| capecitabine | lysine | ≥1.75g | cefixime | lysine | ≥15g |
| cefaclor | lysine | ≥1.75g | cefmetazole | lysine | ≥15g |
| cefixime | lysine | ≥1.75g | cefpodoxime proxetil | lysine | ≥15g |
| cefmetazole | lysine | ≥1.75g | cefroxadine | lysine | ≥15g |
| cefpodoxime proxetil | lysine | ≥1.75g | alfoscerate | lysine | ≥15g |
| cefroxadine | lysine | ≥1.75g | cilazapril | lysine | ≥15g |
| alfoscerate | lysine | ≥1.75g | cimetropium bromide | lysine | ≥15g |
| cilazapril | lysine | ≥1.75g | diacerein | lysine | ≥15g |
| cimetropium bromide | lysine | ≥1.75g | erdosteine | lysine | ≥15g |
| diacerein | lysine | ≥1.75g | famciclovir | lysine | ≥15g |
| erdosteine | lysine | ≥1.75g | gemifloxacin | lysine | ≥15g |
| famciclovir | lysine | ≥1.75g | levosulpiride | lysine | ≥15g |
| gemitloxacin | lysine | ≥1.75g | nabumetone | lysine | ≥15g |
| levosulpiride | lysine | ≥1.75g | oxiracetam | lysine | ≥15g |
| nabumetone | lysine | ≥1.75g | phendimetrazine | lysine | ≥15g |
| oxiracetam | lysine | ≥1.75g | rabeprazole | lysine | ≥15g |
| phendimetrazine | lysine | ≥ 1.75g | roxatidine acetate | lysine | ≥15g |
| rabeprazole | lysine | ≥1.75g | tamsulosin | lysine | ≥15g |
| roxatidine acetate | lysine | ≥1.75g | terazosin | lysine | ≥15g |
| tamsulosin | lysine | ≥1.75g | thioctic | lysine | ≥15g |
| terazosin | lysine | ≥1.75g | tosufloxacin | lysine | ≥15g |
| thioctic | lysine | ≥1.75g | triflusal | lysine | ≥15g |
| tosufloxacin | lysine | ≥1.75g | zaltoprofen | lysine | ≥15g |
| triflusal | lysine | ≥1.75g | etidronic acid | lysine | ≥15g |
| zaltoprofen | lysine | ≥.75g | zoledronic acid | lysine | ≥15g |
| etidronic acid | lysine | ≥1.75g | clodronic acid | lysine | ≥15g |
| zoledronic acid | lysine | ≥1.75g | tiludronic acid | lysine | ≥15g |
| clodronic acid | lysine | ≥1.75g | pamidronic acid | lysine | ≥15g |
| tiludronic acid | lysine | ≥1.75g | alendronic acid | lysine | ≥15g |
| pamidronic acid | lysine | ≥1.75g | risedronic acid | lysine | ≥15 |
| alendronic acid | lysine | ≥1.75g | ibandronic acid | lysine | ≥15g |
| risedronic acid | lysine | ≥1.75g | abacavir | glycine | ≥15g |
| ibandronic acid | lysine | ≥1.75g | acarbose | glycine | ≥15g |
| abacavir | glycine | ≥1.75g | acetazolamide | glycine | ≥15g |
| acarbose | glycine | ≥1.75g | acyclovir | glycine | ≥15g |
| acetazolamide | glycine | ≥1.75g | albuterol (salbutamol) | glycine | ≥15g |
| acyclovir | glycine | ≥1.75g | allopurinol | glycine | ≥15g |
| albuterol (salbutamol) | glycine | ≥1.75g | amiloride | glycine | ≥15g |
| allopurinol | glycine | ≥1.75g | amisulpride | glycine | ≥15g |
| amiloride | glycine | ≥1.75g | amlodipine | glycine | ≥15g |
| amisulpride | glycine | ≥1.75g | amoxicillin | glycine | ≥15g |
| amlodipine | glycine | ≥1.75g | amphetamine | glycine | ≥15g |
| amoxicillin | glycine | ≥1.75g | atenolol | glycine | ≥15g |
| amphetamine | glycine | ≥1.75g | atropine | glycine | ≥15g |
| atenolol | glycine | ≥1.75g | azathioprine | glycine | ≥15g |
| atropine | glycine | ≥1.75g | benserazide | glycine | ≥15g |
| azathioprine | glycine | ≥1.75g | benznidazole | glycine | ≥15g |
| benserazide | glycine | ≥1.75g | camostat | glycine | ≥15g |
| benznidazole | glycine | ≥1.75g | captopril | glycine | ≥15g |
| camostat | glycine | ≥1.75g | cefdinir | glycine | ≥15g |
| captopril | glycine | ≥1.75g | cefotiam hexetil hydrochloride | glycine | ≥15g |
| cefdinir | glycine | ≥1.75g | cefprozil | glycine | ≥15g |
| cefotiam hexetil hydrochloride | glycine | ≥1.75g | cefuroxime axetil | glycine | ≥15g |
| cefprozil | glycine | ≥1.75g | chloramphenicol | glycine | ≥15g |
| cefuroxime axetil | glycine | ≥1.75g | cimetidine | glycine | ≥15g |
| chloramphenicol | glycine | ≥1.75g | ciprofloxacin | glycine | ≥15g |
| cimetidine | glycine | ≥1.75g | codeine | glycine | ≥15g |
| ciprofloxacin | glycine | ≥1.75g | colchicine | glycine | ≥15g |
| codeine | glycine | ≥1.75g | cyclophosphamide | glycine | ≥15g |
| colchicine | glycine | ≥1.75g | dapsone | glycine | ≥15g |
| cyclophosphamide | glycine | ≥1.75g | dexamethasone | glycine | ≥15g |
| dapsone | glycine | ≥1.75g | didanosine | glycine | ≥15g |
| dexamethasone | glycine | ≥1.75g | diethylcarbamazine | glycine | ≥15g |
| didanosine | glycine | ≥1.75g | methionine | glycine | ≥15g |
| diethylcarbamazine | glycine | ≥1.75g | dolasetron | glycine | ≥15g |
| methionine | glycine | ≥1.75g | doxifluridine | glycine | ≥15g |
| dolasetron | glycine | ≥1.75g | doxycycline | glycine | ≥15g |
| doxifluridine | glycine | ≥1.75g | ergonovine | glycine | ≥15g |
| doxycycline | glycine | ≥1.75g | erythromycin ethylsuccinate | glycine | ≥15g |
| ergonovine | glycine | ≥1.75g | ethambutol | glycine | ≥15g |
| erythromycin ethylsuccinate | glycine | ≥1.75g | ethosuximide | glycine | ≥15g |
| ethambutol | glycine | ≥1.75g | famotidine | glycine | ≥15g |
| ethosuximide | glycine | ≥1.75g | fluconazole | glycine | ≥15g |
| famotidine | glycine | ≥1.75g | folic acid | glycine | ≥15g |
| fluconazole | glycine | ≥1.75g | furosemide | glycine | ≥15g |
| folic acid | glycine | ≥1.75g | fusultiamine | glycine | ≥15g |
| furosemide | glycine | ≥1.75g | gabapentin | glycine | ≥15g |
| fursultiamine | glycine | ≥1.75g | glipizide | glycine | ≥15g |
| gabapentin | glycine | ≥1.75g | granisetron | glycine | ≥15g |
| glipizide | glycine | ≥1.75g | griseofulvin | glycine | ≥15g |
| granisetron | glycine | ≥1.75g | hydralazine | glycine | ≥15g |
| griseofulvin | glycine | ≥1.75g | hydrochlorothiazide | glycine | ≥15g |
| hydralazine | glycine | ≥1.75g | imidapril | glycine | ≥15g |
| hydrochlorothiazide | glycine | ≥1.75g | isoniazid | glycine | ≥15g |
| imidapril | glycine | ≥1.75g | lamivudine | glycine | ≥15g |
| isoniazid | glycine | ≥1.75g | 1-carbocysteine | glycine | ≥15g |
| lamivudine | glycine | ≥1.75g | levetiracetam | glycine | ≥15g |
| l-carbocysteine | glycine | ≥1.75g | levofloxacin | glycine | ≥15g |
| levetiracetam | glycine | ≥1.75g | linezolid | glycine | ≥15g |
| levofloxacin | glycine | ≥1.75g | lisinopril | glycine | ≥15g |
| linezolid | glycine | ≥1.75g | losartan | glycine | ≥15g |
| lisinopril | glycine | ≥1.75g | methotrexate | glycine | ≥15g |
| losartan | glycine | ≥1.75g | methyldopa | glycine | ≥15g |
| methotrexate | glycine | ≥1.75g | s-methylmethionine | glycine | ≥15g |
| methyldopa | glycine | ≥1.75g | metoclopramide | glycine | ≥15g |
| s-methylmethionine | glycine | ≥1.75g | metronidazole | glycine | ≥15g |
| metoclopramide | glycine | ≥1.75g | moxifloxacin | glycine | ≥15g |
| metronidazole | glycine | ≥1.75g | nalidixic acid | glycine | ≥15g |
| moxifloxacin | glycine | ≥1.75g | nicorandil | glycine | ≥15g |
| nalidixic acid | glycine | ≥1.75g | nifurtimox | glycine | ≥15g |
| nicorandil | glycine | ≥1.75g | nitrofurantoin | glycine | ≥15g |
| nifurtimox | glycine | ≥1.75g | nizatidine | glycine | ≥15g |
| nitrofurantoin | glycine | ≥1.75g | nystatin | glycine | ≥15g |
| nizatidine | glycine | ≥1.75g | ondansetron | glycine | ≥15g |
| nystatin | glycine | ≥1.75g | oseltamivir | glycine | ≥15g |
| ondansetron | glycine | ≥1.75g | oxcarbazepine | glycine | ≥15g |
| oseltamivir | glycine | ≥1.75g | penicillamine | glycine | ≥15g |
| oxcarbazepine | glycine | ≥1.75g | perindopril | glycine | ≥15g |
| penicillamine | glycine | ≥1.75g | phenobarbital | glycine | ≥15g |
| perindopril | glycine | ≥1.75g | phenoxymethylpenicill in | glycine | ≥15g |
| phenobarbital | glycine | ≥1.75g | pravastatin sodium | glycine | ≥15g |
| phenoxymethylpenicill in | glycine | ≥1.75g | prednisolone | glycine | ≥15g |
| pravastatin sodium | glycine | ≥1.75g | primaquine | glycine | ≥15g |
| prednisolone | glycine | ≥1.75g | procaterol | glycine | ≥15g |
| primaquine | glycine | ≥1.75g | propylthiouracil | glycine | ≥15g |
| procaterol | glycine | ≥1.75g | pseudoephedrine | glycine | ≥15g |
| propylthiouracil | glycine | ≥1.75g | pyrazinamide | glycine | ≥15g |
| pseudoephedrine | glycine | ≥1.75g | pyridostigmine bromide | glycine | ≥15g |
| pyrazinamide | glycine | ≥1.75g | pyridoxine hydrochloride | glycine | ≥15g |
| pyridostigmine bromide | glycine | ≥1.75g | ranitidine | glycine | ≥15g |
| pyridoxine hydrochloride | glycine | ≥1.75g | ribavirin | glycine | ≥15g |
| ranitidine | glycine | ≥1.75g | riboflavin | glycine | ≥15g |
| ribavirin | glycine | ≥1.75g | rizatriptan | glycine | ≥15g |
| riboflavin | glycine | ≥1.75g | stavudine | glycine | ≥15g |
| rizatriptan | glycine | ≥1.75g | sulfadiazine | glycine | ≥15g |
| stavudine | glycine | ≥1.75g | sulfamethoxazole | glycine | ≥15g |
| sulfadiazine | glycine | ≥1.75g | sultamicillin | glycine | ≥15g |
| sulfamethoxazole | glycine | ≥1.75g | sumatriptan | glycine | ≥15g |
| sultamicillin | glycine | ≥1.75g | taltirelin | glycine | ≥15g |
| sumatriptan | glycine | ≥1.75g | tegafur | glycine | ≥15g |
| taltirelin | glycine | ≥1.75g | tenofovir disoproxil | glycine | ≥15g |
| tegafur | glycine | ≥1.75g | theophylline | glycine | ≥15g |
| tenofovir disoproxil | glycine | ≥1.75g | thiamine | glycine | ≥15g |
| theophylline | glycine | ≥1.75g | trimetazidine | glycine | ≥15g |
| thiamine | glycine | ≥1.75g | trimethoprim | glycine | ≥15g |
| trimetazidine | glycine | ≥1.75g | voglibose | glycine | ≥15g |
| trimethoprim | glycine | ≥1.75g | zidovudine | glycine | ≥15g |
| voglibose | glycine | ≥1.7sag | zolmitriptan | glycine | ≥15g |
| zidovudine | glycine | ≥1.75g | acetylcarnitine | glycine | ≥15g |
| zolmitriptan | glycine | ≥1.75g | capecitabine | glycine | ≥15g |
| acetylcarnitine | glycine | ≥1.75g | cefaclor | glycine | ≥15g |
| capecitabine | glycine | ≥1.75g | cefixime | glycine , | ≥15g |
| cefaclor | glycine | ≥1.75g | cefmetazole | glycine | ≥**1**5g |
| cefixime | glycine | ≥1.75g | cefpodoxime proxetil | glycine | ≥15g |
| cefmetazole | glycine | ≥1.75g | cefroxadine | glycine | ≥15g |
| cefpodoxime proxetil | glycine | ≥1.75g | alfoscerate | glycine | ≥15g |
| cefroxadine | glycine | ≥1.75g | cilazapril | glycine | ≥15g |
| alfoscerate | glycine | ≥1.75g | cimetropium bromide | glycine | ≥15g |
| cilazapril | glycine | ≥1.75g | diacerein | glycine | ≥15g |
| cimetropium bromide | glycine | ≥1.75g | erdosteine | glycine | ≥15g |
| diacerein | glycine | ≥1.75g | famciclovir | glycine | ≥15g |
| erdosteine | glycine | ≥1.75g | gemifloxacin | glycine | ≥15g |
| famciclovir | glycine | ≥1.75g | levosulpiride | glycine | ≥15g |
| gemifloxacin | glycine | ≥1.75g | nabumetone | glycine | ≥15g |
| levosulpiride | glycine | ≥1.75g | oxiracetam | glycine | ≥15g |
| nabumetone | glycine | ≥1.75g | phendimetrazine | glycine | ≥15g |
| oxiracetam | glycine | ≥1.75g | rabeprazole | glycine | ≥15g |
| phendimetrazine | glycine | ≥1.75g | roxatidine acetate | glycine | ≥15g |
| rabeprazole | glycine | ≥1.75g | tamsulosin | glycine | ≥15g |
| roxatidine acetate | glycine | ≥1.75g | terazosin | glycine | ≥15g |
| tamsulosin | glycine | ≥1.75g | thioctic | glycine | ≥15g |
| terazosin | glycine | ≥1.75g | tosufloxacin | glycine | ≥15g |
| thioctic | glycine | ≥1.75g | triflusal | glycine | ≥15g |
| tosufloxacin | glycine | ≥1.75g | zaltoprofen | glycine | ≥15g |
| triflusal | glycine | ≥1.75g | etidronic acid | glycine | ≥15g |
| zaltoprofen | glycine | ≥1.75g | zoledronic acid | glycine | ≥15g |
| etidronic acid | glycine | ≥1.75g | clodronic acid | glycine | ≥15g |
| zoledronic acid | glycine | ≥1.75g | tiludronic acid | glycine | ≥15g |
| clodronic acid | glycine | ≥1.75g | pamidronic acid | glycine | ≥15g |
| tiludronic acid | glycine | ≥1.75g | alendronic acid | glycine | ≥15g |
| pamidronic acid | glycine | ≥1.75g | risedronic acid | glycine | ≥15g |
| alendronic acid | glycine | ≥1.75g | ibandronic acid | glycine | ≥15g |
| risedronic acid | glycine | ≥1.75g | abacavir | lysine | 5g to 20g |
| ibandronic acid | glycine | ≥1.75g | acarbose | lysine | 5g to 20g |
| abacavir | lysine | ≥2g | acetazolamide | lysine | 5g to 20g |
| acarbose | lysine | ≥2g | acyclovir | lysine | 5g to 20g |
| acetazolamide | lysine | ≥2g | albuterol (salbutamol) | lysine | 5g to 20g |
| acyclovir | lysine | ≥2g | allopurinol | lysine | 5g to 20g |
| albuterol (salbutamol) | lysine | ≥2g | amiloride | lysine | 5g to 20g |
| allopurinol | lysine | ≥2g | amisulpride | lysine | 5g to 20g |
| amiloride | lysine | ≥2g | amlodipine | lysine | 5g to 20g |
| amisulpride | lysine | ≥2g | amoxicillin | lysine | 5g to 20g |
| amlodipine | lysine | ≥2g | amphetamine | lysine | 5g to 20g |
| amoxicillin | lysine | ≥2g | atenolol | lysine | 5g to 20g |
| amphetamine | lysine | ≥2g | atropine | lysine | 5g to 20g |
| atenolol | lysine | ≥2g | azathioprine | lysine | 5g to 20g |
| atropine | lysine | ≥2g | benserazide | lysine | 5g to 20g |
| azathioprine | lysine | ≥2g | benznidazole | lysine | 5g to 20g |
| benserazide | lysine | ≥2g | camostat | lysine | 5g to 20g |
| benznidazole | lysine | ≥2g | captopril | lysine | 5g to 20g |
| camostat | lysine | ≥2g | cefdinir | lysine | 5g to 20g |
| captopril | lysine | ≥2g | cefotiam hexetil | lysine | 5g to 20g |
| | | | hydrochloride | | |
| cefdinir | lysine | ≥2g | cefprozil | lysine | 5g to 20g |
| cefotiam hexetil hydrochloride | lysine | ≥2g | cefuroxime axetil | lysine | 5g to 20g |
| cefprozil | lysine | ≥2g | chloramphenicol | lysine | 5g to 20g |
| cefuroxime axetil | lysine | ≥2g | cimetidine | lysine | 5g to 20g |
| chloramphenicol | lysine | ≥2g | ciprofloxacin | lysine | 5g to 20g |
| cimetidine | lysine | ≥2g | codeine | lysine | 5g to 20g |
| ciprofloxacin | lysine | ≥2g | colchicine | lysine | 5g to 20g |
| codeine | lysine | ≥2g | cyclophosphamide | lysine | 5g to 20g |
| colchicine | lysine | ≥2g | dapsone | lysine | 5g to 20g |
| cyclophosphamide | lysine | ≥2g | dexamethasone | lysine | 5g to 20g |
| dapsone | lysine | ≥2g | didanosine | lysine | 5g to 20g |
| dexamethasone | lysine | ≥2g | diethylcarbamazine | lysine | 5g to 20g |
| didanosine | lysine | ≥2g | methionine | lysine | 5g to 20g |
| diethylcarbamazine | lysine | ≥2g | dolasetron | lysine | 5g to 20g |
| methionine | lysine | ≥2g | doxifluridine | lysine | 5g to 20g |
| dolasetron | lysine | ≥2g | doxycycline | lysine | 5g to 20g |
| doxifluridine | lysine | ≥2g | ergonovine | lysine | 5g to 20g |
| doxycycline | lysine | ≥2g | erythromycin ethylsuccinate | lysine | 5g to 20g |
| ergonovine | lysine | ≥2g | ethambutol | lysine | 5g to 20g |
| erythromycin ethylsuccinate | lysine | ≥2g | ethosuximide | lysine | 5g to 20g |
| ethambutol | lysine | ≥2g | famotidine | lysine | 5g to 20g |
| ethosuximide | lysine | ≥2g | fluconazole | lysine | 5g to 20g |
| famotidine | lysine | ≥2g | folic acid | lysine | 5g to 20g |
| fluconazole | lysine | ≥2g | furosemide | lysine | 5g to 20g |
| folic acid | lysine | ≥2g | fursultiamine | lysine | 5g to 20g |
| furosemide | lysine | ≥2g | gabapentin | lysine | 5g to 20g |
| fursultiamine | lysine | ≥2g | glipizide | lysine | 5g to 20g |
| gabapentin | lysine | ≥2g | granisetron | lysine | 5g to 20g |
| glipizide | lysine | ≥2g | griseofulvin | lysine | 5g to 20g |
| granisetron | lysine | ≥2g | hydralazine | lysine | 5g to 20g |
| griseofulvin | lysine | ≥2g | hydrochlorothiazide | lysine | 5g to 20g |
| hydralazine | lysine | ≥2g | imidapril | lysine | 5g to 20g |
| hydrochlorothiazide | lysine | ≥2g | isoniazid | lysine | 5g to 20g |
| imidapril | lysine | ≥2g | lamivudine | lysine | 5g to 20g |
| isoniazid | lysine | ≥2g | 1-carbocysteine | lysine | 5g to 20g |
| lamivudine | lysine | ≥2g | levetiracetam | lysine | 5g to 20g |
| 1-carbocysteine | lysine | ≥2g | levofloxacin | lysine | 5g to 20g |
| levetiracetam | lysine | ≥2g | linezolid | lysine | 5g to 20g |
| levofloxacin | lysine | ≥2g | lisinopril | lysine | 5g to 20g |
| linezolid | lysine | ≥2g | losartan | lysine | 5g to 20g |
| lisinopril | lysine | ≥2g | methotrexate | lysine | 5g to 20g |
| losartan | lysine | ≥2g | methyldopa | lysine | 5g to 20g |
| methotrexate | lysine | ≥2g | s-methylmethionine | lysine | 5g to 20g |
| methyldopa | lysine | ≥2g | metoclopramide | lysine | 5g to 20g |
| s-methylmethionine | lysine | ≥2g | metronidazole | lysine | 5g to 20g |
| metoclopramide | lysine | ≥2g | moxifloxacin | lysine | 5g to 20g |
| metronidazole | lysine | ≥2g | nalidixic acid | lysine | 5g to 20g |
| moxifloxacin | lysine | ≥2g | nicorandil | lysine | 5g to 20g |
| nalidixic acid | lysine | ≥2g | nifurtimox | lysine | 5g to 20g |
| nicorandil | lysine | ≥2g | nitrofurantoin | lysine | 5g to 20g |
| nifurtimox | lysine | ≥2g | nizatidine | lysine | 5g to 20g |
| nitrofurantoin | lysine | ≥2g | nystatin | lysine | 5g to 20g |
| nizatidine | lysine | ≥2g | ondansetron | lysine | 5g to 20g |
| nystatin | lysine | ≥2g | oseltamivir | lysine | 5g to 20g |
| ondansetron | lysine | ≥2g | oxcarbazepine | lysine | 5g to 20g |
| oseltamivir | lysine | ≥2g | penicillamine | lysine | 5g to 20g |
| oxcarbazepine | lysine | ≥2g | perindopril | lysine | 5g to 20g |
| penicillamine | lysine | ≥2g | phenobarbital | lysine | 5g to 20g |
| perindopril | lysine | ≥2g | phenoxymethylpenicill in | lysine | 5g to 20g |
| phenobarbital | lysine | ≥2g | pravastatin sodium | lysine | 5g to 20g |
| phenoxymethylpenicill in | lysine | ≥2g | prednisolone | lysine | 5g to 20g |
| pravastatin sodium | lysine | ≥2g | primaquine | lysine | 5g to 20g |
| prednisolone | lysine | ≥2g | procaterol | lysine | 5g to 20g |
| primaquine | lysine | ≥2g | propylthiouracil | lysine | 5g to 20g |
| procaterol | lysine | ≥2g | pseudoephedrine | lysine | 5g to 20g |
| propylthiouracil | lysine | ≥2g | pyrazinamide | lysine | 5g to 20g |
| pseudoephedrine | lysine | ≥2g | pyridostigmine bromide | lysine | 5g to 20g |
| pyrazinamide | lysine | ≥2g | pyridoxine hydrochloride | lysine | 5g to 20g |
| pyridostigmine bromide | lysine | ≥2g | ranitidine | lysine | 5g to 20g |
| pyridoxine hydrochloride | lysine | ≥2g | ribavirin | lysine | 5g to 20g |
| ranitidine | lysine | ≥2g | riboflavin | lysine | 5g to 20g |
| ribavirin | lysine | ≥2g | rizatriptan | lysine | 5g to 20g |
| riboflavin | lysine | ≥2g | stavudine | lysine | 5g to 20g |
| rizatriptan | lysine | ≥2g | sulfadiazine | lysine | 5g to 20g |
| stavudine | lysine | ≥2g | sulfamethoxazole | lysine | 5g to 20g |
| sulfadiazine | lysine | ≥2g | sultamicillin | lysine | 5g to 20g |
| sulfamethoxazole | lysine | ≥2g | sumatriptan | lysine | 5g to 20g |
| sultamicillin | lysine | ≥2g | taltirelin | lysine, | 5g to 20g |
| sumatriptan | lysine | ≥2g | tegafur | lysine | 5g to 20g |
| taltirelin | lysine | ≥2g | tenofovir disoproxil | lysine | 5g to 20g |
| tegafur | lysine | ≥2g | theophylline | lysine | 5g to 20g |
| tenofovir disoproxil | lysine | ≥2g | thiamine | lysine | 5g to 20g |
| theophylline | lysine | ≥2g | trimetazidine | lysine | 5g to 20g |
| thiamine | lysine | ≥2g | trimethoprim | lysine | 5g to 20g |
| trimetazidine | lysine | ≥2g | voglibose | lysine | 5g to 20g |
| trimethoprim | lysine | ≥2g | zidovudine | lysine | 5g to 20g |
| voglibose | lysine | ≥2g | zolmitriptan | lysine | 5g to 20g |
| zidovudine | lysine | ≥2g | acetylcarnitine | lysine | 5g to 20g |
| zolmitriptan | lysine | ≥2g | capecitabine | lysine | 5g to 20g |
| acetylcarnitine | lysine | ≥2g | cefaclor | lysine | 5g to 20g |
| capecitabine | lysine | ≥2g | cefixime | lysine | 5g to 20g |
| cefaclor | lysine | ≥2g | cefmetazole | lysine | 5g to 20g |
| cefixime | lysine | ≥2g | cefpodoxime proxetil | lysine | 5g to 20g |
| cefmetazole | lysine | ≥2g | cefroxadine | lysine | 5g to 20g |
| cefpodoxime proxetil | lysine | ≥2g | alfoscerate | lysine | 5g to 20g |
| cefroxadine | lysine | ≥2g | cilazapril | lysine | 5g to 20g |
| alfoscerate | lysine | ≥2g | cimetropium bromide | lysine | 5g to 20g |
| cilazapril | lysine | ≥2g | diacerein | lysine | 5g to 20g |
| cimetropium bromide | lysine | ≥2g | erdosteine | lysine | 5g to 20g |
| diacerein | lysine | ≥2g | famciclovir | lysine | 5g to 20g |
| erdosteine | lysine | ≥2g | gemifloxacin | lysine | 5g to 20g |
| famciclovir | lysine | ≥2g | levosulpiride | lysine | 5g to 20g |
| gemifloxacin | lysine | ≥2g | nabumetone | lysine | 5g to 20g |
| levosulpiride | lysine | ≥2g | oxiracetam | lysine | 5g to 20g |
| nabumetone | lysine | >2g | phendimetrazine | lysine | 5g to 20g |
| oxiracetam | lysine | ≥2g | rabeprazole | lysine | 5g to 20g |
| phendimetrazine | lysine | ≥2g | roxatidine acetate | lysine | 5g to 20g |
| rabeprazole | lysine | ≥2g | tamsulosin | lysine | 5g to 20g |
| roxatidine acetate | lysine | ≥2g | terazosin | lysine | 5g to 20g |
| tamsulosin | lysine | ≥2g | thioctic | lysine | 5g to 20g |
| terazosin | lysine | ≥2g | tosufloxacin | lysine | 5g to 20g |
| thioctic | lysine | ≥2g | triflusal | lysine | 5g to 20g |
| tosufloxacin | lysine | ≥2g | zaltoprofen | lysine | 5g to 20g |
| triflusal | lysine | ≥2g | etidronic acid | lysine | 5g to 20g |
| zaltoprofen | lysine | ≥2g | zoledronic acid | lysine | 5g to 20g |
| etidronic acid | lysine | ≥2g | clodronic acid | lysine | 5g to 20g |
| zoledronic acid | lysine | ≥2g | tiludronic acid | lysine | 5g to 20g |
| clodronic acid | lysine | ≥2g | pamidronic acid | lysine | 5g to 20g |
| tiludronic acid | lysine | ≥2g | alendronic acid | lysine | 5g to 20g |
| pamidronic acid | lysine | ≥2g | risedronic acid | lysine | 5g to 20g |
| alendronic acid | lysine | ≥2g | ibandronic acid | lysine | 5g to 20g |
| risedronic acid | lysine | ≥2g | abacavir | glycine | 5g to 20g |
| ibandronic acid | lysine | ≥2g | acarbose | glycine | 5g to 20g |
| abacavir | glycine | ≥2g | acetazolamide | glycine | 5g to 20g |
| acarbose | glycine | ≥2g | acyclovir | glycine | 5g to 20g |
| acetazolamide | glycine | ≥2g | albuterol (salbutamol) | glycine | 5g to 20g |
| acyclovir | glycine | ≥2g | allopurinol | glycine | 5g to 20g |
| albuterol (salbutamol) | glycine | ≥2g | amiloride | glycine | 5g to 20g |
| allopurinol | glycine | ≥2g | amisulpride | glycine | 5g to 20g |
| amiloride | glycine | ≥2g | amlodipine | glycine | 5g to 20g |
| amisulpride | glycine | ≥2g | amoxicillin | glycine | 5g to 20g |
| amlodipine | glycine | ≥2g | amphetamine | glycine | 5g to 20g |
| amoxicillin | glycine | ≥2g | atenolol | glycine | 5g to 20g |
| amphetamine | glycine | ≥2g | atropine | glycine | 5g to 20g |
| atenolol | glycine | ≥2g | azathioprine | glycine | 5g to 20g |
| atropine | glycine | ≥2g | benserazide | glycine | 5g to 20g |
| azathioprine | glycine | ≥2g | benznidazole | glycine | 5g to 20g |
| benserazide | glycine | ≥2g | camostat | glycine | 5g to 20g |
| benznidazole | glycine | ≥2g | captopril | glycine | 5g to 20g |
| camostat | glycine | ≥2g | cefdinir | glycine | 5g to 20g |
| captopril | glycine | ≥2g | cefotiam hexetil hydrochloride | glycine | 5g to 20g |
| cefdinir | glycine | ≥2g | cefprozil | glycine | 5g to 20g |
| cefotiam hexetil hydrochloride | glycine | ≥2g | cefuroxime axetil | glycine | 5g to 20g |
| cefprozil | glycine | ≥2g | chloramphenicol | glycine | 5g to 20g |
| cefuroxime axetil | glycine | ≥2g | cimetidine | glycine | 5g to 20g |
| chloramphenicol | glycine | ≥2g | ciprofloxacin | glycine | 5g to 20g |
| cimetidine | glycine | ≥2g | codeine | glycine | 5g to 20g |
| ciprofloxacin | glycine | ≥2g | colchicine | glycine | 5g to 20g |
| codeine | glycine | ≥2g | cyclophosphamide | glycine | 5g to 20g |
| colchicine | glycine | ≥2g | dapsone | glycine | 5g to 20g |
| cyclophosphamide | glycine | ≥2g | dexamethasone | glycine | 5g to 20g |
| dapsone | glycine | ≥2g | didanosine | glycine | 5g to 20g |
| dexamethasone | glycine | ≥2g | diethylcarbamazine | glycine | 5g to 20g |
| didanosine | glycine | ≥2g | methionine | glycine | 5g to 20g |
| diethylcarbamazine | glycine | ≥2g | dolasetron | glycine | 5g to 20g |
| methionine | glycine | ≥2g | doxifluridine | glycine | 5g to 20g |
| dolasetron | glycine | ≥2g | doxycycline | glycine | 5g to 20g |
| doxifluridine | glycine | ≥2g | ergonovine | glycine | 5g to 20g |
| doxycycline | glycine | ≥2g | erythromycin ethylsuccinate | glycine | 5g to 20g |
| ergonovine | glycine | ≥2g | ethambutol | glycine | 5g to 20g |
| erythromycin ethylsuccinate | glycine | ≥2g | ethosuximide | glycine | 5g to 20g |
| ethambutol | glycine | ≥2g | famotidine | glycine | 5g to 20g |
| ethosuximide | glycine | ≥2g | fluconazole | glycine | 5g to 20g |
| famotidine | glycine | ≥2g | folic acid | glycine | 5g to 20g |
| fluconazole | glycine | ≥2g | furosemide | glycine | 5g to 20g |
| folic acid | glycine | ≥2g | fursultiamine | glycine | 5g to 20g |
| furosemide | glycine | ≥2g | gabapentin | glycine | 5g to 20g |
| fursultiamine | glycine | >2g | glipizide | glycine | 5g to 20g |
| gabapentin | glycine | ≥2g | granisetron | glycine | 5g to 20g |
| glipizide | glycine | ≥2g | griseofulvin | glycine | 5g to 20g |
| granisetron | glycine | ≥2g | hydralazine | glycine | 5g to 20g |
| griseofulvin | glycine | ≥2g | hydrochlorothiazide | glycine | 5g to 20g |
| hydralazine | glycine | ≥2g | imidapril | glycine | 5g to 20g |
| hydrochlorothiazide | glycine | ≥2g | isoniazid | glycine | 5g to 20g |
| imidapril | glycine | ≥2g | lamivudine | glycine | 5g to 20g |
| isoniazid | glycine | ≥2g | 1-carbocysteine | glycine | 5g to 20g |
| lamivudine | glycine | ≥2g | levetiracetam | glycine | 5g to 20g |
| 1-carbocysteine | glycine | ≥2g | levofloxacin | glycine | 5g to 20g |
| levetiracetam | glycine | ≥2g | linezolid | glycine | 5g to 20g |
| levofloxacin | glycine | ≥2g | lisinopril | glycine | 5g to 20g |
| linezolid | glycine | ≥2g | losartan | glycine | 5g to 20g |
| lisinopril | glycine | ≥2g | methotrexate | glycine | 5g to 20g |
| losartan | glycine | ≥2g | methyldopa | glycine | 5g to 20g |
| methotrexate | glycine | ≥2g | s-methylmethionine | glycine | 5g to 20g |
| methyldopa | glycine | ≥2g | metoclopramide | glycine | 5g to 20g |
| s-methylmethionine | glycine | ≥2g | metronidazole | glycine | 5g to 20g |
| metoclopramide | glycine | ≥2g | moxifloxacin | glycine | 5g to 20g |
| metronidazole | glycine | ≥2g | nalidixic acid | glycine | 5g to 20g |
| moxifloxacin | glycine | ≥2g | nicorandil | glycine | 5g to 20g |
| nalidixic acid | glycine | ≥2g | nifurtimox | glycine | 5g to 20g |
| nicorandil | glycine | ≥2g | nitrofurantoin | glycine | 5g to 20g |
| nifurtimox | glycine | ≥2g | nizatidine | glycine | 5g to 20g |
| nitrofurantoin | glycine | ≥2g | nystatin | glycine | 5g to 20g |
| nizatidine | glycine | ≥**2**g | ondansetron | glycine | 5g to 20g |
| nystatin | glycine | ≥2g | oseltamivir | glycine | 5g to 20g |
| ondansetron | glycine | ≥2g | oxcarbazepine | glycine | 5g to 20g |
| oseltamivir | glycine | ≥2g | penicillamine | glycine | 5g to 20g |
| oxcarbazepine | glycine | ≥2g | perindopril | glycine | 5g to 20g |
| penicillamine | glycine | ≥2g | phenobarbital | glycine | 5g to 20g |
| perindopril | glycine | ≥2g | phenoxymethylpenicillin | glycine | 5g to 20g |
| phenobarbital | glycine | ≥2g | pravastatin sodium | glycine | 5g to 20g |
| phenoxymethylpenicill in | glycine | ≥2g | prednisolone | glycine | 5g to 20g |
| pravastatin sodium | glycine | ≥2g | primaquine | glycine | 5g to 20g |
| prednisolone | glycine | ≥2g | procaterol | glycine | 5g to 20g |
| primaquine | glycine | ≥2g | propylthiouracil | glycine | 5g to 20g |
| procaterol | glycine | ≥2g | pseudoephedrine | glycine | 5g to 20g |
| propylthiouracil | glycine | ≥2g | pyrazinamide | glycine | 5g to 20g |
| pseudoephedrine | glycine | ≥2g | pyridostigmine bromide | glycine | 5g to 20g |
| pyrazinamide | glycine | ≥2g | pyridoxine hydrochloride | glycine | 5g to 20g |
| pyridostigmine bromide | glycine | ≥2g | ranitidine | glycine | 5g to 20g |
| pyridoxine hydrochloride | glycine | ≥2g | ribavirin | glycine | 5g to 20g |
| ranitidine | glycine | ≥2g | riboflavin | glycine | 5g to 20g |
| ribavirin | glycine | ≥2g | rizatriptan | glycine | 5g to 20g |
| riboflavin | glycine | ≥2g | stavudine | glycine | 5g to 20g |
| rizatriptan | glycine | ≥2g | sulfadiazine | glycine | 5g to 20g |
| stavudine | glycine | ≥2g | sulfamethoxazole | glycine | 5g to 20g |
| sulfadiazine | glycine | ≥2g | sultamicillin | glycine | 5g to 20g |
| sulfamethoxazole | glycine | ≥2g | sumatriptan | glycine | 5g to 20g |
| sultamicillin | glycine | ≥2g | taltirelin | glycine | 5g to 20g |
| sumatriptan | glycine | ≥2g | tegafur | glycine | 5g to 20g |
| taltirelin | glycine | ≥2g | tenofovir disoproxil | glycine | 5g to 20g |
| tegafur | glycine | ≥2g | theophylline | glycine | 5g to 20g |
| tenofovir disoproxil | glycine | ≥2g | thiamine | glycine | 5g to 20g |
| theophylline | glycine | ≥2g | trimetazidine | glycine | 5g to 20g |
| thiamine | glycine | ≥2g | trimethoprim | glycine | 5g to 20g |
| trimetazidine | glycine | ≥2g | voglibose | glycine | 5g to 20g |
| trimethoprim | glycine | ≥2g | zidovudine | glycine | 5g to 20g |
| voglibose | glycine | >2g | zolmitriptan | glycine | 5g to 20g |
| zidovudine | glycine | ≥2g | acetylcarnitine | glycine | 5g to 20g |
| zolmitriptan | glycine | ≥2g | capecitabine | glycine | 5g to 20g |
| acetylcarnitine | glycine | ≥2g | cefaclor | glycine | 5g to 20g |
| capecitabine | glycine | ≥2g | cefixime | glycine | 5g to 20g |
| cefaclor | glycine | ≥2g | cefmetazole | glycine | 5g to 20g |
| cefixime | glycine | ≥2g | cefpodoxime proxetil | glycine | 5g to 20g |
| cefmetazole | glycine | ≥2g | cefroxadine | glycine | 5g to 20g |
| cefpodoxime proxetil | glycine | ≥2g | alfoscerate | glycine | 5g to 20g |
| cefroxadine | glycine | ≥2g | cilazapril | glycine | 5g to 20g |
| alfoscerate | glycine | ≥2g | cimetropium bromide | glycine | 5g to 20g |
| cilazapril | glycine | ≥2g | diacerein | glycine | 5g to 20g |
| cimetropium bromide | glycine | ≥2g | erdosteine | glycine | 5g to 20g |
| diacerein | glycine | ≥2g | famciclovir | glycine | 5g to 20g |
| erdosteine | glycine | ≥2g | gemifloxacin | glycine | 5g to 20g |
| famciclovir | glycine | ≥2g | levosulpiride | glycine | 5g to 20g |
| gemifloxacin | glycine | ≥2g | nabumetone | glycine | 5g to 20g |
| levosulpiride | glycine | ≥2g | oxiracetam | glycine | 5g to 20g |
| nabumetone | glycine | ≥2g | phendimetrazine | glycine | 5g to 20g |
| oxiracetam | glycine | ≥2g | rabeprazole | glycine | 5g to 20g |
| phendimetrazine | glycine | ≥2g | roxatidine acetate | glycine | 5g to 20g |
| rabeprazole | glycine | ≥2g | tamsulosin | glycine | 5g to 20g |
| roxatidine acetate | glycine | ≥2g | terazosin | glycine | 5g to 20g |
| tamsulosin | glycine | ≥2g | thioctic | glycine | 5g to 20g |
| terazosin | glycine | ≥2g | tosufloxacin | glycine | 5g to 20g |
| thioctic | glycine | ≥2g | triflusal | glycine | 5g to 20g |
| tosufloxacin | glycine | ≥2g | zaltoprofen | glycine | 5g to 20g |
| triflusal | glycine | ≥2g | etidronic acid | glycine | 5g to 20g |
| zaltoprofen | glycine | ≥2g | zoledronic acid | glycine | 5g to 20g |
| etidronic acid | glycine | ≥2g | clodronic acid | glycine | 5g to 20g |
| zoledronic acid | glycine | ≥2g | tiludronic acid | glycine | 5g to 20g |
| clodronic acid | glycine | ≥2g | pamidronic acid | glycine | 5g to 20g |
| tiludronic acid | glycine | ≥2g | alendronic acid | glycine | 5g to 20g |
| pamidronic acid | glycine | ≥2g | risedronic acid | glycine | 5g to 20g |
| alendronic acid | glycine | ≥2g | ibandronic acid | glycine | 5g to 20g |
| risedronic acid | glycine | ≥2g | | | |
| ibandronic acid | glycine | ≥2g | | | |

**Table 12. Particular compositions comprising: a bisphosphonic acid (left column), either in the form of a crystalline molecular complex (e.g., salt or cocrystal) with a coformer or in the form of a free acid(middle column), and an additional coformer (right column). Each row of the above table represents an individual composition.**

| **Bisphosphonic Acid** | **Molecular Complex Coformer** | **Additional Coformer as Excipient** |
|---|---|---|
| zoledronic acid | sodium | L-lysine |
| alendronic acid | sodium | L-lysine |
| ibandronic acid | sodium | L-lysine |
| risedronic acid | sodium | L-lysine |
| tiludronic acid | sodium | L-lysine |
| zoledronic acid | sodium | DL-lysine |
| alendronic acid | sodium | DL-lysine |
| ibandronic acid | sodium | DL-lysine |
| risedronic acid | sodium | DL-lysine |
| tiludronic acid | sodium | DL-lysine |
| zoledronic acid | sodium | glycine |
| alendronic acid | sodium | glycine |
| ibandronic acid | sodium | glycine |
| risedronic acid | sodium | glycine |
| tiludronic acid | sodium | glycine |
| zoledronic acid | ammonium | L-lysine |
| alendronic acid | ammonium | L-lysine |
| ibandronic acid | ammonium | L-lysine |
| risedronic acid | ammonium | L-lysine |
| tiludronic acid | ammonium | L-lysine |
| zoledronic acid | ammonium | DL-lysine |
| alendronic acid | ammonium | DL-lysine |
| ibandronic acid | ammonium | DL-lysine |
| risedronic acid | ammonium | DL-lysine |
| tiludronic acid | ammonium | DL-lysine |
| zoledronic acid | ammonium | glycine |
| alendronic acid | ammonium | glycine |
| ibandronic acid | ammonium | glycine |
| risedronic acid | ammonium | glycine |
| tiludronic acid | ammonium | glycine |
| zoledronic acid | ammonia | L-lysine |
| alendronic acid | ammonia | L-lysine |
| ibandronic acid | ammonia | L-lysine |
| risedronic acid | ammonia | L-lysine |
| tiludronic acid | ammonia | L-lysine |
| zoledronic acid | ammonia | DL-lysine |
| alendronic acid | ammonia | DL-lysine |
| ibandronic acid | ammonia | DL-lysine |
| risedronic acid | ammonia | DL-lysine |
| tiludronic acid | ammonia | DL-lysine |
| zoledronic acid | ammonia | glycine |
| alendronic acid | ammonia | glycine |
| ibandronic acid | ammonia | glycine |
| risedronic acid | ammonia | glycine |
| tiludronic acid | ammonia | glycine |
| zoledronic acid | L-lysine | L-lysine |
| alendronic acid | L-lysine | L-lysine |
| ibandronic acid | L-lysine | L-lysine |
| risedronic acid | L-lysine | L-lysine |
| tiludronic acid | L-lysine | L-lysine |
| zoledronic acid | L-lysine | DL-lysine |
| alendronic acid | L-lysine | DL-lysine |
| ibandronic acid | L-lysine | DL-lysine |
| risedronic acid | L-lysine | DL-lysine |
| tiludronic acid | L-lysine | DL-lysine |
| zoledronic acid | L-lysine | glycine |
| alendronic acid | L-lysine | glycine |
| ibandronic acid | L-lysine | glycine |
| risedronic acid | L-lysine | glycine |
| tiludronic acid | L-lysine | glycine |
| zoledronic acid | DL-lysine | L-lysine |
| alendronic acid | DL-lysine | L-lysine |
| ibandronic acid | DL-lysine | L-lysine |
| risedronic acid | DL-lysine | L-lysine |
| riludronic acid | DL-lysine | L-lysine |
| zoledronic acid | DL-lysine | DL-lysine |
| alendronic acid | DL-lysine | DL-lysine |
| ibandronic acid | DL-lysine | DL-lysine |
| risedronic acid | DL-lysine | DL-lysine |
| tiludronic acid | DL-lysine | DL-lysine |
| zoledronic acid | DL--lysine | glycine |
| alendronic acid | DL-lysine | glycine |
| ibandronic acid | DL-lysine | glycine |
| risedronic acid | DL-lysine | glycine |
| tiludronic acid | DL-lysine | glycine |
| zoledronic acid | nicotinamide | L-lysine |
| alendronic acid | nicotinamide | L-lysine |
| ibandronic acid | nicotinamide | L-lysine |
| risedronic acid | nicotinamide | L-lysine |
| tiludronic acid | nicotinamide | L-lysine |
| zoledronic acid | nicotinamide | DL-lysine |
| alendronic acid | nicotinamide | DL-lysine |
| ibandronic acid | nicotinamide | DL-lysine |
| risedronic acid | nicotinamide | DL-lysine |
| tiludronic acid | nicotinamide | DL-lysine |
| zoledronic acid | nicotinamide | glycine |
| alendronic acid | nicotinamide | glycine |
| ibandronic acid | nicotinamide | glycine |
| risedronic acid | nicotinamide | glycine |
| tiludronic acid | nicotinamide | glycine |
| zoledronic acid | adenine | L-lysine |
| alendronic acid | adenine | L-lysine |
| ibandronic acid | adenine | L-lysine |
| risedronic acid | adenine | L-lysine, |
| tiludronic acid | adenine | L-lysine |
| zoledronic acid | adenine | DL-lysine |
| alendronic acid | adenine | DL-lysine |
| ibandronic acid | adenine | DL-lysine |
| risedronic acid | adenine | DL-lysine |
| tiludronic acid | adenine | DL-lysine |
| zoledronic acid | adenine | glycine |
| alendronic acid | adenine | glycine |
| ibandronic acid | adenine | glycine |
| risedronic acid | adenine | glycine |
| tiludronic acid | adenine | glycine |
| zoledronic acid | glycine | L-lysine |
| alendronic acid | glycine | L-lysine |
| ibandronic acid | glycine | L-lysine |
| risedronic acid | glycine | L-lysine |
| tiludronic acid | glycine | L-lysine |
| zoledronic acid | glycine | DL-lysine |
| alendronic acid | glycine | DL-lysine |
| ibandronic acid | glycine | DL-lysine |
| risedronic acid | glycine | DL-lysine |
| tiludronic acid | glycine | DL-lysine |
| zoledronic acid | glycine | glycine |
| alendronic acid | glycine | glycine |
| ibandronic acid | glycine | glycine |
| risedronic acid | glycine | glycine |
| tiludronic acid | glycine | glycine |
| zoledronic acid | free acid | L-lysine |
| alendronic acid | free acid | L-lysine |
| ibandronic acid | free acid | L-lysine |
| risedronic acid | free acid | L-lysine |
| tiludronic acid | free acid | L-lysine |
| zoledronic acid | free acid | DL-lysine |
| alendronic acid | free acid | DL-lysine |
| ibandronic acid | free acid | DL-lysine |
| risedronic acid | free acid | DL-lysine |
| tiludronic acid | free acid | DL-lysine |
| zoledronic acid | free acid | glycine |
| alendronic acid | free acid | glycine |
| ibandronic acid | free acid | glycine |
| risedronic acid | free acid | glycine |
| tiludronic acid | free acid | glycine |

**Table 13. Particular compositions comprising: (from left to right) zoledronic acid (either in the form of a crystalline molecular complex (e.g., salt or cocrystal) with a coformer or in the form of a free acid), an additional coformer, and the ratio of the additional coformer to bisphosphonic acid (by mass). Each row of the above table repreents an individual composition.**

| **Bisphosphonic Acid** | **Molecular Complex Coformer** | **Additional Coformer** | **Mass Ratio of Additional Coformer:Bisphosphic Acid** |
|---|---|---|---|
| zoledronic acid | sodium | L-lysine | ≥5:1 |
| zoledronic acid | sodium | L-lysine | ≥50:1 |
| zoledronic acid | sodium | L-lysine | ≥750:1 |
| zoledronic acid | sodium | L-lysine | ≥2500:1 |
| zoledronic acid | sodium | L-lysine | ≥5000:1 |
| zoledronic acid | sodium | DL-lysine | ≥5:1 |
| zoledronic acid | sodium | DL-lysine | ≥:50:1 |
| zoledronic acid | sodium | DL-lysine | ≥750:1 |
| zoledronic acid | sodium | DL-lysine | ≥2500:1 |
| zoledronic acid | sodium | DL-lysine | ≥5000:1 |
| zoledronic acid | sodium | glycine | ≥5:1 |
| zoledronic acid | sodium | glycine | ≥50:1 |
| zoledronic acid | sodium | glycine | ≥750: |
| zoledronic acid | sodium | glycine | ≥2500:1 |
| zoledronic acid | sodium | glycine | ≥5000:1 |
| zoledronic acid | ammonium | L-lysine | ≥5:1 |
| zoledronic acid | ammonium | L-lysine | ≥50:1 |
| zoledronic acid | ammonium | L-lysine | ≥750:1 |
| zoledronic acid | ammonium | L-lysine | ≥2500:1 |
| zoledronic acid | ammonium | L-lysine | ≥5000:1 |
| zoledronic acid | ammonium | DL-lysine | ≥5:1 |
| zoledronic acid | ammonium | DL-lysine | ≥50:1 |
| zoledronic acid | ammonium | DL-lysine | ≥750:1 |
| zoledronic acid | ammonium | DL-lysine | ≥2500:1 |
| zoledronic acid | ammonium | DL-lysine | ≥5000:1 |
| zoledronic acid | ammonium | glycine | ≥5:1 |
| zoledronic acid | ammonium | glycine | ≥50:1 |
| zoledronic acid | ammonium | glycine | ≥750:1 |
| zoledronic acid | ammonium | glycine | ≥2500:1 |
| zoledronic acid | ammonium | glycine | ≥5000:1 |
| zoledronic acid | ammonia | L-lysine | ≥5:1 |
| zoledronic acid | ammonia | L-lysine | ≥50:1 |
| zoledronic acid | ammonia | L-lysine | ≥750:1 |
| zoledronic acid | ammonia | L-lysine | ≥2500:1 |
| zoledronic acid | ammonia | L-lysine | ≥5000:1 |
| zoledronic acid | ammonia | DL-lysine | >5:1 |
| zoledronic acid | ammonia | DL-lysine | ≥50:1 |
| zoledronic acid | ammonia | DL-lysine | ≥750:1 |
| zoledronic acid | ammonia | DL-lysine | ≥2500:1 |
| zoledronic acid | ammonia | DL-lysine | ≥5000:1 |
| zoledronic acid | ammonia | glycine | ≥5:1 |
| zoledronic acid | ammonia | glycine | ≥50:1 |
| zoledronic acid | ammonia | glycine | ≥750:1 |
| zoledronic acid | ammonia | glycine | ≥2500:1 |
| zoledronic acid | ammonia | glycine | ≥5000:1 |
| zoledronic acid | L-lysine | L-lysine | ≥5:1 |
| zoledronic acid | L-lysine | L-lysine | ≥50:1 |
| zoledronic acid | L-lysine | L-lysine | ≥750:1 |
| zoledronic acid | L-lysine | L-lysine | ≥2500:1 |
| zoledronic acid | L-lysine | L-lysine | ≥5000:1 |
| zoledronic acid | L-lysine | DL-lysine | ≥5:1 |
| zoledronic acid | L-lysine | DL-lysine | ≥50:1 |
| zoledronic acid | L-lysine | DL-lysine | ≥750:1 |
| zoledronic acid | L-lysine | DL-lysine | ≥2500:1 |
| zoledronic acid | L-lysine | DL-lysine | ≥5000:1 |
| zoledronic acid | L-lysine | glycine | ≥5:1 |
| zoledronic acid | L-lysine | glycine | ≥50:1 |
| zoledronic acid | L-lysine | glycine | ≥750:1 |
| zoledronic acid | L-lysine | glycine | ≥2500:1 |
| zoledronic acid | L-lysine | glycine | ≥5000:1 |
| zoledronic acid | DL-lysine | L-lysine | ≥5:1 |
| zoledronic acid | DL-lysine | L-lysine | ≥50:1 |
| zoledronic acid | DL-lysine | L-lysine | ≥750:1 |
| zoledronic acid | DL-lysine | L-lysine | ≥2500:1 |
| zoledronic acid | DL-lysine | L-lysine | ≥5000:1 |
| zoledronic acid | DL-lysine | DL-lysine | ≥5:1 |
| zoledronic acid | DL-lysine | DL-lysine | >50:1 |
| zoledronic acid | L-lysine | DL-lysine | ≥750:1 |
| zoledronic acid | DL-lysine | DL-lysine | ≥2500:1 |
| zoledronic acid | D L-lysine | DL-lysine | ≥5000:1 |
| zoledronic acid | DL-lysine | glycine | ≥5:1 |
| zoledronic acid | DL-lysine | glycine | ≥50:1 |
| zoledronic acid | DL-lysine | glycine | ≥750:1 |
| zoledronic acid | DL-lysine | glycine | ≥2500:1 |
| zoledronic acid | DL-lysine | glycine | ≥5000:1 |
| zoledronic acid | nicotinamide | L-lysine | ≥5:1 |
| zoledronic acid | nicotinamide | L-lysine | ≥50:1 |
| zoledronic acid | nicotinamide | L-lysine | ≥750:1 |
| zoledronic acid | nicotinamide | L-lysine | ≥2500:1 |
| zoledronic acid | nicotinamide | L-lysine | ≥5000:1 |
| zoledronic acid | nicotinamide | DL-lysine | ≥5:1 |
| zoledronic acid | nicotinamide | DL-lysine | ≥50:1 |
| zoledronic acid | nicotinamide | DL-lysine | ≥750:1 |
| zoledronic acid | nicotinamide | DL-lysine | ≥2500:1 |
| zoledronic acid | nicotinamide | DL-lysine | ≥5000:1 |
| zoledronic acid | nicotinamide | glycine | ≥5:1 |
| zoledronic acid | nicotinamide | glycine | ≥50:1 |
| zoledronic acid | nicotinamide | glycine | ≥750:1 |
| zoledronic acid | nicotinamide | glycine | ≥2500:1 |
| zoledronic acid | nicotinamide | glycine | ≥5000:1 |
| zoledronic acid | adenine | L-lysine | ≥5:1 |
| zoledronic acid | adenine | L-lysine | ≥50:1 |
| zoledronic acid | adenine | L-lysine | ≥750:1 |
| zoledronic acid | adenine | L-lysine | ≥2500:1 |
| zoledronic acid | adenine | L-lysine | ≥5000:1 |
| zoledronic acid | adenine | DL-lysine | ≥5:1 |
| zoledronic acid | adenine | DL-lysine | ≥50:1 |
| zoledronic acid | adenine | DL-lysine | ≥750:1 |
| zoledronic acid | adenine | DL-lysine | ≥2500:1 |
| zoledronic acid | adenine | DL-lysine | ≥5000:1 |
| zoledronic acid | adenine | glycine | ≥5:1 |
| zoledronic acid | adenine | glycine | ≥50:1 |
| zoledronic acid | adenine | glycine | ≥750:1 |
| zoledronic acid | adenine | glycine | ≥2500:1 |
| zoledronic acid | adenine | glycine | ≥5000:1 |
| zoledronic acid | glycine | L-lysine | ≥5:1 |
| zoledronic acid | glycine | L-lysine | ≥50:1 |
| zoledronic acid | glycine | L-lysine | ≥750:1 |
| zoledronic acid | glycine | L-lysine | ≥2500:1 |
| zoledronic acid | glycine | L-lysine | ≥5000:1 |
| zoledronic acid | glycine | DL-lysine | ≥5:1 |
| zoledronic acid | glycine | DL-lysine | ≥50:1 |
| zoledronic acid | glycine | DL-lysine | ≥750:1 |
| zoledronic acid | glycine | DL-lysine | ≥2500:1 |
| zoledronic acid | glycine | DL-lysine | ≥5000:1 |
| zoledronic acid | glycine | glycine | ≥5:1 |
| zoledronic acid | glycine | glycine | ≥50:1 |
| zoledronic acid | glycine | glycine | ≥750:1 |
| zoledronic acid | glycine | glycine | ≥2500:1 |
| zoledronic acid | glycine | glycine | ≥5000:1 |
| zoledronic acid | free acid | L-lysine | ≥5:1 |
| zoledronic acid | free acid | L-lysine | ≥50:1 |
| zoledronic acid | free acid | L-lysine | ≥750:1 |
| zoledronic acid | free acid | L-lysine | ≥2500:1 |
| zoledronic acid | free acid | L-lysine | ≥5000:1 |
| zoledronic acid | free acid | DL-lysine | ≥5:1 |
| zoledronic acid | free acid | DL-lysine | ≥50:1 |
| zoledronic acid | free acid | DL-lysine | ≥750:1 |
| zoledronic acid | free acid | DL-lysine | ≥2500:1 |
| zoledronic acid | free acid | DL-lysine | ≥5000:1 |
| zoledronic acid | free acid | glycine | ≥5:1 |
| zoledronic acid | free acid | glycine | ≥50:1 |
| zoledronic acid | free acid | glycine | ≥750:1 |
| zoledronic acid | free acid | glycine | ≥2500:1 |
| zoledronic acid | free acid | glycine | ≥5000:1 |

**Table 14. Particular compositions comprising: a crystalline molecular complex (left column), an additional coformer (middle column), and the ratio of the additional coformer to molecular complex coformer (by mass) is indicated in the far right column. Each row of the above table represents an individual composition.**

| **Molecular Complex** | **Additional Coformer** | **Mass Ratio of Additional Coformer:Molecular Complex Coformer** |
|---|---|---|
| zoledronic acid, sodium zoledronate and water complex characterized by an X-ray powder diffraction pattern having peaks at about 8.1, 13.3, 21.5, 24.6, and 25.6 ±0.2 degrees two-theta | L-lysine | ≥5:1 |
| ammonium zoledronic acid salt and water complex characterized by an X-ray powder diffraction pattern having strong peaks at about 11.0, 14.6, 15.4, 19.9, and 29.4 ±0.2 degrees two-theta | L-lysine | ≥5:1 |
| zoledronic diammonia water complex characterized by an X-ray powder diffraction pattern having strong peaks at about 12.2, 13.0, 14.1, 17.1, and 19.3 ±0.2 degrees two-theta | L-lysine | >5:1 |
| zoledronic acid, L-lysine, and water complex characterized by an X-ray powder diffraction pattern having peaks at about 9.0, 14.4, 18.1, 26.0, and 29.6 ±0.2 degrees two-theta | L-lysine | ≥5:1 |
| zoledronic acid, L-lysine, and water complex characterized by an X-ray powder diffraction pattern comprising peaks at about 9.6, 10.7, 14.3, 21.4, 23.5 ±0.2 degrees two theta | L-lysine | ≥5:1 |
| zoledronic acid DL-lysine and water complex characterized by an X-ray powder diffraction pattern comprising peaks at about 8.3, 11.8, 12.3, 15.8, and 20.8 ±0.2 degrees two-theta | L-lysine | ≥5:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | L-lysine | ≥5:1 |
| zoledronic acid, DL-lysine, and water complex characterized by an X-ray powder diffraction pattern comprising peaks at about 9.7, 10.8, 14.4, 18.9, 21.4 ±0.2 degrees two theta | L-lysine | ≥5:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | L-lysine | ≥5:1 |
| zoledronic acid, adenine, and water complex characterized by an X-ray powder diffraction pattern comprising peaks at about 13.6, 15.9, 19.7, 27.9, and 29.5 ±0.2 degrees two-theta | L-lysine | >5:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | L-lysine | ≥5:1 |
| zoledronic acid and glycine complex characterized by an X-ray powder diffraction pattern comprising peaks at about 10.2, 17.8, 19.9, 22.9, and 28.1 ±0.2 degrees two-theta | L-lysine | ≥5:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | L-lysine | ≥40:1 |
| ammonium zoledronic acid salt and water complex characterized by an X-ray powder diffraction pattern having strong peaks at about 11.0, 14.6, 15.4, 19.9, and 29.4 ±0.2 degrees two-theta | L-lysine | ≥40:1 |
| zoledronic diammonia water complex characterized by an X-ray powder diffraction pattern having strong peaks at about 12.2, 13.0, 14.1, 17.1, and 19.3 ±0.2 degrees two-theta | L-lysine | ≥40:1 |
| zoledronic acid, L-lysine, and water complex characterized by an X-ray powder diffraction pattern having peaks at about 9.0, 14.4, 18.1, 26.0, and 29.6 ±0.2 degrees two-theta | L-lysine | ≥40:1 |
| zoledronic acid, L-lysine, and water complex characterized by an X-ray powder diffraction pattern comprising peaks at about 9.6, 10.7, 14.3, 21.4, 23.5 ±0.2 degrees two theta | L-lysine | ≥40:1 |
| zoledronic acid DL-lysine and water complex characterized by an X-ray powder diffraction pattern comprising peaks at about 8.3, 11.8, 12.3, 15.8, and 20.8 ±0.2 degrees two-theta | L-lysine | ≥40:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | L-lysine | ≥40:1 |
| zoledronic acid, DL-lysine, and water complex characterized by an X-ray powder diffraction pattern comprising peaks at about 9.7, 10.8, 14.4, 18.9, 21.4 ±0.2 degrees two theta | L-lysine | >40:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | L-lysine | ≥40:1 |
| zoledronic acid, adenine, and water complex characterized by an X-ray powder diffraction pattern comprising peaks at about 13.6, 15.9, 19.7, 27.9, and 29.5 ±0.2 degrees two-theta | L-lysine | ≥40:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | L-lysine | ≥40:1 |
| zoledronic acid and glycine complex characterized by an X-ray powder diffraction pattern comprising peaks at about 10.2, 17.8, 19.9, 22.9, and 28.1 ±0.2 degrees two-theta | L-lysine | ≥40:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | L-lysine | ≥750:1 |
| ammonium zoledronic acid salt and water complex characterized by an X-ray powder diffraction pattern having strong peaks at about 11.0, 14.6, 15.4, 19.9, and 29.4 ±0.2 degrees two-theta | L-lysine | ≥750:1 |
| zoledronic diammonia water complex characterized by an X-ray powder diffraction pattern having strong peaks at about 12.2, 13.0, 14.1, 17.1, and 19.3 ±0.2 degrees two-theta | L-lysine | ≥750:1 |
| zoledronic acid, L-lysine, and water complex characterized by an X-ray powder diffraction pattern having peaks at about 9.0, 14.4, 18.1, 26.0, and 29.6 ±0.2 degrees two-theta | L-lysine | ≥750:1 |
| zoledronic acid, L-lysine, and water complex characterized by an X-ray powder diffraction pattern comprising peaks at about 9.6, 10.7, 14.3, 21.4, 23.5 ±0.2 degrees two theta | L-lysine | ≥750:1 |
| zoledronic acid DL-lysine and water complex characterized by an X-ray powder diffraction pattern comprising peaks at about 8.3, 11.8, 12.3, 15.8, and 20.8 ±0.2 degrees two-theta | L-lysine | ≥750:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | L-lysine | ≥750:1 |
| zoledronic acid, DL-lysine, and water complex characterized by an X-ray powder diffraction pattern comprising peaks at about 9.7, 10.8, 14.4, 18.9, 21.4±0.2 degrees two theta | L-lysine | >750:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | L-lysine | ≥750:1 |
| zoledronic acid, adenine, and water complex characterized by an X-ray powder diffraction pattern comprising peaks at about 13.6, 15.9, 19.7, 27.9, and 29.5 ±0.2 degrees two-theta | L-lysine | ≥750:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | L-lysine | ≥750:1 |
| zoledronic acid and glycine complex characterized by an X-ray powder diffraction pattern comprising peaks at about 10.2, 17.8, 19.9, 22.9, and 28.1 ±0.2 degrees two-theta | L-lysine | ≥750:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | L-lysine | ≥1000:1 |
| ammonium zoledronic acid salt and water complex characterized by an X-ray powder diffraction pattern having strong peaks at about 11.0, 14.6, 15.4, 19.9, and 29.4 ±0.2 degrees two-theta | L-lysine | ≥1000:1 |
| zoledronic diammonia water complex characterized by an X-ray powder diffraction pattern having strong peaks at about 12.2, 13.0, 14.1, 17.1, and 19.3 ±0.2 degrees two-theta | L-lysine | ≥1000:1 |
| zoledronic acid, L-lysine, and water complex characterized by an X-ray powder diffraction pattern having peaks at about 9.0, 14.4, 18.1, 26.0, and 29.6 ±0.2 degrees two-theta | L-lysine | ≥1000:1 |
| zoledronic acid, L-lysine, and water complex characterized by an X-ray powder diffraction pattern comprising peaks at about 9.6, 10.7, 14.3, 21.4, 23.5 ±0.2 degrees two theta | L-lysine | ≥1000:1 |
| zoledronic acid DL-lysine and water complex characterized by an X-ray powder diffraction pattern comprising peaks at about 8.3, 11.8, 12.3, 15.8, and 20.8 ±0.2 degrees two-theta | L-lysine | ≥1000:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | L-lysine | ≥1000:1 |
| zoledronic acid, DL-lysine, and water complex characterized by an X-ray powder diffraction pattern comprising peaks at about 9.7, 10.8, 14.4, 18.9, 21.4 ±0.2 degrees two theta | L-lysine | ≥1000:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | L-lysine | ≥1000:1 |
| zoledronic acid, adenine, and water complex characterized by an X-ray powder diffraction pattern comprising peaks at about 13.6, 15.9, 19.7, 27.9, and 29.5 ±0.2 degrees two-theta | L-lysine | ≥1000:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | L-lysine | ≥1000:1 |
| zoledronic acid and glycine complex characterized by an X-ray powder diffraction pattern comprising peaks at about 10.2, 17.8, 19.9, 22.9, and 28.1 ±0.2 degrees two-theta | L-lysine | ≥1000:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | L-lysine | ≥1000≥5000:1 |
| ammonium zoledronic acid salt and water complex characterized by an X-ray powder diffraction pattern having strong peaks at about 11.0, 14.6, 15.4, 19.9, and 29.4 ±0.2 degrees two-theta | L-lysine | ≥1000≥5000:1 |
| zoledronic diammonia water complex characterized by an X-ray powder diffraction pattern having strong peaks at about 12.2, 13.0, 14.1, 17.1, and 19.3±0.2 degrees two-theta | L-lysine | ≥1000≥5000:1 |
| zoledronic acid, L-lysine, and water complex characterized by an X-ray powder diffraction pattern having peaks at about 9.0, 14.4, 18.1, 26.0, and 29.6 ±0.2 degrees two-theta | L-lysine | ≥1000≥5000:1 |
| zoledronic acid, L-lysine, and water complex characterized by an X-ray powder diffraction pattern comprising peaks at about 9.6, 10.7, 14.3, 21.4, 23.5 ±0.2 degrees two theta | L-lysine | ≥1000≥5000:1 |
| zoledronic acid DL-lysine and water complex characterized by an X-ray powder diffraction pattern comprising peaks at about 8.3, 11.8, 12.3, 15.8, and 20.8 ±0.2 degrees two-theta | L-lysine | ≥1000≥5000:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | L-lysine | ≥1000≥5000:1 |
| zoledronic acid, DL-lysine, and water complex characterized by an X-ray powder diffraction pattern comprising peaks at about 9.7, 10.8, 14.4, 18.9, 21.4 ±0.2 degrees two theta | L-lysine | ≥1000≥5000:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | L-lysine | ≥1000≥5000:1 |
| zoledronic acid, adenine, and water complex characterized by an X-ray powder diffraction pattern comprising peaks at about 13.6, 15.9, 19.7, 27.9, and 29.5 ±0.2 degrees two-theta | L-lysine | ≥1000≥5000:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | L-lysine | ≥1000≥5000:1 |
| zoledronic acid and glycine complex characterized by an X-ray powder diffraction pattern comprising peaks at about 10.2, 17.8, 19.9, 22.9, and 28.1 ±0.2 degrees two-theta | L-lysine | ≥1000≥5000:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | D,L-lysine | ≥5:1 |
| ammonium zoledronic acid salt and water complex characterized by an X-ray powder diffraction pattern having strong peaks at about 11.0, 14.6, 15.4, 19.9, and 29.4 ±0.2 degrees two-theta | D,L-lysine | >5:1 |
| zoledronic diammonia water complex characterized by an X-ray powder diffraction pattern having strong peaks at about 12.2, 13.0, 14.1, 17.1, and 19.3 ±0.2 degrees two-theta | D,L-lysine | ≥5:1 |
| zoledronic acid, L-lysine, and water complex characterized by an X-ray powder diffraction pattern having peaks at about 9.0, 14.4, 18.1, 26.0, and 29.6 ±0.2 degrees two-theta | D,L-lysine | ≥5:1 |
| zoledronic acid, L-lysine, and water complex characterized by an X-ray powder diffraction pattern comprising peaks at about 9.6, 10.7, 14.3, 21.4, 23.5 ±0.2 degrees two theta | DL-lysine | >5:1 |
| zoledronic acid DL-lysine and water complex characterized by an X-ray powder diffraction pattern comprising peaks at about 8.3, 11.8, 12.3, 15.8, and 20.8 ±0.2 degrees two-theta | DL-lysine | ≥5:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | DL-lysine | >5:1 |
| zoledronic acid, DL-lysine, and water complex characterized by an X-ray powder diffraction pattern comprising peaks at about 9.7, 10.8, 14.4, 18.9, 21.4 ±0.2 degrees two theta | DL-lysine | ≥5:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | DL-lysine | ≥5:1 |
| zoledronic acid, adenine, and water complex characterized by an X-ray powder diffraction pattern comprising peaks at about 13.6, 15.9, 19.7, 27.9, and 29.5 ±0.2 degrees two-theta | DL-lysine | ≥5:1 |
| zoledronic acid, nicotinamide, and water complex characterized by an X-ray powder diffraction pattern comprising peaks at about 13.1, 15.2, 21.0, 23.9, and 26.5±0.2 degrees two-theta | DL-lysine | >5:1 |
| zoledronic acid and glycine complex characterized by an X-ray powder diffraction pattern comprising peaks at about 10.2, 17.8, 19.9, 22.9, and 28.1 ±0.2 degrees two-theta | DL-lysine | ≥5:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | DL-lysine | ≥40:1 |
| ammonium zoledronic acid salt and water complex characterized by an X-ray powder diffraction pattern having strong peaks at about 11.0, 14.6, 15.4, 19.9, and 29.4 ±0.2 degrees two-theta | DL-lysine | ≥40:1 |
| zoledronic diammonia water complex characterized by an X-ray powder diffraction pattern having strong peaks at about 12.2, 13.0, 14.1, 17.1, and 19.3 ±0.2 degrees two-theta | DL-lysine | ≥40:1 |
| zoledronic acid, L-lysine, and water complex characterized by an X-ray powder diffraction pattern having peaks at about 9.0, 14.4, 18.1, 26.0, and 29.6 ±0.2 degrees two-theta | DL-lysine | ≥40:1 |
| zoledronic acid, L-lysine, and water complex characterized by an X-ray powder diffraction pattern comprising peaks at about 9.6, 10.7, 14.3, 21.4, 23.5 ±0.2 degrees two theta | DL-lysine | ≥40:1 |
| zoledronic acid DL-lysine and water complex characterized by an X-ray powder diffraction pattern comprising peaks at about 8.3, 11.8, 12.3, 15.8, and 20.8 ±0.2 degrees two-theta | DL-lysine | ≥40:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | DL-lysine | ≥40:1 |
| zoledronic acid, DL-lysine, and water complex characterized by an X-ray powder diffraction pattern comprising peaks at about 9.7, 10.8, 14.4, 18.9, 21.4 ±0.2 degrees two theta | DL-lysine | ≥40:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | DL-lysine | ≥40:1 |
| zoledronic acid, adenine, and water complex characterized by an X-ray powder diffraction pattern comprising peaks at about 13.6, 15.9, 19.7, 27.9, and 29.5 ±0.2 degrees two-theta | DL-lysine | ≥40:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | DL-lysine | ≥40:1 |
| zoledronic acid and glycine complex characterized by an X-ray powder diffraction pattern comprising peaks at about 10.2, 17.8, 19.9, 22.9, and 28.1 ±0.2 degrees two-theta | DL-lysine | ≥40:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | DL-lysine | ≥750:1 |
| ammonium zoledronic acid salt and water complex characterized by an X-ray powder diffraction pattern having strong peaks at about 11.0, 14.6, 15.4, 19.9, and 29.4 ±0.2 degrees two-theta | DL-lysine | ≥750:1 |
| zoledronic diammonia water complex characterized by an X-ray powder diffraction pattern having strong peaks at about 12.2, 13.0, 14.1, 17.1, and 19.3 ±0.2 degrees two-theta | DL-lysine | ≥750:1 |
| zoledronic acid, L-lysine, and water complex characterized by an X-ray powder diffraction pattern having peaks at about 9.0, 14.4, 18.1, 26.0, and 29.6 ±0.2 degrees two-theta | DL-lysine | ≥750:1 |
| zoledronic acid, L-lysine, and water complex characterized by an X-ray powder diffraction pattern comprising peaks at about 9.6, 10.7, 14.3, 21.4, 23.5 ±0.2 degrees two theta | DL-lysine | ≥750:1 |
| zoledronic acid DL-lysine and water complex characterized by an X-ray powder diffraction pattern comprising peaks at about 8.3, 11.8, 12.3, 15.8, and 20.8 ±0.2 degrees two-theta | DL-lysine | ≥750:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | DL-lysine | ≥750:1 |
| zoledronic acid, DL-lysine, and water complex characterized by an X-ray powder diffraction pattern comprising peaks at about 9.7, 10.8, 14.4, 18.9, 21.4 ±0.2 degrees two theta | DL-lysine | ≥750:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | DL-lysine | ≥750:1 |
| zoledronic acid, adenine, and water complex characterized by an X-ray powder diffraction pattern comprising peaks at about 13.6, 15.9, 19.7, 27.9, and 29.5 ±0.2 degrees two-theta | DL-lysine | ≥750:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | DL-lysine | ≥750:1 |
| zoledronic acid and glycine complex characterized by an X-ray powder diffraction pattern comprising peaks at about 10.2, 17.8, 19.9, 22.9, and 28.1 ±0.2 degrees two-theta | DL-lysine | ≥750:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | DL-lysine | ≥1000:1 |
| ammonium zoledronic acid salt and water complex characterized by an X-ray powder diffraction pattern having strong peaks at about 11.0, 14.6, 15.4, 19.9, and 29.4 ±0.2 degrees two-theta | DL-lysine | ≥1000:1 |
| zoledronic diammonia water complex characterized by an X-ray powder diffraction pattern having strong peaks at about 12.2, 13.0, 14.1, 17.1, and 19.3 ±0.2 degrees two-theta | DL-lysine | ≥1000:1 |
| zoledronic acid, L-lysine, and water complex characterized by an X-ray powder diffraction pattern having peaks at about 9.0, 14.4, 18.1, 26.0, and 29.6±0.2 degrees two-theta | DL-lysine | ≥1000:1 |
| zoledronic acid, L-lysine, and water complex characterized by an X-ray powder diffraction pattern comprising peaks at about 9.6, 10.7, 14.3, 21.4, 23.5 ±0.2 degrees two theta | DL-lysine | ≥1000:1 |
| zoledronic acid DL-lysine and water complex characterized by an X-ray powder diffraction pattern comprising peaks at about 8.3, 11.8, 12.3, 15.8, and 20.8 ±0.2 degrees two-theta | DL-lysine | ≥1000:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | DL-lysine | ≥1000:1 |
| zoledronic acid, DL-lysine, and water complex characterized by an X-ray powder diffraction pattern comprising peaks at about 9.7, 10.8, 14.4, 18.9, 21.4 ±0.2 degrees two theta | DL-lysine | ≥1000:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | DL-lysine | ≥1000:1 |
| zoledronic acid, adenine, and water complex characterized by an X-ray powder diffraction pattern comprising peaks at about 13.6, 15.9, 19.7, 27.9, and 29.5 ±0.2 degrees two-theta | DL-lysine | ≥1000:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | DL-lysine | ≥1000:1 |
| zoledronic acid and glycine complex characterized by an X-ray powder diffraction pattern comprising peaks at about 10.2, 17.8, 19.9, 22.9, and 28.1 ±0.2 degrees two-theta | DL-lysine | ≥1000:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | DL-lysine | ≥5000:1 |
| ammonium zoledronic acid salt and water complex characterized by an X-ray powder diffraction pattern having strong peaks at about 11.0, 14.6, 15.4, 19.9, and 29.4 ±0.2 degrees two-theta | DL-lysine | ≥5000:1 |
| zoledronic diammonia water complex characterized by an X-ray powder diffraction pattern having strong peaks at about 12.2, 13.0, 14.1, 17.4, and 19.3 ±0.2 degrees two-theta | DL-lysine | ≥5000:1 |
| zoledronic acid, L-lysine, and water complex characterized by an X-ray powder diffraction pattern having peaks at about 9.0, 14.4, 18.1, 26.0, and 29.6 ±0.2 degrees two-theta | DL-lysine | ≥5000:1 |
| zoledronic acid, L-lysine, and water complex characterized by an X-ray powder diffraction pattern comprising peaks at about 9.6, 10.7, 14.3, 21.4, 23.5 ±0.2 degrees two theta | DL-lysine | ≥5000:1 |
| zoledronic acid DL-lysine and water complex characterized by an X-ray powder diffraction pattern comprising peaks at about 8.3, 11.8, 12.3, 15.8, and 20.8 ±0.2 degrees two-theta | DL-lysine | ≥5000:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | DL-lysine | ≥5000:1 |
| zoledronic acid, DL-lysine, and water complex characterized by an X-ray powder diffraction pattern comprising peaks at about 9.7, 10.8, 14.4, 18.9, 21.4 ±0.2 degrees two theta | DL-lysine | ≥5000:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | DL-lysine | ≥5000:1 |
| zoledronic acid, adenine, and water complex characterized by an X-ray powder diffraction pattern comprising peaks at about 13.6, 15.9, 19.7, 27.9, and 29.5 ±0.2 degrees two-theta | DL-lysine | ≥5000:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | DL-lysine | ≥5000:1 |
| zoledronic acid and glycine complex characterized by an X-ray powder diffraction pattern comprising peaks at about 10.2, 17.8, 19.9, 22.9, and 28.1 ±0.2 degrees two-theta | DL-lysine | ≥5000:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | glycine | ≥5:1 |
| ammonium zoledronic acid salt and water complex characterized by an X-ray powder diffraction pattern having strong peaks at about 11.0, 14.6, 15.4, 19.9, and 29.4 ±0.2 degrees two-theta | glycine | ≥5:1 |
| zoledronic diammonia water complex characterized by an X-ray powder diffraction pattern having strong peaks at about 12.2, 13.0, 14.1, 17.1, and 19.3 ±0.2 degrees two-theta | glycine | ≥5:1 |
| zoledronic acid, L-lysine, and water complex characterized by an X-ray powder diffraction pattern having peaks at about 9.0, 14.4, 18.1, 26.0, and 29.6 ±0.2 degrees two-theta | glycine | ≥5:1 |
| zoledronic acid, L-lysine, and water complex characterized by an X-ray powder diffraction pattern comprising peaks at about 9.6, 10.7, 14.3, 21.4, 23.5 ±0.2 degrees two theta | glycine | ≥5:1 |
| zoledronic acid DL-lysine and water complex characterized by an X-ray powder diffraction pattern comprising peaks at about 8.3, 11.8, 12.3, 15.8, and 20.8 ±0.2 degrees two-theta | glycine | ≥5:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | glycine | ≥5:1 |
| zoledronic acid, DL-lysine, and water complex characterized by an X-ray powder diffraction pattern comprising peaks at about 9.7, 10.8, 14.4, 18.9, 21.4 ±0.2 degrees two theta | glycine | ≥5:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | glycine | ≥5:1 |
| zoledronic acid, adenine, and water complex characterized by an X-ray powder diffraction pattern comprising peaks at about 13.6, 15.9, 19.7, 27.9, and 29.5 ±0.2 degrees two-theta | glycine | ≥5:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | glycine | ≥5:1 |
| zoledronic acid and glycine complex characterized by an X-ray powder diffraction pattern comprising peaks at about 10.2, 17.8, 19.9, 22.9, and 28.1 ±0.2 degrees two-theta | glycine | ≥5:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | glycine | ≥40:1 |
| ammonium zoledronic acid salt and water complex characterized by an X-ray powder diffraction pattern having strong peaks at about 11.0, 14.6, 15.4, 19.9, and 29.4 ±0.2 degrees two-theta | glycine | ≥40:1 |
| zoledronic diammonia water complex characterized by an X-ray powder diffraction pattern having strong peaks at about 12.2, 13.0, 14.1, 17.1, and 19.3 ±0.2 degrees two-theta | glycine | ≥40:1 |
| zoledronic acid, L-lysine, and water complex characterized by an X-ray powder diffraction pattern having peaks at about 9.0, 14.4, 18.1, 26.0, and 29.6 ±0.2 degrees two-theta | glycine | ≥40:1 |
| zoledronic acid, L-lysine, and water complex characterized by an X-ray powder diffraction pattern comprising peaks at about 9.6, 10.7, 14.3, 21.4, 23.5 ±0.2 degrees two theta | glycine | ≥40:1 |
| zoledronic acid DL-lysine and water complex characterized by an X-ray powder diffraction pattern comprising peaks at about 8.3, 11.8, 12.3, 15.8, and 20.8 ±0.2 degrees two-theta | glycine | ≥40:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | glycine | ≥40:1 |
| zoledronic acid, DL-lysine, and water complex characterized by an X-ray powder diffraction pattern comprising peaks at about 9.7, 10.8, 14.4, 18.9, 21.4 ±0.2 degrees two theta | glycine | ≥40:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | glycine | ≥40:1 |
| zoledronic acid, adenine, and water complex characterized by an X-ray powder diffraction pattern comprising peaks at about 13.6, 15.9, 19.7, 27.9, and 29.5 ±0.2 degrees two-theta | glycine | ≥40:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | glycine | ≥40:1 |
| zoledronic acid and glycine complex characterized by an X-ray powder diffraction pattern comprising peaks at about 10.2, 17.8, 19.9, 22.9, and 28.1 ±0.2 degrees two-theta | glycine | ≥40:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | glycine | ≥750:1 |
| ammonium zoledronic acid salt and water complex characterized by an X-ray powder diffraction pattern having strong peaks at about 11.0, 14.6, 15.4, 19.9, and 29.4 ±0.2 degrees two-theta | glycine | ≥750:1 |
| zoledronic diammonia water complex characterized by an X-ray powder diffraction pattern having strong peaks at about 12.2, 13.0, 14.1, 17.1, and 19.3 ±0.2 degrees two-theta | glycine | ≥750:1 |
| zoledronic acid, L-lysine, and water complex characterized by an X-ray powder diffraction pattern having peaks at about 9.0, 14.4, 18.1, 26.0, and 29.6 ±0.2 degrees two-theta | glycine | ≥750:1 |
| zoledronic acid, L-lysine, and water complex characterized by an X-ray powder diffraction pattern comprising peaks at about 9.6, 10.7, 14.3, 21.4, 23.5 ±0.2 degrees two theta | glycine | ≥750:1 |
| zoledronic acid DL-lysine and water complex characterized by an X-ray powder diffraction pattern comprising peaks at about 8.3, 11.8, 12.3, 15.8, and 20.8 ±0.2 degrees two-theta | glycine | ≥750:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | glycine | ≥750:1 |
| zoledronic acid, DL-lysine, and water complex characterized by an X-ray powder diffraction pattern comprising peaks at about 9.7, 10.8, 14.4, 18.9, 21.4 ±0.2 degrees two theta | glycine | ≥750:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | glycine | ≥750:1 |
| zoledronic acid, adenine, and water complex characterized by an X-ray powder diffraction pattern comprising peaks at about 13.6, 15.9, 19.7, 27.9, and 29.5 ±0.2 degrees two-theta | glycine | ≥750:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | glycine | >750:1 |
| zoledronic acid and glycine complex characterized by an X-ray powder diffraction pattern comprising peaks at about 10.2, 17.8, 19.9, 22.9, and 28.1 ±0.2 degrees two-theta | glycine | ≥750:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | glycine | ≥1000:1 |
| ammonium zoledronic acid salt and water complex characterized by an X-ray powder diffraction pattern having strong peaks at about 11.0, 14.6, 15.4, 19.9, and 29.4 ±0.2 degrees two-theta | glycine | ≥1000:1 |
| zoledronic diammonia water complex characterized by an X-ray powder diffraction pattern having strong peaks at about 12.2, 13.0, 14.1, 17.1, and 19.3 ±0.2 degrees two-theta | glycine | ≥1000:1 |
| zoledronic acid, L-lysine, and water complex characterized by an X-ray powder diffraction pattern having peaks at about 9.0, 14.4, 18.1, 26.0, and 29.6 ±0.2 degrees two-theta | glycine | ≥1000:1 |
| zoledronic acid, L-lysine, and water complex characterized by an X-ray powder diffraction pattern comprising peaks at about 9.6, 10.7, 14.3, 21.4, 23.5 ±0.2 degrees two theta | glycine | ≥1000:1 |
| zoledronic acid DL-lysine and water complex characterized by an X-ray powder diffraction pattern comprising peaks at about 8.3, 11.8, 12.3, 15.8, and 20.8 ±0.2 degrees two-theta | glycine | ≥1000:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | glycine | ≥1000:1 |
| zoledronic acid, DL-lysine, and water complex characterized by an X-ray powder diffraction pattern comprising peaks at about 9.7, 10.8, 14.4, 18.9, 21.4 ±0.2 degrees two theta | glycine | ≥1000:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | glycine | ≥1000:1 |
| zoledronic acid, adenine, and water complex characterized by an X-ray powder diffraction pattern comprising peaks at about 13.6, 15.9, 19.7, 27.9, and 29.5 ±0.2 degrees two-theta | glycine | ≥1000:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | glycine | ≥1000:1 |
| zoledronic acid and glycine complex characterized by an X-ray powder diffraction pattern comprising peaks at about 10.2, 17.8, 19.9, 22.9, and 28.1 ±0.2 degrees two-theta | glycine | ≥1000:1 |
| zoledronic acid, sodium zoledronate and water complex characterized by an X-ray powder diffraction pattern having peaks at about 8.1, 13.3, 21.5, 24.6, and 25.6 ±0.2 degrees two-theta | glycine | ≥5000:1 |
| ammonium zoledronic acid salt and water complex characterized by an X-ray powder diffraction pattern having strong peaks at about 11.0, 14.6, 15.4, 19.9, and 29.4 ±0.2 degrees two-theta | glycine | ≥5000:1 |
| zoledronic diammonia water complex characterized by an X-ray powder diffraction pattern having strong peaks at about 12.2, 13.0, 14.1, 17.1, and 19.3 ±0.2 degrees two-theta | glycine | ≥5000:1 |
| zoledronic acid, L-lysine, and water complex characterized by an X-ray powder diffraction pattern having peaks at about 9.0, 14.4, 18.1, 26.0, and 29.6 ±0.2 degrees two-theta | glycine | ≥5000:1 |
| zoledronic acid, L-lysine, and water complex characterized by an X-ray powder diffraction pattern comprising peaks at about 9.6, 10.7, 14.3, 21.4, 23.5 ±0.2 degrees two theta | glycine | ≥5000:1 |
| zoledronic acid DL-lysine and water complex characterized by an X-ray powder diffraction pattern comprising peaks at about 8.3, 11.8, 12.3, 15.8, and 20.8 ±0.2 degrees two-theta | glycine | ≥5000:1 |
| zoledronic acid, DL-lysine, and water complex characterized by an X-ray powder diffraction pattern comprising peaks at about 9.1, 14.7, 18.0, 21.2, and 26.0 ±0.2 degrees two-theta | glycine | ≥5000:1 |
| zoledronic acid, DL-lysine, and water complex characterized by an X-ray powder diffraction pattern comprising peaks at about 9.7, 10.8, 14.4, 18.9, 21.4 ±0.2 degrees two theta | glycine | ≥5000:1 |
| zoledronic acid, zoledronic, DL-lysine, ethanol, and water complex characterized by an X-ray powder diffraction pattern comprising peaks at about 8.8, 9.7, 17.6, 23.1, and 26.5 ±0.2 degrees two-theta | glycine | ≥5000:1 |
| zoledronic acid, adenine, and water complex characterized by an X-ray powder diffraction pattern comprising peaks at about 13.6, 15.9, 19.7, 27.9, and 29.5 ±0.2 degrees two-theta | glycine | ≥5000:1 |
| zoledronic acid, nicotinamide, and water complex characterized by an X-ray powder diffraction pattern comprising peaks at about 13.1, 15.2, 21.0, 23.9, and 26.5 ±0.2 degrees two-theta | glycine | ≥5000:1 |
| zoledronic acid and glycine complex characterized by an X-ray powder diffraction pattern comprising peaks at about 10.2, 17.8, 19.9, 22.9, and 28.1 ±0.2 degrees two-theta | glycine | ≥5000:1 |

**Table 15. Particular unit doses of a pharmaceutical composition comprising: zoledronic acid (left column), an amino acid, present as either a molecular complex coformer, additional coformer or both molecular complex coformer and additional coformer (middle column), and amount of the amino acid in a unit dose of bisphosphonic acid (right column). Each row of the above table represents an individual composition.**

| **Bisphosphonic Acid** | **Amino Acid** | **Amount of Amino Acid per Unit Dose of Bisphosphonic Acid** |
|---|---|---|
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥log |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mug |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥10g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥10g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥10g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥10g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥5g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥10g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥10g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |
| zoledronic acid | L-lysine | ≥100mg |
| zoledronic acid | L-lysine | ≥1250mg |
| zoledronic acid | L-lysine | ≥1750mg |
| zoledronic acid | L-lysine | ≥5g |
| zoledronic acid | L-lysine | ≥10g |
| zoledronic acid | DL-lysine | ≥100mg |
| zoledronic acid | DL-lysine | ≥1250mg |
| zoledronic acid | DL-lysine | ≥1750mg |
| zoledronic acid | DL-lysine | ≥5g |
| zoledronic acid | DL-lysine | ≥10g |
| zoledronic acid | glycine | ≥100mg |
| zoledronic acid | glycine | ≥1250mg |
| zoledronic acid | glycine | ≥1750mg |
| zoledronic acid | glycine | ≥5g |
| zoledronic acid | glycine | ≥10g |

## Claims

1. A pharmaceutical composition comprising:
a crystalline molecular complex comprising zoledronic acid or a salt thereof and a molecular complex coformer selected from lysine and glycine, and a pharmaceutically acceptable excipient;
further comprising an additional coformer selected from lysine and/or glycine, wherein the mass ratio of additional coformer to molecular complex coformer is between about 2:1 to about 5000:1.

2. The pharmaceutical composition of claim 1, wherein the coformer in the molecular complex is lysine.

3. The pharmaceutical composition of claim 1, wherein the coformer in the molecular complex is glycine.

4. The pharmaceutical composition of any one of claims 1 to 3, wherein the molecular complex coformer and the additional coformer are the same.

5. The pharmaceutical composition of any one of claims 1 to 3, wherein the molecular complex coformer and the additional coformer are different.

6. The pharmaceutical composition of any one of claims 1 to 5, wherein the crystalline molecular complex is selected from the group consisting of:
a. a molecular complex comprising zoledronic acid, lysine and water;
b. a molecular complex consisting of zoledronic acid, lysine and water;
c. a molecular complex consisting of zoledronic acid, L-lysine and water;
d. a molecular complex consisting of zoledronic acid, DL-lysine and water;
e. a molecular complex consisting of zoledronic acid and lysine;
f. a molecular complex consisting of zoledronic acid and L-lysine;
g. a molecular complex consisting of zoledronic acid and DL-lysine;
h. a molecular complex comprising zoledronic acid, glycine and water;
i. a molecular complex consisting of zoledronic acid and glycine;
j. a molecular complex consisting of zoledronic acid, DL-lysine, ethanol, and water.

7. The pharmaceutical composition of claim 6, wherein the crystalline molecular complex is selected from the group consisting of:
a. a crystalline zoledronic acid, L-lysine, and water molecular complex **characterized by** an X-ray powder diffraction pattern comprising peaks at about 9.0, 14.4, 18.1, 26.0, and 29.6 ±0.2 degrees two-theta;
b. a crystalline zoledronic acid, L-lysine, and water molecular complex **characterized by** an X-ray powder diffraction pattern comprising peaks at about 9.6, 10.7, 14.3, 21.4, 23.5 ±0.2 degrees two-theta;
c. a crystalline zoledronic acid DL-lysine and water molecular complex **characterized by** an X-ray powder diffraction pattern comprising peaks at about 8.3, 11.8, 12.3, 15.8, and 20.8 ±0.2 degrees two-theta;
d. a crystalline zoledronic acid, DL-lysine, and water molecular complex **characterized by** an X-ray powder diffraction pattern comprising peaks at about 9.1, 14.7, 18.0, 21.2, and 26.0 ±0.2 degrees two-theta;
e. a crystalline zoledronic acid, DL-lysine, and water molecular complex **characterized by** an X-ray powder diffraction pattern comprising peaks at about 9.7, 10.8, 14.4, 18.9, 21.4 ±0.2 degrees two-theta;
f. a crystalline zoledronic acid, zoledronic, DL-lysine, ethanol, and water complex **characterized by** an X-ray powder diffraction pattern comprising peaks at about 8.8, 9.7, 17.6, 23.1, and 26.5 ±0.2 degrees two-theta; and
g. a crystalline zoledronic acid and glycine molecular complex **characterized by** an X-ray powder diffraction pattern comprising peaks at about 10.2, 17.8, 19.9, 22.9, and 28.1 ±0.2 degrees two-theta;
when collecting the X-ray powder diffraction patterns over an angular range of 3° to 40° 2θ using Cu Kα radiation with λ = 1.540562 Å.

8. The pharmaceutical composition of any one of claims 1 to 7, wherein the pharmaceutical composition is an oral dosage form.

9. The pharmaceutical composition according to claim 8, wherein the composition is an oral dosage form selected from a tablet, a capsule, a solution, or a suspension.

10. The pharmaceutical composition of claim 8 or claim 9, wherein the oral dosage form is enterically coated.

11. The pharmaceutical composition of any one of claims 1 to 10, wherein the pharmaceutical composition is in the form of a unit dose, preferably wherein said unit dose comprises between about 1 mg to about 500 mg of zoledronic acid.

12. The pharmaceutical composition of any one of claims 1 to 11 for use in treating or preventing a disease for which zoledronic acid is indicated.

13. The pharmaceutical composition for use according to claim 12, wherein said disease is selected from the group consisting of: osteoporosis, hypercalcemia, cancer induced bone metastasis, Paget's disease, or adjuvant cancer therapy and neoadjuvant cancer therapy.

14. A pharmaceutical composition as defined in any one of claims 1 to 11 for use in enhancing the oral bioavailability or permeability of zoledronic acid or a salt thereof in a patient in need thereof.

## Patentansprüche

1. Pharmazeutische Zusammensetzung, umfassend:
einen kristallinen Molekülkomplex, der Zoledronsäure oder ein Salz davon und einen Molekülkomplex-Coformer, ausgewählt aus Lysin und Glycin, und einen pharmazeutisch akzeptablen Hilfsstoff umfasst;
ferner einen zusätzlichen Coformer umfassend, der ausgewählt ist aus Lysin und/oder Glycin, wobei das Massenverhältnis des zusätzlichen Coformers zum Molekülkomplex-Coformer etwa 2:1 bis etwa 5000:1 beträgt.

2. Pharmazeutische Zusammensetzung nach Anspruch 1, wobei der Coformer in dem Molekülkomplex Lysin ist.

3. Pharmazeutische Zusammensetzung nach Anspruch 1, wobei der Coformer in dem Molekülkomplex Glycin ist.

4. Pharmazeutische Zusammensetzung nach einem der Ansprüche 1 bis 3, wobei der Molekülkomplex-Coformer und der zusätzliche Coformer gleich sind.

5. Pharmazeutische Zusammensetzung nach einem der Ansprüche 1 bis 3, wobei der Molekülkomplex-Coformer und der zusätzliche Coformer verschieden sind.

6. Pharmazeutische Zusammensetzung nach einem der Ansprüche 1 bis 5, wobei der kristalline Molekülkomplex ausgewählt ist aus der Gruppe bestehend aus:
a. einem Molekülkomplex, der Zoledronsäure, Lysin und Wasser umfasst;
b. einem Molekülkomplex, der aus Zoledronsäure, Lysin und Wasser besteht;
c. einem Molekülkomplex, der aus Zoledronsäure, L-Lysin und Wasser besteht;
d. einem Molekülkomplex, der aus Zoledronsäure, DL-Lysin und Wasser besteht;
e. einem Molekülkomplex, der aus Zoledronsäure und Lysin besteht;
f. einem Molekülkomplex, der aus Zoledronsäure und L-Lysin besteht;
g. einem Molekülkomplex, der aus Zoledronsäure und DL-Lysin besteht;
h. einem Molekülkomplex, der Zoledronsäure, Glycin und Wasser umfasst;
i. einem Molekülkomplex, der aus Zoledronsäure und Glycin besteht;
j. einem Molekülkomplex, der aus Zoledronsäure, DL-Lysin, Ethanol und Wasser besteht.

7. Pharmazeutische Zusammensetzung nach Anspruch 6, wobei der kristalline Molekülkomplex ausgewählt ist aus der Gruppe bestehend aus:
a. einem kristallinen Molekülkomplex aus Zoledronsäure, L-Lysin und Wasser, **gekennzeichnet durch** ein Röntgenpulverdiffraktionsmuster, das Peaks bei etwa 9,0, 14,4, 18,1, 26,0 und 29,6 ± 0,2 Grad unter zwei-theta umfasst;
b. einem kristallinen Molekülkomplex aus Zoledronsäure, L-Lysin und Wasser, **gekennzeichnet durch** ein Röntgenpulverdiffraktionsmuster, das Peaks bei etwa 9,6, 10,7, 14,3, 21,4, 23,5 ± 0,2 Grad unter zwei-theta umfasst;
c. einem kristallinen Molekülkomplex aus Zoledronsäure, DL-Lysin und Wasser, **gekennzeichnet durch** ein Röntgenpulverdiffraktionsmuster, das Peaks bei etwa 8,3, 11,8, 12,3, 15,8 und 20,8 ± 0,2 Grad unter zwei-theta umfasst;
d. einem kristallinen Molekülkomplex aus Zoledronsäure, DL-Lysin und Wasser, **gekennzeichnet durch** ein Röntgenpulverdiffraktionsmuster, das Peaks bei etwa 9,1, 14,7, 18,0, 21,2 und 26,0 ± 0,2 Grad unter zwei-theta umfasst;
e. einem kristallinen Molekülkomplex aus Zoledronsäure, DL-Lysin und Wasser, **gekennzeichnet durch** ein Röntgenpulverdiffraktionsmuster, das Peaks bei etwa 9,7, 10,8, 14,4, 18,9, 21,4 ± 0,2 Grad unter zwei-theta umfasst;
f. einem Kristallkomplex aus Zoledronsäure, Zoledronic, DL-Lysin, Ethanol und Wasser, **gekennzeichnet durch** ein Pulverröntgenbeugungsmuster, das Peaks bei etwa 8,8, 9,7, 17,6, 23,1 und 26,5 ± 0,2 Grad unter zwei-theta umfasst; und;
g. einem kristallinen Molekülkomplex aus Zoledronsäure und Glycin, **gekennzeichnet durch** ein Röntgenpulverdiffraktionsmuster, das Peaks bei etwa 10,2, 17,8, 19,9, 22,9 und 28,1 ± 0,2 Grad unter zwei-theta umfasst;
bei Erfassung der Röntgenpulverdiffraktionsmuster über einem Winkelbereich von 3° bis 40° unter 2θ mittels Cu-Kα-Strahlung mit λ = 1,540562 Å.

8. Pharmazeutische Zusammensetzung nach einem der Ansprüche 1 bis 7, wobei die pharmazeutische Zusammensetzung eine orale Dosisform ist.

9. Pharmazeutische Zusammensetzung nach Anspruch 8, wobei die Zusammensetzung eine orale Dosisform ist, ausgewählt aus einer Tablette, einer Kapsel, einer Lösung oder einer Suspension.

10. Pharmazeutische Zusammensetzung nach Anspruch 8 oder Anspruch 9, wobei die orale Dosisform magensaftresistent beschichtet ist.

11. Pharmazeutische Zusammensetzung nach einem der Ansprüche 1 bis 10, wobei die pharmazeutische Zusammensetzung in Form einer Einheitsdosis vorliegt, wobei vorzugsweise die Einheitsdosis zwischen etwa 1 mg bis etwa 500 mg Zoledronsäure umfasst.

12. Pharmazeutische Zusammensetzung nach einem der Ansprüche 1 bis 11 zur Verwendung bei der Behandlung oder Verhütung einer Krankheit, für die Zoledronsäure indiziert ist.

13. Pharmazeutische Zusammensetzung zur Verwendung nach Anspruch 12, wobei die Krankheit ausgewählt ist aus der Gruppe bestehend aus: Osteoporose, Hyperkalzämie, durch Krebs induzierte Knochenmetastasen, Morbus Paget oder adjuvanter Krebstherapie und neoadjuvanter Krebstherapie.

14. Pharmazeutische Zusammensetzung nach einem der Ansprüche 1 bis 11 zur Verwendung bei der Verbesserung der oralen Bioverfügbarkeit oder Durchlässigkeit von bzw. für Zoledronsäure oder von einem bzw. für ein Salz derselben in einem Patienten, der dessen bedarf.

## Revendications

1. Composition pharmaceutique comprenant :
un complexe moléculaire cristallin comprenant de l'acide zolédronique ou un sel de celui-ci et un co-formeur de complexe moléculaire choisi parmi la lysine et la glycine, et un excipient acceptable sur le plan pharmaceutique ;
comprenant en outre un co-formeur supplémentaire choisi parmi la lysine et/ou la glycine, dans laquelle le rapport massique du co-formeur supplémentaire au co-formeur de complexe moléculaire est compris entre environ 2:1 et environ 5000:1.

2. Composition pharmaceutique selon la revendication 1, dans laquelle le co-formeur dans le complexe moléculaire est la lysine.

3. Composition pharmaceutique selon la revendication 1, dans laquelle le co-formeur dans le complexe moléculaire est la glycine.

4. Composition pharmaceutique selon l'une quelconque des revendications 1 à 3, dans laquelle le co-formeur de complexe moléculaire et le co-formeur supplémentaire sont identiques.

5. Composition pharmaceutique selon l'une quelconque des revendications 1 à 3, dans laquelle le co-formeur de complexe moléculaire et le co-formeur supplémentaire sont différents.

6. Composition pharmaceutique selon l'une quelconque des revendications 1 à 5, dans laquelle le complexe moléculaire cristallin est choisi dans le groupe constitué :
a. d'un complexe moléculaire comprenant de l'acide zolédronique, de la lysine et de l'eau ;
b. d'un complexe moléculaire constitué d'acide zolédronique, de lysine et d'eau ;
c. d'un complexe moléculaire constitué d'acide zolédronique, de L-lysine et d'eau ;
d. d'un complexe moléculaire constitué d'acide zolédronique, de DL-lysine et d'eau ;
e. d'un complexe moléculaire constitué d'acide zolédronique et de lysine ;
f. d'un complexe moléculaire constitué d'acide zolédronique et de L-lysine ;
g. d'un complexe moléculaire constitué d'acide zolédronique et de DL-lysine ;
h. d'un complexe moléculaire comprenant de l'acide zolédronique, de la glycine et de l'eau ;
i. d'un complexe moléculaire constitué d'acide zolédronique et de glycine ;
j. d'un complexe moléculaire constitué d'acide zolédronique, de DL-lysine, d'éthanol et d'eau.

7. Composition pharmaceutique selon la revendication 6, dans laquelle le complexe moléculaire cristallin est choisi dans le groupe constitué :
a. d'un complexe moléculaire cristallin d'acide zolédronique, de la L-lysine et d'eau, **caractérisé par** un profil de diffraction de poudre aux rayons X comprenant des pics à environ 9,0, 14,4, 18,1, 26,0 et 29,6 ±0,2 degrés deux-thêta ;
b. d'un complexe moléculaire cristallin d'acide zolédronique, de la L-lysine et d'eau, **caractérisé par** un profil de diffraction de poudre aux rayons X comprenant des pics à environ 9,6, 10,7, 14,3, 21,4, 23,5 ± 0,2 degrés deux-thêta ;
c. d'un complexe moléculaire cristallin d'acide zolédronique, de la DL-lysine et d'eau, **caractérisé par** un profil de diffraction de poudre aux rayons X comprenant des pics à environ 8,3, 11,8, 12,3, 15,8 et 20,8 ±0,2 degrés deux-thêta ;
d. d'un complexe moléculaire cristallin d'acide zolédronique, de la DL-lysine et d'eau, **caractérisé par** un profil de diffraction de poudre aux rayons X comprenant des pics à environ 9,1, 14,7, 18,0, 21,2 et 26,0 ±0,2 degrés deux-thêta ;
e. d'un complexe moléculaire cristallin d'acide zolédronique, de la DL-lysine et d'eau, **caractérisé par** un profil de diffraction de poudre aux rayons X comprenant des pics à environ 9,7, 10,8, 14,4, 18,9, 21,4 ± 0,2 degrés deux-thêta ;
f. d'un complexe cristallin d'acide zolédronique, zolédronique, de la DL-lysine, d'éthanol et d'eau, **caractérisé par** un profil de diffraction de poudre aux rayons X comprenant des pics à environ 8,8, 9,7, 17,6, 23,1 et 26,5 ± 0,2 degrés deux-thêta ; et
g. d'un complexe moléculaire cristallin d'acide zolédronique et de glycine, **caractérisé, par** un profil de diffraction de poudre aux rayons X comprenant des pics à environ 10,2, 17,8, 19,9, 22,9 et 28,1 ± 0,2 degrés deux-thêta ;
lorsqu'on collecte les motifs de diffraction de poudre aux rayons X sur une plage angulaire de 3 ° à 40 ° 2θ en utilisant un rayonnement Cu Kα avec λ = 1,540562 Å.

8. Composition pharmaceutique selon l'une quelconque des revendications 1 à 7, où la composition pharmaceutique est une forme pharmaceutique orale.

9. Composition pharmaceutique selon la revendication 8, où la composition est une forme pharmaceutique orale choisie parmi un comprimé, une gélule, une solution ou une suspension.

10. Composition pharmaceutique selon la revendication 8 ou la revendication 9, dans laquelle la forme pharmaceutique orale est à enrobage entérique.

11. Composition pharmaceutique selon l'une quelconque des revendications 1 à 10, où la composition pharmaceutique est sous la forme d'une dose unitaire, de préférence dans laquelle ladite dose unitaire comprend entre environ 1 mg et environ 500 mg d'acide zolédronique.

12. Composition pharmaceutique selon l'une quelconque des revendications 1 à 11, destinée à être utilisée pour le traitement ou la prévention d'une maladie pour laquelle l'acide zolédronique est indiqué.

13. Composition pharmaceutique utilisable selon la revendication 12, dans laquelle ladite maladie est choisie dans le groupe constitué de : l'ostéoporose, l'hypercalcémie, des métastases osseuses induites par un cancer, maladie de Paget, ou d'une thérapie du cancer par adjuvant et d'une thérapie du cancer par néoadjuvant.

14. Composition pharmaceutique telle que définie dans l'une quelconque des revendications 1 à 11, destinée à être utilisée pour renforcer la biodisponibilité ou la perméabilité orale de l'acide zolédronique ou d'un sel de celui-ci chez un patient qui en a besoin.
